# EUROPEAN PATENT APPLICATION

(11) **EP 4 592 390 A1**
(43) Date of publication of application: **30.07.2025**
(21) Application number: 23867472.5
(22) Date of filing: 19.09.2023
(51) Int. Cl.: C12N 15/113, C12Q 1/68, A61K 31/713, A61K 47/42

(54) **SPECIFICALLY MODIFIED RNAI REAGENT AND COMPOSITION**

(30) Priority: 20.09.2022 CN 202211147122
(71) Applicant: Shanghai Argo Biopharmaceutical Co., Ltd., Shanghai 201803 (CN)
(72) Inventor: SHU, Dongxu, Ningbo, Zhejiang 315100 (CN); SHAO, Pengcheng Patrick, Warrington Township Pennsylvania 018976 (US)
(74) Representative: Simmons & Simmons LLP (Munich)
(86) International application number: PCT/CN2023/119567
(87) International publication number: WO 2024/061185

(57) **Abstract**

One aspect of the present invention relates to an RNAi (dsRNA) duplex reagent capable of inhibiting the expression of a target gene. The dsRNA duplex has a F-modified nucleotide at a specific position in an antisense strand or sense strand. Other aspects of the present invention relate to a pharmaceutical composition comprising the dsRNA reagent suitable for therapeutic use and a method for inhibiting the expression of a target gene by administering the dsRNA reagent, such as for the treatment of various disease conditions.

## Description

### FIELD

The present invention relates to an RNAi agent with a specific motif that can be used to inhibit the expression of a target gene as well as an RNAi composition for use in the treatment of a disease. In particular, the present invention provides the RNAi agent to inhibit the expression of a target gene associated with various diseases.

### BACKGROUND

RNA interference or "RNAi" technique, as originally proposed by Fire et al., has been an effective means of disease therapy known in the art. The RNAi mechanism is initiated by the generation of longer, non-coding RNAs mediated by the enzyme Dicer. These RNA molecules are loaded into RNA-induced silencing complex (RISC) in which the sense strand (also known as the passenger strand) is discarded and the antisense strand (also known as guide strand) is hybridized with a fully or partially complementary mRNA sequence, and then Ago2-mediated degradation or inhibited translation induces the silencing of mRNA. Oligonucleotides that reduce gene expression via the RNA interference (RNAi) pathway have been developed, for example but not limited to, double-stranded ribonucleic acid (dsRNA) of RNAi oligonucleotides, including short interfering RNA (siRNA), micro RNA (miRNA), and short hairpin RNA (shRNA) of 19-25 nucleotides in size, or antisense oligonucleotides (ASO) of 16-53 nucleotides in size. Advances in RNAi technology and delivery methods have led to increasingly positive results for RNAi-based therapies. Such therapies represent a promising therapeutic direction for a class of diseases, especially against targets considered "undruggable" by small molecular or biological methods. However, such technologies cannot be widely applied due to inherent metabolic problems of natural RNA such as targetability and stability *in vivo.*

Over the past 20 years, great strides have been made in overcoming the inherent metabolic problems of natural RNA by developing various chemical modifications and improved delivery methods applied for RNAi oligonucleotides. The chemical modification of RNAi oligonucleotides plays a vital role in promoting the full exploitation of the potential of such therapeutic regimens by improving their pharmacokinetic and pharmacodynamic properties (Deleavey&Damha, Chem, Biol, 19:937-954, 2012) and preventing the activation of the innate immune system. For example, Choung et al. used siRNA duplexes with alternative modifications, and obtained stable siRNA in serum through 2'-OMe (2'-O-methyl), 2'-F (2'-fluoro), phosphorothioate modified nucleotides, and combinations thereof (2006, Biochemical and Biophysical Research Communications, 342, 919-927), which further comprises modification of nucleotides at other positions to LNA, UNA, etc.. Recently, Nair et al. demonstrated the effectiveness of N-acetylgalactosamine (GalNAc)-conjugated chemically modified siRNA in mediating delivery to liver hepatocytes *in vivo* (J. AM. Chem, SOC, 136: 16958-16961, 2014). These chemically modified siRNAs have been taken into clinical development for the treatment of a variety of human diseases.

Among these modifications, one of the most common chemical modifications is the 2'-OH substitution of the furanose of ribonucleotides, including but not limited to fully chemically modified siRNA employing a combination of 2'-O-methyl (2'-OMe) and 2'-fluoro (2'-F) throughout the duplex. For example, Morrissey et al. used an siRNA duplex containing 2'-F modified residue at the Ago2 cleavage site among other sites and modifications, and achieved compatible silencing compared to unmodified siRNA, but the position of these 2'-F modifications was not determined and whether there was a substantial effect on the silencing of gene activity was uncertain. Choung et al. suggested that 2'-OME should not be used at certain sites in order to increase siRNA stability. Janas et al. noted that there was no evidence to demonstrate the regularity of 2'-F modified siRNA to date (NUCLEIC ACID THER., 26:363-371, 2016; NUCLEIC ACID THER., 27:11-22, 2016). Although modifications such as 2'-F siRNA are well tolerated in clinical trials, the main problem in therapeutic agents of oligonucleotides using chemically modified nucleoside analogs is the potential toxicity associated with non-natural oligonucleotide analogs. It was reported that 2'-F-modified fully phosphorothioated antisense oligonucleotides resulted in cell protein reduction and double-stranded DNA fragmentation, leading to acute hepatotoxicity *in vivo* (NUCLEIC ACID RES., 46:2204-2217, 2018). Furthermore, the larger and naturally derived 2'-OMe modification is known to be more metabolically stable and tolerable than the smaller 2'-F modification. Nevertheless, it remains unclear whether the substitution of smaller 2'-F with 2'-OMe would interfere RNA protein binding and inhibit RNAi activity (Chiu et al., RNA, 9:1034-1048, 2003; Prakash et al., J. MED. CHEM., 48:4247-4253, 2005; Zheng et al., FASEB J., 27:4017-4026, 2013).

Thus, in order to enhance stability and tolerability and minimize toxic side effects of non-natural nucleotide agents without affecting RNAi activity, the prior art discloses further reducing the 2'-F content accompanied by 2'-OME adjustment, and a fine-tuning of the position of 2'-OME and 2'-F has shown good efficacy and duration. A recent report has attempted to optimize the modification mode of a 21/23-length siRNA and Galnac-conjugated platform (Mol. Ther. 26:708-717, 2018). International Patent Application No. WO2021067744 investigated the effect of positions and numbers of 2'-F in hairpin RNA (shRNA) and found that certain positions and numbers of 2'-F in shRNA had an impact on RNAi activity. However, the significance of the specific positions and numbers of 2'-F modification was not fully appreciated in terms of maintaining RNAi activity while promoting stability, tolerance and decreasing toxic side effects.

While great progress has been made in overcoming the inherent metabolic problems of natural RNA through the development of chemical modifications and improved delivery methods, there remains a need in the art for RNAi agents with enhanced *in vivo* efficacy and stability suitable for administration for therapeutic purposes. It is still desirable to minimize the use of non-natural nucleoside analogs such as 2'-F modified nucleosides in therapeutic RNAi and to determine the effect of, in particular, specific positions and numbers of RNAi on the activity, stability, and toxic side effects in, for example, double-stranded ribonucleic acid and antisense oligonucleotides.

### SUMMARY

The present invention is based, in part, on the design of chemical modifications for RNAi agents, which improves the efficacy and/or duration of gene silencing activity *in vivo* of RNAi agents. The modification pattern described herein can be generally applied to a variety of RNAi agents with different sequences and targets. RNAi agents can be used to inhibit expression of a target gene *in vivo,* for example, to achieve therapeutic purposes.

Thus, according to one aspect of the present invention, an RNAi agent for inhibiting expression of a target gene is provided, wherein the RNAi agent is a double-stranded ribonucleic acid (dsRNA) agent comprising a sense strand and an antisense strand complementary to at least a portion of an mRNA corresponding to the target gene, and the antisense strand has 18-30 nucleotides and comprises a sequence represented by formula (X) in the direction of 3'-5':

3'-(N_{X})ₙ Nx Nx Nx Nx N_{F} N_{X} N_{X} N_{X} N_{X} N_{X} N_{X} N_{X} N_{X} N_{X}N_{X} N_{X} N_{F} N_{X}-5' formula (X)

wherein, each N_{F} represents a 2'-fluoro modified nucleotide;
each N_{X} independently represents a modified or an unmodified nucleotide, wherein no more than three N_{X} are 2'-fluoro modified nucleotides;
n can be an integer from 0 to 7.

In some embodiments, an RNAi agent for inhibiting expression of a target gene is provided, wherein the RNAi agent is a double-stranded ribonucleic acid (dsRNA) agent comprising a sense strand and an antisense strand complementary to at least a portion of an mRNA corresponding to the target gene, and the antisense strand has 18-30 nucleotides and comprises a sequence represented by formula (I) in the direction of 3'-5' :

3'-(N_{X})ₙ Nx Nx Nx Nx N_{F} N_{X} N_{X} N_{X} N_{X} N_{X} N_{X} N_{X} N_{X} N_{X}N_{X} N_{X} N_{F} N_{X}-5' formula (I)

wherein, each N_{F} represents a 2'-fluoro modified nucleotide;
each N_{X} independently represents a modified or an unmodified nucleotide, wherein only three N_{X} are 2'-fluoro modified nucleotides, or only one N_{X} is a 2'-fluoro modified nucleotide;
n can be an integer from 0 to 7.

In some embodiments, an RNAi agent for inhibiting expression of a target gene is provided, wherein the RNAi agent is a double-stranded ribonucleic acid (dsRNA) agent comprising a sense strand and an antisense strand complementary to at least a portion of an mRNA corresponding to the target gene, and the antisense strand has 18-30 nucleotides and comprises a sequence represented by formula (I-1) in the direction of 3'-5':

3'-(N_{L})ₙ N_{M1} N_{L} N_{M2} N_{L} N_{F} N_{L} N_{M3} N_{L} N_{M4} N_{L} N_{M5} N_{M6} N_{L} N_{M7} N_{M8} N_{L} N_{F} N_{L}-5' formula (I-1)

wherein, each N_{F} represents a 2'-fluoro modified nucleotide;
N_{M1}, N_{M2}, N_{M3}, N_{M4}, N_{M5}, N_{M6}, N_{M7}, and N_{M8} independently represent a modified or an unmodified nucleotide, wherein only three of N_{M1}, N_{M2}, N_{M3}, N_{M4}, N_{M5}, N_{M6}, N_{M7}, and N_{M8} are 2'-fluoro modified nucleotides, or only one of N_{M1}, N_{M2}, N_{M3}, N_{M4}, N_{M5}, N_{M6}, N_{M7}, and N_{M8} is a 2'-fluoro modified nucleotide;
each N_{L} independently represents a modified or an unmodified nucleotide, wherein the modified nucleotide is not a 2'-fluoro modified nucleotide;
n can be an integer from 0 to 7.

In some embodiments, in the antisense strand represented by formula (I-1) of the dsRNA agent, only three of N_{M1}, N_{M2}, N_{M3}, N_{M4}, N_{M6}, N_{M7}, and N_{M8} are 2'-fluoro modified nucleotides, and N_{M5} is not a 2'-F modified nucleotide.

In some specific embodiments, the antisense strand of the dsRNA agent comprises a sequence represented by formula (II) in the direction of 3'-5':

3'-(N_{L})ₙ N_{M1} N_{L} N_{M2} N_{L} N_{F} N_{L} N_{M3} N_{L} N_{M4} N_{L} N_{M5} N_{F} N_{L} N_{M7} N_{M8} N_{L} N_{F} N_{L}-5' formula (II)

wherein, each N_{F} represents a 2'-fluoro modified nucleotide;
N_{M1}, N_{M2}, N_{M3}, N_{M4}, N_{M5}, N_{M7}, and N_{M8} independently represent a modified or an unmodified nucleotide, wherein only two of N_{M1}, N_{M2}, N_{M3}, and N_{M4} are 2'-fluoro modified nucleotides, and N_{M5}, N_{M7} and N_{M8} are not 2' -fluoro modified nucleotides;
each N_{L} independently represents a modified or an unmodified nucleotide, wherein the modified nucleotide is not a 2'-fluoro modified nucleotide;
n can be an integer from 0 to 7.

In some specific embodiments, the antisense strand of the dsRNA agent comprises a sequence represented by formula (III) in the direction of 3'-5' :

3'-(N_{L})ₙ N_{M1} N_{L} N_{M2} N_{L} N_{F} N_{L} N_{F} N_{L} N_{M4} N_{L} N_{M5} N_{M6} N_{L} N_{M7} N_{M8} N_{L} N_{F} N_{L}-5' formula (III)

wherein, each N_{F} represents a 2'-fluoro modified nucleotide;
N_{M1}, N_{M2}, N_{M4}, N_{M5}, N_{M6}, N_{M7}, and N_{M8} independently represent a modified or an unmodified nucleotide, wherein only two of N_{M1}, N_{M2}, N_{M6}, and N_{M7} are 2'-fluoro modified nucleotides, and N_{M4}, N_{M5} and N_{M8} are not 2'-fluoro modified nucleotides;
each N_{L} independently represents a modified or an unmodified nucleotide, wherein the modified nucleotide is not a 2'-fluoro modified nucleotide;
n can be an integer from 0 to 7.

In some more specific embodiments, the dsRNA agent has 2'-fluoro modified nucleotides at positions 2, 5, 12, 14, and 18 of the antisense strand represented by formula (I-1) counting from the first paired nucleotide at 5' end. In the embodiments, N_{M1}, N_{M3}, and N_{M7} in the antisense strand represented by formula (I-1) of the dsRNA agent are 2'-fluoro modified nucleotides.

In some more specific embodiments, the dsRNA agent has 2'-fluoro modified nucleotides at positions 2, 7, 12, 14, and 16 of the antisense strand represented by formula (I-1) counting from the first paired nucleotide at 5' end. In the embodiments, N_{M2}, N_{M3}, and N_{M6} in the antisense strand represented by formula (I-1) of the dsRNA agent are 2'-fluoro modified nucleotides.

In some more specific embodiments, the dsRNA agent has 2'-fluoro modified nucleotides at positions 2, 4, 10, 14, and 16 of the antisense strand represented by formula (I-1) counting from the first paired nucleotide at 5' end.

In some more specific embodiments, the dsRNA agent has 2'-fluoro modified nucleotides at positions 2, 7, 10, 14, and 16 of the antisense strand represented by formula (I-1) counting from the first paired nucleotide at 5' end.

In some more specific embodiments, the dsRNA agent has 2'-fluoro modified nucleotides at positions 2, 7, 12, 14, and 18 of the antisense strand represented by formula (I-1) counting from the first paired nucleotide at 5' end.

In some more specific embodiments, the dsRNA agent has 2'-fluoro modified nucleotides at positions 2, 5, 12, 14, and 16 of the antisense strand represented by formula (I-1) counting from the first paired nucleotide at 5' end.

In some more specific embodiments, the dsRNA agent has 2'-fluoro modified nucleotides at positions 2, 5, 10, 14, and 16 of the antisense strand represented by formula (I-1) counting from the first paired nucleotide at 5' end.

It will be appreciated that in the embodiments, the dsRNA agent has only five 2'-fluoro modified nucleotides in the antisense strand represented by formula (I-1), formula (II) or formula (III).

In some embodiments, in the antisense strand represented by formula (I-1) of the dsRNA agent, only one of N_{M1}, N_{M2}, N_{M3}, N_{M5}, N_{M6}, and N_{M8} is a 2'-fluoro modified nucleotide, and N_{M4} and N_{M7} are not 2'-fluoro modified nucleotides.

In some embodiments, the antisense strand of the dsRNA agent comprises a sequence represented by formula (IV) in the direction of 3' to 5':

3'-(N_{L})ₙ N_{M1} N_{L} N_{M2} N_{L} N_{F} N_{L} N_{M3} N_{L} N_{M4} N_{L} N_{M5} N_{M6} N_{L} N_{M7} N_{M8} N_{L} N_{F} N_{L}-5' formula (IV)

wherein, each N_{F} represents a 2'-fluoro modified nucleotide;
N_{M1}, N_{M2}, N_{M3}, N_{M4}, N_{M5}, N_{M7}, and N_{M8} independently represent a modified or an unmodified nucleotide, wherein only one of N_{M1}, N_{M2}, N_{M3}, N_{M5}, N_{M6}, and N_{M8} is a 2'-fluoro modified nucleotide, and N_{M4} and N_{M7} are not 2'-fluoro modified nucleotides;
each N_{L} independently represents a modified or an unmodified nucleotide, wherein the modified nucleotide is not a 2'-fluoro modified nucleotide;
n can be an integer from 0 to 7.

In some more specific embodiments, the dsRNA agent has 2'-fluoro modified nucleotides at positions 2, 4, and 14 of the antisense strand represented by formula (IV) counting from the first paired nucleotide at 5' end.

In some more specific embodiments, the dsRNA agent has 2'-fluoro modified nucleotides at positions 2, 8, and 14 of the antisense strand represented by formula (IV) counting from the first paired nucleotide at 5' end.

In some more specific embodiments, the dsRNA agent has 2'-fluoro modified nucleotides at positions 2, 7, and 14 of the antisense strand represented by formula (IV) counting from the first paired nucleotide at 5' end.

In some more specific embodiments, the dsRNA agent has 2'-fluoro modified nucleotides at positions 2, 12, and 14 of the antisense strand represented by formula (IV) counting from the first paired nucleotide at 5' end.

In some more specific embodiments, the dsRNA agent has 2'-fluoro modified nucleotides at positions 2, 14, and 16 of the antisense strand represented by formula (IV) counting from the first paired nucleotide at 5' end.

In some more specific embodiments, the dsRNA agent has 2'-fluoro modified nucleotides at positions 2, 14, and 18 of the antisense strand represented by formula (IV) counting from the first paired nucleotide at 5' end.

In some more specific embodiments, the dsRNA agent has 2'-fluoro modified nucleotides at positions 2, 6, and 14 of the antisense strand counting from the first paired nucleotide at 5' end.

It will be appreciated that in the embodiments, the dsRNA agent has only three 2'-fluoro modified nucleotides in the antisense strand of formula (I-1) or formula (IV).

In some embodiments, an RNAi agent capable of inhibiting expression of a target gene is involved, wherein the RNAi agent is a double-stranded ribonucleic acid (dsRNA) agent comprising a sense strand and an antisense strand complementary to at least a portion of an mRNA corresponding to the target gene, and the antisense strand has 18-30 nucleotides and comprises a sequence represented by formula (I-2) in the direction of 3'-5':

3'-(N_{L})ₙ N_{M1} N_{L} N_{M2} N_{L} N_{F} N_{L} N_{M3} N_{M9} N_{M4} N_{M10} N_{M5} N_{M6} N_{L} N_{M7} N_{M8} N_{L} N_{F} N_{L}-5' formula (I-2)

wherein, each N_{F} represents a 2'-fluoro modified nucleotide;
N_{M1}, N_{M2}, N_{M3}, N_{M4}, N_{M5}, N_{M6}, N_{M7}, N_{M8}, N_{M9} and N_{M10} independently represent a modified or an unmodified nucleotide, wherein only three of N_{M1}, N_{M2}, N_{M3}, N_{M4}, N_{M5}, N_{M6}, N_{M7}, N_{M8}, N_{M9} and N_{M10} are 2'-fluoro modified nucleotides;
each N_{L} independently represents a modified or an unmodified nucleotide, wherein the modified nucleotide is not a 2'-fluoro modified nucleotide;
n can be an integer from 0 to 7.

In some more specific embodiments, in the antisense strand represented by formula (I-2) of the dsRNA agent, N_{M6} and N_{M9} independently represent a 2'-fluoro modified nucleotide, only one of N_{M1} and N_{M2} is a 2'-fluoro modified nucleotide, and N_{M3}, N_{M4}, N_{M5}, N_{M7}, N_{M8} and N_{M10} are not 2'-fluoro modified nucleotides. It will be understood by those skilled in the art that N_{M6} and N_{M9} both are 2'-fluoro modified nucleotides, only one of N_{M1} and N_{M2} is a 2'-fluoro modified nucleotide, and N_{M3}, N_{M4}, N_{M5}, N_{M7} and N_{M8} are not 2'-fluoro modified nucleotides. In the embodiments, it is meant that in the antisense strand, the nucleotides at positions 2, 7, 11, 14 and 16 are 2'-fluoro modified nucleotides, while the nucleotides at other positions are not 2'-fluoro modified nucleotides; alternatively, the nucleotides at positions 2, 7, 11, 14, and 18 are 2'-fluoro modified nucleotides, while the nucleotides at other positions are not 2'-fluoro modified nucleotides.

In some more specific embodiments, in the antisense strand represented by formula (I-2) of the dsRNA agent, N_{M2} and N_{M3} independently represent a 2'-fluoro modified nucleotide, only one of N_{M5}, N_{M6} and N_{M10} is a 2'-fluoro modified nucleotide, and N_{M1}, N_{M4}, N_{M7}, N_{M8} and N_{M9} are not 2'-fluoro modified nucleotides. It will be understood by those skilled in the art that N_{M2} and N_{M3} both are 2'-fluoro modified nucleotides, only one of N_{M5}, N_{M6}, and N_{M10} is a 2' -fluoro modified nucleotide, and N_{M1}, N_{M4}, N_{M7}, N_{M8}, and N_{M9} are not 2' -fluoro modified nucleotides. In the embodiments, it is meant that in the antisense strand, the nucleotides at positions 2, 7, 12, 14, and 16 are 2'-fluoro modified nucleotides, while the nucleotides at other positions are not 2'-fluoro modified nucleotides; alternatively, the nucleotides at positions 2, 8, 12, 14, and 16 are 2'-fluoro modified nucleotides, while the nucleotides at other positions are not 2'-fluoro modified nucleotides; alternatively, the nucleotides at positions 2, 9, 12, 14, and 16 are 2'-fluoro modified nucleotides, while the nucleotides at other positions are not 2'-fluoro modified nucleotides.

In some embodiments, an RNAi agent capable of inhibiting expression of a target gene is involved, wherein the RNAi agent is a double-stranded ribonucleic acid (dsRNA) agent comprising a sense strand and an antisense strand complementary to at least a portion of an mRNA corresponding to the target gene, and the antisense strand has 18-30 nucleotides and comprises a sequence represented by formula (I') in the direction of 3' to 5':

3'-(N_{L})ₙ N_{M1} N_{L} N_{F} N_{L} N_{F} N_{L} N_{M3} N_{L} N_{M4} N_{L} N_{M5} N_{F} N_{L} N_{M7} N_{M8} N_{L} N_{F} N_{L}-5' formula (I')

wherein, each N_{F} represents a 2'-fluoro modified nucleotide;
N_{M1}, N_{M3}, N_{M4}, N_{M5}, N_{M7}, and N_{M8} independently represent a modified or an unmodified nucleotide, wherein no more than one of N_{M1}, N_{M3}, N_{M4}, N_{M5}, N_{M7}, and N_{M8} is a 2'-fluoro modified nucleotide;
each N_{L} independently represents a modified or an unmodified nucleotide, wherein the modified nucleotide is not a 2'-fluoro modified nucleotide;
n can be an integer from 0 to 7.

In some more specific embodiments, in the antisense strand represented by formula (I') of the dsRNA agent, only one of N_{M1}, N_{M3}, N_{M4}, N_{M5}, N_{M7} and N_{M8} is a 2'-fluoro modified nucleotide. Those skilled in the art will understand that in the antisense strand, the nucleotides at positions 2, 7, 14, and 16 are 2'-fluoro modified nucleotides, only one of N_{M1}, N_{M3}, N_{M4}, N_{M5}, N_{M7}, and N_{M8} is a 2'-fluoro modified nucleotide, and the nucleotides at other positions are not 2'-fluoro modified nucleotides, meaning a total of five 2'-fluoro modified nucleotides. By way of non-limiting example, the nucleotides at positions 2, 5, 7, 14, and 16 are 2'-fluoro modified nucleotide, while the nucleotides at other positions are not 2'-fluoro modified nucleotide; the nucleotides at positions 2, 4, 7, 14 and 16 are 2'-fluoro modified nucleotides, while the nucleotides at other positions are not 2'-fluoro modified nucleotides; the nucleotides at positions 2, 7, 8, 14 and 16 are 2'-fluoro modified nucleotides, while the nucleotides at other positions are not 2'-fluoro modified nucleotides; the nucleotides at positions 2, 7, 10, 14 and 16 are 2'-fluoro modified nucleotides, while the nucleotides at other positions are not 2'-fluoro modified nucleotides; the nucleotides at positions 2, 7, 12, 14 and 16 are 2'-fluoro modified nucleotides, while the nucleotides at other positions are not 2'-fluoro modified nucleotides; the nucleotides at positions 2, 7, 14, 16, and 18 are 2'-fluoro modified nucleotides, while the nucleotides at other positions are not 2'-fluoro modified nucleotides.

In some more specific embodiments, in the antisense strand represented by formula (I') of the dsRNA agent, N_{M1}, N_{M3}, N_{M4}, N_{M5}, N_{M7} and N_{M8} are not 2'-fluoro modified nucleotides. Those skilled in the art will understand that in the antisense strand, the nucleotides at positions 2, 7, 14 and 16 are 2'-fluoro modified nucleotides, while the nucleotides at other positions are not 2'-fluoro modified nucleotides, meaning a total of four 2'-fluoro modified nucleotides. In some embodiments, in the antisense strand represented by formula (I) and/or formula (X) of the dsRNA agent, one or more of N_{X} are modified nucleotides independently selected from the group consisting of 2'-fluoro modified nucleotide, 2'-O-methyl modified nucleotide, 2'-deoxy nucleotide, 2',3'-seco nucleotide mimic, locked nucleotide (LNA), unlocked nucleic acid nucleotide (UNA), glycol nucleic acid nucleotide (GNA), bicyclo nucleic acid (BNA), 2'-F-arabino nucleotide, 2'-methoyxyethyl nucleotide (MOE), abasic nucleotide, ribitol, inverted nucleotide, inverted abasic nucleotide (Invab), inverted 2'-OMe nucleotide, inverted 2'-deoxy nucleotide, 2'-amino modified nucleotide, 2'-alkyl modified nucleotide, morpholino nucleotide, 3'-OMe nucleotide, phosphorothioate modified nucleotide, terminal nucleotide linked to a cholesteryl derivative or dodecanoic acid bisdecylamide group, 2'-amino modified nucleotide, phosphoramidate, and non-natural base comprising nucleotide. In some embodiments, in the antisense strand represented by formula (I-1), formula (I-2), formula (I'), formula (II), formula (III) and/or formula (IV) of the dsRNA agent, one or more of N_{L} are modified nucleotides independently selected from the group consisting of 2'-O-methyl modified nucleotide, 2'-deoxy nucleotide, 2',3'-seco nucleotide mimic, locked nucleotide (LNA), unlocked nucleic acid nucleotide (UNA), glycol nucleic acid nucleotide (GNA), bicyclo nucleic acid (BNA), 2'-F-arabino nucleotide, 2'-methoyxyethyl nucleotide (MOE), abasic nucleotide, ribitol, inverted nucleotide, inverted abasic nucleotide (Invab), inverted 2'-OMe nucleotide, inverted 2'-deoxy nucleotide, 2'-amino modified nucleotide, 2'-alkyl modified nucleotide, morpholino nucleotide, 3'-OMe nucleotide, phosphorothioate modified nucleotide, terminal nucleotide linked to a cholesteryl derivative or dodecanoic acid bisdecylamide group, 2'-amino modified nucleotide, phosphoramidate, and non-natural base comprising nucleotide. In some embodiments, N_{L} in the dsRNA agent is a modified nucleotide selected from the group consisting of 2'-O-methyl modified nucleotide, UNA, and Invab.

In some embodiments, the antisense strand represented by formula (I) and/or formula (I') of the dsRNA agent has an E-vinylphosphonate nucleotide at 5' end of the antisense strand.

In some embodiments, in the antisense strand represented by formula (I-1), formula (I-2), formula (I'), formula (II), formula (III) and/or a formula (IV) of the dsRNA agent, N_{M1}, N_{M2}, N_{M3}, N_{M4}, N_{M5}, N_{M6}, N_{M7}, N_{M8}, N_{M9}, and N_{M10} are modified nucleotides independently selected from the group consisting of 2'-fluoro modified nucleotide, 2'-O-methyl modified nucleotide, 2'-deoxy nucleotide, 2',3'-seco nucleotide mimic, locked nucleotide (LNA), unlocked nucleic acid nucleotide (UNA), glycol nucleic acid nucleotide (GNA), bicyclo nucleic acid (BNA), 2'-F-arabino nucleotide, 2'-methoyxyethyl nucleotide, abasic nucleotide, ribitol, inverted nucleotide, inverted abasic nucleotide (Invab), inverted 2'-OMe nucleotide, inverted 2'-deoxy nucleotide, 2'-amino modified nucleotide, 2'-alkyl modified nucleotide, morpholino nucleotide, 3'-OMe nucleotide, phosphorothioate modified nucleotide, terminal nucleotide linked to a cholesteryl derivative or dodecanoic acid bisdecylamide group, 2'-amino modified nucleotide, phosphoramidate, and non-natural base comprising nucleotide. In some embodiments, in the antisense strand represented by formula (I) and/or formula (I') of the dsRNA agent, N_{M1}, N_{M2}, N_{M3}, N_{M4}, N_{M5}, N_{M6}, N_{M7}, and N_{M8} are independently 2'-O methyl modified nucleotides in case of not being 2'-fluoro modified nucleotides.

In some embodiment, all or substantially all of nucleotides in the sense strand and the antisense strand of the dsRNA agent are modified nucleotides.

In some embodiments, the dsRNA agent has a sense strand complementary or substantially complementary to the antisense strand represented by formula (X), formula (I), formula (I-1), formula (I-2), formula (I'), formula (II), formula (III) and/or formula (IV), and the complementary or substantially complementary region is 18-25 nucleotides in length. In some embodiments, the dsRNA agent has a sense strand complementary or substantially complementary to the antisense strand represented by formula (I), and the complementary or substantially complementary region is 18-25 nucleotides in length. In some embodiments, the dsRNA agent has a sense strand complementary or substantially complementary to the antisense strand of formula (I'), and the complementary or substantially complementary region is 18-25 nucleotides in length. In some embodiments, the complementary or substantially complementary region is 18-23 nucleotides in length. In some embodiments, the complementary or substantially complementary region is 19-21 nucleotides in length. In some embodiments, the complementary or substantially complementary region is 18, 19, 20, 21, 22, 23, 24, or 25 nucleotides in length. In some embodiment, the dsRNA agent has a sense strand fully complementary to the antisense strand represented by formula (I). In some embodiment, the dsRNA agent has a sense strand fully complementary to the antisense strand represented by formula (I').

In some embodiments, n is 7 in the dsRNA agent, and it can be understood that the complementary pair of the antisense strand is 25 nucleotides in length. In some specific embodiments, n is 0, 1, 2, 3, 4, 5, or 6 in the dsRNA agent.

In one embodiment, the dsRNA agent of the present invention may comprises one or more of an overhang at the 3' or 5' end or both ends, and the overhang is 1-5 nucleotides in length. In a specific embodiment, the dsRNA agent of the present invention may have an overhang of 1-5 nucleotides in length at 3' end of the antisense strand. In a specific embodiment, the dsRNA agent of the present invention may have an overhang of 1-5 nucleotides in length at 5' end of the sense strand. In a more specific embodiment, the dsRNA agent of the present invention may have an overhang of 1 or 2 nucleotides in length.

In one embodiment, the dsRNA agent of the present invention has a blunt end at 5' end of the antisense strand or at 3' end of the sense strand.

In some embodiment, the sense strand of the dsRNA agent is no more than 40 nucleotides in length. In some embodiment, each strand of that dsRNA agent is no more than 30 nucleotides in length. In some embodiment, each strand of that dsRNA agent is no more than 25 nucleotides in length. In some embodiment, each strand of that dsRNA agent is no more than 23 nucleotides in length. In some embodiment, each strand of that dsRNA agent is 19, 20, or 21 nucleotides in length.

In some embodiment, that dsRNA agent comprises at least one phosphorothioate internucleoside linkage. In some embodiments, the sense strand of the dsRNA agent comprises at least one thiophosphate nucleoside linkage. In some embodiment, the antisense strand of the dsRNA agent comprises at least one phosphorothioate internucleoside linkage. In some embodiment, that sense strand and/or antisense strand of the dsRNA agent comprises 1, 2, 3, 4, 5, or 6 phosphorothioate internucleoside linkages. In some embodiments, the dsRNA agent comprises 1 or 2 phosphorothioate internucleoside linkages at 3' end and/or 5' end of the sense strand and/or the antisense strand.

In some embodiments, the dsRNA agent further comprises one or more targeting groups or linking groups. In some embodiments, the one or more targeting groups or linking groups of the dsRNA agent of the present invention are same or different. The targeting group or linking group can be conjugated to the sense strand, antisense strand, or both strands at 3' end, 5' end, or both ends. In some embodiments, one or more targeting groups or linking groups of the dsRNA agent are conjugated to the sense strand. The distribution, targeting, lifetime, endosomal dissolution characteristics, improved delivery, hybridization, or specificity of the dsRNA agent can be altered by the targeting ligand incorporated, for example, but not limiting, lectin, glycoprotein, lipid or protein, thyrotropin, melanotropin, lectin, glycoprotein, surfactant protein A, mucin carbohydrate, multivalent lactose, multivalent galactose, N-acetyl-galactosamine, N-acetyl-glucosamine, multivalent mannose, multivalent fucose, glycosylated polyaminoacid, multivalent galactose, transferrin, bisphosphonate, polyglutamate, polyaspartate, lipid, cholesterol, steroid, bile acid, folate, vitamin B12, vitamin A, biotin, RGD peptide, RGD peptide mimetic, or aptamer, and other examples include dyes, intercalating agents (e.g. acridines), cross-linkers (e.g. psoralene, mitomycin C), porphyrins (TPPC4, texaphyrin, sapphyrin), polycyclic aromatic hydrocarbons (e.g., phenazine, dihydrophenazine), artificial endonucleases or chelating agents (e.g. EDTA), lipophilic molecules, e.g., cholesterol, cholic acid, adamantane acetic acid, 1-pyrene butyric acid, dihydrotestosterone, 1,3-bis-O(hexadecyl)glycerol, geranyloxyhexyl group, hexadecylglycerol, borneol, menthol, 1,3-propanediol, heptadecyl group, palmitic acid, myristic acid, O3-(oleoyl)lithocholic acid, O3-(oleoyl)cholenic acid, dimethoxytrityl, or phenoxazine) and peptide conjugates (e.g., antennapedia peptide, Tat peptide), alkylating agents, phosphate, amino, mercapto, PEG (e.g., PEG-40K), MPEG, [MPEG]2, polyamino, alkyl, substituted alkyl, radiolabeled markers, enzymes, haptens (e.g., biotin), transport/absorption facilitators (e.g., aspirin, vitamin E, folic acid), synthetic ribonucleases (e.g., imidazole, bisimidazole, histamine, imidazole clusters, acridine-imidazole conjugates, Eu3+ complexes of tetraazamacrocycles), dinitrophenyl, HRP, or AP. In some embodiments, the targeting group or linking group is selected from the group consisting of N-acetyl-galactosamine (GalNAc) and a lipophilic molecule. The lipophilic molecule can be used for delivery in the field of neurology. In some embodiments, the targeting group or linking group of the dsRNA agent is conjugated to the 3' end and/or the 5' end of the sense strand. In some embodiment, the targeting group of the dsRNA agent has the following structure:

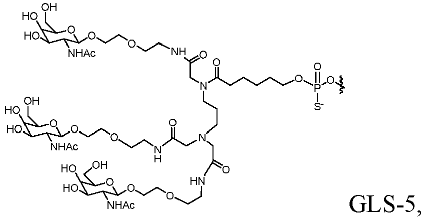

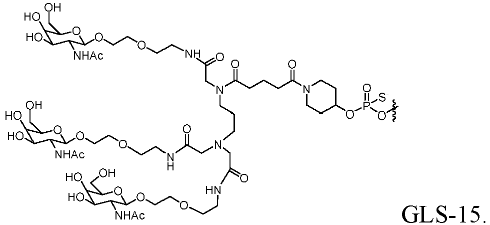

In some embodiments, the dsRNA agent comprises an inverted abasic residue at the 3' end of the antisense strand represented by formula (I) and/or formula (I'). In some embodiments, the sense strand of the dsRNA agent comprises one or two inverted abasic residues at the 3' end or/and 5' end.

In some embodiments, an sequence of inhibitor FXII(F12) gene is expressed in the dsRNA agent. In some embodiments, the dsRNA agent has the sequence shown in Table 2 below.

In some embodiments, the dsRNA agent is an siRNA.

According to another aspect of the present invention, an RNAi agent for inhibiting expression of a target gene is provided, wherein the RNAi agent is double-stranded ribonucleic acid (dsRNA) agent comprising a sense strand and an antisense strand complementary to at least a portion of an mRNA corresponding to the target gene, and the sense strand has 18-40 nucleotides and comprises a sequence represented by formula (V) in the direction of 5'-3':

5'-(N'_{L})_{n'} N'_{L} N'_{L} N'_{L} N'_{N1} N'_{N2} N'_{N3} N'_{N4} N'_{F} N'_{L} N'_{N5}N'_{N6} N'_{L} N'_{L} N'_{L} N'_{L} N'_{L} N'_{L} N'_{L}-3' formula (V)

wherein, each N'_{F} represents a 2'-fluoro modified nucleotide;
N'_{N1}, N'_{N2}, N'_{N3}, N'_{N4}, N'_{N5}, N'_{N6} independently represent a modified or an unmodified nucleotide, wherein at least two of N'_{N1}, N'_{N2}, N'_{N3}, N'_{N4}, N'_{N5}, and N'_{N6} are 2'-fluoro modified nucleotides;
formula (V) has no motif of three or more contiguous 2'-fluoro modified nucleotides and has no more than six 2'-fluoro modified nucleotides;
each N'_{L} independently represents a modified or an unmodified nucleotide;
n' can be an integer from 0-7.

In some embodiments, an RNAi agent for inhibiting expression of a target gene is provided, wherein the RNAi agent is double-stranded ribonucleic acid (dsRNA) agent comprising a sense strand and an antisense strand complementary to at least a portion of an mRNA corresponding to the target gene, and the sense strand has 18-40 nucleotides and comprises a sequence represented by the formula (V') in the direction of 5'-3':

5'-(N'_{L})_{n'} N'_{L} N'_{L} N'_{L} N'_{N1} N'_{N2} N'_{N3} N' _{N4} N'_{F} N'_{L} N'_{N5}N'_{N6} N'_{L} N'_{L} N'_{L} N'_{L} N'_{L} N'_{L} N'_{L}-3' formula (V')

wherein, each N'_{F} represents a 2'-fluoro modified nucleotide;
N'_{N1}, N'_{N2}, N'_{N3}, N'_{N4}, N'_{N5}, and N'_{N6} independently represent a modified or an unmodified nucleotide, wherein only two of N'_{N1}, N'_{N2}, N'_{N3}, N'_{N4}, N'_{N5}, and N'_{N6} are 2'-fluoro modified nucleotides;
each N'_{L} independently represents a modified or an unmodified nucleotide, wherein the modified nucleotide is not a 2'-fluoro modified nucleotide;
n' can be an integer from 0-7.

In some embodiments, the dsRNA agent has 2'-fluoro modified nucleotides at positions 9, 11, and 13 of the sense strand represented by formula (V') counting from the first paired nucleotide at 3' end of the sense strand. In the embodiments, in the sense strand represented by formula (V') of the dsRNA agent, N' _{N3} and N'_{N5} are 2'-fluorine modified nucleotides.

In some embodiments, the dsRNA agent has 2'-fluoro modified nucleotides at positions 8, 11, and 13 of the sense strand represented by formula (V') counting from the first paired nucleotide at 3' end of the sense strand. In the embodiments, in the sense strand represented by formula (V') of the dsRNA agent, N' _{N3} and N'_{N6} are 2'-fluorine modified nucleotides.

In some embodiments, the dsRNA agent has 2'-fluoro modified nucleotides at positions 9, 11 and 12 of the sense strand represented by formula (V') counting from the first paired nucleotide at 3' end of the sense strand.

In some embodiments, the dsRNA agent has 2'-fluoro modified nucleotides at positions 11, 12 and 14 of the sense strand represented by formula (V') counting from the first paired nucleotide at 3' end of the sense strand.

In some embodiments, the dsRNA agent has 2'-fluoro modified nucleotides at positions 11, 12 and 15 of the sense strand represented by formula (V') counting from the first paired nucleotide at 3' end of the sense strand.

It can be understood that in the embodiments, the dsRNA agent has only three 2'-fluorine modified nucleotides in the sense strand represented by formula (V').

In some embodiments, the dsRNA agent has 2'-fluoro modified nucleotides at positions 9, 11, and 13 of the sense strand represented by formula (V) counting from the first paired nucleotide at 3' end of the sense strand. In the embodiments, in the sense strand represented by formula (V) of the dsRNA agent, N' _{N3} and N'_{N5} are 2'-fluorine modified nucleotides.

In some embodiments, the dsRNA agent has 2'-fluoro modified nucleotides at positions 8, 11, and 13 of the sense strand represented by formula (V) counting from the first paired nucleotide at 3' end of the sense strand. In the embodiments, in the sense strand represented by formula (V) of the dsRNA agent, N' _{N3} and N'_{N6} are 2'-fluorine modified nucleotides.

In some embodiments, the dsRNA agent has 2'-fluoro modified nucleotides at positions 9, 11 and 12 of the sense strand represented by formula (V) counting from the first paired nucleotide at 3' end of the sense strand.

In some embodiments, the dsRNA agent has 2'-fluoro modified nucleotides at positions 11, 12 and 14 of the sense strand represented by formula (V) counting from the first paired nucleotide at 3' end of the sense strand.

In some embodiments, the dsRNA agent has 2'-fluoro modified nucleotides at positions 11, 12 and 15 of the sense strand represented by formula (V) counting from the first paired nucleotide at 3' end of the sense strand.

It can be understood that in the embodiments, the sense strand represented by formula (V) of the dsRNA agent has three 2'-fluorine modified nucleotides but a total of no more than six 2'-fluorine modified nucleotides, and has no motif of three or more contiguous 2'-fluorine modified nucleotides.

According to another aspect of the present invention, an RNAi agent for inhibiting expression of a target gene is provided, wherein the RNAi agent is double-stranded ribonucleic acid (dsRNA) agent comprising a sense strand and an antisense strand complementary to at least a portion of an mRNA corresponding to the target gene, and the sense strand has 18-40 nucleotides and comprises a sequence represented by formula (IX) in the direction of 5'-3' :

5'-(N'_{L})_{n'} N'_{L} N'_{L} N'_{L} N'_{N1} N'_{N2} N'_{N3} N'_{N4} N'_{F} N'_{L} N'_{N5}N'_{N6} N'_{N7} N'_{L} N'_{L} N'_{L} N'_{L} N'_{L} N'_{L}-3' formula (IX)

wherein, each N'_{F} represents a 2'-fluoro modified nucleotide;
N'_{N1}, N'_{N2}, N'_{N3}, N'_{N4}, N'_{N5}, N'_{N6}, and N'_{N7} independently represent a modified or an unmodified nucleotide, wherein at least two of N'_{N1}, N' _{N2}, N'_{N3}, N'_{N4}, N'_{N5}, N'_{N6}, and N'_{N7} are 2'-fluoro modified nucleotides;
formula (IX) has no motif of three or more contiguous 2'-fluoro modified nucleotides and has no more than six 2'-fluoro modified nucleotides;
each N'_{L} independently represents a modified or an unmodified nucleotide;
n' can be an integer from 0 to 7.

In some embodiments, in the sense strand represented by formula (V) and/or formula (IX), one or more of N'_{L} are modified nucleotides independently selected from the group consisting of 2'-fluoro modified nucleotide, 2'-O-methyl modified nucleotide, 2'-deoxy nucleotide, 2',3'-seco nucleotide mimic, locked nucleotide (LNA), unlocked nucleic acid nucleotide (UNA), glycol nucleic acid nucleotide (GNA), bicyclo nucleic acid (BNA), 2'-F-arabino nucleotide, 2'-methoyxyethyl nucleotide (MOE), abasic nucleotide, ribitol, inverted nucleotide, inverted abasic nucleotide (Invab), inverted 2'-OMe nucleotide, inverted 2'-deoxy nucleotide, 2'-amino modified nucleotide, 2'-alkyl modified nucleotide, morpholino nucleotide, 3'-OMe nucleotide, phosphorothioate modified nucleotide, terminal nucleotide linked to a cholesteryl derivative or dodecanoic acid bisdecylamide group, 2'-amino modified nucleotide, phosphoramidate, and non-natural base comprising nucleotide. In some embodiments, N'_{L} in formula (V) and/or formula (IX) of the dsRNA agent is a modified nucleotide selected from the group consisting of 2'-O-methyl modified nucleotide, UNA, and Invab

In some embodiments, the dsRNA agent has an E-vinylphosphonate nucleotide at 5' end of the antisense strand.

In some embodiments, in the sense strand represented by formula (V) and/or formula (IX), N'_{N1}, N'_{N2}, N'_{N3}, N'_{N4}, N'_{N5}, N'_{N6}, and N'_{N7} are modified nucleotides independently selected from the group consisting of 2'-fluoro modified nucleotide, 2'-O-methyl modified nucleotide, 2'-deoxy nucleotide, 2',3'-seco nucleotide mimic, locked nucleotide (LNA), unlocked nucleic acid nucleotide (UNA), glycol nucleic acid nucleotide (GNA), bicyclo nucleic acid (BNA), 2'-F-arabino nucleotide, 2'-methoyxyethyl nucleotide, abasic nucleotide, ribitol, inverted nucleotide, inverted abasic nucleotide (Invab), inverted 2'-OMe nucleotide, inverted 2'-deoxy nucleotide, 2'-amino modified nucleotide, 2'-alkyl modified nucleotide, morpholino nucleotide, 3'-OMe nucleotide, phosphorothioate modified nucleotide, terminal nucleotide linked to a cholesteryl derivative or dodecanoic acid bisdecylamide group, 2'-amino modified nucleotide, phosphoramidate, and non-natural base comprising nucleotide. In some embodiments, in the sense strand represented by formula (V) of the dsRNA agent, N'_{N1}, N'_{N2}, N'_{N3}, N'_{N4}, N'_{N5}, and N'_{N6} are preferably 2'-O methyl modified nucleotides in case of not being 2'-fluoro modified nucleotides.

In some embodiment, all or substantially all of nucleotides in the sense strand and the antisense strand of the dsRNA agent are modified nucleotides.

In some embodiments, the dsRNA agent has an antisense strand complementary or substantially complementary to the sense strand represented by formula (V), and the complementary or substantially complementary region is 18-25 nucleotides in length. In some embodiments, the complementary or substantially complementary region is 18-25 nucleotides in length. In some embodiments, the complementary or substantially complementary region is 18-23 nucleotides in length. In some embodiments, the complementary or substantially complementary region is 19-21 nucleotides in length. In some embodiments, the complementary or substantially complementary region is 18, 19, 20, 21, 22, 23, 24, or 25 nucleotides in length. In some embodiment, the dsRNA agent has an antisense strand fully complementary to the sense strand represented by formula (V).

In some embodiments, n' is 7 in the dsRNA agent, and it can be understood that the complementary pair of the sense strand is 25 nucleotides in length. In some specific embodiments, n' is 0, 1, 2, 3, 4, 5, or 6 in the dsRNA agent.

In one embodiment, the dsRNA agent of the present invention may comprises one or more of an overhang at the 3' or 5' end or both ends, and the overhang is 1-5 nucleotides in length. The overhang can be the result of one strand being longer than the other, or the result of two strands of the same length being staggered. The overhang may form one mismatch with the target mRNA, or it may be complementary to the targeted gene sequence or may be other sequences. In a specific embodiment, the dsRNA agent of the present invention may have an overhang of 1-5 nucleotides in length at 3' end of the antisense strand. In a specific embodiment, the dsRNA agent of the present invention may have an overhang of 1-5 nucleotides in length at 5' end of the sense strand. In a more specific embodiment, the dsRNA agent of the present invention may have an overhang of 1 or 2 nucleotides in length.

In one embodiment, the dsRNA agent of the present invention has a blunt end at 5' end of the antisense strand or at 3' end of the sense strand.

In some embodiment, the antisense strand of the dsRNA agent is no more than 30 nucleotides in length. In some embodiment, each strand of that dsRNA agent is no more than 30 nucleotides in length. In some embodiment, each strand of that dsRNA agent is no more than 25 nucleotides in length. In some embodiment, each strand of that dsRNA agent is no more than 23 nucleotides in length. In some embodiment, each strand of that dsRNA agent is 19, 20, or 21 nucleotides in length.

In some embodiment, that dsRNA agent comprises at least one phosphorothioate internucleoside linkage. In some embodiments, the sense strand of the dsRNA agent comprises at least one thiophosphate nucleoside linkage. In some embodiment, the antisense strand of the dsRNA agent comprises at least one phosphorothioate internucleoside linkage. In some embodiment, that sense strand and/or antisense strand of the dsRNA agent comprises 1, 2, 3, 4, 5, or 6 phosphorothioate internucleoside linkages. In some embodiments, the dsRNA agent comprises 1 or 2 phosphorothioate internucleoside linkages at 3' end and/or 5' end of the sense strand and/or the antisense strand.

In some embodiments, the dsRNA agent further comprises one or more targeting groups or linking groups. In some embodiments, the one or more targeting groups or linking groups of the dsRNA agent of the present invention are same or different. The targeting group or linking group can be conjugated to the sense strand, antisense strand, or both strands at 3' end, 5' end, or both ends. In some embodiments, one or more targeting groups or linking groups of the dsRNA agent are conjugated to the sense strand. The distribution, targeting, lifetime, endosomal dissolution characteristics, improved delivery, hybridization, or specificity of the dsRNA agent can be altered by the targeting ligand incorporated, for example, but not limiting, lectin, glycoprotein, lipid or protein, thyrotropin, melanotropin, lectin, glycoprotein, surfactant protein A, mucin carbohydrate, multivalent lactose, multivalent galactose, N-acetyl-galactosamine, N-acetyl-glucosamine, multivalent mannose, multivalent fucose, glycosylated polyaminoacid, multivalent galactose, transferrin, bisphosphonate, polyglutamate, polyaspartate, lipid, cholesterol, steroid, bile acid, folate, vitamin B12, vitamin A, biotin, RGD peptide, RGD peptide mimetic, or aptamer, and other examples include dyes, intercalating agents (e.g. acridines), cross-linkers (e.g. psoralene, mitomycin C), porphyrins (TPPC4, texaphyrin, sapphyrin), polycyclic aromatic hydrocarbons (e.g., phenazine, dihydrophenazine), artificial endonucleases or chelating agents (e.g. EDTA), lipophilic molecules, e.g., cholesterol, cholic acid, adamantane acetic acid, 1-pyrene butyric acid, dihydrotestosterone, 1,3-bis-O(hexadecyl)glycerol, geranyloxyhexyl group, hexadecylglycerol, borneol, menthol, 1,3-propanediol, heptadecyl group, palmitic acid, myristic acid, O3-(oleoyl)lithocholic acid, O3-(oleoyl)cholenic acid, dimethoxytrityl, or phenoxazine) and peptide conjugates (e.g., antennapedia peptide, Tat peptide), alkylating agents, phosphate, amino, mercapto, PEG (e.g., PEG-40K), MPEG, [MPEG]2, polyamino, alkyl, substituted alkyl, radiolabeled markers, enzymes, haptens (e.g., biotin), transport/absorption facilitators (e.g., aspirin, vitamin E, folic acid), synthetic ribonucleases (e.g., imidazole, bisimidazole, histamine, imidazole clusters, acridine-imidazole conjugates, Eu3+ complexes of tetraazamacrocycles), dinitrophenyl, HRP, or AP. In some embodiments, the targeting group or linking group is selected from the group consisting of N-acetyl-galactosamine (GalNAc) and a lipophilic molecule. The lipophilic molecule can be used for delivery in the field of neurology. In some embodiments, the targeting group or linking group of the dsRNA agent is conjugated to the 5' end of the sense strand. In some embodiment, the targeting group of the dsRNA agent has the following structure:

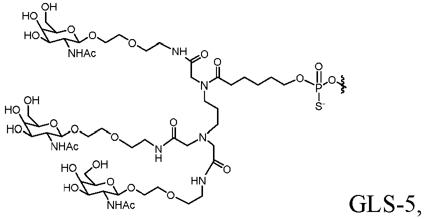

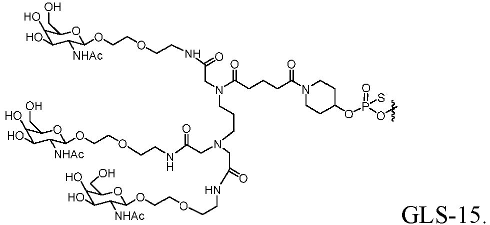

In some embodiments, the dsRNA agent comprises an inverted abasic residue at the 3' end of the sense strand represented by formula (V) and/or formula (IX). In some embodiments, the sense strand represented by formula (V) and/or formula (IX) of the dsRNA agent comprises one or two inverted abasic residues at the 3' end or/and 5' end.

In some embodiments, an sequence of inhibitor FXII(F12) gene is expressed in the dsRNA agent. In some embodiments, the dsRNA agent has the sequence shown in Table 2 below.

In some embodiments, the dsRNA agent is an siRNA.

In yet another aspect of the present invention, an RNAi agent for inhibiting expression of a target gene is provided, wherein the RNAi agent is double-stranded ribonucleic acid (dsRNA) agent comprising a sense strand and an antisense strand complementary to at least a portion of an mRNA corresponding to the target gene, wherein the antisense strand has 18-30 nucleotides, the sense strand has 18-40 nucleotides, and the dsRNA agent comprises an antisense strand represented by formula (I) and a sense strand represented by formula (V);

the antisense strand comprises a sequence represented by formula (I) in the direction of 3'-5':

3'-(N_{X})ₙ N_{X} N_{X} N_{X} N_{X} N_{F} N_{X} N_{X} N_{X} N_{X} N_{X} N_{X} N_{X} N_{X} N_{X}N_{X} N_{X} N_{F} N_{X}-5' formula (I)

the sense strand comprises a sequence represented by formula (V) in the direction of 5'-3':

5'-(N'_{L})_{n'} N' _{L}N'_{L}N'_{L}N'_{N1} N' _{N2} N' _{N3} N' _{N4} N'_{F} N'_{L}N' _{N5}N' _{N6} N'_{L} N'_{L}N'_{L}N'_{L}N'_{L}N'_{L} N'_{L}-3' formula (V)

wherein, each N_{F} represents a 2'-fluoro modified nucleotide;
each N_{X} independently represents a modified or an unmodified nucleotide, wherein only three N_{X} are 2'-fluoro modified nucleotides, or only one N_{X} is a 2'-fluoro modified nucleotide;
each N'_{F} represents a 2'-fluoro modified nucleotide;
N'_{N1}, N'_{N2}, N'_{N3}, N'_{N4}, N'_{N5}, and N'_{N6} independently represent a modified or an unmodified nucleotide, wherein at least two of N'_{N1}, N'_{N2}, N'_{N3}, N'_{N4}, N'_{N5}, and N'_{N6} are 2'-fluoro modified nucleotides;
the sense strand represented by formula (V) has no motif of three or more contiguous 2'-fluoro modified nucleotides and has no more than six 2'-fluoro modified nucleotides;
each N'_{L} independently represents a modified or an unmodified nucleotide;
each n and n' can independently be an integer from 0 to 7.

It can be understood that the dsRNA agent comprises combinations of an antisense strand having any modification described in formula (I) herein and a sense strand having any modification described in formula (V) herein.

Including, but not limited to, in some embodiments, an RNAi agent for inhibiting expression of a target gene is provided, wherein the RNAi agent is double-stranded ribonucleic acid (dsRNA) agent comprising a sense strand and an antisense strand complementary to at least a portion of an mRNA corresponding to the target gene, wherein the antisense strand has 18-30 nucleotides, the sense strand has 18-40 nucleotides, and the dsRNA agent comprises an antisense strand represented by formula (I-1) and a sense strand represented by formula (V);
the antisense strand comprises a sequence represented by formula (I-1) in the direction of 3'-5':

3'-(N_{L})ₙ N_{M1} N_{L} N_{M2} N_{L} N_{F} N_{L} N_{M3} N_{L} N_{M4} N_{L} N_{M5} N_{M6} N_{L} N_{M7} N_{M8} N_{L} N_{F} N_{L}-5' formula (I-1)

the sense strand comprises a sequence represented by formula (V) in the direction of 5'-3':

5'-(N'_{L})_{n'} N' _{L}N'_{L} N'_{L} N'_{N1} N' _{N2} N' _{N3} N' _{N4} N'_{F} N'_{L}N' _{N5}N' _{N6} N'_{L} N'_{L} N'_{L} N'_{L} N'_{L} N'_{L} N'_{L}-3' formula (V)

wherein, each N_{F} represents a 2'-fluoro modified nucleotide;
N_{M1}, N_{M2}, N_{M3}, N_{M4}, N_{M5}, N_{M6}, N_{M7}, and N_{M8} independently represent a modified or an unmodified nucleotide, wherein only three of N_{M1}, N_{M2}, N_{M3}, N_{M4}, N_{M5}, N_{M6}, N_{M7}, and N_{M8} are 2'-fluoro modified nucleotides, or only one of N_{M1}, N_{M2}, N_{M3}, N_{M4}, N_{M5}, N_{M6}, N_{M7}, and N_{M8} is a 2'-fluoro modified nucleotide;
each N_{L} independently represents a modified or an unmodified nucleotide, wherein the modified nucleotide is not a 2'-fluoro modified nucleotide;
each N'_{F} represents a 2'-fluoro modified nucleotide;
N'_{N1}, N'_{N2}, N'_{N3}, N'_{N4}, N'_{N5}, and N'_{N6} independently represent a modified or an unmodified nucleotide, wherein at least two of N'_{N1}, N'_{N2}, N'_{N3}, N'_{N4}, N'_{N5}, and N'_{N6} are 2'-fluoro modified nucleotides;
the sense strand represented by formula (V) has no motif of three or more contiguous 2'-fluoro modified nucleotides and has no more than six 2'-fluoro modified nucleotides;
each N'_{L} independently represents a modified or an unmodified nucleotide;
each n and n' can independently be an integer from 0 to 7.

In some embodiments, the dsRNA agent comprises an antisense strand with the nucleotide sequence represented by formula (I-1) herein and a sense strand with the nucleotide sequence represented by formula (V') herein. In some embodiments, the dsRNA agent comprises an antisense strand with the nucleotide sequence represented by formula (II) herein and a sense strand with the nucleotide sequence represented by formula (V) herein. In some embodiments, the dsRNA agent comprises an antisense strand with the nucleotide sequence represented by formula (III) herein and a sense strand with the nucleotide sequence represented by formula (V) herein. In some embodiments, the dsRNA agent comprises an antisense strand with the nucleotide sequence represented by formula (IV) herein and a sense strand with the nucleotide sequence represented by formula (V) herein. In some embodiments, the dsRNA agent comprises an antisense strand with the nucleotide sequence represented by formula (II) herein and a sense strand with the nucleotide sequence represented by formula (V') herein. In some embodiments, the dsRNA agent comprises an antisense strand with the nucleotide sequence represented by formula (III) herein and a sense strand with the nucleotide sequence represented by formula (V') herein. In some embodiments, the dsRNA agent comprises an antisense strand with the nucleotide sequence represented by formula (IV) herein and a sense strand with the nucleotide sequence represented by formula (V') herein. In some embodiments, the dsRNA agent comprises an antisense strand with the nucleotide sequence represented by formula (I-2) herein and a sense strand with the nucleotide sequence represented by formula (V') herein. In some embodiments, the dsRNA agent comprises an antisense strand with the nucleotide sequence represented by formula (I') herein and a sense strand with the nucleotide sequence represented by formula (V') herein.

In some more specific embodiments, the dsRNA agent has 2'-fluoro modified nucleotides at positions 2, 7, 12, 14, and 16 of the antisense strand represented by formula (I-1) counting from the first paired nucleotide at 5' end, and has 2'-fluoro modified nucleotides at positions 9, 11, and 13 of the sense strand represented by formula (V) or formula (V') counting from the first paired nucleotide at 3' end of the sense strand. In some more specific embodiments, the dsRNA agent has 2'-fluoro modified nucleotides at positions 2, 7, 12, 14, and 16 of the antisense strand represented by formula (I-1) counting from the first paired nucleotide at 5' end, and has 2'-fluoro modified nucleotides at positions 9, 11, and 12 of the sense strand represented by formula (V) or formula (V') counting from the first paired nucleotide at 3' end of the sense strand. In some more specific embodiments, the dsRNA agent has 2'-fluoro modified nucleotides at positions 2, 7, 12, 14, and 16 of the antisense strand represented by formula (I-1) counting from the first paired nucleotide at 5' end, and has 2'-fluoro modified nucleotides at positions 8, 11, and 13 of the sense strand represented by formula (V) or formula (V') counting from the first paired nucleotide at 3' end of the sense strand. In some more specific embodiments, the dsRNA agent has 2'-fluoro modified nucleotides at positions 2, 7, 12, 14, and 16 of the antisense strand represented by formula (I-1) counting from the first paired nucleotide at 5' end, and has 2'-fluoro modified nucleotides at positions 11, 12, and 14 of the sense strand represented by formula (V) or formula (V') counting from the first paired nucleotide at 3' end of the sense strand. In some more specific embodiments, the dsRNA agent has 2'-fluoro modified nucleotides at positions 2, 7, 12, 14, and 16 of the antisense strand represented by formula (I-1) counting from the first paired nucleotide at 5' end, and has 2'-fluoro modified nucleotides at positions 11, 12, and 15 of the sense strand represented by formula (V) or formula (V') counting from the first paired nucleotide at 3' end of the sense strand.

In some more specific embodiments, the dsRNA agent has 2'-fluoro modified nucleotides at positions 2, 5, 12, 14, and 18 of the antisense strand represented by formula (I-1) counting from the first paired nucleotide at 5' end, and has 2'-fluoro modified nucleotides at positions 9, 11, and 13 of the sense strand represented by formula (V) or formula (V') counting from the first paired nucleotide at 3' end of the sense strand. In some more specific embodiments, the dsRNA agent has 2'-fluoro modified nucleotides at positions 2, 5, 12, 14, and 18 of the antisense strand represented by formula (I-1) counting from the first paired nucleotide at 5' end, and has 2'-fluoro modified nucleotides at positions 9, 11, and 12 of the sense strand represented by formula (V) or formula (V') counting from the first paired nucleotide at 3' end of the sense strand. In some more specific embodiments, the dsRNA agent has 2'-fluoro modified nucleotides at positions 2, 5, 12, 14, and 18 of the antisense strand represented by formula (I-1) counting from the first paired nucleotide at 5' end, and has 2'-fluoro modified nucleotides at positions 8, 11, and 13 of the sense strand represented by formula (V) or formula (V') counting from the first paired nucleotide at 3' end of the sense strand. In some more specific embodiments, the dsRNA agent has 2'-fluoro modified nucleotides at positions 2, 5, 12, 14, and 18 of the antisense strand represented by formula (I-1) counting from the first paired nucleotide at 5' end, and has 2'-fluoro modified nucleotides at positions 11, 12, and 14 of the sense strand represented by formula (V) or formula (V') counting from the first paired nucleotide at 3' end of the sense strand. In some more specific embodiments, the dsRNA agent has 2'-fluoro modified nucleotides at positions 2, 5, 12, 14, and 18 of the antisense strand represented by formula (I-1) counting from the first paired nucleotide at 5' end, and has 2'-fluoro modified nucleotides at positions 11, 12, and 15 of the sense strand represented by formula (V) or formula (V') counting from the first paired nucleotide at 3' end of the sense strand.

In some more specific embodiments, the dsRNA agent has 2'-fluoro modified nucleotides at positions 2, 7, 12, 14, and 18 of the antisense strand represented by formula (I-1) counting from the first paired nucleotide at 5' end, and has 2'-fluoro modified nucleotides at positions 9, 11, and 12 of the sense strand represented by formula (V) or formula (V') counting from the first paired nucleotide at 3' end of the sense strand.

In some more specific embodiments, the dsRNA agent has 2'-fluoro modified nucleotides at positions 2, 4, 10, 14, and 16 of the antisense strand represented by formula (I-1) counting from the first paired nucleotide at 5' end, and has 2'-fluoro modified nucleotides at positions 9, 11, and 12 of the sense strand represented by formula (V) or formula (V') counting from the first paired nucleotide at 3' end of the sense strand.

In some more specific embodiments, the dsRNA agent has 2'-fluoro modified nucleotides at positions 2, 7, 10, 14, and 16 of the antisense strand represented by formula (I-1) counting from the first paired nucleotide at 5' end, and has 2'-fluoro modified nucleotides at positions 9, 11, and 12 of the sense strand represented by formula (V) or formula (V') counting from the first paired nucleotide at 3' end of the sense strand.

In some more specific embodiments, the dsRNA agent has 2'-fluoro modified nucleotides at positions 2, 4, and 14 of the antisense strand represented by formula (IV) counting from the first paired nucleotide at 5' end, and has 2'-fluoro modified nucleotides at positions 8, 11, and 13 of the sense strand represented by formula (V) or formula (V') counting from the first paired nucleotide at 3' end of the sense strand.

In some more specific embodiments, the dsRNA agent has 2'-fluoro modified nucleotides at positions 2, 8, and 14 of the antisense strand represented by formula (IV) counting from the first paired nucleotide at 5' end, and has 2'-fluoro modified nucleotides at positions 8, 11, and 13 of the sense strand represented by formula (V) or formula (V') counting from the first paired nucleotide at 3' end of the sense strand. In some more specific embodiments, the dsRNA agent has 2'-fluoro modified nucleotides at positions 2, 7, and 14 of the antisense strand represented by formula (IV) counting from the first paired nucleotide at 5' end, and has 2'-fluoro modified nucleotides at positions 8, 11, and 13 of the sense strand represented by formula (V) or formula (V') counting from the first paired nucleotide at 3' end of the sense strand. In some more specific embodiments, the dsRNA agent has 2'-fluoro modified nucleotides at positions 2, 12, and 14 of the antisense strand represented by formula (IV) counting from the first paired nucleotide at 5' end, and has 2'-fluoro modified nucleotides at positions 8, 11, and 13 of the sense strand represented by formula (V) or formula (V') counting from the first paired nucleotide at 3' end of the sense strand. In some more specific embodiments, the dsRNA agent has 2'-fluoro modified nucleotides at positions 2, 14, and 16 of the antisense strand represented by formula (IV) counting from the first paired nucleotide at 5' end, and has 2'-fluoro modified nucleotides at positions 8, 11, and 13 of the sense strand represented by formula (V) or formula (V') counting from the first paired nucleotide at 3' end of the sense strand. In some more specific embodiments, the dsRNA agent has 2'-fluoro modified nucleotides at positions 2, 14, and 18 of the antisense strand represented by formula (IV) counting from the first paired nucleotide at 5' end, and has 2'-fluoro modified nucleotides at positions 8, 11, and 13 of the sense strand represented by formula (V) or formula (V') counting from the first paired nucleotide at 3' end of the sense strand. In some more specific embodiments, the dsRNA agent has 2'-fluoro modified nucleotides at positions 2, 6, and 14 of the antisense strand represented by formula (IV) counting from the first paired nucleotide at 5' end, and has 2'-fluoro modified nucleotides at positions 8, 11, and 13 of the sense strand represented by formula (V) or formula (V') counting from the first paired nucleotide at 3' end of the sense strand.

In yet another aspect of the present invention, an RNAi agent for inhibiting expression of a target gene is provided, wherein the RNAi agent is double-stranded ribonucleic acid (dsRNA) agent comprising a sense strand and an antisense strand complementary to at least a portion of an mRNA corresponding to the target gene, wherein the antisense strand has 18-30 nucleotides, the sense strand has 18-40 nucleotides, and the dsRNA agent comprises an antisense strand represented by formula (I-2) and a sense strand represented by formula (V);
the antisense strand comprises a sequence represented by formula (I-2) in the direction of 3'-5':

3'-(N_{L})ₙ N_{M1} N_{L} N_{M2} N_{L} N_{F} N_{L} N_{M3} N_{M9} N_{M4}N_{M10} N_{M5} N_{M6} N_{L} N_{M7} N_{M8} N_{L} N_{F} N_{L}-5' formula (I-2)

the sense strand comprises a sequence represented by formula (V) in the direction of 5'-3':

5'-(N'_{L})_{n'} N' _{L}N'_{L}N'_{L}N'_{N1} N' _{N2} N' _{N3} N' _{N4} N'_{F} N'_{L}N' _{N5}N' _{N6} N'_{L} N'_{L}N'_{L}N'_{L}N'_{L}N'_{L} N'_{L}-3' formula (V)

wherein, each N_{F} and N'_{F} represents a 2'-fluoro modified nucleotide;
N_{M1}, N_{M2}, N_{M3}, N_{M4}, N_{M5}, N_{M6}, N_{M7}, N_{M8}, N_{M9} and N_{M10} independently represent a modified or an unmodified nucleotide, wherein only three of N_{M1}, N_{M2}, N_{M3}, N_{M4}, N_{M5}, N_{M6}, N_{M7}, N_{M8}, N_{M9} and N_{M10} are 2'-fluoro modified nucleotides;
N'_{N1}, N'_{N2}, N'_{N3}, N'_{N4}, N'_{N5}, and N'_{N6} independently represent a modified or an unmodified nucleotide, wherein at least two of N'_{N1}, N'_{N2}, N'_{N3}, N'_{N4}, N'_{N5}, and N'_{N6} are 2'-fluoro modified nucleotides;
the sense strand represented by formula (V) has no motif of three or more contiguous 2'-fluoro modified nucleotides and has no more than six 2'-fluoro modified nucleotides;
each N_{L} and N'_{L} independently represents a modified or an unmodified nucleotide;
each n and n' can independently be an integer from 0 to 7.

In yet another aspect of the present invention, an RNAi agent for inhibiting expression of a target gene is provided, wherein the RNAi agent is double-stranded ribonucleic acid (dsRNA) agent comprising a sense strand and an antisense strand complementary to at least a portion of an mRNA corresponding to the target gene, wherein the antisense strand has 18-30 nucleotides, the sense strand has 18-40 nucleotides, and the dsRNA agent comprises an antisense strand represented by formula (I') and a sense strand represented by formula (V);
the antisense strand comprises a sequence represented by formula (I') in the direction of 3'-5':

3'-(N_{L})ₙ N_{M1} N_{L} N_{F} N_{L} N_{F} N_{L} N_{M3} N_{L} N_{M4} N_{L} N_{M5} N_{F} N_{L} N_{M7} N_{M8} N_{L} N_{F} N_{L}-5' formula (I')

the sense strand comprises a sequence represented by formula (V) in the direction of 5'-3':

5'-(N'_{L})_{n'} N' _{L}N'_{L}N'_{L}N'_{N1} N' _{N2} N' _{N3} N' _{N4} N'_{F} N'_{L}N' _{N5}N' _{N6} N'_{L} N'_{L}N'_{L}N'_{L}N'_{L}N'_{L} N'_{L}-3' formula (V)

wherein, each N_{F} and N'_{F} represents a 2'-fluoro modified nucleotide;
N_{M1}, N_{M3}, N_{M4}, N_{M5}, N_{M7}, and N_{M8} independently represent a modified or an unmodified nucleotide, wherein no more than one of N_{M1}, N_{M3}, N_{M4}, N_{M5}, N_{M7}, and N_{M8} is a 2'-fluoro modified nucleotide;
N'_{N1}, N'_{N2}, N'_{N3}, N'_{N4}, N'_{N5}, and N'_{N6} independently represent a modified or an unmodified nucleotide, wherein at least two of N'_{N1}, N'_{N2}, N'_{N3}, N'_{N4}, N'_{N5}, and N'_{N6} are 2'-fluoro modified nucleotides;
the sense strand represented by formula (V) has no motif of three or more contiguous 2'-fluoro modified nucleotides and has no more than six 2'-fluoro modified nucleotides;
each N_{L} and N'_{L} independently represents a modified or an unmodified nucleotide;
each n and n' can independently be an integer from 0 to 7.

It can be understood that the dsRNA agent comprises combinations of an antisense strand having any modification described in formula (X), formula (I), formula (I'), formula (I-1), formula (I-2), formula (II), formula (III), or formula (IV) herein and a sense strand having any modification described in formula (V), formula (IX) or formula (V') herein. It can be further understood that the dsRNA agent comprises combinations of an antisense strand having any modification described in formula (X) herein and a sense strand having any modification described in formula (V) or formula (IX) herein.

Another aspect of the present invention also provides a composition comprising the dsRNA according to any above-mentioned embodiment of the present invention.

In some embodiments, the composition further comprises a pharmaceutically acceptable carrier. Such composition typically comprises one or more RNAi and a pharmaceutically acceptable carrier. As used herein, the term "pharmaceutically acceptable carrier" is intended to include any and all solvents, dispersion media, coatings, antibacterial and antifungal agents, isotonic and absorption delaying agents, and the like, compatible with pharmaceutical administration. The use of such media and agents for pharmaceutically active substances is well known in the art. Except insofar as any conventional media or agent is incompatible with the active compound, use thereof in the compositions is contemplated.

The composition of the present invention can be administered in a variety of ways depending on whether local or systemic therapy is desired and on the area to be treated. Administration may be local (including ophthalmic, vaginal, rectal, intranasal, transdermal), oral, or parenteral. In some embodiments, parenteral administration includes subcutaneous, intravenous, intraarterial, intralymphatic, intrabronchial, intrapleural, intraperitoneal, intracerobrospinal, intramuscular, intrathecal, or intraventricular administration. In some embodiments, the route and site of administration can be selected to enhance targeting. For example, for purpose of targeting myocytes, intramuscular injection into the muscle of interest would be a logical choice. In some embodiments, lung cells can be targeted by administering the RNAi in aerosol form.

In some embodiments, the composition also comprises one or more additional therapeutic agents.

In some embodiments, the composition is packaged in a kit, container, pack, dispenser, pre-filled syringe, or vial.

Another aspect of the present invention provides a cell comprising the dsRNA agent according to any above-mentioned embodiment of the present invention.

In some embodiments, the cell is mammalian cells, optionally human cells.

Another aspect of the present invention provides a method for inhibiting expression of a target gene in a cell, the method comprising:
delivering the dsRNA agent according to any above-mentioned embodiment of the present invention into a subject, such that the dsRNA agent is delivered to a specific target in the subject. The dsRNA agent of the present invention can be used for treating or alleviating conditions, diseases or disorders associated with aberrant expression or activity of a target gene, such as a pathological phenotype caused by overexpression of the gene product. Exemplary target genes include, but are not limited to: LPA, PNPLA3, ASGR1, F7, F12, FXI, APOCIII, APOB, APOL1, TTR, PCSK9, SCAP, KRAS, CD274, PDCD1, C5, ALAS1, HAO1, LDHA, ANGPTL3, SERPINA1, AGT, HAMP, LECT2, EGFR, VEGF, KIF11, AT3, CTNNB1, HMGB1, HIF1A, APP, ATXN2, C9orf72, TARDBP, MAPT (Tau), HTT, SNCA, FUS, ATXN3, SCN9A, SCN10A, CACNA1B, ATXN1, SCAl, SCA7, SCA8, MeCP2, PRNP, SODl, DMPK, and STAT3. The target gene may also include viral genes, such as hepatitis B and C virus genes, human immunodeficiency virus genes, herpes virus genes, and the like. Exemplary diseases include, but are not limited to viral diseases (such as HIV, HBV), neuromuscular diseases, bacterial infections, inflammation, immune diseases, metabolic diseases, liver diseases, kidney diseases, cardiovascular diseases, ophthalmic diseases, lung diseases and rare diseases.

A composition comprising the dsRNA can be delivered to a subject by multiple administration routes. Exemplary administration routes include ophthalmic, vaginal, rectal, intranasal, transdermal, subcutaneous, intravenous, intraarterial, intralymphatic, intrabronchial, intrapleural, intraperitoneal, intracerobrospinal, intramuscular, pulmonary, intrathecal, or intraventricular administration. The RNAi molecule of the present invention can be incorporated into pharmaceutical compositions suitable for administration.

In some embodiments, the dsRNA agent is administered subcutaneously to the subject *in vivo.*

In some embodiments, the dsRNA agent is administered intravenously to the subject *in vivo.*

### BRIEF DESCRIPTION OF SEQUENCES AND DRAWINGS

SEQ ID NO: 1 is FXII mRNA [NCBI Reference Sequence: NM_021489.3. The sequence thereof is shown as below:

1 actcctgggc aggcagcggg gccatcggca gacgccatga cggctctgtt gttcctgggg
61 tctctgctga tgagtctgga tctgacactt tcggctccac catggaaaga ctccaagaaa
121 tttaaggacg cacctgatgg gcccacagtg gttctcactg tggatgggag gctctgccat
181 tttccctttc agtaccaccg tcagctacac cacaaatgca tccacaaagg gcggccaggc
241 tcccgcccct ggtgtgctac cacccccaac tttgatgaag atcagcaatg gggatactgc
301 ttggagccca agaaagtgaa agaccattgc agcaaacaca acccgtgcca caaaggaggg
361 acatgtatca acacccccaa tgggccacac tgtctctgcc ctgaacacct cactgggaaa
421 cattgccaga aagagaaatg ctttgagcct cagcttctca agttcttcca cgagaatgag
481 ctatggttta gaacggggcc aggaggtgtg gccaggtgcg agtgcaaagg ttctgaggct
541 cactgcaagc cggtggccag ccaggcctgc agcatcaatc cgtgccttaa tgggggcagc
601 tgcctcctcg tggaggacca cccactgtgc cgttgcccta caggctacac tggatatttt
661 tgcgacttgg acctttgggc gacctgctat gaaggcaggg ggctcagcta ccggggccag
721 gctggaacta cgcaatcggg tgcgccatgt cagcggtgga ccgtggaggc cacctaccgg
781 aacatgactg agaagcaagc gctaagctgg ggcctgggcc accacgcatt ttgccggaac

 841 ccagataatg acacacgtcc atggtgcttc gtctggagtg gcgacaggct gagctgggac
 901 tattgcggcc tggagcagtg ccagacgcca acgtttgcac ctctagttgt ccctgagagt
 961 caggaggagt ccccgtccca ggcaccatct ctgtcccatg caccaaatga ctcgaccgat
1021 catcagactt ctctgtccaa gaccaacacg atgggctgcg gacagaggtt ccgcaaggga
1081 ctgtcctcgt tcatgcgcgt ggtgggcgga ctagtggctc tgcctgggtc gcacccctac
1141 atcgctgcac tgtactgggg taacaacttc tgcgcgggca gtctcatcgc cccctgttgg
1201 gtgctgaccg cggctcactg cctgcagaat cggccagcgc ccgaggaact gacagtggta
1261 cttggtcaag atcgccacaa ccagagctgc gagtggtgcc agactctggc tgtgcgctcc
1321 taccgccttc acgagggctt ctcctccatc acctaccagc acgacttggc tctgctgcgc
1381 ctgcaggaaa gcaaaaccaa cagttgcgcg atcctgtcac ctcacgttca gcctgtgtgt
1441 ctacccagcg gcgcggcccc accctctgag acagtgctct gcgaggtggc cggctggggt
1501 caccagttcg agggggctga agaatactcc accttcctgc aggaggcaca ggttcccttt
1561 atcgccctgg atcgctgctc caactctaac gtgcacggag acgccattct ccctgggatg
1621 ctttgcgctg gcttcttgga gggaggcacc gatgcctgcc agggtgactc cgggggccct
1681 ctggtgtgtg aggaaggaac tgcagaacat cagctcaccc tgcgcggagt catcagctgg
1741 ggctccggct gtggtgaccg caacaagccc ggagtctaca cagacgtggc caactacctg
1801 gcttggatcc agaagcatat tgcttcataa ctaaccaggc tttatccttc cctccttgtg
1861 tgctccttgg gatgggacga tgaatgtggc atgctgggtc acagtgaagc tagtgccccg
1921 acactggggg cacagaaact caataaagtg ctttgaaaac gttcctcaga a

Table 1 shows the duplexes AD# of the present invention and the sequences of their unmodified sense strand and antisense strand. Table 2 shows the duplexes AD# of the present invention and the sequences of their modified sense strand and antisense strand. The chemical modifications are expressed as the following: uppercase indicates 2'-fluorine modified nucleotide; lowercase indicates 2'-OMe modified nucleotide; "*" indicates a phosphorothioate internucleoside linkage; the delivery molecules used in *in vivo* studies are indicated as "GLX-n" at the 3' or 5' end of each sense strand; and "Invab" indicates inverted abasic nucleotide.

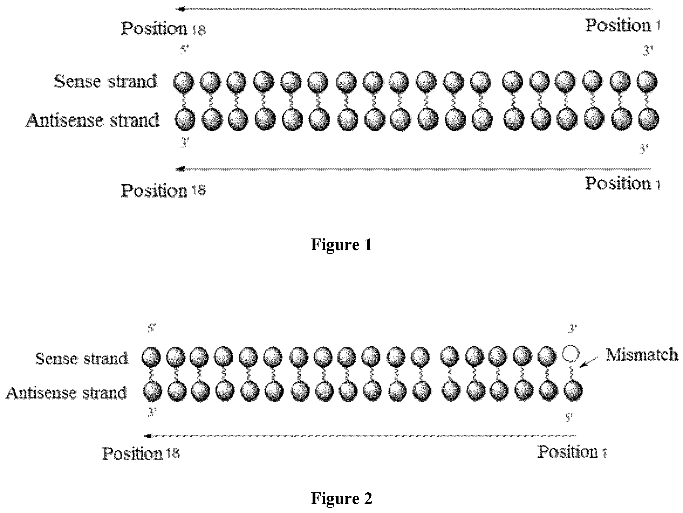

Figure 1 shows the position of the dsRNA sequence of the present invention.

Figure 2 shows the position of the dsRNA sequence containing one mismatched base of the present invention.

Figure 3-1 and Figure 3-2 show the position of the dsRNA sequence containing one overhang of the present invention.

Figure 4 shows the FXII silencing effect of AD00127 of the present invention and positive controls AD00198 and AD00199 after 14 days of use in mice.

### DETAILED DESCRIPTION

In some embodiments of the present invention, for example but not limited to, an RNAi agent capable of inhibiting expression of a target gene is involved in one aspect, wherein the RNAi agent is a double-stranded ribonucleic acid (dsRNA) agent comprising a sense strand and an antisense strand complementary to at least a portion of an mRNA corresponding to the target gene, and the antisense strand has 18-30 nucleotides and comprises a sequence represented by formula (X) in the direction of 3'-5':

3'-(N_{X})ₙ Nx Nx Nx N_{X} N_{F} N_{X} N_{X} N_{X} N_{X} N_{X} N_{X} N_{X} N_{X} N_{X}N_{X} N_{X} N_{F} N_{X}-5' formula (X)

wherein, each N_{F} represents a 2'-fluoro modified nucleotide;
each N_{X} independently represents a modified or an unmodified nucleotide, wherein no more than three N_{X} are 2'-fluoro modified nucleotides;
n can be an integer from 0 to 7.

In some embodiments, an RNAi agent for inhibiting expression of a target gene is provided, wherein the RNAi agent is a double-stranded ribonucleic acid (dsRNA) agent comprising a sense strand and an antisense strand complementary to at least a portion of an mRNA corresponding to the target gene, and the antisense strand has 18-30 nucleotides and comprises a sequence represented by formula (I) in the direction of 3'-5' :

3'-(N_{X})ₙ Nx Nx Nx N_{X} N_{F} N_{X} N_{X} N_{X} N_{X} N_{X} N_{X} N_{X} N_{X} N_{X}N_{X} N_{X} N_{F} N_{X}-5' formula (I)

wherein, each N_{F} represents a 2'-fluoro modified nucleotide;
each N_{X} independently represents a modified or an unmodified nucleotide, wherein only three N_{X} are 2'-fluoro modified nucleotides, or only one N_{X} is a 2'-fluoro modified nucleotide;
n can be an integer from 0 to 7.

In some embodiments, an RNAi agent for inhibiting expression of a target gene is provided, wherein the RNAi agent is a double-stranded ribonucleic acid (dsRNA) agent comprising a sense strand and an antisense strand complementary to at least a portion of an mRNA corresponding to the target gene, and the antisense strand has 18-30 nucleotides and comprises a sequence represented by formula (I-1) in the direction of 3'-5':

3'-(N_{L})ₙ N_{M1} N_{L} N_{M2} N_{L} N_{F} N_{L} N_{M3} N_{L} N_{M4} N_{L} N_{M5} N_{M6} N_{L} N_{M7} N_{M8} N_{L} N_{F} N_{L}-5' formula (I-1)

wherein, each N_{F} represents a 2'-fluoro modified nucleotide;
N_{M1}, N_{M2}, N_{M3}, N_{M4}, N_{M5}, N_{M6}, N_{M7}, and N_{M8} independently represent a modified or an unmodified nucleotide, wherein only three of N_{M1}, N_{M2}, N_{M3}, N_{M4}, N_{M5}, N_{M6}, N_{M7}, and N_{M8} are 2'-fluoro modified nucleotides, or only one of N_{M1}, N_{M2}, N_{M3}, N_{M4}, N_{M5}, N_{M6}, N_{M7}, and N_{M8} is a 2'-fluoro modified nucleotide;
each N_{L} independently represents a modified or an unmodified nucleotide, wherein the modified nucleotide is not a 2'-fluoro modified nucleotide;
n can be an integer from 0 to 7.

In some embodiments, in the antisense strand represented by formula (I-1) of the dsRNA agent, only three of N_{M1}, N_{M2}, N_{M3}, N_{M4}, N_{M6}, N_{M7}, and N_{M8} are 2' -fluoro modified nucleotides, and N_{M5} is not a 2'-F modified nucleotide. It can be understood by one of skill in the art, by way of example but not limitation, that in some non-limiting examples, N_{M1}, N_{M2} and N_{M3} are 2'-fluorine modified nucleotides, and N_{M4}, N_{M5}, N_{M6}, N_{M7} and N_{M8} are not 2'-fluorine modified nucleotides, and it can be understood by one of skill in the art that the nucleotides at positions 2, 12, 14, 16, and 18 of the antisense strand are 2'-fluorine modified nucleotides, while the nucleotides at other positions are not 2'-fluorine modified nucleotides. In another non-limiting example, N_{M2}, N_{M3} and N_{M6} are 2'-fluorine modified nucleotides, and N_{M1}, N_{M4}, N_{M5}, N_{M7} and N_{M8} are not 2'-fluorine modified nucleotides, and it can be understood by one of skill in the art that the nucleotides at positions 2, 7, 12, 14, and 16 of the antisense strand are 2'-fluorine modified nucleotides, while the nucleotides at other positions are not 2'-fluorine modified nucleotides. In another non-limiting example, N_{M1}, N_{M3} and N_{M7} are 2'-fluorine modified nucleotides, and N_{M2}, N_{M4}, N_{M5}, N_{M6} and N_{M8} are not 2' -fluorine modified nucleotides, and it can be understood by one of skill in the art that the nucleotides at positions 2, 5, 12, 14, and 18 of the antisense strand are 2'-fluorine modified nucleotides, while the nucleotides at other positions are not 2'-fluorine modified nucleotides. In another non-limiting example, N_{M1}, N_{M3} and N_{M6} are 2'-fluorine modified nucleotides, and N_{M2}, N_{M4}, N_{M5}, N_{M7} and N_{M8} are not 2'-fluorine modified nucleotides, and it can be understood by one of skill in the art that the nucleotides at positions 2, 7, 12, 14, and 18 of the antisense strand are 2'-fluorine modified nucleotides, while the nucleotides at other positions are not 2'-fluorine modified nucleotides. In another non-limiting example, N_{M2}, N_{M3} and N_{M7} are 2'-fluorine modified nucleotides, and N_{M1}, N_{M4}, N_{M5}, N_{M6} and N_{M8} are not 2'-fluorine modified nucleotides, and it can be understood by one of skill in the art that the nucleotides at positions 2, 5, 12, 14, and 16 of the antisense strand are 2'-fluorine modified nucleotides, while the nucleotides at other positions are not 2'-fluorine modified nucleotides. In another non-limiting example, N_{M2}, N_{M4} and N_{M7} are 2'-fluorine modified nucleotides, and N_{M1}, N_{M3}, N_{M5}, N_{M6} and N_{M8} are not 2' -fluorine modified nucleotides, and it can be understood by one of skill in the art that the nucleotides at positions 2, 5, 10, 14, and 16 of the antisense strand are 2'-fluorine modified nucleotides, while the nucleotides at other positions are not 2'-fluorine modified nucleotides.

In some embodiments, the antisense strand of the dsRNA agent comprises a sequence represented by formula (II) in the direction of 3'-5':

3'-(N_{L})ₙ N_{M1} N_{L} N_{M2} N_{L} N_{F} N_{L} N_{M3} N_{L} N_{M4} N_{L} N_{M5} N_{F} N_{L} N_{M7} N_{M8} N_{L} N_{F} N_{L}-5' formula (II)

wherein, each N_{F} represents a 2'-fluoro modified nucleotide;
N_{M1}, N_{M2}, N_{M3}, N_{M4}, N_{M5}, N_{M7}, and N_{M8} independently represent a modified or an unmodified nucleotide, wherein only two of N_{M1}, N_{M2}, N_{M3}, and N_{M4} are 2'-fluoro modified nucleotides, and N_{M5}, N_{M7} and N_{M8} are not 2' -fluoro modified nucleotides;
each N_{L} independently represents a modified or an unmodified nucleotide, wherein the modified nucleotide is not a 2'-fluoro modified nucleotide;
n can be an integer from 0 to 7.

In some non-limiting examples, in the antisense strand represented by formula (II) of the dsRNA agent, N_{M1}, N_{M2}, N_{M3}, N_{M4}, N_{M5}, N_{M7}, and N_{M8} independently represent a modified or an unmodified nucleotide, wherein only two of N_{M1}, N_{M2}, N_{M3}, and N_{M4} are 2' -fluoro modified nucleotides, and N_{M5}, N_{M7} and N_{M8} are not 2'-fluoro modified nucleotides. It can be understood by one of skill in the art, by way of example but not limitation, that in some non-limiting examples, N_{M1} and N_{M2} are 2'-fluorine modified nucleotides, and N_{M3}, N_{M4}, N_{M5}, N_{M7} and N_{M8} are not 2'-fluorine modified nucleotides, and it can be understood by one of skill in the art that the nucleotides at positions 2, 7, 14, 16, and 18 of the antisense strand are 2'-fluorine modified nucleotides, while the nucleotides at other positions are not 2'-fluorine modified nucleotides. In another non-limiting example, N_{M2} and N_{M3} are 2'-fluorine modified nucleotides, and N_{M1}, N_{M4}, N_{M5}, N_{M7} and N_{M8} are not 2'-fluorine modified nucleotides, and it can be understood by one of skill in the art that the nucleotides at positions 2, 7, 12, 14, and 16 of the antisense strand are 2'-fluorine modified nucleotides, while the nucleotides at other positions are not 2'-fluorine modified nucleotides. In another non-limiting example, N_{M1} and N_{M3} are 2'-fluorine modified nucleotides, and N_{M2}, N_{M4}, N_{M5}, N_{M7} and N_{M8} are not 2'-fluorine modified nucleotides, and it can be understood by one of skill in the art that the nucleotides at positions 2, 7, 12, 14, and 18 of the antisense strand are 2'-fluorine modified nucleotides, while the nucleotides at other positions are not 2'-fluorine modified nucleotides. In another non-limiting example, N_{M2} and N_{M4} are 2'-fluorine modified nucleotides, and N_{M1}, N_{M3}, N_{M5}, N_{M7} and N_{M8} are not 2' -fluorine modified nucleotides, and it can be understood by one of skill in the art that the nucleotides at positions 2, 7, 10, 14, and 16 of the antisense strand are 2'-fluorine modified nucleotides, while the nucleotides at other positions are not 2'-fluorine modified nucleotides.

In some embodiments, the antisense strand of the dsRNA agent comprises a sequence represented by formula (III) in the direction of 3'-5':

3'-(N_{L})ₙ N_{M1} N_{L} N_{M2} N_{L} N_{F} N_{L} N_{F} N_{L} N_{M4} N_{L} N_{M5} N_{M6} N_{L} N_{M7} N_{M8} N_{L} N_{F} N_{L}-5' formula (III)

wherein, each N_{F} represents a 2'-fluoro modified nucleotide;
N_{M1}, N_{M2}, N_{M4}, N_{M5}, N_{M6}, N_{M7}, and N_{M8} independently represent a modified or an unmodified nucleotide, wherein only two of N_{M1}, N_{M2}, N_{M6}, and N_{M7} are 2'-fluoro modified nucleotides, and Nᵤ₄, N_{M5} and N_{M8} are not 2'-fluoro modified nucleotides;
each N_{L} independently represents a modified or an unmodified nucleotide, wherein the modified nucleotide is not a 2'-fluoro modified nucleotide;
n can be an integer from 0 to 7.

In some non-limiting examples, in the antisense strand represented by formula (III) of the dsRNA agent, N_{M1}, N_{M2}, N_{M4}, N_{M5}, N_{M6}, N_{M7} and N_{M8} independently represent a modified or an unmodified nucleotide, wherein only two of N_{M1}, N_{M2}, N_{M6}, and N_{M7} are 2'-fluorine modified nucleotide, and N_{M4}, N_{M5}, and N_{M8} are not 2' -fluorine modified nucleotides. It can be understood by one of skill in the art, by way of example but not limitation, that in some non-limiting examples, in the antisense strand represented by formula (III) of the dsRNA agent, N_{M1} and N_{M7} are 2'-fluorine modified nucleotides, and N_{M2}, N_{M4}, N_{M5}, N_{M6} and N_{M8} are not 2'-fluorine modified nucleotides, and it can be understood by one of skill in the art that the nucleotides at positions 2, 5, 12, 14, and 18 of the antisense strand are 2'-fluorine modified nucleotides, while the nucleotides at other positions are not 2'-fluorine modified nucleotides. In another non-limiting example, in the antisense strand represented by formula (III) of the dsRNA agent, N_{M2} and N_{M6} are 2'-fluorine modified nucleotides, and N_{M1}, N_{M4}, N_{M5}, N_{M7} and N_{M8} are not 2'-fluorine modified nucleotides, and it can be understood by one of skill in the art that the nucleotides at positions 2, 7, 12, 14, and 16 of the antisense strand are 2'-fluorine modified nucleotides, while the nucleotides at other positions are not 2'-fluorine modified nucleotides. In another non-limiting example, in the antisense strand represented by formula (III) of the dsRNA agent, N_{M3} and N_{M7} are 2'-fluorine modified nucleotides, and N_{M1}, N_{M4}, N_{M5}, N_{M6} and N_{M8} are not 2'-fluorine modified nucleotides, and it can be understood by one of skill in the art that the nucleotides at positions 2, 5, 12, 14, and 16 of the antisense strand are 2'-fluorine modified nucleotides, while the nucleotides at other positions are not 2'-fluorine modified nucleotides. In another non-limiting example, in the antisense strand represented by formula (III) of the dsRNA agent, N_{M4} and N_{M7} are 2'-fluorine modified nucleotides, and N_{M1}, N_{M3}, N_{M5}, N_{M6} and N_{M8} are not 2'-fluorine modified nucleotides, and it can be understood by one of skill in the art that the nucleotides at positions 2, 5, 10, 14, and 16 of the antisense strand are 2'-fluorine modified nucleotides, while the nucleotides at other positions are not 2'-fluorine modified nucleotides.

In some non-liniting examples, the dsRNA agent has 2'-fluoro modified nucleotides at positions 2, 7, 12, 14, and 16 of the antisense strand represented by formula (I-1) counting from the first paired nucleotide at 5' end, and it is understood that the dsRNA agent has no 2'-fluorine modified nucleotides at other positions of the antisense strand.

As used herein, the positions of the antisense strand represented by formula (I-1) counting from the first paired nucleotide at 5' end generally refer to the positions counting from the blunt end of the first paired nucleotide N_{L} at 5' end of the antisense strand, as a non-limiting example, in formula (Ia), 3'-(N_{L})ₙ N_{M1} N_{L} N_{F} N_{L} N_{F} N_{L} N_{F} N_{L} N_{M4} N_{L} N_{M5} N_{F} N_{L} N_{M7} N_{M8} N_{L} N_{F} N_{L} (at position 1)-5' formula (Ia); and it will be further understood that the first mismatch in the dsRNA agent is also considered as a pairing, also referred to as position 1.

In some specific embodiments, the dsRNA agent has 2'-fluoro modified nucleotides at positions 2, 7, 12, 14, and 16 of the antisense strand represented by formula (I-1) counting from the first paired nucleotide at 5' end. In some specific embodiments, the dsRNA agent has 2'-fluoro modified nucleotides at positions 2, 7, 12, 14, and 18 of the antisense strand represented by formula (I-1) counting from the first paired nucleotide at 5' end. In some specific embodiments, the dsRNA agent has 2'-fluoro modified nucleotides at positions 2, 5, 12, 14, and 18 of the antisense strand represented by formula (I-1) counting from the first paired nucleotide at 5' end. In some specific embodiments, the dsRNA agent has 2'-fluoro modified nucleotides at positions 2, 4, 10, 14, and 16 of the antisense strand represented by formula (I-1) counting from the first paired nucleotide at 5' end. In some specific embodiments, the dsRNA agent has 2'-fluoro modified nucleotides at positions 2, 7, 10, 14, and 16 of the antisense strand represented by formula (I-1) counting from the first paired nucleotide at 5' end. It will be understood by one of skill in the art that in the specific embodiments, the antisense strand represented by formula (I-1) of the dsRNA agent has only five 2'-fluorine modified nucleotides.

In some embodiments, in the antisense strand represented by formula (I-1) of the dsRNA agent, only one of N_{M1}, N_{M2}, N_{M3}, N_{M5}, N_{M6}, and N_{M8} is a 2'-fluorine modified nucleotide, and N_{M4} and N_{M7} are not 2' -fluorine modified nucleotides.

In some non-limiting examples, in the antisense strand represented by formula (I-1) of the dsRNA agent, only one of N_{M1}, N_{M2}, N_{M3}, N_{M5}, N_{M6}, and N_{M8} are 2'-fluorine modified nucleotide, and N_{M4} and N_{M7} are not 2'-fluorine modified nucleotides. It can be understood by one of skill in the art, by way of example but not limitation, that in some non-limiting examples, N_{M2} is a 2'-fluorine modified nucleotide, and N_{M1}, N_{M3}, N_{M4}, N_{M5}, N_{M6}, N_{M7} and N_{M8} are not 2'-fluorine modified nucleotides, and it can be understood by one of skill in the art that in the antisense strand represented by formula (I-1) of the dsRNA agent, counting from the first paired nucleotide at 5' end, the nucleotides at positions 2, 14 and 16 are 2'-fluorine modified nucleotides, while the nucleotides at other positions are not 2'-fluorine modified nucleotides. In another non-limiting example, N_{M3} is a 2'-fluorine modified nucleotide, and N_{M1}, N_{M2}, N_{M4}, N_{M5}, N_{M6}, N_{M7} and N_{M8} are not 2'-fluorine modified nucleotides, and it can be understood by one of skill in the art that in the antisense strand represented by formula (I-1) of the dsRNA agent, counting from the first paired nucleotide at 5' end, the nucleotides at positions 2, 12 and 14 are 2'-fluorine modified nucleotides, while the nucleotides at other positions are not 2'-fluorine modified nucleotides. In another non-limiting example, N_{M5} is a 2'-fluorine modified nucleotide, and N_{M1}, N_{M2}, N_{M3}, N_{M4}, N_{M6}, N_{M7} and N_{M8} are not 2'-fluorine modified nucleotides, and it can be understood by one of skill in the art that in the antisense strand represented by formula (I-1) of the dsRNA agent, counting from the first paired nucleotide at 5' end, the nucleotides at positions 2, 8 and 14 are 2'-fluorine modified nucleotides, while the nucleotides at other positions are not 2'-fluorine modified nucleotides. In another non-limiting example, N_{M8} is a 2'-fluorine modified nucleotide, and N_{M1}, N_{M2}, N_{M3}, N_{M4}, N_{M5}, N_{M6} and N_{M7} are not 2' -fluorine modified nucleotides, and it can be understood by one of skill in the art that in the antisense strand represented by formula (I-1) of the dsRNA agent, counting from the first paired nucleotide at 5' end, the nucleotides at positions 2, 4 and 14 are 2'-fluorine modified nucleotides, while the nucleotides at other positions are not 2'-fluorine modified nucleotides. It will be understood by one of skill in the art that in the specific embodiments, the antisense strand represented by formula (I-1) of the dsRNA agent has only three 2'-fluorine modified nucleotides.

In some embodiments, the antisense strand of the dsRNA agent comprises a sequence represented by formula (IV) in the direction of 3' to 5':

3'-(N_{L})ₙ N_{M1} N_{L} N_{M2} N_{L} N_{F} N_{L} N_{M3} N_{L} N_{M4} N_{L} N_{M5} N_{M6} N_{L} N_{M7} N_{M8} N_{L} N_{F} N_{L}-5' formula (IV)

wherein, each N_{F} represents a 2'-fluoro modified nucleotide;
N_{M1}, N_{M2}, Nₘ₃, N_{M4}, N_{M5}, N_{M7}, and N_{M8} independently represent a modified or an unmodified nucleotide, wherein only one of N_{M1}, N_{M2}, N_{M3}, N_{M5}, N_{M6}, and N_{M8} is a 2'-fluoro modified nucleotide, and N_{M4} and N_{M7} are not 2'-fluoro modified nucleotides;
each N_{L} independently represents a modified or an unmodified nucleotide, wherein the modified nucleotide is not a 2'-fluoro modified nucleotide;
n can be an integer from 0 to 7.

In some specific embodiments, the dsRNA agent has 2'-fluoro modified nucleotides at positions 2, 4, and 14 of the antisense strand represented by formula (IV) counting from the first paired nucleotide at 5' end. In some specific embodiments, the dsRNA agent has 2'-fluoro modified nucleotides at positions 2, 8, and 14 of the antisense strand represented by formula (IV) counting from the first paired nucleotide at 5' end. In some specific embodiments, the dsRNA agent has 2'-fluoro modified nucleotides at positions 2, 12, and 14 of the antisense strand represented by formula (IV) counting from the first paired nucleotide at 5' end. In some specific embodiments, the dsRNA agent has 2'-fluoro modified nucleotides at positions 2, 14, and 16 of the antisense strand represented by formula (IV) counting from the first paired nucleotide at 5' end. In some specific embodiments, the dsRNA agent has 2'-fluoro modified nucleotides at positions 2, 14, and 18 of the antisense strand represented by formula (IV) counting from the first paired nucleotide at 5' end. In some specific embodiments, the dsRNA agent has 2'-fluoro modified nucleotides at positions 2, 4, and 14 of the antisense strand represented by formula (IV) counting from the first paired nucleotide at 5' end. It will be understood by one of skill in the art that in the specific embodiments, the antisense strand represented by formula (IV) of the dsRNA agent has only three 2'-fluorine modified nucleotides. In some more specific embodiments, the dsRNA agent has 2'-fluoro modified nucleotides at positions 2, 6, and 14 of the antisense strand counting from the first paired nucleotide at 5' end.

In some embodiments, an RNAi agent capable of inhibiting expression of a target gene is involved, wherein the RNAi agent is a double-stranded ribonucleic acid (dsRNA) agent comprising a sense strand and an antisense strand complementary to at least a portion of an mRNA corresponding to the target gene, and the antisense strand has 18-30 nucleotides and comprises a sequence represented by formula (I-2) in the direction of 3'-5':

3'-(N_{L})ₙ N_{M1} N_{L} N_{M2} N_{L} N_{F} N_{L} N_{M3} N_{M9} N_{M4} N_{M10} N_{M5} N_{M6} N_{L} N_{M7} N_{M8} N_{L} N_{F} N_{L}-5' formula (I-2)

wherein, each N_{F} represents a 2'-fluoro modified nucleotide;
N_{M1}, N_{M2}, N_{M3}, N_{M4}, N_{M5}, N_{M6}, N_{M7}, N_{M8}, N_{M9} and N_{M10} independently represent a modified or an unmodified nucleotide, wherein only three of N_{M1}, N_{M2}, N_{M3}, N_{M4}, N_{M5}, N_{M6}, N_{M7}, N_{M8}, N_{M9} and N_{M10} are 2'-fluoro modified nucleotides;
each N_{L} independently represents a modified or an unmodified nucleotide, wherein the modified nucleotide is not a 2'-fluoro modified nucleotide;
n can be an integer from 0 to 7.

In some specific embodiments, the dsRNA agent has 2'-fluorine modified nucleotides at positions 2, 7, 11, 14 and 16 of the antisense strand represented by formula (I-2) counting from the first paired nucleotide at 5' end. In some specific embodiments, the dsRNA agent has 2'-fluorine modified nucleotides at positions 2, 7, 11, 14 and 18 of the antisense strand represented by formula (I-2) counting from the first paired nucleotide at 5' end. In some specific embodiments, the dsRNA agent has 2'-fluorine modified nucleotides at positions 2, 7, 11, 14 and 18 of the antisense strand represented by formula (I-2) counting from the first paired nucleotide at 5' end. In some specific embodiments, the dsRNA agent has 2'-fluorine modified nucleotides at positions 2, 7, 12, 14 and 16 of the antisense strand represented by formula (I-2) counting from the first paired nucleotide at 5' end. In some specific embodiments, the dsRNA agent has 2'-fluorine modified nucleotides at positions 2, 8, 12, 14 and 16 of the antisense strand represented by formula (I-2) counting from the first paired nucleotide at 5' end. In some specific embodiments, the dsRNA agent has 2'-fluorine modified nucleotides at positions 2, 9, 12, 14 and 16 of the antisense strand represented by formula (I-2) counting from the first paired nucleotide at 5' end.

In some embodiments, an RNAi agent capable of inhibiting expression of a target gene is involved, wherein the RNAi agent is a double-stranded ribonucleic acid (dsRNA) agent comprising a sense strand and an antisense strand complementary to at least a portion of an mRNA corresponding to the target gene, and the antisense strand has 18-30 nucleotides and comprises a sequence represented by formula (I') in the direction of 3' to 5':

3'-(N_{L})ₙ N_{M1} N_{L} N_{F} N_{L} N_{F} N_{L} N_{M3} N_{L} N_{M4} N_{L} N_{M5} N_{F} N_{L} N_{M7} N_{M8} N_{L} N_{F} N_{L}-5' formula (I')

wherein, each N_{F} represents a 2'-fluoro modified nucleotide;
N_{M1}, Nₘ₃, N_{M4}, N_{M5}, N_{M7}, and N_{M8} independently represent a modified or an unmodified nucleotide, wherein no more than one of N_{M1}, N_{M3}, N_{M4}, N_{M5}, N_{M7}, and N_{M8} is a 2'-fluoro modified nucleotide;
each N_{L} independently represents a modified or an unmodified nucleotide, wherein the modified nucleotide is not a 2'-fluoro modified nucleotide;
n can be an integer from 0 to 7.

In some more specific embodiments, in the antisense strand represented by formula (I') of the dsRNA agent, no more than one of N_{M1}, N_{M3}, N_{M4}, N_{M5}, N_{M7} and N_{M8} is a 2'-fluoro modified nucleotide, and it will be understood by those of skill in the art that only one or none of N_{M1}, N_{M3}, N_{M4}, N_{M5}, N_{M7} and N_{M8} is a 2'-fluoro modified nucleotide, meaning that the antisense strand represented by formula (I') has only four or five 2'-fluorine modified nucleotides and the nucleotides at positions 2, 7, 14, and 16 are 2'-fluoro modified nucleotides. In some specific embodiments, the dsRNA agent has 2'-fluorine modified nucleotides at positions 2, 5, 7, 14 and 16 of the antisense strand represented by formula (I-2) counting from the first paired nucleotide at 5' end. In some specific embodiments, the dsRNA agent has 2'-fluorine modified nucleotides at positions 2, 4, 7, 14 and 16 of the antisense strand represented by formula (I-2) counting from the first paired nucleotide at 5' end. In some specific embodiments, the dsRNA agent has 2'-fluorine modified nucleotides at positions 2, 7, 8, 14 and 16 of the antisense strand represented by formula (I-2) counting from the first paired nucleotide at 5' end. In some specific embodiments, the dsRNA agent has 2'-fluorine modified nucleotides at positions 2, 7, 10, 14 and 16 of the antisense strand represented by formula (I-2) counting from the first paired nucleotide at 5' end. In some specific embodiments, the dsRNA agent has 2'-fluorine modified nucleotides at positions 2, 7, 12, 14 and 16 of the antisense strand represented by formula (I-2) counting from the first paired nucleotide at 5' end. In some specific embodiments, the dsRNA agent has 2'-fluorine modified nucleotides at positions 2, 7, 14, 16 and 18 of the antisense strand represented by formula (I-2) counting from the first paired nucleotide at 5' end. In some specific embodiments, the dsRNA agent has 2'-fluorine modified nucleotides at positions 2, 7, 14 and 16 of the antisense strand represented by formula (I-2) counting from the first paired nucleotide at 5' end. In some embodiments, in the antisense strand represented by formula (X) and/or formula (I) of the dsRNA agent, one or more of N_{X} are modified nucleotides independently selected from the group consisting of 2'-fluoro modified nucleotide, 2'-O-methyl modified nucleotide, 2'-deoxy nucleotide, 2',3'-seco nucleotide mimic, locked nucleotide (LNA), unlocked nucleic acid nucleotide (UNA), glycol nucleic acid nucleotide (GNA), bicyclo nucleic acid (BNA), 2'-F-arabino nucleotide, 2'-methoyxyethyl nucleotide (MOE), abasic nucleotide, ribitol, inverted nucleotide, inverted abasic nucleotide (Invab), inverted 2'-OMe nucleotide, inverted 2'-deoxy nucleotide, 2'-amino modified nucleotide, 2'-alkyl modified nucleotide, morpholino nucleotide, 3'-OMe nucleotide, phosphorothioate modified nucleotide, terminal nucleotide linked to a cholesteryl derivative or dodecanoic acid bisdecylamide group, 2'-amino modified nucleotide, phosphoramidate, and non-natural base comprising nucleotide.

In some embodiments, in the antisense strand represented by formula (I-1), formula (I-2), formula (I'), formula (II), formula (III) and/or formula (IV) of the dsRNA agent, one or more of N_{L} are modified nucleotides independently selected from the group consisting of 2'-O-methyl modified nucleotide, 2'-deoxy nucleotide, 2',3'-seco nucleotide mimic, locked nucleotide (LNA), unlocked nucleic acid nucleotide (UNA), glycol nucleic acid nucleotide (GNA), bicyclo nucleic acid (BNA), 2'-F-arabino nucleotide, 2'-methoyxyethyl nucleotide (MOE), abasic nucleotide, ribitol, inverted nucleotide, inverted abasic nucleotide (Invab), inverted 2'-OMe nucleotide, inverted 2'-deoxy nucleotide, 2'-amino modified nucleotide, 2'-alkyl modified nucleotide, morpholino nucleotide, 3'-OMe nucleotide, phosphorothioate modified nucleotide, terminal nucleotide linked to a cholesteryl derivative or dodecanoic acid bisdecylamide group, 2'-amino modified nucleotide, phosphoramidate, and non-natural base comprising nucleotide.

In some embodiments, in the antisense strand represented by formula (I-1), formula (I-2), formula (I'), formula (II), formula (III) and/or formula (IV) of the dsRNA agent, one or more of N_{L} are modified nucleotides independently selected from the group consisting of 2'-O-methyl modified nucleotide, GNA, LNA, UNA, and Invab. In some preferred embodiments, in the antisense strand represented by formula (I) of the dsRNA agent, one or more of N_{L} are modified nucleotides independently selected from the group consisting of 2'-O-methyl modified nucleotide.

In some embodiments, in the antisense strand represented by formula (I-1), formula (I-2), formula (I'), formula (II), formula (III) and/or a formula (IV) of the dsRNA agent, N_{M1}, N_{M2}, N_{M3}, N_{M4}, N_{M5}, N_{M6}, N_{M7}, N_{M8}, N_{M9}, and N_{M10} are modified nucleotides independently selected from the group consisting of 2'-fluoro modified nucleotide, 2'-O-methyl modified nucleotide, 2'-deoxy nucleotide, 2',3'-seco nucleotide mimic, locked nucleotide (LNA), unlocked nucleic acid nucleotide (UNA), glycol nucleic acid nucleotide (GNA), bicyclo nucleic acid (BNA), 2'-F-arabino nucleotide, 2'-methoyxyethyl nucleotide, abasic nucleotide, ribitol, inverted nucleotide, inverted abasic nucleotide (Invab), inverted 2'-OMe nucleotide, inverted 2'-deoxy nucleotide, 2'-amino modified nucleotide, 2'-alkyl modified nucleotide, morpholino nucleotide, 3'-OMe nucleotide, phosphorothioate modified nucleotide, terminal nucleotide linked to a cholesteryl derivative or dodecanoic acid bisdecylamide group, 2'-amino modified nucleotide, phosphoramidate, and non-natural base comprising nucleotide. In some embodiments, in the antisense strand represented by formula (I-1) of the dsRNA agent, N_{M1}, N_{M2}, N_{M4}, N_{M5}, N_{M6}, N_{M7}, and/or N_{M8} are preferably 2'-O methyl modified nucleotides in case of not being 2'-fluoro modified nucleotides.

In some non-limiting examples, in the antisense strand represented by formula (III) of the dsRNA agent, N_{M1} and N_{M7} are 2'-fluorine modified nucleotides, and N_{M2}, N_{M4}, N_{M5}, N_{M6} and/or N_{M8} represent an unmodified nucleotide, or a modified nucleotide independently selected from the group consisting of 2'-O-methyl modified nucleotide, 2'-deoxy nucleotide, 2',3'-seco nucleotide mimic, locked nucleotide (LNA), unlocked nucleic acid nucleotide (UNA), glycol nucleic acid nucleotide (GNA), bicyclo nucleic acid (BNA), 2'-F-arabino nucleotide, 2'-methoyxyethyl nucleotide, abasic nucleotide, ribitol, inverted nucleotide, inverted abasic nucleotide (Invab), inverted 2'-OMe nucleotide, inverted 2'-deoxy nucleotide, 2'-amino modified nucleotide, 2'-alkyl modified nucleotide, morpholino nucleotide, 3'-OMe nucleotide, phosphorothioate modified nucleotide, terminal nucleotide linked to a cholesteryl derivative or dodecanoic acid bisdecylamide group, 2'-amino modified nucleotide, phosphoramidate, and non-natural base comprising nucleotide. In some preferred embodiments, in the dsRNA agent, N_{M2}, N_{M4}, N_{M5}, N_{M6} and/or N_{M8} represent an unmodified nucleotide, or a modified nucleotide independently selected from the group consisting of 2'-O-methyl modified nucleotide, UNA, GNA, and LNA. In some preferred embodiments, in the dsRNA agent, N_{M2}, N_{M4}, N_{M5}, N_{M6} and/or N_{M8} represent a modified nucleotide or an unmodified nucleotide, preferably 2'-O-methyl modified nucleotide.

In some non-limiting examples, in the antisense strand represented by formula (III) of the dsRNA agent, N_{M2} and N_{M6} are 2'-fluorine modified nucleotides, and N_{M1}, N_{M4}, N_{M5}, N_{M7} and/or N_{M8} represent an unmodified nucleotide, or a modified nucleotide independently selected from the group consisting of 2'-O-methyl modified nucleotide, 2'-deoxy nucleotide, 2',3'-seco nucleotide mimic, locked nucleotide (LNA), unlocked nucleic acid nucleotide (UNA), glycol nucleic acid nucleotide (GNA), bicyclo nucleic acid (BNA), 2'-F-arabino nucleotide, 2'-methoyxyethyl nucleotide, abasic nucleotide, ribitol, inverted nucleotide, inverted abasic nucleotide (Invab), inverted 2'-OMe nucleotide, inverted 2'-deoxy nucleotide, 2'-amino modified nucleotide, 2'-alkyl modified nucleotide, morpholino nucleotide, 3'-OMe nucleotide, phosphorothioate modified nucleotide, terminal nucleotide linked to a cholesteryl derivative or dodecanoic acid bisdecylamide group, 2'-amino modified nucleotide, phosphoramidate, and non-natural base comprising nucleotide. In some preferred embodiments, in the dsRNA agent, N_{M1}, N_{M4}, N_{M5}, N_{M7} and/or N_{M8} represent an unmodified nucleotide, or a modified nucleotide independently selected from the group consisting of 2'-O-methyl modified nucleotide, UNA, GNA, and LNA. In some preferred embodiments, in the dsRNA agent, N_{M1}, N_{M4}, N_{M5}, N_{M7} and/or N_{M8} represent a modified nucleotide or an unmodified nucleotide, preferably 2'-O-methyl modified nucleotide.

It can be understood that in the antisense strand represented by formula (II) or formula (III) of the dsRNA agent of the present invention, N_{M1}, N_{M2}, N_{M3}, N_{M4}, N_{M5}, N_{M6}, N_{M7} and/or N_{M8} all have such modification characteristics.

In some non-limiting examples, in the antisense strand represented by formula (IV) of the dsRNA agent of the present invention, N_{M1}, N_{M2}, N_{M3}, N_{M4}, N_{M5}, N_{M6}, N_{M7} and/or N_{M8} independently represent a modified nucleotide or an unmodified nucleotide, wherein N_{M1} is a 2'-fluorine modified nucleotide, and N_{M2}, Nₘ₃, N_{M4}, N_{M5}, N_{M6}, N_{M7} and/or N_{M8} are not 2'-fluorine modified nucleotides and are independently selected from the group consisting of 2'-O-methyl modified nucleotide, 2'-deoxy nucleotide, 2',3'-seco nucleotide mimic, locked nucleotide (LNA), unlocked nucleic acid nucleotide (UNA), glycol nucleic acid nucleotide (GNA), bicyclo nucleic acid (BNA), 2'-F-arabino nucleotide, 2'-methoyxyethyl nucleotide, abasic nucleotide, ribitol, inverted nucleotide, inverted abasic nucleotide (Invab), inverted 2'-OMe nucleotide, inverted 2'-deoxy nucleotide, 2'-amino modified nucleotide, 2'-alkyl modified nucleotide, morpholino nucleotide, 3'-OMe nucleotide, phosphorothioate modified nucleotide, terminal nucleotide linked to a cholesteryl derivative or dodecanoic acid bisdecylamide group, 2'-amino modified nucleotide, phosphoramidate, and non-natural base comprising nucleotide. In some preferred embodiments, in the antisense strand represented by formula (IV) of the dsRNA agent, one or more of N_{M2}, N_{M3}, N_{M4}, N_{M5}, N_{M6}, N_{M7} and/or N_{M8} are modified nucleotides independently selected from the group consisting of 2'-O-methyl modified nucleotide, UNA, GNA, and LNA. In some preferred embodiments, in the antisense strand represented by formula (IV) of the dsRNA agent, N_{M2}, N_{M3}, N_{M4}, N_{M5}, N_{M6}, N_{M7} and/or N_{M8} are 2'-O-methyl modified nucleotides.

In some non-limiting examples, in the antisense strand represented by formula (IV) of the dsRNA agent of the present invention, N_{M1}, N_{M2}, N_{M3}, N_{M4}, N_{M5}, N_{M6}, N_{M7} and/or N_{M8} independently represent a modified nucleotide or an unmodified nucleotide, wherein N_{M2} is a 2'-fluorine modified nucleotide, and N_{M1}, Nₘ₃, N_{M4}, N_{M5}, N_{M6}, N_{M7} and/or N_{M8} are not 2'-fluorine modified nucleotides and are independently selected from the group consisting of 2'-O-methyl modified nucleotide, 2'-deoxy nucleotide, 2',3'-seco nucleotide mimic, locked nucleotide (LNA), unlocked nucleic acid nucleotide (UNA), glycol nucleic acid nucleotide (GNA), bicyclo nucleic acid (BNA), 2'-F-arabino nucleotide, 2'-methoyxyethyl nucleotide, abasic nucleotide, ribitol, inverted nucleotide, inverted abasic nucleotide (Invab), inverted 2'-OMe nucleotide, inverted 2'-deoxy nucleotide, 2'-amino modified nucleotide, 2'-alkyl modified nucleotide, morpholino nucleotide, 3'-OMe nucleotide, phosphorothioate modified nucleotide, terminal nucleotide linked to a cholesteryl derivative or dodecanoic acid bisdecylamide group, 2'-amino modified nucleotide, phosphoramidate, and non-natural base comprising nucleotide. In some preferred embodiments, in the antisense strand represented by formula (IV) of the dsRNA agent, one or more of N_{M1}, N_{M3}, N_{M4}, N_{M5}, N_{M6}, N_{M7} and/or N_{M8} are modified nucleotides independently selected from the group consisting of 2'-O-methyl modified nucleotide, UNA, GNA, and LNA. In some preferred embodiments, in the antisense strand represented by formula (IV) of the dsRNA agent, N_{M1}, N_{M3}, N_{M4}, N_{M5}, N_{M6}, N_{M7} and/or N_{M8} are 2'-O-methyl modified nucleotides.

In some non-limiting examples, in the antisense strand represented by formula (IV) of the dsRNA agent of the present invention, N_{M1}, N_{M2}, N_{M3}, N_{M4}, N_{M5}, N_{M6}, N_{M7} and/or N_{M8} independently represent a modified nucleotide or an unmodified nucleotide, wherein N_{M3} is a 2'-fluorine modified nucleotide, and N_{M1}, N_{M2}, N_{M4}, N_{M5}, N_{M6}, N_{M7} and/or N_{M8} are not 2'-fluorine modified nucleotides and are independently selected from the group consisting of 2'-O-methyl modified nucleotide, 2'-deoxy nucleotide, 2',3'-seco nucleotide mimic, locked nucleotide (LNA), unlocked nucleic acid nucleotide (UNA), glycol nucleic acid nucleotide (GNA), bicyclo nucleic acid (BNA), 2'-F-arabino nucleotide, 2'-methoyxyethyl nucleotide, abasic nucleotide, ribitol, inverted nucleotide, inverted abasic nucleotide (Invab), inverted 2'-OMe nucleotide, inverted 2'-deoxy nucleotide, 2'-amino modified nucleotide, 2'-alkyl modified nucleotide, morpholino nucleotide, 3'-OMe nucleotide, phosphorothioate modified nucleotide, terminal nucleotide linked to a cholesteryl derivative or dodecanoic acid bisdecylamide group, 2'-amino modified nucleotide, phosphoramidate, and non-natural base comprising nucleotide. In some preferred embodiments, in the antisense strand represented by formula (IV) of the dsRNA agent, one or more of N_{M1}, N_{M2}, N_{M4}, N_{M5}, N_{M6}, N_{M7} and/or N_{M8} are modified nucleotides independently selected from the group consisting of 2'-O-methyl modified nucleotide, UNA, GNA, and LNA. In some preferred embodiments, in the antisense strand represented by formula (IV) of the dsRNA agent, N_{M1}, N_{M2}, N_{M4}, N_{M5}, N_{M6}, N_{M7} and/or N_{M8} are 2'-O-methyl modified nucleotides.

In some non-limiting examples, in the antisense strand represented by formula (IV) of the dsRNA agent of the present invention, N_{M1}, N_{M2}, N_{M3}, N_{M4}, N_{M5}, N_{M6}, N_{M7} and/or N_{M8} independently represent a modified nucleotide or an unmodified nucleotide, wherein N_{M6} is a 2'-fluorine modified nucleotide, and N_{M1}, N_{M2}, Nₘ₃, N_{M4}, N_{M5}, N_{M7} and/or N_{M8} are not 2'-fluorine modified nucleotides and are independently selected from the group consisting of 2'-O-methyl modified nucleotide, 2'-deoxy nucleotide, 2',3'-seco nucleotide mimic, locked nucleotide (LNA), unlocked nucleic acid nucleotide (UNA), glycol nucleic acid nucleotide (GNA), bicyclo nucleic acid (BNA), 2'-F-arabino nucleotide, 2'-methoyxyethyl nucleotide, abasic nucleotide, ribitol, inverted nucleotide, inverted abasic nucleotide (Invab), inverted 2'-OMe nucleotide, inverted 2'-deoxy nucleotide, 2'-amino modified nucleotide, 2'-alkyl modified nucleotide, morpholino nucleotide, 3'-OMe nucleotide, phosphorothioate modified nucleotide, terminal nucleotide linked to a cholesteryl derivative or dodecanoic acid bisdecylamide group, 2'-amino modified nucleotide, phosphoramidate, and non-natural base comprising nucleotide. In some preferred embodiments, in the antisense strand represented by formula (IV) of the dsRNA agent, one or more of N_{M1}, N_{M2}, N_{M4}, N_{M5}, N_{M6}, N_{M7} and/or N_{M8} are modified nucleotides independently selected from the group consisting of 2'-O-methyl modified nucleotide, UNA, GNA, and LNA. In some preferred embodiments, in the antisense strand represented by formula (IV) of the dsRNA agent, N_{M1}, N_{M2}, N_{M4}, N_{M5}, N_{M6}, N_{M7} and/or N_{M8} are 2'-O-methyl modified nucleotides.

It can be understood that in the antisense strand represented by formula (IV) of the dsRNA agent of the present invention, N_{M1}, N_{M2}, Nₘ₃, N_{M4}, N_{M5}, N_{M6}, N_{M7} and/or N_{M8} all have such modification characteristics.

In some non-limiting examples, in the antisense strand represented by formula (I-2) of the dsRNA agent of the present invention, N_{M1}, N_{M2}, N_{M3}, N_{M4}, N_{M5}, N_{M6}, N_{M7}, N_{M8}, N_{M9} and/or N_{M10} independently represent a modified nucleotide or an unmodified nucleotide, wherein N_{M2}, N_{M6} and N_{M9} are 2'-fluorine modified nucleotides, and N_{M1}, N_{M3}, N_{M4}, N_{M5}, N_{M7}, N_{M8}, and/or N_{M10} are not 2'-fluorine modified nucleotides and are independently selected from the group consisting of 2'-O-methyl modified nucleotide, 2'-deoxy nucleotide, 2',3'-seco nucleotide mimic, locked nucleotide (LNA), unlocked nucleic acid nucleotide (UNA), glycol nucleic acid nucleotide (GNA), bicyclo nucleic acid (BNA), 2'-F-arabino nucleotide, 2'-methoyxyethyl nucleotide, abasic nucleotide, ribitol, inverted nucleotide, inverted abasic nucleotide (Invab), inverted 2'-OMe nucleotide, inverted 2'-deoxy nucleotide, 2'-amino modified nucleotide, 2'-alkyl modified nucleotide, morpholino nucleotide, 3'-OMe nucleotide, phosphorothioate modified nucleotide, terminal nucleotide linked to a cholesteryl derivative or dodecanoic acid bisdecylamide group, 2'-amino modified nucleotide, phosphoramidate, and non-natural base comprising nucleotide. In some preferred embodiments, in the antisense strand represented by formula (I-2) of the dsRNA agent, one or more of N_{M1}, N_{M3}, N_{M4}, N_{M5}, N_{M7}, N_{M8}, and/or N_{M10} are modified nucleotides independently selected from the group consisting of 2'-O-methyl modified nucleotide, UNA, GNA, and LNA. In some preferred embodiments, in the antisense strand represented by formula (I-2) of the dsRNA agent, N_{M1}, N_{M3}, N_{M4}, N_{M5}, N_{M7}, N_{M8}, and/or N_{M10} are 2'-O-methyl modified nucleotides. It can be understood that in the antisense strand represented by formula (I-2) of the dsRNA agent of the present invention, all have such modification characteristics.

In some non-limiting examples, in the antisense strand represented by formula (I') of the dsRNA agent of the present invention, N_{M1}, N_{M2}, Nₘ₃, N_{M4}, N_{M5}, N_{M6}, N_{M7}, and/or N_{M8} independently represent a modified nucleotide or an unmodified nucleotide, wherein the modified nucleotide is independently selected from the group consisting of 2'-O-methyl modified nucleotide, 2'-deoxy nucleotide, 2',3'-seco nucleotide mimic, locked nucleotide (LNA), unlocked nucleic acid nucleotide (UNA), glycol nucleic acid nucleotide (GNA), bicyclo nucleic acid (BNA), 2'-F-arabino nucleotide, 2'-methoyxyethyl nucleotide, abasic nucleotide, ribitol, inverted nucleotide, inverted abasic nucleotide (Invab), inverted 2'-OMe nucleotide, inverted 2'-deoxy nucleotide, 2'-amino modified nucleotide, 2'-alkyl modified nucleotide, morpholino nucleotide, 3'-OMe nucleotide, phosphorothioate modified nucleotide, terminal nucleotide linked to a cholesteryl derivative or dodecanoic acid bisdecylamide group, 2'-amino modified nucleotide, phosphoramidate, and non-natural base comprising nucleotide. In some preferred embodiments, in the antisense strand represented by formula (I') of the dsRNA agent, one or more of N_{M1}, N_{M3}, N_{M4}, N_{M5}, N_{M7}, and/or N_{M8} are modified nucleotides independently selected from the group consisting of 2'-O-methyl modified nucleotide, UNA, GNA, and LNA. In some preferred embodiments, in the antisense strand represented by formula (I-2) of the dsRNA agent, N_{M1}, N_{M3}, N_{M4}, N_{M5}, N_{M7}, and/or N_{M8} are 2'-O-methyl modified nucleotides. In some non-limiting examples, in the antisense strand represented by formula (I') of the dsRNA agent of the present invention, N_{M1}, N_{M2}, Nₘ₃, N_{M4}, N_{M5}, N_{M6}, N_{M7}, and/or N_{M8} independently represent a modified nucleotide or an unmodified nucleotide, wherein the modified nucleotide is independently selected from the group consisting of 2'-O-methyl modified nucleotide, 2'-deoxy nucleotide, 2',3'-seco nucleotide mimic, locked nucleotide (LNA), unlocked nucleic acid nucleotide (UNA), glycol nucleic acid nucleotide (GNA), bicyclo nucleic acid (BNA), 2'-F-arabino nucleotide, 2'-methoyxyethyl nucleotide, abasic nucleotide, ribitol, inverted nucleotide, inverted abasic nucleotide (Invab), inverted 2'-OMe nucleotide, inverted 2'-deoxy nucleotide, 2'-amino modified nucleotide, 2'-alkyl modified nucleotide, morpholino nucleotide, 3'-OMe nucleotide, phosphorothioate modified nucleotide, terminal nucleotide linked to a cholesteryl derivative or dodecanoic acid bisdecylamide group, 2'-amino modified nucleotide, phosphoramidate, and non-natural base comprising nucleotide. In some preferred embodiments, in the antisense strand represented by formula (I') of the dsRNA agent, N_{M7} is a 2'-fluoro modified nucleotide, N_{M1}, N_{M2}, Nₘ₃, N_{M4}, N_{M5}, N_{M6} and/or N_{M8} are not 2' -fluoro modified nucleotides, and one or more of N_{M1}, N_{M2}, N_{M3}, N_{M4}, N_{M5}, N_{M6} and/or N_{M8} are modified nucleotides independently selected from the group consisting of 2'-O-methyl modified nucleotide, UNA, GNA, and LNA. In some preferred embodiments, in the antisense strand represented by formula (I') of the dsRNA agent, N_{M1}, N_{M2}, Nₘ₃, N_{M4}, N_{M5}, N_{M6} and/or N_{M8} are 2'-O-methyl modified nucleotides.

In some embodiments, in the sense strand complementary or substantially complementary to the antisense strand represented by formula (I) of the dsRNA agent, all or substantially all of nucleotides are modified nucleotides. In some embodiments, in the sense strand of the dsRNA agent, all nucleotides are modified nucleotides selected from the group consisting of 2'-fluoro modified nucleotide, 2'-O-methyl modified nucleotide, 2'-deoxy nucleotide, 2',3'-seco nucleotide mimic, locked nucleotide (LNA), unlocked nucleic acid nucleotide (UNA), glycol nucleic acid nucleotide (GNA), bicyclo nucleic acid (BNA), 2'-F-arabino nucleotide, 2'-methoyxyethyl nucleotide, abasic nucleotide, ribitol, inverted nucleotide, inverted abasic nucleotide (Invab), inverted 2'-OMe nucleotide, inverted 2'-deoxy nucleotide, 2'-amino modified nucleotide, 2'-alkyl modified nucleotide, morpholino nucleotide, 3'-OMe nucleotide, phosphorothioate modified nucleotide, terminal nucleotide linked to a cholesteryl derivative or dodecanoic acid bisdecylamide group, 2'-amino modified nucleotide, phosphoramidate, and non-natural base comprising nucleotide.

In some embodiments, in the sense strand complementary or substantially complementary to the antisense strand represented by formula (I') of the dsRNA agent, all or substantially all of nucleotides are modified nucleotides. In some embodiments, in the sense strand of the dsRNA agent, all nucleotides are modified nucleotides selected from the group consisting of 2'-fluoro modified nucleotide, 2'-O-methyl modified nucleotide, 2'-deoxy nucleotide, 2',3'-seco nucleotide mimic, locked nucleotide (LNA), unlocked nucleic acid nucleotide (UNA), glycol nucleic acid nucleotide (GNA), bicyclo nucleic acid (BNA), 2'-F-arabino nucleotide, 2'-methoyxyethyl nucleotide, abasic nucleotide, ribitol, inverted nucleotide, inverted abasic nucleotide (Invab), inverted 2'-OMe nucleotide, inverted 2'-deoxy nucleotide, 2'-amino modified nucleotide, 2'-alkyl modified nucleotide, morpholino nucleotide, 3'-OMe nucleotide, phosphorothioate modified nucleotide, terminal nucleotide linked to a cholesteryl derivative or dodecanoic acid bisdecylamide group, 2'-amino modified nucleotide, phosphoramidate, and non-natural base comprising nucleotide.

In some embodiments, in the sense strand and the antisense strand of the dsRNA agent, all or substantially all of nucleotides are modified nucleotides.

In some embodiments, the dsRNA agent has a sense strand complementary or substantially complementary to the antisense strand represented by formula (I), and the complementary or substantially complementary region is 18-25 nucleotides in length. In some embodiments, the complementary or substantially complementary region of the dsRNA agent is 18-23 nucleotides in length. In some embodiments, the complementary or substantially complementary region of the dsRNA agent is 19-21 nucleotides in length. In some other embodiments, the complementary or substantially complementary region can be 18-25 nucleotides in length, 19-25 nucleotides in length, 19-23 nucleotides in length, or 19-21 nucleotides in length. In another example, in some embodiments, the complementary or substantially complementary region is 18, 19, 20, or 21 nucleotides in length.

In some embodiments, the dsRNA agent has a sense strand complementary or substantially complementary to the antisense strand represented by formula (X), and the complementary or substantially complementary region is 18-25 nucleotides in length. In some embodiments, the complementary or substantially complementary region of the dsRNA agent is 18-23 nucleotides in length. In some embodiments, the complementary or substantially complementary region of the dsRNA agent is 19-21 nucleotides in length. In some other embodiments, the complementary or substantially complementary region can be 18-25 nucleotides in length, 19-25 nucleotides in length, 19-23 nucleotides in length, or 19-21 nucleotides in length. In another example, in some embodiments, the complementary or substantially complementary region is 18, 19, 20, or 21 nucleotides in length.

In some embodiment, the dsRNA agent has a sense strand fully complementary to the antisense strand represented by formula (X).

In some embodiment, the dsRNA agent has a sense strand fully complementary to the antisense strand represented by formula (I).

In some embodiments, the antisense strand represented by formula (X) of the dsRNA agent is fully or substantially complementary to the target gene.

In some embodiments, the antisense strand represented by formula (I) of the dsRNA agent is fully or substantially complementary to the target gene.

In some embodiments, the dsRNA agent of the present invention contains no mismatches. In certain embodiments, the target gene dsRNA agent of the present invention contains no more than 1 mismatch (such mismatch does not affect the counting start position of the pairing). In some embodiments, the dsRNA agent of the present invention contains no more than 2 mismatches. In certain embodiments, the dsRNA agent of the present invention contains no more than 3 mismatches. In some embodiments of the invention, the antisense strand of the dsRNA agent contains a mismatch to a target sequence that is not located in the center of the complementary region. In some embodiments, the antisense strand of the dsRNA agent contains 1, 2, 3, 4 or 5 more mismatches located within the last 5, 4, 3, 2 or 1 nucleotide of either or both the 5' end or 3' end of the complementary region.

In some embodiments, each strand of the dsRNA agent of the present invention is no more than 30 nucleotides in length.

In some embodiments, each strand of the dsRNA agent of the present invention is no more than 25 nucleotides in length.

In some embodiments, each strand of the dsRNA agent of the present invention is no more than 23 nucleotides in length.

In some embodiments, each strand of the dsRNA agent of the present invention is 19, 20 or 21 nucleotides in length.

In one embodiment, the dsRNA agent of the present invention has two blunt ends at both ends of the sense strand and the antisense strand. For example, each strand of the dsRNA agent is fully paired to form a blunt end structure.

In one embodiment, the antisense strand represented by formula (I) of the dsRNA agent has 1-5 unpaired nucleotide overhangs at 3' end, such as 1, 2, 3, 4 or 5 unpaired nucleotide overhangs. The overhang can be the result of one strand being longer than the other, or the result of two strands of the same length being staggered. The overhang may form one mismatch with the target mRNA, or it may be complementary to the targeted gene sequence or may be other sequences. The first and second strand can also be connected, for example, by other base to form a hairpin or by other non-base linkers.

In certain embodiments, the sense strand complementary or partially complementary to the antisense strand represented by formula (I) of the dsRNA agent has a blunt end at 3' end and/or 5' end.

In some embodiments, the sense strand complementary or partially complementary to the antisense strand represented by formula (I) of the dsRNA agent has 1-5 unpaired nucleotide overhangs at 3' end and/or 5' end, such as 1, 2, 3, 4 or 5 unpaired nucleotide overhangs. In certain embodiments, the sense strand complementary or partially complementary to the antisense strand represented by formula (I) of the dsRNA agent has a blunt end at 3' end and/or 5' end.

In some embodiments, the overhang of unpaired nucleotides in the sense strand and/or antisense strand of the dsRNA agent of the present invention is 2-5 nucleotides in length, 1-5 nucleotides in length, 2-5 nucleotides in length, 1-4 nucleotides in length, 2-4 nucleotides in length, 1-3 nucleotides in length, 2-3 nucleotides in length or 1-2 nucleotides in length.

In one embodiment, one or more nucleotides in the overhang of the dsRNA agent of the present invention can be independently a modified nucleotide or an unmodified nucleotide, and the modified nucleotide includes but is not limited to: 2'-fluoro modified nucleotide, 2'-O-methyl modified nucleotide, 2'-deoxy nucleotide, 2',3'-seco nucleotide mimic, locked nucleotide (LNA), unlocked nucleic acid nucleotide (UNA), glycol nucleic acid nucleotide (GNA), bicyclo nucleic acid (BNA), 2'-F-arabino nucleotide, 2'-methoyxyethyl nucleotide, abasic nucleotide, ribitol, inverted nucleotide, inverted abasic nucleotide (Invab), inverted 2'-OMe nucleotide, inverted 2'-deoxy nucleotide, 2'-amino modified nucleotide, 2'-alkyl modified nucleotide, morpholino nucleotide, 3'-OMe nucleotide, phosphorothioate modified nucleotide, terminal nucleotide linked to a cholesteryl derivative or dodecanoic acid bisdecylamide group, 2'-amino modified nucleotide, phosphoramidate, and non-natural base comprising nucleotide.

In one embodiment, both ends of the overhang of the sense strand, antisense strand or both strands of the overhang of the dsRNA agent of the present invention can be phosphorylated.

In one embodiment, the overhang of the dsRNA agent of the present invention exists at 3' end or 5' end of the sense strand, antisense strand or both strands. In one embodiment, a 3' overhang exists in the antisense strand. In one embodiment, a 5' overhang exists in the sense strand.

In one embodiment, the dsRNA agent of the present invention may comprise only one single overhang, which can enhance the interference activity of the dsRNA without affecting its overall stability. For example, the single-stranded overhang is located at the 5' end of the sense strand, or alternatively, at the 3' end of antisense strand, and correspondingly, the dsRNA agent may also have a blunt end located at the 5' end of the antisense strand (or the 3' end of the sense strand), or vice versa.

In one embodiment, the 5' end of the antisense strand or the 3' end of the sense strand is blunt end in the dsRNA agent of the present invention.

In some embodiments, the dsRNA agent of the present invention comprises at least one phosphorothioate internucleoside linkage.

In some embodiments, the sense strand of the dsRNA agent of the present invention comprises at least one phosphorothioate internucleoside linkage.

In some embodiments, the antisense strand of the dsRNA agent of the present invention comprises at least one phosphorothioate internucleoside linkage.

In some embodiments, the sense strand and/or the antisense strand of the dsRNA agent of the present invention comprises 1, 2, 3, 4, 5, 6, 7, 8, 9, 10, 11, 12, 13, 14, 15, 16, 17 or 18 phosphorothioate internucleoside linkages.

In one embodiment, the sense strand of the dsRNA agent of the present invention comprises 1-10 blocks with 2-10 phosphorothioate internucleoside linkages separated by 1, 2, 3, 4, 5, 6, 7, 8, 9, 10, 11, 12, 13, 14, 15, 16, 17 or 18 phosphate internucleoside linkages, wherein one of the phosphorothioate internucleoside linkages is located at any position in the oligonucleotide sequence, and the sense strand is paired with the antisense strand comprising any combination of phosphorothioate internucleoside linkages, methylphosphonate internucleoside linkages, and phosphate internucleoside linkages or the antisense strand comprising phosphorothioate linkage or phosphate linkage.

In one embodiment, the antisense strand of the dsRNA agent of the present invention comprises 1-10 blocks with 2-10 phosphorothioate internucleoside linkages separated by 1, 2, 3, 4, 5, 6, 7, 8, 9, 10, 11, 12, 13, 14, 15, 16, 17 or 18 phosphate internucleoside linkages, wherein one of the phosphorothioate internucleoside linkages is located at any position in the oligonucleotide sequence, and the antisense strand is paired with the sense strand comprising any combination of phosphorothioate internucleoside linkage, methylphosphonate internucleoside linkage and phosphate internucleoside linkages or the sense strand comorising phosphorothioate linkage or phosphate linkage.

In one embodiment, the dsRNA agent of the present invention further comprises one or more phosphorothioate internucleoside linkage modifications in the internal region of 1-10 duplexes of the sense strand and/or the antisense strand. For example, at least 2, 3, 4, 5, 6, 7, 8, 9 or 10 nucleotides at positions 8-16 of the duplex region counting from the 3' end of the sense strand can be linked via the phosphorothioate internucleoside linkage. The dsRNA can optionally further comprise one or more phosphorothioate internucleotide linkage modifications in 1-10 end positions.

In one embodiment, the dsRNA agent of the present invention further comprises 1-5 phosphorothioate internucleotide linkage or methylphosphonate internucleotide linkage modifications at positions 1-5 of the sense strand and further comprises 1-5 phosphorothioate internucleotide linkage or methylphosphonate internucleotide linkage modifications at positions 18-23 of the sense strand (counting from the paired nucleotide at 3' end of the sense strand as used herein), and further comprises 1-2 phosphorothioate internucleotide linkages at positions 1-2 of the antisense strand and further comprises 1-5 phosphorothioate internucleotide linkages in positions 18-23 of the antisense strand (counting from the blunt end at 5' end of the antisense strand as used herein). In one embodiment, the dsRNA agent of the present invention comprises 1 or 2 phosphorothioate internucleotide linkage modifications at 3' end and/or 5' end of the sense strand or the antisense strand. It can be understood in the art that the positions at 3' end and/or 5' end means counting sequentially from the first nucleotide at the 3' end or 5' end of the sense strand or the antisense strand in any length. For a non-limiting example, that the antisense strand has two phosphorothioate internucleotide linkage modifications at the 5' end refers to that there are phosphorothioate internucleotide linkage modifications between the first paired nucleotide and the second nucleotide, between the second nucleotide and the third nucleotide from the 5' end of the antisense strand.

In one embodiment, the dsRNA agent of the present invention comprises the phosphorothioate internucleotide linkage modification in the overhang. For example, the overhang comprises two nucleotides with a phosphorothioate internucleotide linkage between the two nucleotides. Internucleotide linkage modification can also be made to link the overhang nucleotide with the terminal-paired nucleotide in the duplex region. It should be understood in the art that the linking of the overhang nucleotide and the terminal-paired nucleotide in the duplex region refers to the linking of the overhang nucleotide and terminal N_{L} or N_{L}' unless otherwise specified. For example, at least 2, 3, 4 or all nucleotides in the overhang may be linked by phosphorothioate internucleotide linkages, and optionally, there may be another phosphorothioate internucleotide linkage that links the overhang nucleotide with a paired nucleotide next to the overhang nucleotide. For example, there may be at least two phosphorothioate internucleotide linkages between the terminal three nucleotides, where two of the three nucleotides are overhang nucleotides and the third one is a paired nucleotide next to the overhang nucleotide. Preferably, these three terminal nucleotides may be at the 3' end of the antisense strand. In one embodiment, this is also the case where the dsRNA agent of the present invention comprises the phosphorothioate linkage modification in the overhang of the sense strand.

In some embodiments, the sense strand and/or the antisense strand of the dsRNA agent of the present invention has 1 or 2 inverted abasic residue nucleotides. In some embodiments, the sense strand and/or the antisense strand of the dsRNA agent of the present invention comprises 1 or 2 inverted abasic residues at 3' end or/and 5' end. In some embodiments, the sense strand of the dsRNA agent of the present invention comprises an inverted abasic residue at the 3' end.

In some embodiments, SEQ ID NO: 1 is FXII mRNA [NCBI reference sequence: NM_021489.3; Unmodified duplex AD00127.um has a sense strand of AACUCAAUAAAGUGCUUUGAA (SEQ ID NO: 2) and an antisense strand of UUCAAAGCACUUUAUUGAGUU (SEQ ID NO: 3), where the complementary Pos(20) is at position 1938 in NM_021489.3; Unmodified duplex AD00549.um has a sense strand of GCCCAAGAAAGUGAAAGACCA (SEQ ID NO: 4) and an antisense strand of UGGUCUUUCACUUUCLTUGGGC (SEQ ID NO: 5), where the complementary Pos(20) is at position 307 in NM_021489.3.

According to another aspect of the present invention, an RNAi agent for inhibiting expression of a target gene is provided, wherein the RNAi agent is double-stranded ribonucleic acid (dsRNA) agent comprising a sense strand and an antisense strand complementary to at least a portion of an mRNA corresponding to the target gene, and the sense strand has 18-40 nucleotides and comprises a sequence represented by formula (V) in the direction of 5'-3':

5'-(N'_{L})_{n'} N'_{L} N'_{L} N'_{L} N'_{N1} N'_{N2} N'_{N3} N'_{N4} N'_{F} N'_{L} N'_{N5}N'_{N6} N'_{L} N'_{L} N'_{L} N'_{L} N'_{L} N'_{L} N'_{L}-3' formula (V)

wherein, each N'_{F} represents a 2'-fluoro modified nucleotide;
N'_{N1}, N'_{N2}, N'_{N3}, N'_{N4}, N'_{N5}, N'_{N6} independently represent a modified or an unmodified nucleotide, wherein at least two of N'_{N1}, N'_{N2}, N'_{N3}, N'_{N4}, N'_{N5}, and N'_{N6} are 2'-fluoro modified nucleotides;
formula (V) has no motif of three or more contiguous 2'-fluoro modified nucleotides and has no more than six 2'-fluoro modified nucleotides;
each N'_{L} independently represents a modified or an unmodified nucleotide;
n' can be an integer from 0-7.

In some embodiments, an RNAi agent for inhibiting expression of a target gene is provided, wherein the RNAi agent is double-stranded ribonucleic acid (dsRNA) agent comprising a sense strand and an antisense strand complementary to at least a portion of an mRNA corresponding to the target gene, and the sense strand has 18-40 nucleotides and comprises a sequence represented by the formula (V') in the direction of 5'-3':

5'-(N'_{L})_{n'} N'_{L} N'_{L} N'_{L} N'_{N1} N'_{N2} N' _{N3} N' _{N4} N'_{F} N'_{L} N'_{N5}N'_{N6} N'_{L} N'_{L} N'_{L} N'_{L} N'_{L} N'_{L} N'_{L}-3' formula (V')

wherein, each N'_{F} represents a 2'-fluoro modified nucleotide;
N'_{N1}, N'_{N2}, N'_{N3}, N'_{N4}, N'_{N5}, and N'_{N6} independently represent a modified or an unmodified nucleotide, wherein only two of N'_{N1}, N'_{N2}, N'_{N3}, N'_{N4}, N'_{N5}, and N'_{N6} are 2'-fluoro modified nucleotides;
each N'_{L} independently represents a modified or an unmodified nucleotide, wherein the modified nucleotide is not a 2'-fluoro modified nucleotide;
n' can be an integer from 0-7.

In some non-limiting embodiments, in the sense strand represented by formula (V) of the dsRNA agent, N'_{N1}, N'_{N2}, N'_{N3}, N'_{N4}, N'_{N5}, and/or N'_{N6} are independently represent a modified nucleotide or an unmodified nucleotide, wherein N'_{N1} and N'_{N2} are 2'-fluorine modified nucleotides. It can be understood by those skilled in the art that the dsRNA agent has 2'-fluoro modified nucleotides at positions 11, 14, and 15 of the sense strand represented by formula (V) counting from the first paired nucleotide at 3' end. It can be understood that the dsRNA agent may have 2'-fluoro modified nucleotides at other positions of the sense strand, but the dsRNA agent has a total of six 2'-fluoro modified nucleotides and has no motif of three or more contiguous 2'-fluoro modified nucleotides. In some non-limiting embodiments, in the sense strand represented by formula (V') of the dsRNA agent, N'_{N1}, N'_{N2}, N'_{N3}, N'_{N4}, N'_{N5}, and/or N'_{N6} are independently represent a modified nucleotide or an unmodified nucleotide, wherein N'_{N1} and N'_{N2} are 2'-fluorine modified nucleotides, and N'_{N3}, N'_{N4}, N'_{N5}, and N'_{N6} are not 2' -fluorine modified nucleotides. It can be understood by those skilled in the art that the dsRNA agent has 2'-fluoro modified nucleotides at positions 11, 14, and 15 of the sense strand represented by formula (V) counting from the first paired nucleotide at 3' end, and has no 2'-fluoro modified nucleotides at other positions.

In some non-limiting embodiments, in the sense strand represented by formula (V) of the dsRNA agent, N'_{N3} and N'_{N6} are 2'-fluorine modified nucleotides. It can be understood by those skilled in the art that the dsRNA agent has 2'-fluoro modified nucleotides at positions 8, 11, and 13 of the sense strand represented by formula (V) counting from the first paired nucleotide at 3' end, and the dsRNA agent may have 2'-fluoro modified nucleotides at other positions of the sense strand, but the dsRNA agent has no more than six 2'-fluoro modified nucleotides in total and has no motif of three or more contiguous 2'-fluoro modified nucleotides. In some non-limiting embodiments, in the sense strand represented by formula (V') of the dsRNA agent, N'_{N1}, N'_{N2}, N'_{N3}, N'_{N4}, N'_{N5}, and/or N'_{N6} are independently represent a modified nucleotide or an unmodified nucleotide, wherein N'_{N3} and N'_{N6} are 2'-fluorine modified nucleotides, and N'_{N1}, N'_{N2}, N'_{N4}, and N'_{N5} are not 2'-fluorine modified nucleotides. It can be understood by those skilled in the art that the dsRNA agent has 2'-fluoro modified nucleotides at positions 8, 11, and 13 of the sense strand represented by formula (V) counting from the first paired nucleotide at 3' end, and has no 2'-fluoro modified nucleotides at other positions.

In some non-limiting embodiments, in the sense strand represented by formula (V) of the dsRNA agent, N'_{N1}, N'_{N2}, N'_{N3}, N'_{N4}, N'_{N5}, and/or N'_{N6} are independently represent a modified nucleotide or an unmodified nucleotide, wherein N'_{N3} and N'_{N5} are 2'-fluorine modified nucleotides. It can be understood by those skilled in the art that the dsRNA agent has 2'-fluoro modified nucleotides at positions 9, 11, and 13 of the sense strand represented by formula (V) counting from the first paired nucleotide at 3' end, and the dsRNA agent may have 2'-fluoro modified nucleotides at other positions of the sense strand, but the dsRNA agent has no more than six 2'-fluoro modified nucleotides in total and has no motif of three or more contiguous 2'-fluoro modified nucleotides. In some non-limiting embodiments, in the sense strand represented by formula (V') of the dsRNA agent, N'_{N1}, N'_{N2}, N'_{N3}, N'_{N4}, N'_{N5}, and/or N'_{N6} are independently represent a modified nucleotide or an unmodified nucleotide, wherein N'_{N3} and N'_{N5} are 2'-fluorine modified nucleotides, and N'_{N1}, N'_{N2}, N'_{N4}, and N'_{N6} are not 2'-fluorine modified nucleotides. It can be understood by those skilled in the art that the dsRNA agent has 2'-fluoro modified nucleotides at positions 9, 11, and 13 of the sense strand represented by formula (V) counting from the first paired nucleotide at 3' end, and has no 2'-fluoro modified nucleotides at other positions.

In some specific embodiments, the dsRNA agent has 2'-fluoro modified nucleotides at positions 9, 11, and 13 of the sense strand represented by formula (V) counting from the first paired nucleotide at 3' end of the sense strand. It can be understood that the dsRNA agent may have 2'-fluoro modified nucleotides at other positions of the sense strand, but the dsRNA agent has no more than six 2'-fluoro modified nucleotides in total and has no motif of three or more contiguous 2'-fluoro modified nucleotides. In some specific embodiments, the dsRNA agent has 2'-fluoro modified nucleotides at positions 9, 11, and 13 of the sense strand represented by formula (V') counting from the first paired nucleotide at 3' end of the sense strand, and it can be understood that the dsRNA agent has no 2'-fluoro modified nucleotides at other positions of the sense strand.

The motif of three contiguous 2' fluorine-modified nucleotides can be understood as that contiguous 3 nucleotides in one strand of the dsRNA agent are simultaneously modified by 2'-fluorine, for example, in the XYYYX sequence, all Y are 2'-fluorine modified nucleotides, and X can be modified differently.

As used herein, the positions of the sense strand represented by formula (V) counting from the first paired nucleotide at 3' end generally refer to the positions counting from the blunt end of the first paired nucleotide N'_{L} at 3' end of the sense strand, as a non-limiting example, in formula (Va), Np'-5'-(N'_{L})ₙ -(N'_{L})ₙ N_{L} N'_{L} N'_{L} N'_{N1} N'_{N2} N'_{N3} N'_{N4} N'_{F} N'_{L} N'_{N5}N'_{N6} N'_{L} N'_{L} N'_{L} N'_{L} N'_{L} N'_{L} N'_{L} (at position 1)-3' formula (Va); and it will be further understood that the first mismatch in the dsRNA agent is also considered as a pairing, also referred to as position 1

In some specific embodiments, the dsRNA agent has 2'-fluoro modified nucleotides at positions 9, 11 and 13 of the sense strand represented by formula (V') counting from the first paired nucleotide at 3' end of the sense strand. In some specific embodiments, the dsRNA agent has 2'-fluoro modified nucleotides at positions 9, 11 and 12 of the sense strand represented by formula (V') counting from the first paired nucleotide at 3' end of the sense strand. In some specific embodiments, the dsRNA agent has 2'-fluoro modified nucleotides at positions 8, 11 and 13 of the sense strand represented by formula (V') counting from the first paired nucleotide at 3' end of the sense strand. In some specific embodiments, the dsRNA agent has 2'-fluoro modified nucleotides at positions 11, 12 and 14 of the sense strand represented by formula (V') counting from the first paired nucleotide at 3' end of the sense strand. In some specific embodiments, the dsRNA agent has 2'-fluoro modified nucleotides at positions 11, 12 and 15 of the sense strand represented by formula (V') counting from the first paired nucleotide at 3' end of the sense strand. It will be understood by one of skill in the art that in the specific embodiments, the sense strand represented by formula (V') of the dsRNA agent has only three 2'-fluorine modified nucleotides.

According to another aspect of the present invention, an RNAi agent for inhibiting expression of a target gene is provided, wherein the RNAi agent is double-stranded ribonucleic acid (dsRNA) agent comprising a sense strand and an antisense strand complementary to at least a portion of an mRNA corresponding to the target gene, and the sense strand has 18-40 nucleotides and comprises a sequence represented by formula (IX) in the direction of 5'-3' :

5'-(N'_{L})_{n'} N'_{L} N'_{L} N'_{L} N'_{N1} N'_{N2} N'_{N3} N'_{N4} N'_{F} N'_{L} N'_{N5}N'_{N6} N'_{N7} N'_{L} N'_{L} N'_{L} N'_{L} N'_{L} N'_{L}-3' formula (IX)

wherein, each N'_{F} represents a 2'-fluoro modified nucleotide;
N'_{N1}, N'_{N2}, N'_{N3}, N'_{N4}, N'_{N5}, N'_{N6}, and N'_{N7} independently represent a modified or an unmodified nucleotide, wherein at least two of N'_{N1}, N' _{N2}, N'_{N3}, N'_{N4}, N'_{N5}, N'_{N6}, and N'_{N7} are 2'-fluoro modified nucleotides;
formula (V) has no motif of three or more contiguous 2'-fluoro modified nucleotides and has no more than six 2'-fluoro modified nucleotides;
each N'_{L} independently represents a modified or an unmodified nucleotide;
n' can be an integer from 0 to 7.

In some specific embodiments, the dsRNA agent has 2'-fluoro modified nucleotides at positions 7, 9, 11 and 13 of the sense strand represented by formula (IX) counting from the first paired nucleotide at 3' end of the sense strand. It can be understood by those skilled in the art that in the specific embodiments, the dsRNA agent has only four 2'-fluorine modified nucleotides in the sense strand represented by formula (IX).

In some embodiments, in the sense strand represented by formula (V) and/or formula (IX), one or more of N'_{L} are modified nucleotides independently selected from the group consisting of 2'-fluoro modified nucleotide, 2'-O-methyl modified nucleotide, 2'-deoxy nucleotide, 2',3'-seco nucleotide mimic, locked nucleotide (LNA), unlocked nucleic acid nucleotide (UNA), glycol nucleic acid nucleotide (GNA), bicyclo nucleic acid (BNA), 2'-F-arabino nucleotide, 2'-methoyxyethyl nucleotide, abasic nucleotide, ribitol, inverted nucleotide, inverted abasic nucleotide (Invab), inverted 2'-OMe nucleotide, inverted 2'-deoxy nucleotide, 2'-amino modified nucleotide, 2'-alkyl modified nucleotide, morpholino nucleotide, 3'-OMe nucleotide, phosphorothioate modified nucleotide, terminal nucleotide linked to a cholesteryl derivative or dodecanoic acid bisdecylamide group, 2'-amino modified nucleotide, phosphoramidate, and non-natural base comprising nucleotide. In some embodiments, N'_{L} of the dsRNA agent is a modified nucleotide selected from the group consisting of 2'-O-methyl modified nucleotide, UNA, and Invab.

In some embodiments, in the sense strand represented by formula (V) and/or formula (IX) of the dsRNA agent, in some non-limiting embodiments, in the sense strand represented by formula (V) of the dsRNA agent, N'_{N1}, N'_{N2}, N'_{N3}, N'_{N4}, N'_{N5}, N'_{N6}, and N'_{N7} independently represent a modified nucleotide or an unmodified nucleotide, and the modified nucleotide is independently selected from the group consisting of 2'-fluoro modified nucleotide, 2'-O-methyl modified nucleotide, 2'-deoxy nucleotide, 2',3'-seco nucleotide mimic, locked nucleotide (LNA), unlocked nucleic acid nucleotide (UNA), glycol nucleic acid nucleotide (GNA), bicyclo nucleic acid (BNA), 2'-F-arabino nucleotide, 2'-methoyxyethyl nucleotide, abasic nucleotide, ribitol, inverted nucleotide, inverted abasic nucleotide (Invab), inverted 2'-OMe nucleotide, inverted 2'-deoxy nucleotide, 2'-amino modified nucleotide, 2'-alkyl modified nucleotide, morpholino nucleotide, 3'-OMe nucleotide, phosphorothioate modified nucleotide, terminal nucleotide linked to a cholesteryl derivative or dodecanoic acid bisdecylamide group, 2'-amino modified nucleotide, phosphoramidate, and non-natural base comprising nucleotide. In some embodiments, in the sense strand represented by formula (V') of the dsRNA agent, N'_{N1}, N'_{N2}, N'_{N3}, N'_{N4}, N'_{N5}, and N'_{N6} are independently preferably 2'-O methyl modified nucleotides, UNA, GNA or LNA in case of not being 2'-fluoro modified nucleotides.

In some non-limiting embodiments, in the sense strand represented by formula (V') of the dsRNA agent, N'_{N3} and N'_{N6} are 2'-fluorine modified nucleotides, and N'_{N1}, N'_{N2}, N'_{N4} and N'_{N5} are not 2'-fluorine modified nucleotides. N'_{N1}, N'_{N2}, N'_{N4} and N'_{N5} are modified nucleotides independently selected from the group consisting of 2'-O-methyl modified nucleotide, 2'-deoxy nucleotide, 2',3'-seco nucleotide mimic, locked nucleotide (LNA), unlocked nucleic acid nucleotide (UNA), glycol nucleic acid nucleotide (GNA), bicyclo nucleic acid (BNA), 2'-F-arabino nucleotide, 2'-methoyxyethyl nucleotide, abasic nucleotide, ribitol, inverted nucleotide, inverted abasic nucleotide (Invab), inverted 2'-OMe nucleotide, inverted 2'-deoxy nucleotide, 2'-amino modified nucleotide, 2'-alkyl modified nucleotide, morpholino nucleotide, 3'-OMe nucleotide, phosphorothioate modified nucleotide, terminal nucleotide linked to a cholesteryl derivative or dodecanoic acid bisdecylamide group, 2'-amino modified nucleotide, phosphoramidate, and non-natural base comprising nucleotide. In some preferred embodiments, N'_{N1}, N'_{N2}, N'_{N4} and N'_{N5} independently represent a modified nucleotide or an unmodified nucleotide, and one or more are modified nucleotides independently selected from the group consisting of 2'-O-methyl modified nucleotide, UNA, GNA, and LNA. In some preferred embodiments, N'_{N1}, N'_{N2}, N'_{N4} and N'_{N5} of the dsRNA agent independently represent a modified nucleotide or an unmodified nucleotide and are 2'-O-methyl modified nucleotide.

In some non-limiting embodiments, in the sense strand represented by formula (V') of the dsRNA agent, N'_{N3} and N'_{N5} are 2'-fluorine modified nucleotides, and N'_{N1}, N'_{N2}, N'_{N4} and N'_{N6} are not 2'-fluorine modified nucleotides. N'_{N1}, N'_{N2}, N'_{N4} and N'_{N6} are modified nucleotides independently selected from the group consisting of 2'-O-methyl modified nucleotide, 2'-deoxy nucleotide, 2',3'-seco nucleotide mimic, locked nucleotide (LNA), unlocked nucleic acid nucleotide (UNA), glycol nucleic acid nucleotide (GNA), bicyclo nucleic acid (BNA), 2'-F-arabino nucleotide, 2'-methoyxyethyl nucleotide, abasic nucleotide, ribitol, inverted nucleotide, inverted abasic nucleotide (Invab), inverted 2'-OMe nucleotide, inverted 2'-deoxy nucleotide, 2'-amino modified nucleotide, 2'-alkyl modified nucleotide, morpholino nucleotide, 3'-OMe nucleotide, phosphorothioate modified nucleotide, terminal nucleotide linked to a cholesteryl derivative or dodecanoic acid bisdecylamide group, 2'-amino modified nucleotide, phosphoramidate, and non-natural base comprising nucleotide. In some preferred embodiments, N'_{N1}, N'_{N2}, N'_{N4} and N'_{N6} independently represent a modified nucleotide or an unmodified nucleotide, and one or more are modified nucleotides independently selected from the group consisting of 2'-O-methyl modified nucleotide, UNA, GNA, and LNA. In some preferred embodiments, N'_{N1}, N'_{N2}, N'_{N4} and N'_{N6} of the dsRNA agent independently represent a modified nucleotide or an unmodified nucleotide and are 2'-O-methyl modified nucleotide.

In some embodiments, in the antisense strand complementary or substantially complementary to the sense strand represented by formula (V) of the dsRNA agent, all or substantially all of nucleotides are modified nucleotides. In some embodiments, in the antisense strand of the dsRNA agent, all nucleotides are modified nucleotides. The nucleotides in the antisense strand are modified nucleotides independently selected from the group consisting of 2'-fluoro modified nucleotide, 2'-O-methyl modified nucleotide, 2'-deoxy nucleotide, 2',3'-seco nucleotide mimic, locked nucleotide (LNA), unlocked nucleic acid nucleotide (UNA), glycol nucleic acid nucleotide (GNA), bicyclo nucleic acid (BNA), 2'-F-arabino nucleotide, 2'-methoyxyethyl nucleotide, abasic nucleotide, ribitol, inverted nucleotide, inverted abasic nucleotide (Invab), inverted 2'-OMe nucleotide, inverted 2'-deoxy nucleotide, 2'-amino modified nucleotide, 2'-alkyl modified nucleotide, morpholino nucleotide, 3'-OMe nucleotide, phosphorothioate modified nucleotide, terminal nucleotide linked to a cholesteryl derivative or dodecanoic acid bisdecylamide group, 2'-amino modified nucleotide, phosphoramidate, and non-natural base comprising nucleotide.

In some embodiments, all or substantially all of nucleotides in the sense strand and the antisense strand of the dsRNA agent of the present invention are modified nucleotides.

In some embodiments, in the sense strand represented by formula (V) of the dsRNA agent of the present invention, the phosphate modified nucleotide is phosphorothioate modified nucleotide. In some embodiments, in the sense strand represented by formula (V) of the dsRNA agent of the present invention, the phosphate modified nucleotide is 5'-phosphorothioate modified nucleotide.

In some embodiments, the sense strand represented by formula (V) of the dsRNA agent of the present invention comprises an E-vinylphosphonate nucleotide at the 5' end of the sense strand.

In some embodiments, the dsRNA agent of the present invention has an antisense strand complementary or substantially complementary to the sense strand represented by formula (V), and the complementary region is 18-25 nucleotides in length. In some embodiments, the complementary region is 18-23 nucleotides in length. In some embodiments, the complementary region is 19-21 nucleotides in length. In other embodiments, for example, the complementary region can be 18-25 nucleotides in length, 19-25 nucleotides in length, 19-23 nucleotides in length, or 19-21 nucleotides in length. In another example, in some embodiments, the complementary region is 18, 19, 20 or 21 nucleotides in length.

In some embodiments, n' is 7 in the dsRNA agent, and it can be understood that the complementary pair of the sense strand is 25 nucleotides in length. In some specific embodiments, n' is 0, 1, 2, 3, 4, 5, 6 or 7 in the dsRNA agent.

In some embodiments, the dsRNA agent has an antisense strand fully complementary to the sense strand represented by formula (V).

In some embodiments, the dsRNA agent of the present invention contains no mismatches. In certain embodiments, the target gene dsRNA agent of the present invention contains no more than 1 mismatch (such mismatch does not affect the counting start position of the pairing). In some embodiments, the dsRNA agent of the present invention contains no more than 2 mismatches. In certain embodiments, the dsRNA agent of the present invention contains no more than 3 mismatches. In some embodiments of the invention, the antisense strand of the dsRNA agent contains a mismatch to a target sequence that is not located in the center of the complementary region. In some embodiments, the antisense strand of the dsRNA agent contains 1, 2, 3, 4 or more mismatches located within the last 5, 4, 3, 2 or 1 nucleotide of either or both the 5' end or 3' end of the complementary region..

In some embodiments, the antisense strand of the dsRNA agent of the present invention is no more than 30 nucleotides in length. In some embodiments, each strand of the dsRNA agent of the present invention is no more than 30 nucleotides in length. In some embodiments, each strand of the dsRNA agent of the present invention is no more than 25 nucleotides in length. In some embodiments, each strand of the dsRNA agent of the present invention is no more than 23 nucleotides in length. In some embodiments, each strand of the dsRNA of the present invention is 19, 20 or 21 nucleotides in length.

In some embodiments, the dsRNA agent has a blunt end at the 5' end of the sense strand. In one embodiment, the dsRNA agent of the present invention has two blunt ends at both ends of the sense strand and the antisense strand. For example, each strand of the dsRNA agent is fully paired to form a blunt end structure.

In some embodiments, the sense strand represented by formula (V) of the RNAi agent has 1-5 unpaired nucleotide overhangs at 3' end, such as 1, 2, 3, 4 or 5 unpaired nucleotide overhangs.

In some embodiments, the antisense strand complementary or partially complementary to formula (V) of the dsRNA agent has 1-5 unpaired nucleotide overhangs at 3' end and/or 5' end, such as 1, 2, 3, 4 or 5 unpaired nucleotide overhangs. In some embodiments, the antisense strand complementary or partially complementary to formula (V) of the dsRNA agent has a blunt end at 3' end and/or 5' end.

In some embodiments, the overhang of unpaired nucleotides in the sense strand and/or antisense strand of the dsRNA agent of the present invention is 2-5 nucleotides in length, 1-5 nucleotides in length, 1-4 nucleotides in length, 2-4 nucleotides in length, 1-3 nucleotides in length, 2-3 nucleotides in length or 1-2 nucleotides in length. The overhang can be the result of one strand being longer than the other, or the result of two strands of the same length being staggered. The overhang may form one mismatch with the target mRNA, or it may be complementary to the targeted gene sequence or may be other sequences. The first and second strand can also be connected, for example, by other base to form a hairpin or by other non-base linkers.

In one embodiment, one or more nucleotides in the overhang of the dsRNA agent of the present invention can be independently a modified nucleotide or an unmodified nucleotide, and the modified nucleotide includes but is not limited to: 2'-fluoro modified nucleotide, 2'-O-methyl modified nucleotide, 2'-deoxy nucleotide, 2',3'-seco nucleotide mimic, locked nucleotide (LNA), unlocked nucleic acid nucleotide (UNA), glycol nucleic acid nucleotide (GNA), bicyclo nucleic acid (BNA), 2'-F-arabino nucleotide, 2'-methoyxyethyl nucleotide, abasic nucleotide, ribitol, inverted nucleotide, inverted abasic nucleotide (Invab), inverted 2'-OMe nucleotide, inverted 2'-deoxy nucleotide, 2'-amino modified nucleotide, 2'-alkyl modified nucleotide, morpholino nucleotide, 3'-OMe nucleotide, phosphorothioate modified nucleotide, terminal nucleotide linked to a cholesteryl derivative or dodecanoic acid bisdecylamide group, 2'-amino modified nucleotide, phosphoramidate, and non-natural base comprising nucleotide.

In one embodiment, both ends of the overhang of the sense strand, antisense strand or both strands of the overhang of the dsRNA agent of the present invention can be phosphorylated.

In one embodiment, the overhang of the dsRNA agent of the present invention exists at 5' end or 3' end of the sense strand, antisense strand or both strands. In one embodiment, the overhang exists at 3' end of the antisense strand. In one embodiment, the overhang exists at 5' end of the sense strand.

In one embodiment, the dsRNA agent of the present invention may comprise only one single overhang, which can enhance the interference activity of the dsRNA without affecting its overall stability. For example, the single-stranded overhang is located at the 5' end of the sense strand, or alternatively, at the 3' end of antisense strand, and correspondingly, the dsRNA agent may also have a blunt end located at the 5' end of the antisense strand (or the 3' end of the sense strand), or vice versa.

In one embodiment, the 5' end of the antisense strand or the 3' end of the sense strand is blunt end in the dsRNA agent of the present invention.

In some embodiments, the dsRNA agent of the present invention comprises at least one phosphorothioate internucleoside linkage.

In some embodiments, the sense strand of the dsRNA agent of the present invention comprises at least one phosphorothioate internucleoside linkage.

In some embodiments, the antisense strand of the dsRNA agent of the present invention comprises at least one phosphorothioate internucleoside linkage.

In some embodiments, the sense strand and/or the antisense strand of the dsRNA agent of the present invention comprises 1, 2, 3, 4, 5, 6, 7, 8, 9, 10, 11, 12, 13, 14, 15, 16, 17 or 18 phosphorothioate internucleoside linkages.

In one embodiment, the sense strand of the dsRNA agent of the present invention comprises 1-10 blocks with 2-10 phosphorothioate internucleoside linkages separated by 1, 2, 3, 4, 5, 6, 7, 8, 9, 10, 11, 12, 13, 14, 15, 16, 17 or 18 phosphate internucleoside linkages, wherein one of the phosphorothioate internucleoside linkages is located at any position in the oligonucleotide sequence, and the sense strand is paired with the antisense strand comprising any combination of phosphorothioate internucleoside linkages, methylphosphonate internucleoside linkages, and phosphate internucleoside linkages or the antisense strand comprising phosphorothioate linkage or phosphate linkage.

In one embodiment, the antisense strand of the dsRNA agent of the present invention comprises 1-10 blocks with 2-10 phosphorothioate internucleoside linkages separated by 1, 2, 3, 4, 5, 6, 7, 8, 9, 10, 11, 12, 13, 14, 15, 16, 17 or 18 phosphate internucleoside linkages, wherein one of the phosphorothioate internucleoside linkages is located at any position in the oligonucleotide sequence, and the antisense strand is paired with the sense strand comprising any combination of phosphorothioate internucleoside linkage, methylphosphonate internucleoside linkage and phosphate internucleoside linkages or the sense strand comorising phosphorothioate linkage or phosphate linkage.

In one embodiment, the dsRNA agent of the present invention further comprises one or more phosphorothioate internucleoside linkage modifications in the internal region of 1-10 duplexes of the sense strand and/or the antisense strand. For example, at least 2, 3, 4, 5, 6, 7, 8, 9 or 10 nucleotides at positions 8-16 of the duplex region counting from the 3' end of the sense strand can be linked via the phosphorothioate internucleoside linkage. The dsRNA can optionally further comprise one or more phosphorothioate internucleotide linkage modifications in 1-10 end positions.

In one embodiment, the dsRNA agent of the present invention further comprises 1-5 phosphorothioate internucleotide linkage or methylphosphonate internucleotide linkage modifications at positions 1-5 of the sense strand and further comprises 1-5 phosphorothioate internucleotide linkage or methylphosphonate internucleotide linkage modifications at positions 18-23 of the sense strand (counting from the paired nucleotide at 3' end of the sense strand as used herein), and further comprises 1-2 phosphorothioate internucleotide linkages at positions 1-2 of the antisense strand and further comprises 1-5 phosphorothioate internucleotide linkages in positions 18-23 of the antisense strand (counting from the blunt end at 5' end of the antisense strand as used herein). In one embodiment, the dsRNA agent of the present invention comprises 1 or 2 phosphorothioate internucleotide linkage modifications at 3' end and/or 5' end of the sense strand or the antisense strand.

In one embodiment, the dsRNA agent of the present invention comprises the phosphorothioate internucleotide linkage modification in the overhang. It should be understood in the art that the overhang of the dsRNA agent of the present invention refers to the unpaired nucleotide motif unless otherwise specified. For example, the overhang comprises two nucleotides with a phosphorothioate internucleotide linkage between the two nucleotides. Internucleotide linkage modification can also be made to link the overhang nucleotide with the terminal-paired nucleotide in the duplex region. It should be understood in the art that the linking of the overhang nucleotide and the terminal-paired nucleotide in the duplex region refers to the linking of the overhang nucleotide and terminal N_{L} or N_{L}' unless otherwise specified. For example, at least 2, 3, 4 or all nucleotides in the overhang may be linked by phosphorothioate internucleotide linkages, and optionally, there may be another phosphorothioate internucleotide linkage that links the overhang nucleotide with a paired nucleotide next to the overhang nucleotide. For example, there may be at least two phosphorothioate internucleotide linkages between the terminal three nucleotides, where two of the three nucleotides are overhang nucleotides and the third one is a paired nucleotide next to the overhang nucleotide. Preferably, these three terminal nucleotides may be at the 3' end of the antisense strand. In one embodiment, this is also the case where the dsRNA agent of the present invention comprises the phosphorothioate linkage modification in the overhang of the sense strand

In some embodiments, the sense strand and/or the antisense strand of the dsRNA agent of the present invention has 1 or 2 inverted abasic residue nucleotides. In some embodiments, the sense strand and/or the antisense strand of the dsRNA agent of the present invention comprises 1 or 2 inverted abasic residues at 3' end or/and 5' end. In some embodiments, the sense strand of the dsRNA agent of the present invention comprises an inverted abasic residue at the 3' end.

In some embodiments, the dsRNA agent further comprises one or more targeting groups or linking groups. In some embodiments, the one or more targeting groups or linking groups of the dsRNA agent of the present invention are same or different. The targeting group or linking group can be conjugated to the sense strand, antisense strand, or both strands at 3' end, 5' end, or both ends. In some embodiments, one or more targeting groups or linking groups of the RNAi agent are conjugated to the sense strand. The distribution, targeting, lifetime, endosomal dissolution characteristics, improved delivery, hybridization, or specificity of the RNAi agent can be altered by the targeting ligand incorporated, for example, but not limiting, lectin, glycoprotein, lipid or protein, thyrotropin, melanotropin, lectin, glycoprotein, surfactant protein A, mucin carbohydrate, multivalent lactose, multivalent galactose, N-acetyl-galactosamine, N-acetyl-glucosamine, multivalent mannose, multivalent fucose, glycosylated polyaminoacid, multivalent galactose, transferrin, bisphosphonate, polyglutamate, polyaspartate, lipid, cholesterol, steroid, bile acid, folate, vitamin B12, vitamin A, biotin, RGD peptide, RGD peptide mimetic, or aptamer, and other examples include dyes, intercalating agents (e.g. acridines), cross-linkers (e.g. psoralene, mitomycin C), porphyrins (TPPC4, texaphyrin, sapphyrin), polycyclic aromatic hydrocarbons (e.g., phenazine, dihydrophenazine), artificial endonucleases or chelating agents (e.g. EDTA), lipophilic molecules, e.g., cholesterol, cholic acid, adamantane acetic acid, 1-pyrene butyric acid, dihydrotestosterone, 1,3-bis-O(hexadecyl)glycerol, geranyloxyhexyl group, hexadecylglycerol, borneol, menthol, 1,3-propanediol, heptadecyl group, palmitic acid, myristic acid, O3-(oleoyl)lithocholic acid, O3-(oleoyl)cholenic acid, dimethoxytrityl, or phenoxazine) and peptide conjugates (e.g., antennapedia peptide, Tat peptide), alkylating agents, phosphate, amino, mercapto, PEG (e.g., PEG-40K), MPEG, [MPEG]2, polyamino, alkyl, substituted alkyl, radiolabeled markers, enzymes, haptens (e.g., biotin), transport/absorption facilitators (e.g., aspirin, vitamin E, folic acid), synthetic ribonucleases (e.g., imidazole, bisimidazole, histamine, imidazole clusters, acridine-imidazole conjugates, Eu3+ complexes of tetraazamacrocycles), dinitrophenyl, HRP, or AP. In some embodiments, the targeting group or linking group is selected from the group consisting of N-acetyl-galactosamine (GalNAc) and a lipophilic molecule. The lipophilic molecule can be used for delivery in the field of neurology. In some embodiments, the targeting group or linking group of the dsRNA agent is conjugated to the 5' end of the sense strand.

Another non-limiting example of a delivery agent that can be used in the embodiments of the present invention to deliver the dsRNA agent of the present invention to a cell, tissue, and/or subject is a GalNAc-containing agent, which is linked to the dsRNA agent of the present invention and delivers the dsRNA agent to a cell, tissue and/or subject. Examples of certain other delivery agents comprising GalNAc that may be used in certain embodiments of the method and composition of the present invention are disclosed in PCT application WO2020191183 A1. A non-limiting example of a GalNAc targeting ligand that can be used in the composition and method of the present invention to deliver the dsRNA agent to a cell is a targeting ligand cluster. Examples of the targeting ligand cluster mentioned herein include a GalNAc ligand with phosphodiester bonds (GLO) and a GalNAc ligand with phosphorothioate bonds (GLS). The term "GLX-n" used herein may denote a linked GalNAC-containing compound, for example, but not limited to: GLS-1, GLS-2, GLS-3, GLS-4, GLS-5, GLS-6, GLS-7, GLS-8, GLS-9, GLS-10, GLS-11, GLS-12, GLS-13, GLS-14, GLS-15, GLS-16, GLO-1, GLO-2, GLO-3, GLO-4, GLO-5, GLO-6, GLO-7, GLO-8, GLO-9, GLO-10, GLO-11, GLO-12, GLO-13, GLO-14, GLO-15 and GLO-16, and the structure of each is shown below. It should be understood that any RNAi and dsRNA molecules of the present invention may be linked to GLS-1, GLS-2, GLS-3, GLS-4, GLS-5, GLS-6, GLS-7, GLS-8, GLS-9, GLS-10, GLS-11, GLS-12, GLS-13, GLS-14, GLS-15, GLS-16, GLO-1, GLO-2, GLO-3, GLO-4, GLO-5, GLO-6, GLO-7, GLO-8, GLO-9, GLO-10, GLO-11, GLO-12, GLO-13, GLO-14, GLO-15 or GLO-16. In some preferred examples, the targeting groups are GLS-5 and GLS-15 described herein.

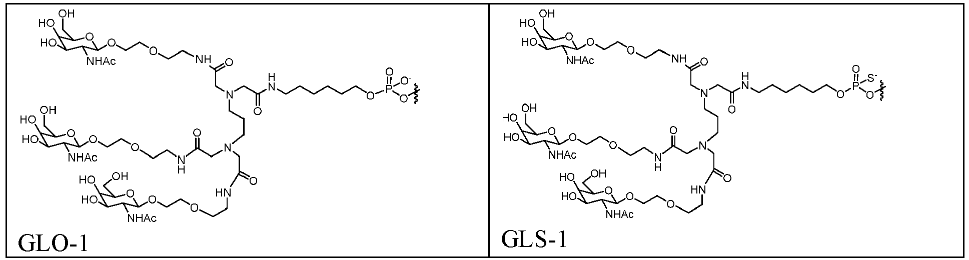

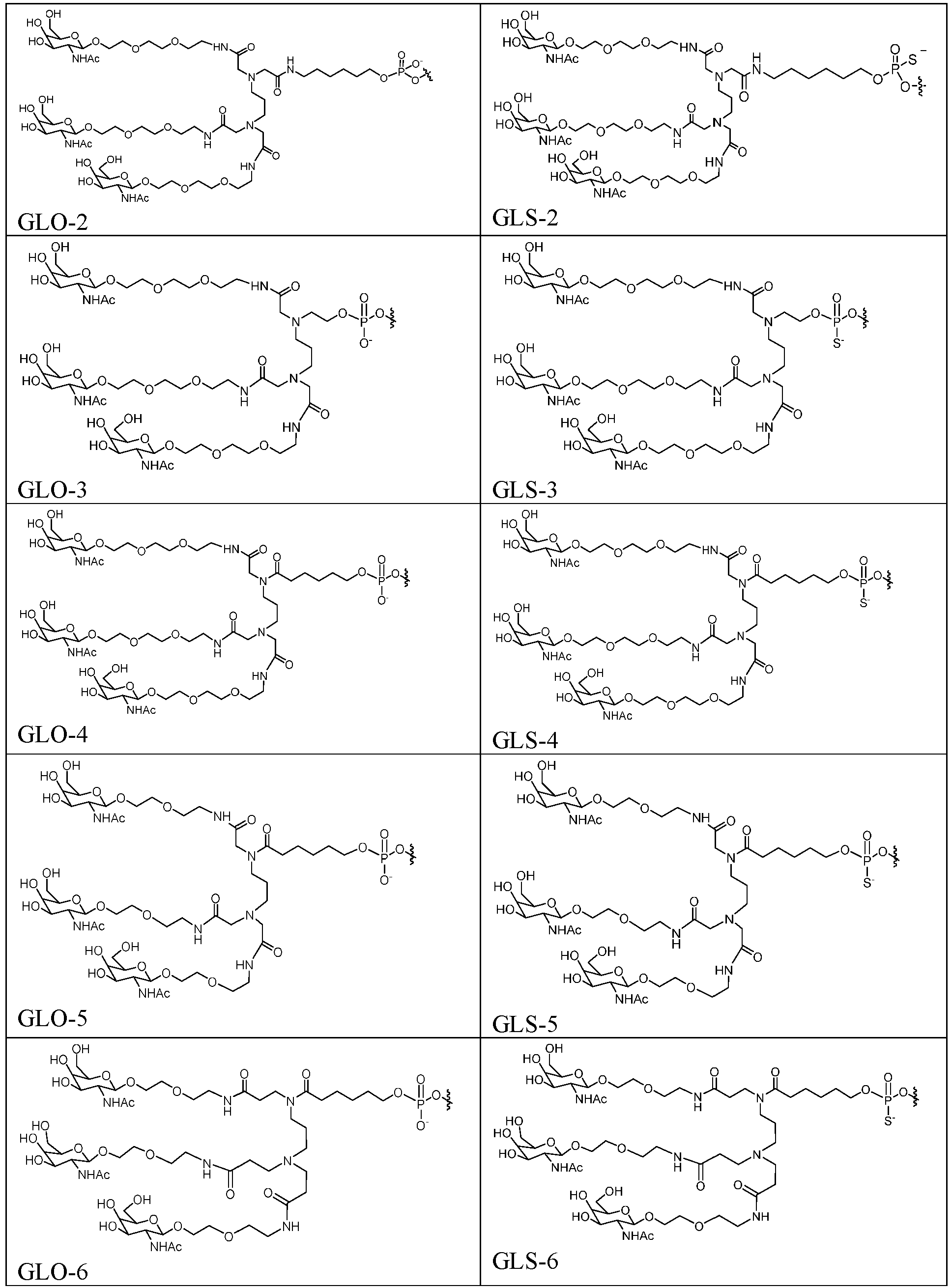

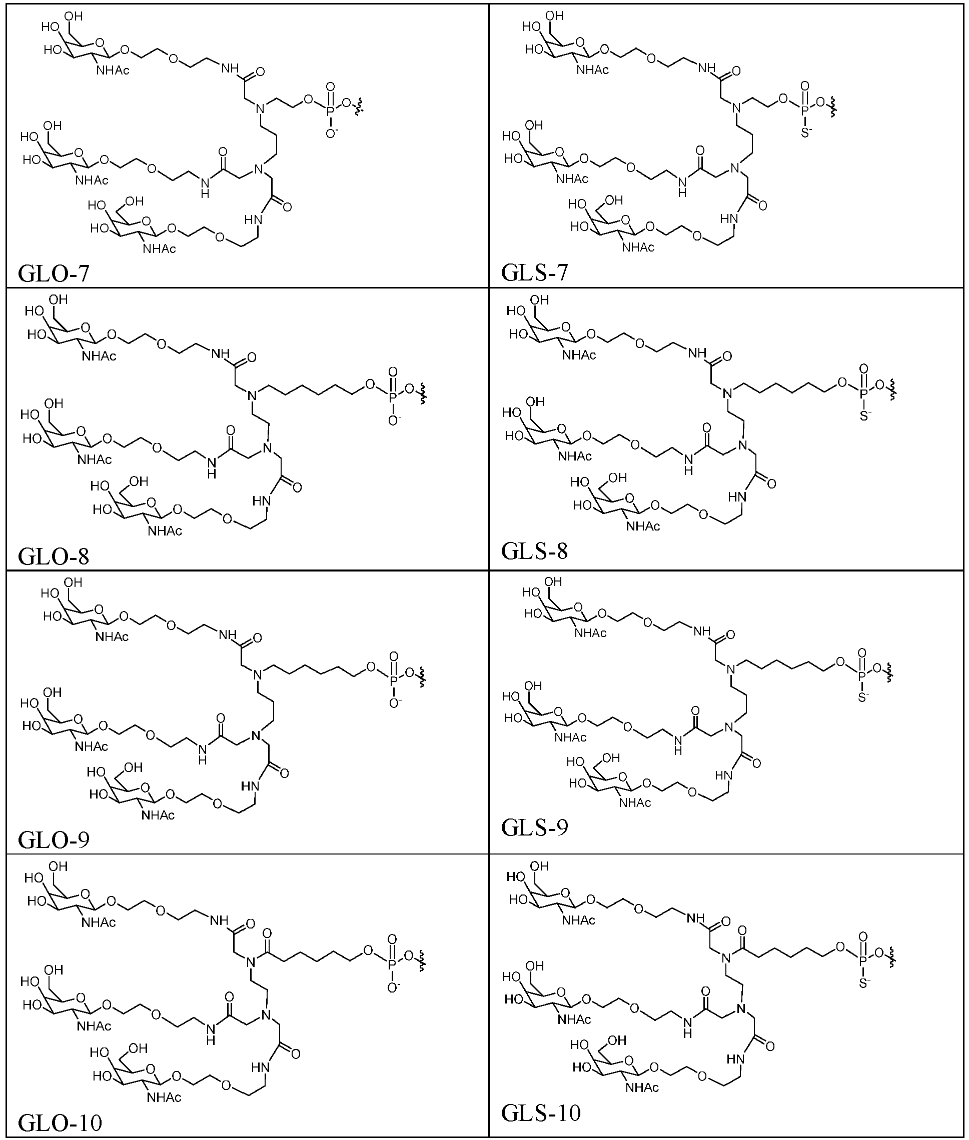

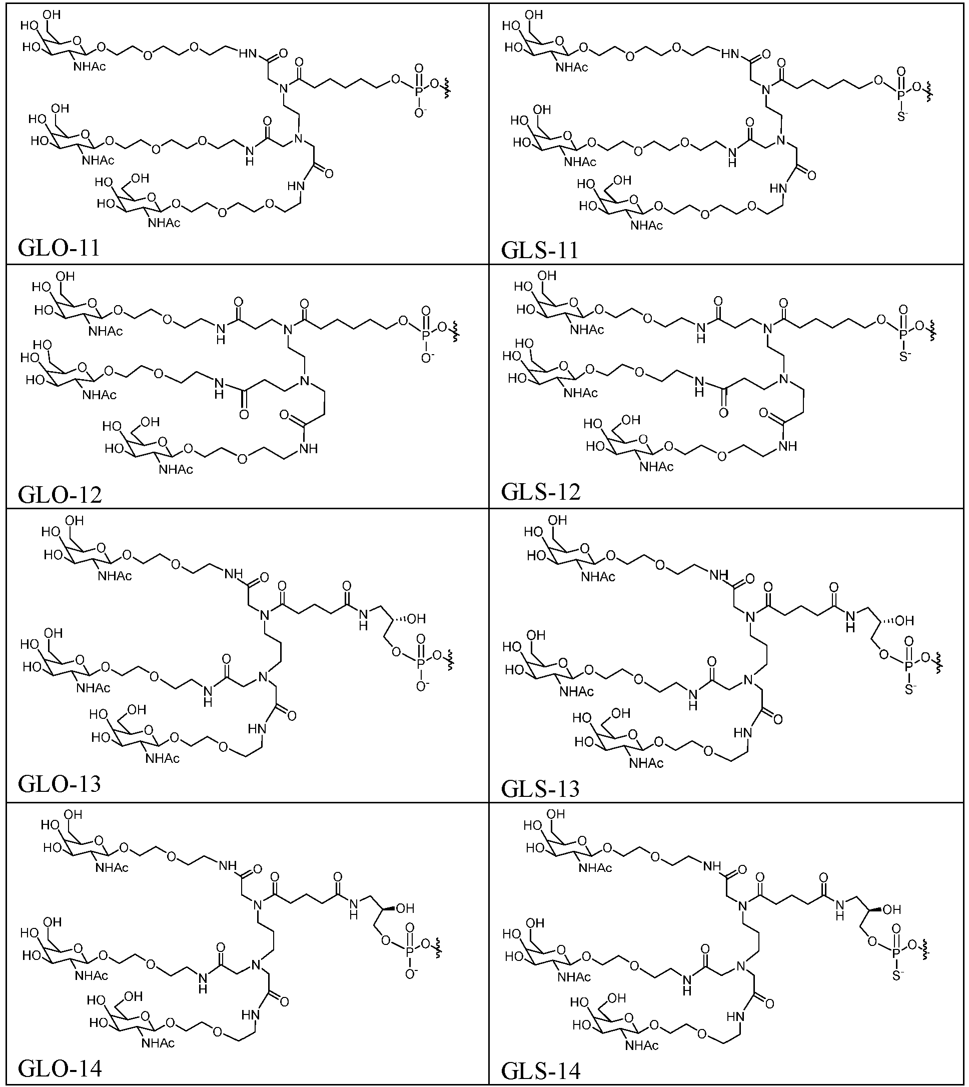

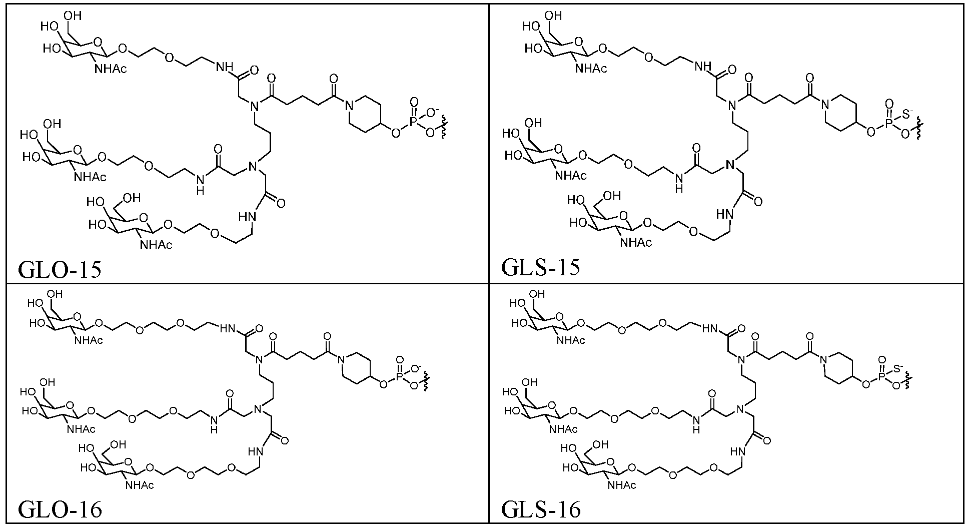

In some embodiments, the targeting group has the following structure: 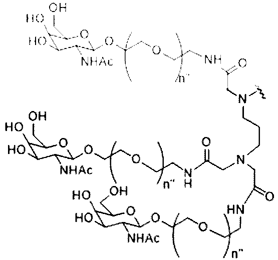 n" independently selected from the integer 1 or 2.

In some embodiments, the dsRNA comprises an inverted abasic residue at the 3' end of the sense strand represented by formula (V'). In some embodiments, the dsRNA agent comprises one or two inverted abasic residues at the 3' end or/and 5' end of the sense strand represented by formula (V').

**Table 1 shows the sequences of the sense strand and the antisense strand of some unmodified FXII RNAi agents of the present invention, and all sequences are shown from 5' to 3'.**

| Duplex AD# | sense sequence 5' to 3' | SEQ ID NO | antisense sequence 5' to 3' | SEQ ID NO | pos(20) in NM 021489.3 |
|---|---|---|---|---|---|
| AD00127.um | AACUCAAUAAAGUGCUUUGAA | 2 | UUCAAAGCACUUUAUUGAGUU | 3 | 1938 |
| AD00549.um | GCCCAAGAAAGUGAAAGACCA | 4 | UGGUCUUUCACUUUCUUGGGC | 5 | 307 |

**Table 2 shows the sequences of the sense strand and the antisense strand of some chemically modified FXII RNAi agents of the present invention. All sequences are shown from 5' to 3', and these sequences are used in some in vitro testing studies described herein. The chemical modifications are expressed as the following: uppercase indicates 2'-fluorine modified nucleotide; lowercase indicates 2'-OMe modified nucleotide; "*" indicates a phosphorothioate internucleoside linkage; "Invab" indicates inverted abasic nucleotide; and L0 is the trivalent GalNAc ligand cluster from literature. (trivalent GalNAc as in Jayaprakash, et al., (2014) J. Am. Chem. Soc., 136, 16958-16961).**

| Duplex AD# | Sense sequences (5' to 3') | 2F modification position | SEQ ID NO | Antisense sequences (5' to 3') | 2F modification position | SEQ ID NO |
|---|---|---|---|---|---|---|
| AD00127 | a*a*cucaauAaAgUgcuuuga*a-L0 | 9,11,13 | 6 | u*U*caaaGcacuUuAuUgag*u*u | 2,7,12,14,16 | 7 |
| AD00198 | a*a*cucaauAAAgugcuuuga*a-L0 | 11,12,13 | 8 | u*U*cAaAgCaCuUuAuUgAg*U *u | 2,4,6,8,10, 12,14,16,18,20 | 9 |
| AD00199 | a*a*cucaAuAAAgugcuuuga*a- L0 | 11,12,13,15 | 10 | u*U*caaAgcacuuuAuUgag*u*u | 2,6,14,16 | 11 |
| AD00539 | (GLS-15)*(Invab)*aacucaAuAAAgugcuuugaa*(Invab) | 11,12,13,15 | 12 | u*U*caaAgcacuuuAuUgag*u*u | 2,6,14,16 | 13 |
| AD00540 | (GLS-15)*(Invab)*aacucaauAaAgUgcuuugaa*(Invab) | 9,11,13 | 14 | u*U*caaaGcacuUuAuUgag*u*u | 2,7,12,14,16 | 15 |
| AD00541 | (GLS-15)*(Invab)*aacucaauaAAgUgcuuugaa*(Invab) | 9,11,12 | 16 | u*U*caaaGcacuUuAuUgag*u*u | 2,7,12,14,16 | 17 |
| AD00542 | (GLS-15)*(Invab)*aacucaauAaAguGcuuugaa*(Invab) | 8,11,13 | 18 | u*U*caaaGcacuUuAuUgag*u*u | 2,7,12,14,16 | 19 |
| AD00545 | (GLS-15)*(Invab)*aacucaauaAAgUgcuuugaa*(Invab) | 9,11,12 | 20 | u*U*caaaGcacuUuAuugAg*u*u | 2,7,12,14,18 | 21 |
| AD00546 | (GLS-15)*(Invab)*aacucaauaAAgUgcuuugaa*(Invab) | 9,11,12 | 22 | u*U*caAagcacuUuAuugAg*u*u | 2,5,12,14,18 | 23 |
| AD00547 | (GLS-15)*(Invab)*aacucaauaAAgUgcuuugaa*(Invab) | 9,11,12 | 24 | u*U*cAaagcaCuuuAuUgag*u*u | 2,4,10,14,16 | 25 |
| AD00548 | (GLS-15)*(Invab)*aacucaauaAAgUgcuuugaa*(Invab) | 9,11,12 | 26 | u*U*caaaGeaCuuuAuUgag*u*u | 2,7,10,14,16 | 27 |
| AD00549 | (GLS-15)*(Invab)*gcccaaGaAAGugaaagacca*(Invab) | 11,12,13,15 | 28 | u*G*gucUuucacuuUcUugg*g*c | 2,6,14,16 | 29 |
| AD00550 | (GLS-15)*(Invab)*gcccaagaAaGuGaaagacca*(Invab) | 9,11,13 | 30 | u*G*gucuUucacUuUcUugg*g*c | 2,7,12,14,16 | 31 |
| AD00551 | (GLS-15)*(Invab)*gcccaagaaAGuGaaagacca*(Invab) | 9,11,12 | 32 | u*G*gucuUucacUuUcUugg*g*c | 2,7,12,14,16 | 33 |
| AD00552 | (GLS-15)*(Invab)*gcccaagaAaGugAaagacca*(Invab) | 8,11,13 | 34 | u*G*gucuUucacUuUcUugg*g*c | 2,7,12,14,16 | 35 |
| AD00555 | (GLS-15)*(Invab)*gcccaagaaAGuGaaagacca*(Invab) | 9,11,12 | 36 | u*G*gucuUucacUuUcuuGg*g*c | 2,7,12,14,18 | 37 |
| AD00556 | (GLS-15)*(Invab)*gcccaagaaAGuGaaagacca*(Invab) | 9,11,12 | 38 | u*G*guCuuucacUuUcuuGg*g*c | 2,5,12,14,18 | 39 |
| AD00558 | (GLS-15)*(Invab)*gcccaagaaAGuGaaagacca*(Invab) | 9,11,12 | 40 | u*G*gucuUucAcuuUcUugg*g*c | 2,7,10,14,16 | 41 |
| AD00698 | (GLS-15)*(Invab)*aacucaAuAAAgugcuuuga*a*(Invab) | 11,12,13,15 | 42 | u*U*caaAgcacuuuAuUgag*u*u | 2,6,14,16 | 43 |
| AD00699 | (GLS-15)*(Invab)*aacucaauAaAgUgcuuuga*a*(Invab) | 9,11,13 | 44 | u*U*caaaGcacuUuAuUgag*u*u | 2,7,12,14,16 | 45 |
| AD00700 | (GLS-15)*(Invab)*aacucaauAaAguGecuuuga*a*(Invab) | 8,11,13 | 46 | u*U*caaaGcacuUuAuUgag*u*u | 2,7,12,14,16 | 47 |
| AD00716 | (GLS-15)*(Invab)*gcccaaGaAAGugaaagacc*a*(Invab) | 11,12,13,15 | 48 | u*G*gucUuucacuuUcUugg*g*c | 2,6,14,16 | 49 |
| AD00722 | (GLS-15)*(Invab)*gcccaagaAaGugAaagacc*a*(Invab) | 8,11,13 | 50 | u*G*gucuuucacuuUcUugg*g*c | 2,14,16 | 51 |
| AD00723 | (GLS-15)*(Invab)*gcccaagaAaGugAaagacc*a*(Invab) | 8,11,13 | 52 | u*G*gucuuucacuuUcuuGg*g*c | 2,14,18 | 53 |
| AD00808 | (GLS-15)*(Invab)*aacucaauAaAgUgcuuuga*a*(Invab) | 9,11,13 | 54 | u*U*caAagcacuUuAuugAg*u*u | 2,5,12,14,18 | 55 |
| AD00810 | (GLS-15)*(Invab)*gcccaagaAaGuGaaagacc*a*(Invab) | 9,11,13 | 56 | u*G*guCuuucacUuUcuuGg*g*c | 2,5,12,14,18 | 57 |
| AD00813 | (GLS-15)*(Invab)*gcccaagaAaGugAaagacc*a*(Invab) | 8,11,13 | 58 | u*G*guCuuucacUuUcuuGg*g*c | 2,5,12,14,18 | 59 |
| AD00864 | (GLS-15)*(Invab)*gcccaagaAaGuGaAagacc*a*(Invab) | 7, 9,11,13 | 60 | u*G*gucuUucacUuUcUugg*g*c | 2,7,12,14,16 | 61 |
| AD00865 | (GLS-15)*(Invab)*gcccaaGaAaGuGaAagacc*a*(Invab) | 7, 9,11,13,15 | 62 | u*G*gucuUucacUuUcUugg*g*c | 2,7,12,14,16 | 63 |
| AD00866 | (GLS-15)*(Invab)*gcccaagaAAGugaaaGacc*a*(Invab) | 5, 11,12, 13 | 64 | u*G*gucuUucacUuUcUugg*g*c | 2,7,12,14,16 | 65 |
| AD00867 | (GLS-15)*(Invab)*gcccAaGaAAGugaaaGacc*a*(Invab) | 5, 11,12,13,15, 17 | 66 | u*G*gucuUucacUuUcUugg*g*c | 2,7,12,14,16 | 67 |
| AD00868 | (GLS-15)*(Invab)*gcccaaGaAAGugaaAgaCc*a*(Invab) | 3, 6, 11,12,13,15 | 68 | u*G*gucuUucacUuUcUugg*g*c | 2,7,12,14,16 | 69 |
| AD00869 | (GLS-15)*(Invab)*gcccaagaaAGuGaaagacc*a*(Invab) | 9,11,12 | 70 | u*G*gucuUucacUuUcUugg*g*c | 2,7,12,14,16 | 71 |
| AD00870 | (GLS-15)*(Invab)*gcccaagaAaGuGaaagacc*a*(Invab) | 9,11,13 | 72 | u*G*gucuUucaCuuUcUugg*g*c | 2,7,11,14,16 | 73 |
| AD00871 | (GLS-15)*(Invab)*gcccaagaAaGuGaaagacc*a*(Invab) | 9,11,13 | 74 | u*G*gucuUucAcuuUcUugg*g*c | 2,7,10,14,16 | 75 |
| AD00872 | (GLS-15)*(Invab)*gcccaagaAaGuGaaagacc*a*(Invab) | 9,11,13 | 76 | u*G*gucuUucacuUUcUugg*g*c | 2,7,13,14,16 | 77 |
| AD00928 | (GLS-15)*(Invab)*aacucaauAaAgUgCuuuga*a*(Invab) | 7, 9,11,13 | 78 | u*U*caaaGcacuUuAuUgag*u*u | 2,7,12,14,16 | 79 |
| AD00929 | (GLS-15)*(Invab)*aacucaAuAaAgUgCuuuga*a*(Invab) | 7, 9,11,13,15 | 80 | u*U*caaaGcacuUuAuUgag*u*u | 2,7,12,14,16 | 81 |
| AD00930 | (GLS-15)*(Invab)*aacucaauAAAgugcuUuga*a*(Invab) | 5, 11,12, 13 | 82 | u*U*caaaGcacuUuAuUgag*u*u | 2,7,12,14,16 | 83 |
| AD00931 | (GLS-15)*(Invab)*aacuCaAuAAAgugcuUuga*a*(Invab) | 5, 11,12,13,15, 17 | 84 | u*U*caaaGcacuUuAuUgag*u*u | 2,7,12,14,16 | 85 |
| AD00932 | (GLS-15)*(Invab)*aacucaAuAAAgugcUuuGa*a*(Invab) | 3, 6, 11,12,13,15 | 86 | u*U*caaaGcacuUuAuUgag*u*u | 2,7,12,14,16 | 87 |
| AD00933 | (GLS-15)*(Invab)*aacucaauaAAgUgcuuuga*a*(Invab) | 9,11,12 | 88 | u*U*caaaGcacuUuAuUgag*u*u | 2,7,12,14,16 | 89 |
| AD00934 | (GLS-15)*(Invab)*aacucaauAaAgUgcuuuga*a*(Invab) | 9,11,13 | 90 | u*U*caaaGcacUuuAuUgag*u*u | 2,7,11,14,16 | 91 |
| AD00935 | (GLS-15)*(Invab)*aacucaauAaAgUgcuuuga*a*(Invab) | 9,11,13 | 92 | u*U*caaaGeaCuuuAuUgag*u*u | 2,7,10,14,16 | 93 |
| AD00936 | (GLS-15)*(Invab)*aacucaauAaAgUgcuuuga*a*(Invab) | 9,11,13 | 94 | u*U*caAagcacuUuAuUgag*u*u | 2,5,12,14,16 | 95 |
| AD00937 | (GLS-15)*(Invab)*aacucaauAaAgUgcuuuga*a*(Invab) | 9,11,13 | 96 | u*U*caAagcacUuuAuUgag*u*u | 2,5,11,14,16 | 97 |
| AD00938 | (GLS-15)*(Invab)*aacucaauAaAgUgcuuuga*a*(Invab) | 9,11,13 | 98 | u*U*caAagcaCuuuAuUgag*u*u | 2,5,10,14,16 | 99 |
| AD00939 | (GLS-15)*(Invab)*aacucaauAaAguGcuuuga*a*(Invab) | 8,11,13 | 100 | u*U*caaaGcacUuuAuUgag*u*u | 2,7,11,14,16 | 101 |
| AD00940 | (GLS-15)*(Invab)*aacucaauAaAguGcuuuga*a*(Invab) | 8,11,13 | 102 | u*U*caaaGeaCuuuAuUgag*u*u | 2,7,10,14,16 | 103 |
| AD00941 | (GLS-15)*(Invab)*aacucaauAaAguGcuuuga*a*(Invab) | 8,11,13 | 104 | u*U*caAagcacuUuAuUgag*u*u | 2,5,12,14,16 | 105 |
| AD00942 | (GLS-15)*(Invab)*aacucaauAaAguGcuuuga*a*(Invab) | 8,11,13 | 106 | u*U*caAagcacUuuAuUgag*u*u | 2,5,11,14,16 | 107 |
| AD00943 | (GLS-15)*(Invab)*aacucaauAaAguGcuuuga*a*(Invab) | 8,11,13 | 108 | u*U*caAagcaCuuuAuUgag*u*u | 2,5,10,14,16 | 109 |
| AD00933 | (GLS-15)*(Invab)*aacucaauaAAgUgcuuuga*a*(Invab) | 9,11,12 | 110 | u*U*caaaGcacuUuAuUgag*u*u | 2,7,12,14,16 | 111 |
| AD00934 | (GLS-15)*(Invab)*aacucaauAaAgUgcuuuga*a*(Invab) | 9,11,13 | 112 | u*U*caaaGcacUuuAuUgag*u*u | 2,7,11,14,16 | 113 |
| AD00935 | (GLS-15)*(Invab)*aacucaauAaAgUgcuuuga*a*(Invab) | 9,11,13 | 114 | u*U*caaaGeaCuuuAuUgag*u*u | 2,7,10,14,16 | 115 |
| AD00936 | (GLS-15)*(Invab)*aacucaauAaAgUgcuuuga*a*(Invab) | 9,11,13 | 116 | u*U*caAagcacuUuAuUgag*u*u | 2,5,12,14,16 | 117 |
| AD01029 | (GLS-15)*(Invab)*aacucaauaAAgUgcuuuga*a*(Invab) | 9,11,12 | 118 | u*U*caaaGcacUuuAuUgag*u*u | 2,7,11,14,16 | 119 |
| AD01030 | (GLS-15)*(Invab)*aacucaauAaAgUgcuuuga*a*(Invab) | 9,11,13 | 120 | u*U*caaaGcacuuuAuUgag*u*u | 2, 7, 14, 16 | 121 |
| AD01031 | (GLS-15)*(Invab)*aacucaauAaAgUgcuuuga*a*(Invab) | 9,11,13 | 122 | u*U*caaaGcacuUuAuugAg*u*u | 2,7,12,14,18 | 123 |
| AD01032 | (GLS-15)*(Invab)*aacucaauAaAgUgcuuuga*a*(Invab) | 9,11,13 | 124 | u*U*caaaGcacUuuAuugAg*u*u | 2,7,11,14,18 | 125 |
| AD01033 | (GLS-15)*(Invab)*aacucaauAaAgUgcuuuga*a*(Invab) | 9,11,13 | 126 | u*U*cAaagcacUuuAuUgag*u*u | 2,4,11,14,16 | 127 |
| AD01034 | (GLS-15)*(Invab)*aacucaauAaAgUgcuuuga*a*(Invab) | 9,11,13 | 128 | u*U*caaagcacuUuAuUgAg*u*u | 2,12,14,16,18 | 129 |
| AD01035 | (GLS-15)*(Invab)*aacucaauAaAgUgcuuuga*a*(Invab) | 9,11,13 | 130 | u*U*caaagcacUuuAuUgAg*u*u | 2,11,14,16,18 | 131 |
| AD01036 | (GLS-15)*(Invab)*aacucaauAaAgUgcuuuga*a*(Invab) | 9,11,13 | 132 | u*U*cAaaGcacuuuAuUgag*u*u | 2, 4, 7, 14, 16 | 133 |
| AD01037 | (GLS-15)*(Invab)*aacucaauAaAgUgcuuuga*a*(Invab) | 9,11,13 | 134 | u*U*caaaGcacuuuAuUgAg*u*u | 2, 7, 14, 16,18 | 135 |
| AD01221 | (GLS-15)*(Invab)*gaaacuCaAUAaagugcuuu*a*(Invab) | 11,12,13,15 | 136 | u*A*aagCacuuuauUgAguu*u*c | 2,6,14,16 | 137 |
| AD01222 | (GLS-15)*(Invab)*gaaacucaAuAaAgugcuuu*a*(Invab) | 9,11,13 | 138 | u*A*aagcAcuuuAuUgAguu*u*c | 2,7,12,14,16 | 139 |
| AD01223 | (GLS-15)*(Invab)*gaaacucaAuAaaGugcuuu*a*(Invab) | 8,11,13 | 140 | u*A*aagcAcuuuAuUgAguu*u*c | 2,7,12,14,16 | 141 |
| AD01224 | (GLS-15)*(Invab)*gaaacucaaUAaAgugcuuu*a*(Invab) | 9,11,12 | 142 | u*A*aagcAcuuuAuUgAguu*u*c | 2,7,12,14,16 | 143 |
| AD01225 | (GLS-15)*(Invab)*gaaacucaAuAaAgugcuuu*a*(Invab) | 9,11,13 | 144 | u*A*aagcAcuuUauUgAguu*u*c | 2,7,11,14,16 | 145 |
| AD01226 | (GLS-15)*(Invab)*gaaacucaAuAaAgugcuuu*a*(Invab) | 9,11,13 | 146 | u*A*aagcAcuUuauUgAguu*u*c | 2,7,10,14,16 | 147 |
| AD01227 | (GLS-15)*(Invab)*gaaacucaAuAaAgugcuuu*a*(Invab) | 9,11,13 | 148 | u*A*aaGcacuuuAuUgAguu*u*c | 2,5,12,14,16 | 149 |
| AD01228 | (GLS-15)*(Invab)*gaaacucaaUAaAgugcuuu*a*(Invab) | 9,11,12 | 150 | u*A*aagcAcuuuAuUgagUu*u*c | 2,7,12,14,18 | 151 |
| AD01229 | (GLS-15)*(Invab)*gaaacucaaUAaAgugcuuu*a*(Invab) | 9,11,12 | 152 | u*A*aagcAcuUuauUgAguu*u*c | 2,7,10,14,16 | 153 |
| AD01230 | (GLS-15)*(Invab)*gaaacucaaUAaAgugcuuu*a*(Invab) | 9,11,12 | 154 | u*A*aagcAcuuUauUgAguu*u*c | 2,7,11,14,16 | 155 |
| AD01231 | (GLS-15)*(Invab)*gaaacucaAuAaAgugcuuu*a*(Invab) | 9,11,13 | 156 | u*A*aagcAcuuuauUgAguu*u*c | 2, 7, 14, 16 | 157 |
| AD01232 | (GLS-15)*(Invab)*gaaacucaAuAaAgugcuuu*a*(Invab) | 9,11,13 | 158 | u*A*aagcAcuuuAuUgagUu*u*c | 2,7,12,14,18 | 159 |
| AD01233 | (GLS-15)*(Invab)*gaaacucaAuAaAgugcuuu*a*(Invab) | 9,11,13 | 160 | u*A*aagcAcuuUauUgagUu*u*c | 2,7,11,14,18 | 161 |
| AD01234 | (GLS-15)*(Invab)*gaaacucaAuAaAgugcuuu*a*(Invab) | 9,11,13 | 162 | u*A*aAgcacuuUauUgAguu*u*c | 2,4,11,14,16 | 163 |
| AD01235 | (GLS-15)*(Invab)*gaaacucaAuAaAgugcuuu*a*(Invab) | 9,11,13 | 164 | u*A*aagcacuuuAuUgAgUu*u*c | 2,12,14,16,18 | 165 |
| AD01236 | (GLS-15)*(Invab)*gaaacucaAuAaAgugcuuu*a*(Invab) | 9,11,13 | 166 | u*A*aagcacuuUauUgAgUu*u*c | 2,11,14,16,18 | 167 |
| AD01237 | (GLS-15)*(Invab)*gaaacucaAuAaAgugcuuu*a*(Invab) | 9,11,13 | 168 | u*A*aAgcAcuuuauUgAguu*u*c | 2,4,7,14,16 | 169 |
| AD01238 | (GLS-15)*(Invab)*gaaacucaAuAaAgugcuuu*a*(Invab) | 9,11,13 | 170 | u*A*aagcAcuuuauUgAgUu*u*c | 2, 7, 14, 16,18 | 171 |
| AD01221 | (GLS-15)*(Invab)*gaaacuCaAUAaagugcuuu*a*(Invab) | 11,12,13,15 | 172 | u*A*aagCacuuuauUgAguu*u*c | 2,6,14,16 | 173 |
| AD01221 | (GLS-15)*(Invab)*gaaacuCaAUAaagugcuuu*a*(Invab) | 11,12,13,15 | 174 | u*A*aagCacuuuauUgAguu*u*c | 2,6,14,16 | 175 |
| AD01222 | (GLS-15)*(Invab)*gaaacucaAuAaAgugcuuu*a*(Invab) | 9,11,13 | 176 | u*A*aagcAcuuuAuUgAguu*u*c | 2,7,12,14,16 | 177 |
| AD01222 | (GLS-15)*(Invab)*gaaacucaAuAaAgugcuuu*a*(Invab) | 9,11,13 | 178 | u*A*aagcAcuuuAuUgAguu*u*c | 2,7,12,14,16 | 179 |
| AD01223 | (GLS-15)*(Invab)*gaaacucaAuAaaGugcuuu*a*(Invab) | 8,11,13 | 180 | u*A*aagcAcuuuAuUgAguu*u*c | 2,7,12,14,16 | 181 |
| AD01224 | (GLS-15)*(Invab)*gaaacucaaUAaAgugcuuu*a*(Invab) | 9,11,12 | 182 | u*A*aagcAcuuuAuUgAguu*u*c | 2,7,12,14,16 | 183 |
| AD01225 | (GLS-15)*(Invab)*gaaacucaAuAaAgugcuuu*a*(Invab) | 9,11,13 | 184 | u*A*aagcAcuuUauUgAguu*u*c | 2,7,11,14,16 | 185 |
| AD01227 | (GLS-15)*(Invab)*gaaacucaAuAaAgugcuuu*a*(Invab) | 9,11,13 | 186 | u*A*aaGcacuuuAuUgAguu*u*c | 2,5,12,14,16 | 187 |
| AD01230 | (GLS-15)*(Invab)*gaaacucaaUAaAgugcuuu*a*(Invab) | 9,11,12 | 188 | u*A*aagcAcuuUauUgAguu*u*c | 2,7,11,14,16 | 189 |
| AD00699 | (GLS-15)*(Invab)*aacucaauAaAgUgcuuuga*a*(Invab) | 9,11,13 | 190 | u*U*caaaGcacuUuAuUgag*u*u | 2,7,12,14,16 | 191 |
| AD01987 | ((GLS-15))*(Invab)*aacucaauAaAgUgcuuuga*a*(Invab) | 9,11,13 | 192 | u*U*caaagCacuUuAuUgag*u*u | 2,8,12,14,16 | 193 |
| AD01988 | ((GLS-15))*(Invab)*aacucaauAaAgUgcuuuga*a*(Invab) | 9,11,13 | 194 | u*U*caaagcAcuUuAuUgag*u*u | 2,9,12,14,16 | 195 |
| AD00699 | (GLS-15)*(Invab)*aacucaauAaAgUgcuuuga*a*(Invab) | 9,11,13 | 196 | u*U*caaaGcacuUuAuUgag*u*u | 2,7,12,14,16 | 197 |
| AD01987 | ((GLS-15))*(Invab)*aacucaauAaAgUgcuuuga*a*(Invab) | 9,11,13 | 198 | u*U*caaagCacuUuAuUgag*u*u | 2,8,12,14,16 | 199 |
| AD01988 | ((GLS-15))*(Invab)*aacucaauAaAgUgcuuuga*a*(Invab) | 9,11,13 | 200 | u*U*caaagcAcuUuAuUgag*u*u | 2,9,12,14,16 | 201 |

In some embodiments, SEQ ID NO: 1 is FXII mRNA [NCBI reference sequence: NM_021489.3. For example, AD00540 and AD00548 are the specific modified nucleotide sequences of the unmodified duplexes AD00127.um with a sense strand of AACUCAAUAAAGUGCUUUGAA (SEQ ID NO: 2) and an antisense strand of UUCAAAGCACUUUAUUGAGUU (SEQ ID NO: 3), where the complementary Pos(20) is at position 1938 in NM_021489.3. For example, AD00549 and AD00558 are the specific modified nucleotide sequences of the unmodified duplex AD00549.um with a sense strand of GCCCAAGAAAGUGAAAGACCA (SEQ ID NO: 4) and an antisense strand of UGGUCUUUCACUUUCLTUGGGC (SEQ ID NO: 5), where the complementary Pos(20) is at position 307 in NM_021489.3.

In another aspect of the present invention, an RNAi agent for inhibiting expression of a target gene is provided, wherein the RNAi agent is double-stranded ribonucleic acid (dsRNA) agent comprising a sense strand and an antisense strand complementary to at least a portion of an mRNA corresponding to the target gene, wherein the antisense strand has 18-30 nucleotides, the sense strand has 18-40 nucleotides, and the dsRNA agent comprises an antisense strand represented by formula (X) and a sense strand represented by formula (V).

The antisense strand comprises a sequence represented by formula (X) in the direction of 3'-5':

3'-(N_{X})ₙ Nx Nx Nx N_{X} N_{F} N_{X} N_{X} N_{X} N_{X} N_{X} N_{X} N_{X} N_{X} N_{X}N_{X} N_{X} N_{F} N_{X}-5' formula (X)

The sense strand comprises a sequence represented by formula (V) in the direction of 5'-3':

5'-(N'_{L})_{n'} N'_{L}N'_{L} N'_{L} N'_{N1} N' _{N2} N' _{N3} N' _{N4} N'_{F} N'_{L} N' _{N5}N' _{N6} N'_{L} N'_{L} N'_{L} N'_{L} N'_{L} N'_{L} N'_{L}-3' formula (V)

wherein, each N_{F} represents a 2'-fluoro modified nucleotide;
each N_{X} independently represents a modified or an unmodified nucleotide, wherein no more than three N_{X} are 2'-fluoro modified nucleotides;
each N'_{F} represents a 2'-fluoro modified nucleotide;
N'_{N1}, N'_{N2}, N'_{N3}, N'_{N4}, N'_{N5}, and N'_{N6} independently represent a modified or an unmodified nucleotide, wherein at least two of N'_{N1}, N'_{N2}, N'_{N3}, N'_{N4}, N'_{N5}, and N'_{N6} are 2'-fluoro modified nucleotides;
the sense strand represented by formula (V) has no motif of three or more contiguous 2'-fluoro modified nucleotides and has no more than six 2'-fluoro modified nucleotides;
each N'_{L} independently represents a modified or an unmodified nucleotide;
each n and n' can independently be an integer from 0 to 7.

In some embodiments, an RNAi agent for inhibiting expression of a target gene is provided, wherein the RNAi agent is double-stranded ribonucleic acid (dsRNA) agent comprising a sense strand and an antisense strand complementary to at least a portion of an mRNA corresponding to the target gene, wherein the antisense strand has 18-30 nucleotides, the sense strand has 18-40 nucleotides, and the dsRNA agent comprises an antisense strand represented by formula (I) and a sense strand represented by formula (V).

The antisense strand comprises a sequence represented by formula (I) in the direction of 3'-5':

3'-(N_{X})ₙ Nx Nx Nx N_{X} N_{F} N_{X} N_{X} N_{X} N_{X} N_{X} N_{X} N_{X} N_{X} N_{X}N_{X} N_{X} N_{F} N_{X}-5' formula (I)

The sense strand comprises a sequence represented by formula (V) in the direction of 5'-3':

5'-(N'_{L})_{n'} N' _{L}N'_{L}N'_{L}N'_{N1} N' _{N2} N' _{N3} N' _{N4} N'_{F} N'_{L}N' _{N5}N' _{N6} N'_{L} N'_{L}N'_{L}N'_{L}N'_{L}N'_{L} N'_{L}-3' formula (V)

wherein, each N_{F} represents a 2'-fluoro modified nucleotide;
each N_{X} independently represents a modified or an unmodified nucleotide, wherein only three N_{X} are 2'-fluoro modified nucleotides, or only one N_{X} is a 2'-fluoro modified nucleotide;
each N'_{F} represents a 2'-fluoro modified nucleotide;
N'_{N1}, N'_{N2}, N'_{N3}, N'_{N4}, N'_{N5}, and N'_{N6} independently represent a modified or an unmodified nucleotide, wherein at least two of N'_{N1}, N'_{N2}, N'_{N3}, N'_{N4}, N'_{N5}, and N'_{N6} are 2'-fluoro modified nucleotides;
the sense strand represented by formula (V) has no motif of three or more contiguous 2'-fluoro modified nucleotides and has no more than six 2'-fluoro modified nucleotides;
each N'_{L} independently represents a modified or an unmodified nucleotide;
each n and n' can independently be an integer from 0 to 7.

It can be understood that the dsRNA agent comprises combinations of an antisense strand having any modification described in formula (I) herein and a sense strand having any modification described in formula (V) herein.

In some embodiments, an RNAi agent for inhibiting expression of a target gene is provided, wherein the RNAi agent is double-stranded ribonucleic acid (dsRNA) agent comprising a sense strand and an antisense strand complementary to at least a portion of an mRNA corresponding to the target gene, wherein the antisense strand has 18-30 nucleotides, the sense strand has 18-40 nucleotides, and the dsRNA agent comprises an antisense strand represented by formula (I-1) and a sense strand represented by formula (V);
the antisense strand comprises a sequence represented by formula (I-1) in the direction of 3'-5':

3'-(N_{L})ₙ N_{M1} N_{L} N_{M2} N_{L} N_{F} N_{L} N_{M3} N_{L} N_{M4} N_{L} N_{M5} N_{M6} N_{L} N_{M7} N_{M8} N_{L} N_{F} N_{L}-5' formula (I-1)

the sense strand comprises a sequence represented by formula (V) in the direction of 5'-3':

5'-(N'_{L})_{n'} N' _{L}N'_{L}N'_{L}N'_{N1} N' _{N2} N' _{N3} N' _{N4} N'_{F} N'_{L}N' _{N5}N' _{N6} N'_{L} N'_{L}N'_{L}N'_{L}N'_{L}N'_{L} N'_{L}-3' formula (V)

wherein, each N_{F} represents a 2'-fluoro modified nucleotide;
N_{M1}, N_{M2}, N_{M3}, N_{M4}, N_{M5}, N_{M6}, N_{M7}, and N_{M8} independently represent a modified or an unmodified nucleotide, wherein only three of N_{M1}, N_{M2}, N_{M3}, N_{M4}, N_{M5}, N_{M6}, N_{M7}, and N_{M8} are 2'-fluoro modified nucleotides, or only one of N_{M1}, N_{M2}, N_{M3}, N_{M4}, N_{M5}, N_{M6}, N_{M7}, and N_{M8} is a 2'-fluoro modified nucleotide;
each N_{L} independently represents a modified or an unmodified nucleotide, wherein the modified nucleotide is not a 2'-fluoro modified nucleotide;
each N'_{F} represents a 2'-fluoro modified nucleotide;
N'_{N1}, N'_{N2}, N'_{N3}, N'_{N4}, N'_{N5}, and N'_{N6} independently represent a modified or an unmodified nucleotide, wherein at least two of N'_{N1}, N'_{N2}, N'_{N3}, N'_{N4}, N'_{N5}, and N'_{N6} are 2'-fluoro modified nucleotides;
the sense strand represented by formula (V) has no motif of three or more contiguous 2'-fluoro modified nucleotides and has no more than six 2'-fluoro modified nucleotides;
each N'_{L} independently represents a modified or an unmodified nucleotide;
each n and n' can independently be an integer from 0 to 7.

In some embodiments, an RNAi agent for inhibiting expression of a target gene is provided, wherein the RNAi agent is double-stranded ribonucleic acid (dsRNA) agent comprising a sense strand and an antisense strand complementary to at least a portion of an mRNA corresponding to the target gene, wherein the antisense strand has 18-30 nucleotides, the sense strand has 18-40 nucleotides, and the dsRNA agent comprises an antisense strand represented by formula (I-1) and a sense strand represented by formula (V');
the antisense strand comprises a sequence represented by formula (I-1) in the direction of 3'-5':

3'-(N_{L})ₙ N_{M1} N_{L} N_{M2} N_{L} N_{F} N_{L} N_{M3} N_{L} N_{M4} N_{L} N_{M5} N_{M6} N_{L} N_{M7} N_{M8} N_{L} N_{F} N_{L}-5' formula (I-1)

the sense strand comprises a sequence represented by formula (V') in the direction of 5'-3':

5'-(N'_{L})_{n'} N'_{L} N'_{L} N'_{L} N'_{N1} N'_{N2} N'_{N3} N' _{N4} N'_{F} N'_{L} N'_{N5}N' _{N6} N'_{L} N'_{L} N'_{L} N'_{L} N'_{L} N'_{L} N'_{L}-3' formula (V')

wherein, each N_{F} represents a 2'-fluoro modified nucleotide;
N_{M1}, N_{M2}, N_{M3}, N_{M4}, N_{M5}, N_{M6}, N_{M7}, and N_{M8} independently represent a modified or an unmodified nucleotide, wherein only three of N_{M1}, N_{M2}, N_{M3}, N_{M4}, N_{M5}, N_{M6}, N_{M7}, and N_{M8} are 2'-fluoro modified nucleotides, or only one of N_{M1}, N_{M2}, N_{M3}, N_{M4}, N_{M5}, N_{M6}, N_{M7}, and N_{M8} is a 2'-fluoro modified nucleotide;
each N_{L} independently represents a modified or an unmodified nucleotide, wherein the modified nucleotide is not a 2'-fluoro modified nucleotide;
each N'_{F} represents a 2'-fluoro modified nucleotide;
N'_{N1}, N'_{N2}, N'_{N3}, N'_{N4}, N'_{N5}, and N'_{N6} independently represent a modified or an unmodified nucleotide, wherein at least two of N'_{N1}, N'_{N2}, N'_{N3}, N'_{N4}, N'_{N5}, and N'_{N6} are 2'-fluoro modified nucleotides;
each N'_{L} independently represents a modified or an unmodified nucleotide, wherein the modified nucleotide is not a 2'-fluoro modified nucleotide;
each n and n' can independently be an integer from 0 to 7.

In some embodiments, the dsRNA agent comprises an antisense strand with the nucleotide sequence represented by formula (II) herein and a sense strand with the nucleotide sequence represented by formula (V) herein.

In some embodiments, the dsRNA agent comprises an antisense strand with the nucleotide sequence represented by formula (III) herein and a sense strand with the nucleotide sequence represented by formula (V) herein.

In some embodiments, the dsRNA agent comprises an antisense strand with the nucleotide sequence represented by formula (IV) herein and a sense strand with the nucleotide sequence represented by formula (V) herein.

In some embodiments, the dsRNA agent comprises an antisense strand with the nucleotide sequence represented by formula (II) herein and a sense strand with the nucleotide sequence represented by formula (V') herein.

In some embodiments, the dsRNA agent comprises an antisense strand with the nucleotide sequence represented by formula (III) herein and a sense strand with the nucleotide sequence represented by formula (V') herein.

In some embodiments, the dsRNA agent comprises an antisense strand with the nucleotide sequence represented by formula (IV) herein and a sense strand with the nucleotide sequence represented by formula (V') herein.

In some more specific embodiments, the dsRNA agent has 2'-fluoro modified nucleotides at positions 2, 7, 12, 14, and 16 of the antisense strand represented by formula (I-1) counting from the first paired nucleotide at 5' end, and has 2'-fluoro modified nucleotides at positions 9, 11, and 13 of the sense strand represented by formula (V) counting from the first paired nucleotide at 3' end of the sense strand, and the sense strand has no motif of three or more contiguous 2'-fluoro modified nucleotides and has no more than six 2'-fluoro modified nucleotides. In some more specific embodiments, the dsRNA agent has 2'-fluoro modified nucleotides at positions 2, 7, 12, 14, and 16 of the antisense strand represented by formula (I-1) counting from the first paired nucleotide at 5' end, and has 2'-fluoro modified nucleotides at positions 9, 11, and 13 of the sense strand represented by formula (V') counting from the first paired nucleotide at 3' end of the sense strand, and it can be understood that the sense strand represented by formula (V') has no 2'-fluoro modified nucleotides at other positions.

In some more specific embodiments, the dsRNA agent has 2'-fluoro modified nucleotides at positions 2, 7, 12, 14, and 16 of the antisense strand represented by formula (I-1) counting from the first paired nucleotide at 5' end, and has 2'-fluoro modified nucleotides at positions 9, 11, and 12 of the sense strand represented by formula (V) counting from the first paired nucleotide at 3' end of the sense strand, and the sense strand has no motif of three or more contiguous 2'-fluoro modified nucleotides and has no more than six 2'-fluoro modified nucleotides. In some more specific embodiments, the dsRNA agent has 2'-fluoro modified nucleotides at positions 2, 7, 12, 14, and 16 of the antisense strand represented by formula (I-1) counting from the first paired nucleotide at 5' end, and has 2'-fluoro modified nucleotides at positions 9, 11, and 12 of the sense strand represented by formula (V') counting from the first paired nucleotide at 3' end of the sense strand, and it can be understood that the sense strand represented by formula (V') has no 2'-fluoro modified nucleotides at other positions.

In some more specific embodiments, the dsRNA agent has 2'-fluoro modified nucleotides at positions 2, 7, 12, 14, and 16 of the antisense strand represented by formula (I-1) counting from the first paired nucleotide at 5' end, and has 2'-fluoro modified nucleotides at positions 8, 11, and 13 of the sense strand represented by formula (V) counting from the first paired nucleotide at 3' end of the sense strand, and the sense strand has no motif of three or more contiguous 2'-fluoro modified nucleotides and has no more than six 2'-fluoro modified nucleotides. In some more specific embodiments, the dsRNA agent has 2'-fluoro modified nucleotides at positions 2, 7, 12, 14, and 16 of the antisense strand represented by formula (I-1) counting from the first paired nucleotide at 5' end, and has 2'-fluoro modified nucleotides at positions 8, 11, and 13 of the sense strand represented by formula (V') counting from the first paired nucleotide at 3' end of the sense strand, and it can be understood that the sense strand represented by formula (V') has no 2'-fluoro modified nucleotides at other positions.

In some more specific embodiments, the dsRNA agent has 2'-fluoro modified nucleotides at positions 2, 7, 12, 14, and 16 of the antisense strand represented by formula (I-1) counting from the first paired nucleotide at 5' end, and has 2'-fluoro modified nucleotides at positions 11, 12, and 14 of the sense strand represented by formula (V) counting from the first paired nucleotide at 3' end of the sense strand.

In some more specific embodiments, the dsRNA agent has 2'-fluoro modified nucleotides at positions 2, 7, 12, 14, and 16 of the antisense strand represented by formula (I-1) counting from the first paired nucleotide at 5' end, and has 2'-fluoro modified nucleotides at positions 11, 12, and 15 of the sense strand represented by formula (V) counting from the first paired nucleotide at 3' end of the sense strand.

In some more specific embodiments, the dsRNA agent has 2'-fluoro modified nucleotides at positions 2, 5, 12, 14, and 18 of the antisense strand represented by formula (I-1) counting from the first paired nucleotide at 5' end, and has 2'-fluoro modified nucleotides at positions 9, 11, and 13 of the sense strand represented by formula (V) counting from the first paired nucleotide at 3' end of the sense strand. In some more specific embodiments, the dsRNA agent has 2'-fluoro modified nucleotides at positions 2, 5, 12, 14, and 18 of the antisense strand represented by formula (I-1) counting from the first paired nucleotide at 5' end, and has 2'-fluoro modified nucleotides at positions 9, 11, and 13 of the sense strand represented by formula (V') counting from the first paired nucleotide at 3' end of the sense strand.

In some more specific embodiments, the dsRNA agent has 2'-fluoro modified nucleotides at positions 2, 5, 12, 14, and 18 of the antisense strand represented by formula (I-1) counting from the first paired nucleotide at 5' end, and has 2'-fluoro modified nucleotides at positions 9, 11, and 13 of the sense strand represented by formula (V) counting from the first paired nucleotide at 3' end of the sense strand. In some more specific embodiments, the dsRNA agent has 2'-fluoro modified nucleotides at positions 2, 5, 12, 14, and 18 of the antisense strand represented by formula (I-1) counting from the first paired nucleotide at 5' end, and has 2'-fluoro modified nucleotides at positions 9, 11, and 13 of the sense strand represented by formula (V') counting from the first paired nucleotide at 3' end of the sense strand.

In some more specific embodiments, the dsRNA agent has 2'-fluoro modified nucleotides at positions 2, 5, 12, 14, and 18 of the antisense strand represented by formula (I-1) counting from the first paired nucleotide at 5' end, and has 2'-fluoro modified nucleotides at positions 9, 11, and 12 of the sense strand represented by formula (V) counting from the first paired nucleotide at 3' end of the sense strand.

In some more specific embodiments, the dsRNA agent has 2'-fluoro modified nucleotides at positions 2, 5, 12, 14, and 18 of the antisense strand represented by formula (I-1) counting from the first paired nucleotide at 5' end, and has 2'-fluoro modified nucleotides at positions 8, 11, and 13 of the sense strand represented by formula (V) counting from the first paired nucleotide at 3' end of the sense strand.

In some more specific embodiments, the dsRNA agent has 2'-fluoro modified nucleotides at positions 2, 5, 12, 14, and 18 of the antisense strand represented by formula (I-1) counting from the first paired nucleotide at 5' end, and has 2'-fluoro modified nucleotides at positions 8, 11, and 13 of the sense strand represented by formula (V') counting from the first paired nucleotide at 3' end of the sense strand.

In some more specific embodiments, the dsRNA agent has 2'-fluoro modified nucleotides at positions 2, 5, 12, 14, and 18 of the antisense strand represented by formula (I-1) counting from the first paired nucleotide at 5' end, and has 2'-fluoro modified nucleotides at positions 11, 12, and 14 of the sense strand represented by formula (V) counting from the first paired nucleotide at 3' end of the sense strand.

In some more specific embodiments, the dsRNA agent has 2'-fluoro modified nucleotides at positions 2, 5, 12, 14, and 18 of the antisense strand represented by formula (I-1) counting from the first paired nucleotide at 5' end, and has 2'-fluoro modified nucleotides at positions 11, 12, and 15 of the sense strand represented by formula (V) counting from the first paired nucleotide at 3' end of the sense strand.

In some more specific embodiments, the dsRNA agent has 2'-fluoro modified nucleotides at positions 2, 7, 12, 14, and 18 of the antisense strand represented by formula (I-1) counting from the first paired nucleotide at 5' end, and has 2'-fluoro modified nucleotides at positions 9, 11, and 12 of the sense strand represented by formula (V) counting from the first paired nucleotide at 3' end of the sense strand.

In some more specific embodiments, the dsRNA agent has 2'-fluoro modified nucleotides at positions 2, 4, 10, 14, and 16 of the antisense strand represented by formula (I-1) counting from the first paired nucleotide at 5' end, and has 2'-fluoro modified nucleotides at positions 9, 11, and 12 of the sense strand represented by formula (V) counting from the first paired nucleotide at 3' end of the sense strand.

In some more specific embodiments, the dsRNA agent has 2'-fluoro modified nucleotides at positions 2, 7, 10, 14, and 16 of the antisense strand represented by formula (I-1) counting from the first paired nucleotide at 5' end, and has 2'-fluoro modified nucleotides at positions 9, 11, and 12 of the sense strand represented by formula (V) counting from the first paired nucleotide at 3' end of the sense strand.

In some more specific embodiments, the dsRNA agent has 2'-fluoro modified nucleotides at positions 2, 5, 12, 14, and 16 of the antisense strand represented by formula (I-1) counting from the first paired nucleotide at 5' end, and has 2'-fluoro modified nucleotides at positions 9, 11, and 13 of the sense strand represented by formula (V) counting from the first paired nucleotide at 3' end of the sense strand.

In some embodiments, an RNAi agent for inhibiting expression of a target gene is provided, wherein the RNAi agent is double-stranded ribonucleic acid (dsRNA) agent comprising a sense strand and an antisense strand complementary to at least a portion of an mRNA corresponding to the target gene, wherein the antisense strand has 18-30 nucleotides, the sense strand has 18-40 nucleotides, and the dsRNA agent comprises an antisense strand represented by formula (I-2) and a sense strand represented by formula (V).

In some embodiments, an RNAi agent for inhibiting expression of a target gene is provided, wherein the RNAi agent is double-stranded ribonucleic acid (dsRNA) agent comprising a sense strand and an antisense strand complementary to at least a portion of an mRNA corresponding to the target gene, wherein the antisense strand has 18-30 nucleotides, the sense strand has 18-40 nucleotides, and the dsRNA agent comprises an antisense strand represented by formula (I') and a sense strand represented by formula (V).

In some more specific embodiments, the dsRNA agent has 2'-fluoro modified nucleotides at positions 2, 4, and 14 of the antisense strand represented by formula (IV) counting from the first paired nucleotide at 5' end, and has 2'-fluoro modified nucleotides at positions 8, 11, and 13 of the sense strand represented by formula (V) counting from the first paired nucleotide at 3' end of the sense strand.

In some more specific embodiments, the dsRNA agent has 2'-fluoro modified nucleotides at positions 2, 8, and 14 of the antisense strand represented by formula (IV) counting from the first paired nucleotide at 5' end, and has 2'-fluoro modified nucleotides at positions 8, 11, and 13 of the sense strand represented by formula (V) counting from the first paired nucleotide at 3' end of the sense strand.

In some more specific embodiments, the dsRNA agent has 2'-fluoro modified nucleotides at positions 2, 7, and 14 of the antisense strand represented by formula (IV) counting from the first paired nucleotide at 5' end, and has 2'-fluoro modified nucleotides at positions 8, 11, and 13 of the sense strand represented by formula (V) counting from the first paired nucleotide at 3' end of the sense strand.

In some more specific embodiments, the dsRNA agent has 2'-fluoro modified nucleotides at positions 2, 12, and 14 of the antisense strand represented by formula (IV) counting from the first paired nucleotide at 5' end, and has 2'-fluoro modified nucleotides at positions 8, 11, and 13 of the sense strand represented by formula (V) counting from the first paired nucleotide at 3' end of the sense strand.

In some more specific embodiments, the dsRNA agent has 2'-fluoro modified nucleotides at positions 2, 14, and 16 of the antisense strand represented by formula (IV) counting from the first paired nucleotide at 5' end, and has 2'-fluoro modified nucleotides at positions 8, 11, and 13 of the sense strand represented by formula (V) counting from the first paired nucleotide at 3' end of the sense strand.

In some more specific embodiments, the dsRNA agent has 2'-fluoro modified nucleotides at positions 2, 14, and 18 of the antisense strand represented by formula (IV) counting from the first paired nucleotide at 5' end, and has 2'-fluoro modified nucleotides at positions 8, 11, and 13 of the sense strand represented by formula (V) counting from the first paired nucleotide at 3' end of the sense strand.

In some more specific embodiments, the dsRNA agent has 2'-fluoro modified nucleotides at positions 2, 6, and 14 of the antisense strand counting from the first paired nucleotide at 5' end, and has 2'-fluoro modified nucleotides at positions 8, 11, and 13 of the sense strand represented by formula (V) counting from the first paired nucleotide at 3' end of the sense strand.

In some more specific embodiments, the dsRNA agent has 2'-fluoro modified nucleotides at positions 2, 7, 11, 14, and 16 of the antisense strand represented by formula (I-2) counting from the first paired nucleotide at 5' end, and has 2'-fluoro modified nucleotides at positions 9, 11, and 13 of the sense strand represented by formula (V) counting from the first paired nucleotide at 3' end of the sense strand.

In some more specific embodiments, the dsRNA agent has 2'-fluoro modified nucleotides at positions 2, 7, 11, 14, and 18 of the antisense strand represented by formula (I-2) counting from the first paired nucleotide at 5' end, and has 2'-fluoro modified nucleotides at positions 9, 11, and 13 of the sense strand represented by formula (V) counting from the first paired nucleotide at 3' end of the sense strand.

In some more specific embodiments, the dsRNA agent has 2'-fluoro modified nucleotides at positions 2, 9, 12, 14, and 16 of the antisense strand represented by formula (I-2) counting from the first paired nucleotide at 5' end, and has 2'-fluoro modified nucleotides at positions 9, 11, and 13 of the sense strand represented by formula (V) counting from the first paired nucleotide at 3' end of the sense strand.

In some more specific embodiments, the dsRNA agent has 2'-fluoro modified nucleotides at positions 2, 8, 12, 14, and 16 of the antisense strand represented by formula (I-2) counting from the first paired nucleotide at 5' end, and has 2'-fluoro modified nucleotides at positions 9, 11, and 13 of the sense strand represented by formula (V) counting from the first paired nucleotide at 3' end of the sense strand.

In some more specific embodiments, the dsRNA agent has 2'-fluoro modified nucleotides at positions 2, 7, 12, 14, and 16 of the antisense strand represented by formula (I-2) counting from the first paired nucleotide at 5' end, and has 2'-fluoro modified nucleotides at positions 9, 11, and 12 of the sense strand represented by formula (V) counting from the first paired nucleotide at 3' end of the sense strand.

In some embodiments, the dsRNA agent comprises combinations of an antisense strand with the nucleotide sequence represented by formula (X) herein and a sense strand with the nucleotide sequence represented by formula (V) herein, including but not limited to other dsRNA modifications described herein.

It can be understood that the dsRNA agent herein comprises combinations of an antisense strand with any nucleotide sequence described herein and a sense strand with any nucleotide sequence described herein, including but not limited to other dsRNA modifications described herein. For example, including but not limited to: formula (X) is combined with formula (V), formula (V') and formula (IX), respectively; formula (IX) is combined with formula (X), formula (I), formula (I'), formula (I-1), formula (I-2), formula (II), formula (III) and formula (IV), respectively.

In some embodiments, the dsRNA agent comprises combinations of an antisense strand with the nucleotide sequence represented by formula (I) herein and a sense strand with the nucleotide sequence represented by formula (V) herein, including but not limited to other dsRNA modifications described herein.

In some embodiments, in the antisense strand represented by formula (I-1), formula (I'), formula (I-2), formula (II), formula (III) and/or formula (IV) of the dsRNA agent, one or more of N_{L} are modified nucleotides independently selected from the group consisting of 2'-O-methyl modified nucleotide, 2'-deoxy nucleotide, 2',3'-seco nucleotide mimic, locked nucleotide (LNA), unlocked nucleic acid nucleotide (UNA), glycol nucleic acid nucleotide (GNA), bicyclo nucleic acid (BNA), 2'-F-arabino nucleotide, 2'-methoyxyethyl nucleotide (MOE), abasic nucleotide, ribitol, inverted nucleotide, inverted abasic nucleotide (Invab), inverted 2'-OMe nucleotide, inverted 2'-deoxy nucleotide, 2'-amino modified nucleotide, 2'-alkyl modified nucleotide, morpholino nucleotide, 3'-OMe nucleotide, phosphorothioate modified nucleotide, terminal nucleotide linked to a cholesteryl derivative or dodecanoic acid bisdecylamide group, 2'-amino modified nucleotide, phosphoramidate, and non-natural base comprising nucleotide. In some embodiments, N'_{L} in the dsRNA agent is selected from the group consisting of 2'-O-methyl modified nucleotide, UNA, and Invab.

In some embodiments, in the sense strand represented by formula (V) and/or formula (IX) of the dsRNA agent, one or more of N'_{L} are modified nucleotides independently selected from the group consisting of 2'-fluoro modified nucleotide, 2'-O-methyl modified nucleotide, 2'-deoxy nucleotide, 2',3'-seco nucleotide mimic, locked nucleotide (LNA), unlocked nucleic acid nucleotide (UNA), glycol nucleic acid nucleotide (GNA), bicyclo nucleic acid (BNA), 2'-F-arabino nucleotide, 2'-methoyxyethyl nucleotide (MOE), abasic nucleotide, ribitol, inverted nucleotide, inverted abasic nucleotide (Invab), inverted 2'-OMe nucleotide, inverted 2'-deoxy nucleotide, 2'-amino modified nucleotide, 2'-alkyl modified nucleotide, morpholino nucleotide, 3'-OMe nucleotide, phosphorothioate modified nucleotide, terminal nucleotide linked to a cholesteryl derivative or dodecanoic acid bisdecylamide group, 2'-amino modified nucleotide, phosphoramidate, and non-natural base comprising nucleotide. In some embodiments, N'_{L} in the dsRNA agent is selected from the group consisting of 2'-O-methyl modified nucleotide, UNA, and Invab.

In some embodiments, in the antisense strand represented by formula (I) of the dsRNA agent, one or more of N_{X} are modified nucleotides independently selected from the group consisting of 2'-fluoro modified nucleotide, 2'-O-methyl modified nucleotide, 2'-deoxy nucleotide, 2',3'-seco nucleotide mimic, locked nucleotide (LNA), unlocked nucleic acid nucleotide (UNA), glycol nucleic acid nucleotide (GNA), bicyclo nucleic acid (BNA), 2'-F-arabino nucleotide, 2'-methoyxyethyl nucleotide (MOE), abasic nucleotide, ribitol, inverted nucleotide, inverted abasic nucleotide (Invab), inverted 2'-OMe nucleotide, inverted 2'-deoxy nucleotide, 2'-amino modified nucleotide, 2'-alkyl modified nucleotide, morpholino nucleotide, 3'-OMe nucleotide, phosphorothioate modified nucleotide, terminal nucleotide linked to a cholesteryl derivative or dodecanoic acid bisdecylamide group, 2'-amino modified nucleotide, phosphoramidate, and non-natural base comprising nucleotide. In some embodiments, N'_{L} in the dsRNA agent is selected from the group consisting of 2'-O-methyl modified nucleotide, UNA, and Invab.

In some embodiments, in some non-limiting examples of the dsRNA agent, N'_{N1}, N'_{N2}, N'_{N3}, N'_{N4}, N'_{N5} and/or N'_{N6} in the sense strand and N_{M1}, N_{M2}, N_{M3}, N_{M4}, N_{M5}, N_{M6}, N_{M7} and/or N_{M8} in the antisense strand are independently represent an unmodified nucleotide, or a modified nucleotide independently selected from the group consisting of 2'-fluoro modified nucleotide, 2'-O-methyl modified nucleotide, 2'-deoxy nucleotide, 2',3'-seco nucleotide mimic, locked nucleotide (LNA), unlocked nucleic acid nucleotide (UNA), glycol nucleic acid nucleotide (GNA), bicyclo nucleic acid (BNA), 2'-F-arabino nucleotide, 2'-methoyxyethyl nucleotide (MOE), abasic nucleotide, ribitol, inverted nucleotide, inverted abasic nucleotide (Invab), inverted 2'-OMe nucleotide, inverted 2'-deoxy nucleotide, 2'-amino modified nucleotide, 2'-alkyl modified nucleotide, morpholino nucleotide, 3'-OMe nucleotide, phosphorothioate modified nucleotide, terminal nucleotide linked to a cholesteryl derivative or dodecanoic acid bisdecylamide group, 2'-amino modified nucleotide, phosphoramidate, and non-natural base comprising nucleotide. In some embodiments, N'_{N1}, N'_{N2}, N'_{N3}, N'_{N4}, N'_{N5} and/or N'_{N6} in the sense strand represented by formula (V) and N_{M1}, N_{M2}, N_{M3}, N_{M4}, N_{M5}, N_{M6}, N_{M7} and/or N_{M8} are preferably 2'-O-methyl-modified nucleotide, UNA, GNA, or LNA in case of not being 2'-fluoro modified nucleotides.

In some embodiments, in the dsRNA agent, the antisense strand represented by formula (I) is complementary or substantially complementary to the sense strand represented by formula (V). In some embodiments, in the sense strand or the antisense strand of the dsRNA agent, all nucleotides are modified nucleotides.

In some embodiments, in the antisense strand represented by formula (I) and the sense strand represented by formula (V) of the dsRNA agent of the present invention, the phosphate modified nucleotide is phosphorothioate modified nucleotide.

In some embodiments, the dsRNA agent of the present invention comprises an E-vinylphosphonate nucleotide at the 5' end of the antisense strand.

In some embodiments, in the dsRNA agent of the present invention, the sense strand is complementary or substantially complementary to the antisense strand. In some embodiments, the complementary or substantially complementary region of the dsRNA agent of the present invention is 18-25 nucleotides in length. In some embodiments, the complementary region is 18-23 nucleotides in length. In some embodiments, the complementary region is 19-21 nucleotides in length. In yet other embodiments, for example, the complementary region can be 18-25 nucleotides in length, 19-25 nucleotides in length, 19-23 nucleotides in length, or 19-21 nucleotides in length. In another example, in some embodiments, the complementary region is 18, 19, 20 or 21 nucleotides in length.

In some embodiments, the dsRNA agent has an antisense strand fully complementary to the sense strand.

In some embodiments, the dsRNA agent of the present invention contains no mismatches. In certain embodiments, the target gene dsRNA agent of the present invention contains no more than 1 mismatch (such mismatch does not affect the counting start position of the pairing). In some embodiments, the dsRNA agent of the present invention contains no more than 2 mismatches. In certain embodiments, the dsRNA agent of the present invention contains no more than 3 mismatches. In some embodiments of the invention, the antisense strand of the dsRNA agent contains a mismatch to a target sequence that is not located in the center of the complementary region. In some embodiments, the antisense strand of the dsRNA agent contains 1, 2, 3, 4 or more mismatches located within the last 5, 4, 3, 2 or 1 nucleotide of either or both the 5' end or 3' end of the complementary region.

In some embodiments, each strand of the dsRNA agent of the present invention is no more than 30 nucleotides in length. In some embodiments, each strand of the dsRNA agent of the present invention is no more than 25 nucleotides in length. In some embodiments, each strand of the dsRNA agent of the present invention is no more than 23 nucleotides in length. In some embodiments, each strand of the dsRNA of the present invention is 19, 20 or 21 nucleotides in length.

In some embodiments, the dsRNA agent has a blunt end at the 5' end of the sense strand. In one embodiment, the dsRNA agent of the present invention has two blunt ends at both ends of the sense strand and the antisense strand. For example, each strand of the dsRNA agent is fully paired to form a blunt end structure.

In some embodiments, the sense strand represented by formula (V) of the RNAi agent has 1-5 unpaired nucleotide overhangs at 5' end, such as 1, 2, 3, 4 or 5 unpaired nucleotide overhangs.

In some embodiments, the antisense strand complementary or partially complementary to formula (V) of the dsRNA agent has 1-5 unpaired nucleotide overhangs at 3' end and/or 5' end, such as 1, 2, 3, 4 or 5 unpaired nucleotide overhangs. In some embodiments, the antisense strand complementary or partially complementary to formula (V) of the dsRNA agent has a blunt end at 3' end and/or 5' end.

In some embodiments, the overhang of unpaired nucleotides in the sense strand and/or antisense strand of the dsRNA agent of the present invention is 2-5 nucleotides in length, 1-5 nucleotides in length, 1-4 nucleotides in length, 2-4 nucleotides in length, 1-3 nucleotides in length, 2-3 nucleotides in length or 1-2 nucleotides in length. The overhang can be the result of one strand being longer than the other, or the result of two strands of the same length being staggered. The overhang may form one mismatch with the target mRNA, or it may be complementary to the targeted gene sequence or may be other sequences. The first and second strand can also be connected, for example, by other base to form a hairpin or by other non-base linkers.

In one embodiment, one or more nucleotides in the overhang of the dsRNA agent of the present invention can be independently a modified nucleotide or an unmodified nucleotide, and the modified nucleotide includes but is not limited to: 2'-fluoro modified nucleotide, 2'-O-methyl modified nucleotide, 2'-deoxy nucleotide, 2',3'-seco nucleotide mimic, locked nucleotide (LNA), unlocked nucleic acid nucleotide (UNA), glycol nucleic acid nucleotide (GNA), bicyclo nucleic acid (BNA), 2'-F-arabino nucleotide, 2'-methoyxyethyl nucleotide, abasic nucleotide, ribitol, inverted nucleotide, inverted abasic nucleotide (Invab), inverted 2'-OMe nucleotide, inverted 2'-deoxy nucleotide, 2'-amino modified nucleotide, 2'-alkyl modified nucleotide, morpholino nucleotide, 3'-OMe nucleotide, phosphorothioate modified nucleotide, terminal nucleotide linked to a cholesteryl derivative or dodecanoic acid bisdecylamide group, 2'-amino modified nucleotide, phosphoramidate, and non-natural base comprising nucleotide.

In one embodiment, both ends of the overhang of the sense strand, antisense strand or both strands of the overhang of the dsRNA agent of the present invention can be phosphorylated.

In one embodiment, the overhang of the dsRNA agent of the present invention exists at 5' end or 3' end of the sense strand, antisense strand or both strands. In one embodiment, the overhang exists at 3' end of the antisense strand. In one embodiment, the overhang exists at 5' end of the sense strand.

In one embodiment, the 5' end of the antisense strand or the 3' end of the sense strand is blunt end in the dsRNA agent of the present invention.

In some embodiments, the dsRNA agent of the present invention comprises at least one phosphorothioate internucleoside linkage.

In some embodiments, the sense strand of the dsRNA agent of the present invention comprises at least one phosphorothioate internucleoside linkage.

In some embodiments, the antisense strand of the dsRNA agent of the present invention comprises at least one phosphorothioate internucleoside linkage.

In some embodiments, the sense strand and/or the antisense strand of the dsRNA agent of the present invention comprises 1, 2, 3, 4, 5, 6, 7, 8, 9, 10, 11, 12, 13, 14, 15, 16, 17 or 18 phosphorothioate internucleoside linkages.

In one embodiment, the sense strand or the antisense strand of the dsRNA agent of the present invention comprises 1-10 blocks with 2-10 phosphorothioate internucleoside linkages separated by 1, 2, 3, 4, 5, 6, 7, 8, 9, 10, 11, 12, 13, 14, 15, 16, 17 or 18 phosphate internucleoside linkages, wherein one of the phosphorothioate internucleoside linkages is located at any position in the oligonucleotide sequence, and the sense strand is paired with the antisense strand comprising any combination of phosphorothioate internucleoside linkages, methylphosphonate internucleoside linkages, and phosphate internucleoside linkages or the antisense strand comprising phosphorothioate linkage or phosphate linkage.

In one embodiment, the dsRNA agent of the present invention further comprises 1-5 phosphorothioate internucleotide linkage or methylphosphonate internucleotide linkage modifications at positions 1-5 of the sense strand and further comprises 1-5 phosphorothioate internucleotide linkage or methylphosphonate internucleotide linkage modifications at positions 18-23 of the sense strand (counting from the paired nucleotide at 3' end of the sense strand as used herein), and further comprises 1-2 phosphorothioate internucleotide linkages at positions 1-2 of the antisense strand and further comprises 1-5 phosphorothioate internucleotide linkages in positions 18-23 of the antisense strand (counting from the blunt end at 5' end of the antisense strand as used herein). In one embodiment, the dsRNA agent of the present invention comprises 1 or 2 phosphorothioate internucleotide linkage modifications at 3' end and/or 5' end of the sense strand or the antisense strand. In one embodiment, the dsRNA agent of the present invention comprises 1 or 2 phosphorothioate internucleotide linkage modifications at the 3' end and/or 5' end of the sense strand or antisense strand.

In one embodiment, the dsRNA agent of the present invention comprises the phosphorothioate internucleotide linkage modification in the overhang.

In some embodiments, the sense strand and/or the antisense strand of the dsRNA agent of the present invention has 1 or 2 inverted abasic residue nucleotides. In some embodiments, the sense strand and/or the antisense strand of the dsRNA agent of the present invention comprises 1 or 2 inverted abasic residues at 3' end or/and 5' end. In some embodiments, the sense strand of the dsRNA agent of the present invention comprises an inverted abasic residue at the 3' end.

In some embodiments, the dsRNA agent further comprises one or more targeting groups or linking groups. In some embodiments, one or more targeting groups or linking groups of the RNAi agent are conjugated to the sense strand. In some embodiments, the targeting group or linking group includes N-acetyl-galactosamine (GalNAc). In some embodiments, the targeting group or linking group is conjugated to the 5' end of the sense strand.

Another non-limiting example of a delivery agent that can be used in the embodiments of the present invention to deliver the dsRNA agent of the present invention to a cell, tissue, and/or subject is a GalNAc-containing agent, which is linked to the dsRNA agent of the present invention and delivers the dsRNA agent to a cell, tissue and/or subject. Examples of the targeting ligand cluster mentioned herein include a GalNAc ligand with phosphodiester bonds (GLO) and a GalNAc ligand with phosphorothioate bonds (GLS). The term "GLX-n" used herein may denote a linked GalNAC-containing compound, for example, but not limited to: GLS-1, GLS-2, GLS-3, GLS-4, GLS-5, GLS-6, GLS-7, GLS-8, GLS-9, GLS-10, GLS-11, GLS-12, GLS-13, GLS-14, GLS-15, GLS-16, GLO-1, GLO-2, GLO-3, GLO-4, GLO-5, GLO-6, GLO-7, GLO-8, GLO-9, GLO-10, GLO-11, GLO-12, GLO-13, GLO-14, GLO-15 and GLO-16, and the structure of each is shown herein. It should be understood that any RNAi and dsRNA molecules of the present invention may be linked to GLS-1, GLS-2, GLS-3, GLS-4, GLS-5, GLS-6, GLS-7, GLS-8, GLS-9, GLS-10, GLS-11, GLS-12, GLS-13, GLS-14, GLS-15, GLS-16, GLO-1, GLO-2, GLO-3, GLO-4, GLO-5, GLO-6, GLO-7, GLO-8, GLO-9, GLO-10, GLO-11, GLO-12, GLO-13, GLO-14, GLO-15 or GLO-16. In some preferred examples, the targeting groups are GLS-5 and GLS-15 described herein.

In some embodiments, the sense strand and the antisense strand of the dsRNA agent of the present invention are partially or fully complementary.

Another aspect of the present invention also provides a composition comprising any embodiment of the above-mentioned dsRNA agent of the present invention.

### Carrier

Certain embodiments of the invention include use of pharmaceutical compositions containing a pharmaceutically acceptable carrier. As used herein, a "pharmaceutical composition" comprises a pharmacologically effective amount of a dsRNA agent or antisense polynucleotide agent of the invention and a pharmaceutically acceptable carrier. The term "pharmaceutically acceptable carrier" refers to a carrier for administration of a therapeutic agent. Such carriers include, but are not limited to, saline, buffered saline, dextrose, water, glycerol, ethanol, and combinations thereof. The term specifically excludes cell culture medium. For drugs administered orally, pharmaceutically acceptable carriers include, but are not limited to pharmaceutically acceptable excipients such as inert diluents, disintegrating agents, binding agents, lubricating agents, sweetening agents, flavoring agents, coloring agents and preservatives. Suitable inert diluents include sodium and calcium carbonate, sodium and calcium phosphate, and lactose, while corn starch and alginic acid are suitable disintegrating agents. Binding agents may include starch and gelatin, while the lubricating agent, if present, will generally be magnesium stearate, stearic acid or talc. If desired, the tablets may be coated with a material such as glyceryl monostearate or glyceryl distearate, to delay absorption in the gastrointestinal tract. Agents included in drug formulations are described further herein below.

Pharmaceutically acceptable carriers include diluents, fillers, salts, buffers, stabilizers, solubilizers and other materials that are well-known in the art. Exemplary pharmaceutically acceptable carriers are described in U.S. Pat. No. 5,211,657 and others are known by those skilled in the art. Such preparations may routinely contain salt, buffering agents, preservatives, compatible carriers, and optionally other therapeutic agents. When used in medicine, the salts should be pharmaceutically acceptable, but non-pharmaceutically acceptable salts may conveniently be used to prepare pharmaceutically-acceptable salts thereof and are not excluded from the scope of the invention. Such pharmacologically and pharmaceutically-acceptable salts include, but are not limited to, those prepared from the following acids: hydrochloric, hydrobromic, sulfuric, nitric, phosphoric, maleic, acetic, salicylic, citric, formic, malonic, succinic, and the like. Also, pharmaceutically-acceptable salts can be prepared as alkaline metal or alkaline earth salts, such as sodium, potassium or calcium salts.

Therapeutic formulations of dsRNA agents or antisense polynucleotide agents may be prepared for storage by mixing the molecule or compound having the desired degree of purity with optional pharmaceutically acceptable carriers, excipients or stabilizers [Remington's Pharmaceutical Sciences 21st edition, (2006)], in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as TWEEN^{®}, PLURONICS^{®} or polyethylene glycol (PEG).

### Administration methods

In some embodiments, the tissue to which the compound is administered is a tissue in which the target gene -associated disease or condition is present or is likely to arise, non-limiting examples of which are the liver or kidney. Direct tissue administration may be achieved by direct injection or other means. Many orally delivered compounds naturally travel to and through the liver and kidneys and some embodiments of treatment methods of the invention include oral administration of one or more dsRNA agents to a subject. dsRNA agents or target gene antisense polynucleotide agents, either alone or in conjunction with other therapeutic agents, may be administered once, or alternatively they may be administered in a plurality of administrations. If administered multiple times, the dsRNA agent or antisense polynucleotide agent may be administered via different routes. For example, though not intended to be limiting, a first (or first several) administrations may be made via subcutaneous means and one or more additional administrations may be oral and/or systemic administrations.

For embodiments of the invention in which it is desirable to administer a target gene dsRNA agent or antisense polynucleotide agent systemically, the target gene dsRNA agent or antisense polynucleotide agent may be formulated for parenteral administration by injection, e.g., by bolus injection or continuous infusion. Formulations for injection may be presented in unit dosage form, e.g., in ampoules or in multi-dose containers, with or without an added preservative. dsRNA agent formulations (also referred to as pharmaceutical compositions) may take such forms as suspensions, solutions or emulsions in oily or aqueous vehicles, and may contain formulatory agents such as suspending, stabilizing and/or dispersing agents.

Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like. Lower doses will result from other forms of administration, such as intravenous administration. In the event that a response in a subject is insufficient at the initial doses applied, higher doses (or effectively higher doses by a different, more localized delivery route) may be employed to the extent that patient tolerance permits. Multiple doses per day may be used as needed to achieve appropriate systemic or local levels of one or more target gene dsRNA agents or antisense polynucleotide agents and to achieve appropriate reduction in target gene activity.

In yet other embodiments, methods of the invention include use of a delivery vehicle such as biocompatible microparticle, nanoparticle, or implant suitable for implantation into a recipient, e.g., a subject. Exemplary bioerodible implants that may be useful in accordance with this method are described in PCT Publication No. WO 95/24929 (incorporated by reference herein), which describes a biocompatible, biodegradable polymeric matrix for containing a biological macromolecule.

Both non-biodegradable and biodegradable polymeric matrices can be used in methods of the invention to deliver one or more dsRNA agents or antisense polynucleotide agents to a subject. In some embodiments, a matrix may be biodegradable. Matrix polymers may be natural or synthetic polymers. A polymer can be selected based on the period of time over which release is desired, generally in the order of a few hours to a year or longer. Typically, release over a period ranging from between a few hours and three to twelve months can be used. The polymer optionally is in the form of a hydrogel that can absorb up to about 90% of its weight in water and further, optionally is cross-linked with multivalent ions or other polymers.

In general, dsRNA agents or antisense polynucleotide agents may be delivered in some embodiments of the invention using the bioerodible implant by way of diffusion, or by degradation of the polymeric matrix. Exemplary synthetic polymers for such use are well known in the art. Biodegradable polymers and non-biodegradable polymers can be used for delivery of dsRNA agents or antisense polynucleotide agents using art-known methods. Bioadhesive polymers such as bioerodible hydrogels (see H. S. Sawhney, C. P. Pathak and J. A. Hubell in Macromolecules, 1993, 26, 581-587, the teachings of which are incorporated by reference herein) may also be used to deliver dsRNA agents or antisense polynucleotide agents for treatment of a target gene-associated disease or condition. Additional suitable delivery systems can include time-release, delayed release or sustained release delivery systems. Such systems can avoid repeated administrations of a dsRNA agent orantisense polynucleotide agent, increasing convenience to the subject and the medical care professional. Many types of release delivery systems are available and known to those of ordinary skill in the art. (See for example: U.S. Pat. Nos. 5,075,109; 4,452,775; 4,675,189; 5,736,152; 3,854,480; 5,133,974; and 5,407,686 (the teaching of each of which is incorporated herein by reference). In addition, pump-based hardware delivery systems can be used, some of which are adapted for implantation.

Use of a long-term sustained release implant may be suitable for prophylactic treatment of subjects and for subjects at risk of developing a recurrent target gene-associated disease or condition. Long-term release, as used herein, means that the implant is constructed and arranged to deliver a therapeutic level of a dsRNA agent or antisense polynucleotide agent for at least up to 10 days, 20 days, 30 days, 60 days, 90 days, six months, a year, or longer. Long-term sustained release implants are well-known to those of ordinary skill in the art and include some of the release systems described above.

### Effective amounts

Methods of the invention, in some aspects comprise contacting a cell with a dsRNA agent or antisense polynucleotide agent in an effective amount to reduce gene expression in the contacted cell. Certain embodiments of methods of the invention comprise administering a dsRNA agent or an antisense polynucleotide agent to a subject in an amount effective to reduce gene expression and treat a -associated disease or condition in the subject. An "effective amount" used in terms of reducing expression of target gene and/or for treating a target gene-associated disease or condition, is an amount necessary or sufficient to realize a desired biologic effect. For example, an effective amount of a dsRNA agent or antisense polynucleotide agent to treat a target gene-associated disease or condition could be that amount necessary to (i) slow or halt progression of the disease or condition; or (ii) reverse, reduce, or eliminate one or more symptoms of the disease or condition. In some aspects of the invention, an effective amount is that amount of a target gene dsRNA agent or target gene antisense polynucleotide agent that when administered to a subject in need of a treatment of a target gene-associated disease or condition, results in a therapeutic response that prevents and/or treats the disease or condition. According to some aspects of the invention, an effective amount is that amount of a target gene dsRNA agent or target gene antisense polynucleotide agent of the invention that when combined or co-administered with another therapeutic treatment for a target gene-associated disease or condition, results in a therapeutic response that prevents and/or treats the disease or condition. In some embodiments of the invention, a biologic effect of treating a subject with a target gene dsRNA agent or target gene antisense polynucleotide agent of the invention may be the amelioration and or absolute elimination of symptoms resulting from the target gene-associated disease or condition. In some embodiments of the invention, a biologic effect is the complete abrogation of the target gene-associated disease or condition, as evidenced for example, by a diagnostic test that indicates the subject is free of the target gene-associated disease or condition. In some embodiments, an effective amount is an amount that results in a desired response, such as an amount that reduces a cell, tissue, and/or associated disease or disorder in a subject suffering from the disease or disorder. In some embodiments, determining the amount administered to a subject method for treating a target gene-associated disease or disorder with a target gene dsRNA agent or a target gene antisense polynucleotide agent of the present invention, by assessing and/or monitoring one or more of the target gene-associated disease or disorder in a subject A variety of "physiological characteristics" to carry out.

It will be recognized that gene silencing may be determined in any cell expressing , either constitutively or by genomic engineering, and by any appropriate assay. In some embodiments of the invention, gene expression is reduced by at least 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, or 100% by administration of a dsRNA agent of the invention. In some embodiments of the invention, gene expression is reduced by at between 5% and 10%, 5% and 25%, 10% and 50%, 10% and 75%, 25% and 75%, 25% and 100%, or 50% and 100% by administration of a dsRNA agent of the invention.

DsRNA agents and antisense polynucleotide agents of the invention are delivered in pharmaceutical compositions in dosages sufficient to inhibit expression of genes. In certain embodiments of the invention, a dose of dsRNA agent or antisense polynucleotide agent is in a range of 0.01 to 200.0 milligrams per kilogram body weight of the recipient per day, generally in the range of 1 to 50 mg per kilogram body weight, 5 to 40 mg/kg body weight, 10 to 30 mg/kg body weight, 1 to 20 mg/kg body weight, 1 to 10 mg/kg body weight, 4 to 15 mg/kg body weight per day, inclusive. For example, the dsRNA agent or antisense polynucleotide agent can be administered in an amount that is from about 0.01 mg/kg, 0.05 mg/kg, 0.1 mg/kg, 0.2 mg/kg, 0.3 mg/kg, 0.4 mg/kg, 0.5 mg/kg, 1 mg/kg, 1.1 mg/kg, 1.2 mg/kg, 1.3 mg/kg, 1.4 mg/kg, 1.5 mg/kg, 1.6 mg/kg, 1.7 mg/kg, 1.8 mg/kg, 1.9 mg/kg, 2 mg/kg, 2.1 mg/kg, 2.2 mg/kg, 2.3 mg/kg, 2.4 mg/kg, 2.5 mg/kg, 2.6 mg/kg, 2.7 mg/kg, 2.8 mg/kg, 2.9 mg/kg, 3.0 mg/kg, 3.1 mg/kg, 3.2 mg/kg, 3.3 mg/kg, 3.4 mg/kg, 3.5 mg/kg, 3.6 mg/kg, 3.7 mg/kg, 3.8 mg/kg, 3.9 mg/kg, 4 mg/kg, 4.1 mg/kg, 4.2 mg/kg, 4.3 mg/kg, 4.4 mg/kg, 4.5 mg/kg, 4.6 mg/kg, 4.7 mg/kg, 4.8 mg/kg, 4.9 mg/kg, 5 mg/kg, 5.1 mg/kg, 5.2 mg/kg, 5.3 mg/kg, 5.4 mg/kg, 5.5 mg/kg, 5.6 mg/kg, 5.7 mg/kg, 5.8 mg/kg, 5.9 mg/kg, 6 mg/kg, 6.1 mg/kg, 6.2 mg/kg, 6.3 mg/kg, 6.4 mg/kg, 6.5 mg/kg, 6.6 mg/kg, 6.7 mg/kg, 6.8 mg/kg, 6.9 mg/kg, 7 mg/kg, 7.1 mg/kg, 7.2 mg/kg, 7.3 mg/kg, 7.4 mg/kg, 7.5 mg/kg, 7.6 mg/kg, 7.7 mg/kg, 7.8 mg/kg, 7.9 mg/kg, 8 mg/kg, 8.1 mg/kg, 8.2 mg/kg, 8.3 mg/kg, 8.4 mg/kg, 8.5 mg/kg, 8.6 mg/kg, 8.7 mg/kg, 8.8 mg/kg, 8.9 mg/kg, 9 mg/kg, 9.1 mg/kg, 9.2 mg/kg, 9.3 mg/kg, 9.4 mg/kg, 9.5 mg/kg, 9.6 mg/kg, 9.7 mg/kg, 9.8 mg/kg, 9.9 mg/kg, 10 mg/kg, 11 mg/kg, 12 mg/kg, 13mg/kg, 14 mg/kg, 15 mg/kg, 16 mg/kg, 17 mg/kg, 18 mg/kg, 19 mg/kg, 20 mg/kg, 21 mg/kg, 22 mg/kg, 23mg/kg, 24 mg/kg, 25 mg/kg, 26 mg/kg, 27 mg/kg, 28 mg/kg, 29 mg/kg, 30 mg/kg, 31 mg/kg, 32 mg/kg, 33mg/kg, 34 mg/kg, 35 mg/kg, 36 mg/kg, 37 mg/kg, 38 mg/kg, 39 mg/kg, 40 mg/kg, 41 mg/kg, 42 mg/kg, 43mg/kg, 44 mg/kg, 45 mg/kg, 46 mg/kg, 47 mg/kg, 48 mg/kg, 49 mg/kg, through 50 mg/kg body per single dose.

Various factors may be considered in the determination of dosage and timing of delivery of a dsRNA agent of the invention. The absolute amount of a dsRNA agent or antisense polynucleotide agent delivered will depend upon a variety of factors including a concurrent treatment, the number of doses and the individual subject parameters including age, physical condition, size and weight. These are factors well known to those of ordinary skill in the art and can be addressed with no more than routine experimentation. In some embodiments, a maximum dose can be used, that is, the highest safe dose according to sound medical judgment.

Methods of the invention may in some embodiments include administering to a subject 1, 2, 3, 4, 5, 6, 7, 8, 9, 10, or more doses of a dsRNA agent or antisense polynucleotide agent. In some instances, a pharmaceutical compound, (e.g., comprising a dsRNA agent or comprising a antisense polynucleotide agent) can be administered to a subject at least daily, every other day, weekly, every other week, monthly, etc. Doses may be administered once per day or more than once per day, for example, 2, 3, 4, 5, or more times in one 24 hour period. A pharmaceutical composition of the invention may be administered once daily, or the dsRNA agent or antisense polynucleotide agent may be administered as two, three, or more sub-doses at appropriate intervals throughout the day or even using continuous infusion or delivery through a controlled release formulation. In some embodiments of methods of the invention, a pharmaceutical composition of the invention is administered to a subject one or more times per day, one or more times per week, one or more times per month, or one or more times per year.

Certain embodiments of the invention include use of pharmaceutical compositions containing a dsRNA agent or antisense polynucleotide agent and a pharmaceutically acceptable carrier. The pharmaceutical composition containing the dsRNA agent or antisense polynucleotide agent can be used in methods of the invention to reduce gene expression and activity in a cell and is useful to treat a -associated disease or condition. Such pharmaceutical compositions can be formulated based on the mode of delivery. Non-limiting examples of formulations for modes of delivery are: a composition formulated for subcutaneous delivery, a composition formulated for systemic administration via parenteral delivery, a composition formulated for intravenous (IV) delivery, a composition formulated for intrathecal delivery, a composition formulated for direct delivery into brain, etc. Administration of a pharmaceutic composition of the invention to deliver a dsRNA agent or antisense polynucleotide agent into a cell may be done using one or more means such as: topical (e.g., by a transdermal patch), pulmonary, e.g., by inhalation or insufflation of powders or aerosols, including by nebulizer; intratracheal, intranasal, epidermal and transdermal, oral or parenteral. Parenteral administration includes intravenous, intraarterial, subcutaneous, intraperitoneal or intramuscular injection or infusion; subdermal, e.g., via an implanted device; or intracranial, e.g., by intraparenchymal, intrathecal or intraventricular, administration. A dsRNA agent or antisense polynucleotide agent can also be delivered directly to a target tissue, for example directly into the liver, directly into a kidney, etc. It will be understood that "delivering a dsRNA agent" or "delivering a antisense polynucleotide agent" into a cell encompasses delivering a dsRNA agent or antisense polynucleotide agent, respectively, directly as well as expressing a dsRNA agent in a cell from an encoding vector that is delivered into a cell, or by any suitable means with which the dsRNA or antisense polynucleotide agent becomes present in a cell. Preparation and use of formulations and means for delivering inhibitory RNAs are well known and routinely used in the art.

In some embodiments, the composition also includes one or more additional therapeutic agents. In some embodiments of the invention, a composition of the invention may include one or more dsRNA agent and optionally one or more of a pharmaceutically acceptable carrier, a delivery agent, a targeting agent, detectable label, etc. A non-limiting example of a targeting agent that may be useful according to some embodiments of methods of the invention is an agent that directs a dsRNA agent of the invention to and/or into a cell to be treated. A targeting agent of choice will depend upon such elements as: the nature of the target gene-associated disease or condition, and on the cell type being targeted. In a non-limiting example, in some embodiments of the invention it may be desirable to target a dsRNA agent to and/or into a liver cell. It will be understood that in some embodiments of methods of the invention, a therapeutic agent comprises a dsRNA agent with only a delivery agent, such as a delivery agent comprising N-Acetylgalactosamine (GalNAc), without any additional attached elements. For example, in some aspects of the invention a dsRNA agent may be attached to a delivery compound comprising GalNAc and included in a composition comprising a pharmaceutically acceptable carrier and administered to a cell or subject without any detectable labels, or targeting agents, etc. attached to the dsRNA agent.

In cases where a dsRNA agent of the invention is administered with and/or attached to one or more delivery agents, targeting agents, labeling agents, etc. a skilled artisan will be aware of and able to select and use suitable agents for use in methods of the invention. Labeling agents may be used in certain methods of the invention to determine the location of a dsRNA agent in cells and tissues and may be used to determine a cell, tissue, or organ location of a treatment composition comprising a dsRNA agent that has been administered in methods of the invention. Procedures for attaching and utilizing labeling agents such as enzymatic labels, dyes, radiolabels, etc. are well known in the art. It will be understood that in some embodiments of compositions and methods of the invention, a labeling agent is attached to one or both of a sense polynucleotide and an antisense polynucleotide included in a dsRNA agent.

In some embodiments, the compositions are packaged in kits, containers, wrappers, dispensers, pre-filled syringes, or vials. Also within the scope of the invention are kits that comprise one or more dsRNA agents and/or antisense polynucleotide agents and instructions for its use in methods of the invention. Kits of the invention may include one or more of a target gene dsRNA agent, sense polynucleotide, and antisense polynucleotide agent that may be used to treat a associated disease or condition. Kits containing one or more target gene dsRNA agents, sense polynucleotides, and antisense polynucleotide agents can be prepared for use in treatment methods of the invention. Components of kits of the invention may be packaged either in aqueous medium or in lyophilized form. A kit of the invention may comprise a carrier being compartmentalized to receive in close confinement therein one or more container means or series of container means such as test tubes, vials, flasks, bottles, syringes, or the like. A first container means or series of container means may contain one or more compounds such as a dsRNA agent and/or sense or antisense polynucleotide agent. A second container means or series of container means may contain a targeting agent, a labelling agent, a delivery agent, etc. that may be included as a portion of a dsRNA agent and/or antisense polynucleotide to be administered in an embodiment of a treatment method of the invention.

A kit of the invention may also include instructions. Instructions typically will be in written form and will provide guidance for carrying-out a treatment embodied by the kit and for making a determination based upon that treatment.

### Cells, Subjects, and Controls

Methods of the invention may be used in conjunction with cells, tissues, organs and/or subjects. In some aspects of the invention a subject is a human or vertebrate mammal including but not limited to a dog, cat, horse, cow, goat, mouse, rat, and primate, e.g., monkey. Thus, the invention can be used to treat target gene -associated diseases or conditions in human and non-human subjects

In some aspects of the invention a subject may be a farm animal, a zoo animal, a domesticated animal or non-domesticated animal and methods of the invention can be used in veterinary prevention and treatment regimens. In some embodiments of the invention, the subject is a human and methods of the invention can be used in human prevention and treatment regimens.

Non-limiting examples of subjects to which the present invention can be applied are subjects who are diagnosed with, suspected of having, or at risk of having a disease or condition associated with a higher than desirable target gene expression and/or activity, also referred to as "elevated levels of target gene expression". Non-limiting examples of diseases and conditions associated with a higher than desirable levels of target gene expression and/or activity are described elsewhere herein. Methods of the invention may be applied to a subject who, at the time of treatment, has been diagnosed as having the disease or condition associated with a higher than desirable target gene expression and/or activity, or a subject who is considered to be at risk for having or developing a disease or condition associated with a higher than desirable target gene expression and/or activity. In some aspects of the invention a disease or condition associated with a higher than desirable target gene level of expression and/or activity is an acute disease or condition, and in certain aspects of the invention a disease or condition associated with a higher than desirable target gene level of expression and/or activity is a chronic disease or condition.

In another non-limiting example, administration of the target gene dsRNA agent of the present invention to treat a disease or disorder caused by or associated with the activation of the target gene, or a symptom or progression thereof in response to the inactivation of the target gene disease or disorder. The term "target gene-associated disease" includes diseases, disorders or conditions that benefit from decreased expression of a target gene.

A cell to which methods of the invention may be applied include cells that are *in vitro, in vivo, ex vivo* cells. Cells may be in a subject, in culture, and/or in suspension, or in any other suitable state or condition. A cell to which a method of the invention may be applied can be a liver cell, a hepatocyte, a cardiac cell, a pancreatic cell, a cardiovascular cell, kidney cell or other type of vertebrate cell, including human and non-human mammalian cells. In certain aspects of the invention, a cell to which methods of the invention may be applied is a healthy, normal cell that is not known to be a disease cell. In certain embodiments of the invention a cell to which methods and compositions of the invention are applied to a liver cell, a hepatocyte, a cardiac cell, a pancreatic cell, a cardiovascular cell, and/or a kidney cell. In certain aspects of the invention, a control cell is a normal cell, but it will be understood that a cell having a disease or condition may also serve as a control cell in particular circumstances for example to compare results in a treated cell having a disease or condition versus an untreated cell having the disease or condition, etc.

A level of target gene polypeptide activity can be determined and compared to control level of target gene polypeptide activity, according to methods of the invention. A control may be a predetermined value, which can take a variety of forms. It can be a single cut-off value, such as a median or mean. It can be established based upon comparative groups, such as in groups having normal levels of target gene polypeptide and/or target gene polypeptide activity and groups having increased levels of target gene polypeptide and/or target gene polypeptide activity. Another non-limiting example of comparative groups may be groups having one or more symptoms of or a diagnosis of an target gene -associated disease or condition; groups without having one or more symptoms of or a diagnosis of the disease or condition; groups of subjects to whom an siRNA treatment of the invention has been administered; groups of subjects to whom an siRNA treatment of the invention has not been administered. Typically, a control may be based on apparently healthy normal individuals in an appropriate age bracket or apparently healthy cells. It will be understood that controls according to the invention may be, in addition to predetermined values, samples of materials tested in parallel with the experimental materials. Examples include samples from control populations or control samples generated through manufacture to be tested in parallel with the experimental samples. In some embodiments of the invention, a control may include a cell or subject not contacted or treated with a target gene dsRNA agent of the invention and in such instances, a control level of target gene polypeptide and/or target gene polypeptide activity can be compared to a level of target gene polypeptide and/or target gene polypeptide activity in a cell or subject contacted with an target gene dsRNA agent or target gene antisense polynucleotide agent of the invention.

In some embodiments of the invention a level of target gene polypeptide determined for a subject can be a control level against which a level of target gene polypeptide determined for the same subject at a different time is compared. In a non-limiting example, a level of target gene is determined in a biological sample obtained from a subject who has not been administered target gene treatment of the invention. In some embodiments, the biological sample is a serum sample. The level of target gene polypeptide determined in the sample obtained from the subject can serve as a baseline or control value for the subject. After one or more administrations of an target gene dsRNA agent to the subject in a treatment method of the invention, one or more additional serum samples can be obtained from the subject and the level of target gene polypeptide in the subsequent sample or samples can be compared to the control/baseline level for the subject. Such comparisons can be used to assess onset, progression, or recession of an target gene associated disease or condition in the subject. For example, a level of target gene polypeptide in the baseline sample obtained from the subject that is higher than a level obtained from the same subject after the subject has been administered an target gene dsRNA agent or target gene antisense polynucleotide agent of the invention indicates regression of the target gene-associated disease or condition and indicates efficacy of the administered target gene dsRNA agent of the invention for treatment of the target gene-associated disease or condition.

In some aspects of the invention, values of one or more of a level of target gene polypeptide and/ortarget gene polypeptide activity determined for a subject may serve as control values for later comparison of levels of target gene polypeptide and/or target gene activity, in that same subject, thus permitting assessment of changes from a "baseline" target gene polypeptide activity in a subject. Thus, an initial target gene polypeptide level and/or initial target gene polypeptide activity level may be present and/or determined in a subject and methods and compounds of the invention may be used to decrease the level of target gene polypeptide and/or target gene polypeptide activity in the subject, with the initial level serving as a control level for that subject.

Using methods of the invention, target gene dsRNA agents and/or target gene antisense polynucleotide agents of the invention may be administered to a subject. Efficacy of the administration and treatment of the invention can be assessed when a level of target gene polypeptide in a serum sample obtained from a subject is decreased by at least 0.5%, 1%, 5%, 10%, 20%, 30%, 40%, 50%, 60%, 70%, 80%, 90%, 95%, or more compared to a pre-administration level of target gene polypeptide in a serum sample obtained from the subject at a prior time point, or compared to a non-contacted control level, for example a level of target gene polypeptide in a control serum sample. It will be understood that a level of target gene polypeptide and a level of target gene polypeptide activity both correlate with a level of target gene expression. Certain embodiments of methods of the invention comprise administering an target gene dsRNA and/or target gene antisense agent of the invention to a subject in an amount effective to inhibit target gene gene expression and thereby reduce a level of target gene polypeptide and reduce a level of target gene polypeptide activity in the subject. In some embodiments of the methods of the invention, contacting (also referred to herein as treatment) of a cell with an siRNA agent of the invention results in inhibition of target gene expression in the cell by at least about 1%, 2%, 3%, 4%, 5%, 6%, 7%, 8%, 9%, 10%, 11%, 12%, 13%, 14%, 15%, 16%, 17%, 18%, 19%, 20%, 21% , 22%, 23%, 24%, 25%, 26%, 27%, 28%, 29%, 30%, 31%, 32%, 33%, 34%, 35%, 36%, 37%, 38 %, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71% , 72%, 73%, 74%, 75%, 76%, 77%, 78%, 79%, 80%, 81%, 82%, 83%, 84%, 85%, 86%, 87%, 88 %, 89%, 90%, 91%, 92%, 93%, 94%, 95%, 96%, 97%, 98%, 99%, or about 100%, for example, to a level below assay detection.

Some embodiments of the invention, include determining presence, absence, and/or an amount (also referred to herein as a level) of target gene polypeptide in one or more biological samples obtained from one or more subjects. The determination can be used to assess efficacy of a treatment method of the invention. For example, methods and compositions of the invention can be used to determine a level of target gene polypeptide in a biological sample obtained from a subject previously treated with administration of an target gene dsRNA agent and/or an target gene antisense agent of the invention. A level of target gene polypeptide determined in a serum sample obtained from the treated subject that is lower by at least 0.5%, 1%, 5%, 10%, 20%, 30%, 40%, 50%, 60%, 70%, 80%, 90%, 95%, or more compared to a pretreatment level of target gene polypeptide determined for the subject, or compared to a non-contacted control biological sample level, indicates a level of efficacy of the treatment administered to the subject.

In some embodiments of the invention a physiological characteristic of an target gene-associated disease or condition determined for a subject can be a control determination against which a determination of the physiological characteristic in the same subject at a different time is compared. The Ahemolysis pathological feature is measured from a subject mRNA level determined in the sample obtained from the subject can serve as a baseline or control value for the subject. After one or more administrations of an target gene dsRNA agent to the subject in a treatment method of the invention, one or more additional serum samples can be obtained from the subject and target gen mRNA level and/or target gene protein level in the subsequent sample or samples are compared to the control/baseline level and/or ratio, respectively, for the subject. Such comparisons can be used to assess onset, progression, or recession of target gen associated disease or condition in the subject. For example, thrombus level in the baseline sample obtained from the subject that is higher than thrombus determined in a sample obtained from the same subject after the subject has been administered an target gene dsRNA agent or antisense polynucleotide agent of the invention indicates regression of the target gene-associated disease or condition and indicates efficacy of the administered target gene dsRNA agent of the invention for treatment of the target gene associated disease or condition.

Some embodiments of the invention, include determining presence, absence, and/or a change in a physiological characteristic of a target gene -associated disease or condition using methods such as but not limited to: (1) assessing one or more biological samples obtained from one or more subjects for the physiological characteristic; (2) imaging a subject (for example but not limited to obtaining a liver image); and (3) or physical examination of the subject. The determination can be used to assess efficacy of a treatment method of the invention.

### The term

As used herein, the term "RNAi" is also known in the art and may be referred to as "siRNA" which is also a short strand of "RNAi". As used herein, the term "RNAi" refers to an agent that comprises RNA and mediates targeted cleavage of an RNA transcript via an RNA-induced silencing complex (RISC) pathway. As is known in the art, an RNAi a target region refers to a contiguous portion of the nucleotide sequence of an mRNA molecule formed during the transcription of a gene, including messenger RNA (mRNA) that is a product of RNA processing of a primary transcription product. The target portion of the sequence will be at least long enough to serve as a substrate for RNAi-directed cleavage at or near that portion. A target sequence may be from 8-30 nucleotides long (inclusive), from 10 - 30 nucleotides long (inclusive), from 12 - 25 nucleotides long (inclusive), from 15 - 23 nucleotides long (inclusive), from 16 -23 nucleotides long (inclusive), or from 18 - 23 nucleotides long (inclusive), including all shorter lengths within each stated range. In some embodiments of the invention, a target sequence is 9, 10, 11, 12, 13, 14, 15, 16, 17, 18, 19, 20, 21, 22, 23, 24, 25, or 26 nucleotides long. In certain embodiment a target sequence is between 9 and 26 nucleotides long (inclusive), including all sub-ranges and integers there between. For example, though not intended to be limiting, in certain embodiments of the invention a target sequence is 8, 9, 10, 11, 12, 13, 14, 15, 16, 17, 18, 19, 20, 21, 22, 23, 24, 25, 26, 27, 28, 29, or 30 nucleotides long, with the sequence fully or at least substantially complementary to at least part of an RNA transcript of target gene.

As used herein, a "dsRNA agent" means a composition that contains an RNA or RNA-like (e.g., chemically modified RNA) oligonucleotide molecule that is capable of degrading or inhibiting translation of messenger RNA (mRNA) transcripts of a target mRNA in a sequence specific manner. Although not wishing to be limited to a particular theory, dsRNA agents of the invention may operate through the RNA interference mechanism (i.e., inducing RNA interference through interaction with the RNA interference pathway machinery (RNA-induced silencing complex or RISC) of mammalian cells), or by any alternative mechanism(s) or pathway(s). Methods for silencing genes in plant, invertebrate, and vertebrate cells are well known in the art [see, for example, (Sharp et al., Genes Dev. 2001, 15:485; Bernstein, et al., (2001) Nature 409:363; Nykanen, et al., (2001) Cell 107:309; and Elbashir, et al., (2001) Genes Dev. 15:188)], the disclosure of each of which is incorporated herein by reference in its entirety.

As used herein, DsRNA agents disclosed herein are comprised of a sense strand and an antisense strand, and include, but are not limited to: short interfering RNAs (siRNAs), RNAi agents, micro RNAs (miRNAs), short hairpin RNAs (shRNA), and dicer substrates. The antisense strand of the dsRNA agents described herein is at least partially complementary to the mRNA being targeted. It is understood in the art that different lengths of dsRNA duplex structure can be used to inhibit target gene expression. For example, dsRNAs having a duplex structure of 19, 20, 21, 22, and 23 base pairs are known to be effective to induce RNA interference (Elbashir et al., EMBO 2001, 20:6877-6888). It is also known in the art that shorter or longer RNA duplex structures are also effective to induce RNA interference.

As used herein, certain embodiments of the compositions and methods of the invention comprise single-stranded RNA in the composition and/or administer single-stranded RNA to a subject. Single-stranded antisense molecules that may be included in certain compositions of the invention and/or administered in certain methods of the invention are referred to herein as "single-stranded antisense agents" or "antisense polynucleotide agents" , "antisense single-stranded nucleotide reagent" is also referred to as "ASO". Single-stranded sense molecules that may be included in certain compositions and/or administered in certain methods of the invention are referred to herein as "single-stranded sense agents" or "sense polynucleotide agents. "

The term "base sequence" is used herein in reference to a polynucleotide sequence without chemical modifications or delivery compounds.

As used herein, the term "matching position" in a sense and an antisense strand are the positions in each strand that "pair" when the two strands are duplexed strands. For example, in a 21 nucleobases sense strand and a 21 nucleobases antisense strand, nucleobase in position 1 of the sense strand and position 21 in the antisense strand are in "matching positions". In yet another non-limiting example in a 23 nucleobases sense strand and a 23 nucleobases antisense strand, nucleobase 2 of the sense strand and position 22 of the antisense strand are in matching positions. In another non-limiting example, in an 18 nucleobases sense strand and an 18 nucleobases antisense strand, nucleobase in position 1 of the sense strand and nucleobase 18 in the antisense strand are in matching positions, and nucleobase 4 in the sense strand and nucleobase 15 in the antisense strand are in matching positions. A skilled artisan will understand how to identify matching positions in sense and antisense strands that are or will be duplexed strands and paired strands.

### Mismatches

It is known to skilled in art, mismatches are tolerated for efficacy in dsRNA, especially the mismatches are within terminal region of dsRNA. Certain mismatches tolerate better, for example mismatches with wobble base pairs G:U and A:C are tolerated better for efficacy (Du et el., A systematic analysis of the silencing effects of an active siRNA at all single-nucleotide mismatched target sites. Nucleic Acids Res. 2005 Mar 21;33(5):1671-7. Doi: 10.1093/nar/gki312. Nucleic Acids Res. 2005;33(11):3698).

### Complementarity

As used herein, unless otherwise indicated, the term "complementary," when used to describe a first nucleotide sequence (e.g., dsRNA agent sense strand or targeted mRNA) in relation to a second nucleotide sequence (e.g., dsRNA agent antisense strand or a single-stranded antisense polynucleotide), means the ability of an oligonucleotide or polynucleotide including the first nucleotide sequence to hybridize [form base pair hydrogen bonds under mammalian physiological conditions (or similar conditions *in vitro*)] and form a duplex or double helical structure under certain conditions with an oligonucleotide or polynucleotide including the second nucleotide sequence. Other conditions, such as physiologically relevant conditions as can be encountered inside an organism, can apply. A skilled artisan will be able to determine the set of conditions most appropriate for a test of complementarity of two sequences in accordance with the ultimate application of the hybridized nucleotides. Complementary sequences include Watson-Crick base pairs or non-Watson-Crick base pairs and include natural or modified nucleotides or nucleotide mimics, at least to the extent that the above hybridization requirements are fulfilled. Sequence identity or complementarity is independent of modification.

Complementary sequences, for example, within a target gene dsRNA as described herein, include base-pairing of the oligonucleotide or polynucleotide comprising a first nucleotide sequence to an oligonucleotide or polynucleotide comprising a second nucleotide sequence over the entire length of one or both nucleotide sequences. Such sequences can be referred to as "fully complementary" with respect to each other herein. It will be understood that in embodiments when two oligonucleotides are designed to form, upon hybridization, one or more single stranded overhangs, such overhangs are not regarded herein as mismatches with regard to the determination of complementarity. For example, a target gene dsRNA agent comprising one oligonucleotide 19 nucleotides in length and another oligonucleotide 20 nucleotides in length, wherein the longer oligonucleotide comprises a sequence of 19 nucleotides that is fully complementary to the shorter oligonucleotide, can yet be referred to as "fully complementary" for the purposes described herein. Thus, as used herein, "fully complementary" means that all (100%) of the bases in a contiguous sequence of a first polynucleotide will hybridize with the same number of bases in a contiguous sequence of a second polynucleotide.

The term "substantially complementary" as used herein means that in a hybridized pair of nucleobase sequences, at least about 85%, but not all, of the bases in a contiguous sequence of a first polynucleotide will hybridize with the same number of bases in a contiguous sequence of a second polynucleotide. The term "substantially complementary" can be used in reference to a first sequence with respect to a second sequence if the two sequences include one or more, for example at least 1, 2, 3, 4, or 5 mismatched base pairs upon hybridization for a duplex up to 15, 16, 17, 18, 19, 20, 21, 22, 23, 24, 25, 26, 27, 28, 29 or 30 base pairs (bp), while retaining the ability to hybridize under the conditions most relevant to their ultimate application, e.g., inhibition of target gene expression via a RISC pathway. The term, "partially complementary" may be used herein in reference to a hybridized pair of nucleobase sequences, in which at least 75%, but not all, of the bases in a contiguous sequence of a first polynucleotide will hybridize with the same number of bases in a contiguous sequence of a second polynucleotide. In some embodiments, "partially complementary" means at least 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% of the bases in a contiguous sequence of a first polynucleotide will hybridize with the same number of bases in a contiguous sequence of a second polynucleotide.

The terms "complementary," "fully complementary," "substantially complementary," and "partially complimentary" are used herein in reference to the base matching between the sense strand and the antisense strand of a target gene dsRNA agent, between the antisense strand of a target gene dsRNA agent and a sequence of a target mRNA, or between a single-stranded antisense oligonucleotide and a sequence of a target gene mRNA. It will be understood that the term "antisense strand of a target gene dsRNA agent" may refer to the same sequence of an "target gene antisense polynucleotide agent".

As used herein, the term "substantially identical" or "substantial identity" used in reference to a nucleic acid sequence means a nucleic acid sequence comprising a sequence with at least about 85% sequence identity or more, preferably at least 90%, at least 91%, at least 92%, at least 93%, at least 94%, at least 95%, at least 96%, at least 97%, at least 98%, or at least 99%, compared to a reference sequence. Percentage of sequence identity is determined by comparing two optimally aligned sequences over a comparison window. The percentage is calculated by determining the number of positions at which the identical nucleic acid base occurs in both sequences to yield the number of matched positions, dividing the number of matched positions by the total number of positions in the window of comparison and multiplying the result by 100 to yield the percentage of sequence identity.

As used herein, the term "strand comprising a sequence" means an oligonucleotide comprising a chain of nucleotides that is described by the sequence referred to using the standard nucleotide nomenclature. The term "double-stranded RNA" or "dsRNA," as used herein, refers to an RNAi that includes an RNA molecule or complex of molecules having a hybridized duplex region comprising two anti-parallel and substantially or fully complementary nucleic acid strands, which are referred to as having "sense" and "antisense" orientations with respect to a target RNA. The duplex region can be of any length that permits specific degradation of a desired target RNA through a RISC pathway, but will typically range from 9 to 30 base pairs in length, e.g., 15-30 base pairs in length. Considering a duplex between 9 and 30 base pairs, the duplex can be any length in this range, for example, 9, 10, 11, 12, 13, 14, 15, 16, 17, 18, 19, 20, 21, 22, 23, 24, 25, 26, 27, 28, 29, or 30, and any sub-range therein between, including, but not limited to 15-30 base pairs, 15-26 base pairs, 15-23 base pairs, 15-22 base pairs, 15-21 base pairs, 15-20 base pairs, 15-19 base pairs, 15-18 base pairs, 15-17 base pairs, 18-30 base pairs, 18-26 base pairs, 18-23 base pairs, 18-22 base pairs, 18-21 base pairs, 18-20 base pairs, 19-30 base pairs, 19-26 base pairs, 19-23 base pairs, 19-22 base pairs, 19-21 base pairs, 19-20 base pairs, 20-30 base pairs, 20-26 base pairs, 20-25 base pairs, 20-24 base pairs, 20-23 base pairs, 20-22 base pairs, 20-21 base pairs, 21-30 base pairs, 21-26 base pairs, 21-25 base pairs, 21-24 base pairs, 21-23 base pairs, or 21-22 base pairs. dsRNA agents generated in the cell by processing with Dicer and similar enzymes are generally in the range of 19-22 base pairs in length. One strand of the duplex region of a target gene dsDNA agent comprises a sequence that is substantially complementary to a region of a target RNA. The two strands forming the duplex structure can be from a single RNA molecule having at least one self-complementary region, or can be formed from two or more separate RNA molecules. Where the duplex region is formed from two strands of a single molecule, the molecule can have a duplex region separated by a single stranded chain of nucleotides (herein referred to as a "hairpin loop") between the 3'-end of one strand and the 5'-end of the respective other strand forming the duplex structure. In some embodiments of the invention, a hairpin look comprises at least 1, 2, 3, 4, 5, 6, 7, 8, 9, 10, 11, 12, 13, 14, 15, 16, 17, 18, 19, 20, or more unpaired nucleotides. Where the two substantially complementary strands of a dsRNA agent are comprised by separate RNA molecules, those molecules need not, but can be covalently connected. Where the two strands are connected covalently by means other than a hairpin loop, the connecting structure is referred to as a "linker."

### Base pairing

The term "base pairing", in some non-limiting interpretations herein, for example: dsRNA reagents may contain complementary or mismatched base pairs in sense and antisense sequences.

### Blunt and overhang

In some embodiments of the invention a dsRNA agent may include a sense and antisense sequence that have no-unpaired nucleotides or nucleotide analogs at one or both terminal ends of the dsRNA agent. An end with no unpaired nucleotides is referred to as a "blunt end" and as having no nucleotide overhang. If both ends of a dsRNA agent are blunt, the dsRNA is referred to as "blunt ended." In some embodiments of the invention, a first end of a dsRNA agent is blunt, in some embodiments a second end of a dsRNA agent is blunt, and in certain embodiments of the invention, both ends of a dsRNA agent are blunt.

In some embodiments of dsRNA agents of the invention, the dsRNA does not have one or two blunt ends. In such instances there is at least one unpaired nucleotide at the end of a strand of a dsRNA agent. For example, when a 3'-end of one strand of a dsRNA extends beyond the 5'-end of the other strand, or vice versa, there is a nucleotide overhang. A dsRNA can comprise an overhang of at least e1, 2, 3, 4, 5, 6, or more nucleotides. A nucleotide overhang can comprise or consist of a nucleotide/nucleoside analog, including a deoxynucleotide/nucleoside. It will be understood that in some embodiments a nucleotide overhang is on a sense strand of a dsRNA agent, on an antisense strand of a dsRNA agent, or on both ends of a dsRNA agent and nucleotide(s) of an overhang can be present on the 5' end, 3' end or both ends of either an antisense or sense strand of a dsRNA.

As used herein, the term "antisense strand" or "guide strand" refers to the strand of the dsRNA agent that includes a region that is substantially complementary to a target sequence. As used herein the term "sense strand," or "passenger strand" refers to the strand of a dsRNA agent that includes a region that is substantially complementary to a region of the antisense strand of the dsRNA agent.

### Modifications

In some embodiments of the invention the RNA of a traget gene RNAi agent is chemically modified to enhance stability and/or one or more other beneficial characteristics. Nucleic acids in certain embodiments of the invention may be synthesized and/or modified by methods well established in the art, for example, those described in "Current protocols in Nucleic Acid Chemistry," Beaucage, S. L. et al. (Eds.), John Wiley & Sons, Inc., New York, N.Y., USA, which is incorporated herein by reference. Modifications that can be present in certain embodiments of dsRNA agents of the invention include, for example, (a) end modifications, e.g., 5' end modifications (phosphorylation, conjugation, inverted linkages, etc.) , 3' end modifications (conjugation, DNA nucleotides, inverted linkages, etc.), (b) base modifications, e.g., replacement with stabilizing bases, destabilizing bases, or bases that base pair with an expanded repertoire of partners, removal of bases (abasic nucleotides), or conjugated bases, (c) sugar modifications (e.g., at the 2' position or 4' position) or replacement of the sugar, as well as (d) backbone modifications, including modification or replacement of the phosphodiester linkages. Specific examples of RNA compounds useful in certain embodiments of traget gene dsRNA agents, antisense polynucleotides, and sense polynucleotides of the invention include, but are not limited to RNAs comprising modified backbones or no natural internucleoside linkages. As a non-limiting example, an RNA having a modified backbone may not have a phosphorus atom in the backbone. RNAs that do not have a phosphorus atom in their internucleoside backbone may be referred to as oligonucleosides. In certain embodiments of the invention, a modified RNA has a phosphorus atom in its internucleoside backbone.

It will be understood that the term "RNA molecule" or "RNA" or "ribonucleic acid molecule" encompasses not only RNA molecules as expressed or found in nature, but also analogs and derivatives of RNA comprising one or more ribonucleotide/ribonucleoside analogs or derivatives as described herein or as known in the art. The terms "ribonucleoside" and "ribonucleotide" may be used interchangeably herein. An RNA molecule can be modified in the nucleobase structure or in the ribose-phosphate backbone structure, e.g., as described herein below, and molecules comprising ribonucleoside analogs or derivatives must retain the ability to form a duplex.

As used herein, the term " fluoro modified nucleotide" refers to a nucleotide in which the hydroxyl group at the 2' position of the ribose group of a nucleotide is replaced by fluoro, and "non-fluoro modified nucleotide" refers to a nucleoside Nucleotides or nucleotide analogs formed by replacing the hydroxyl group at the 2' position of the ribose group of an acid with a non-fluoro group. "Nucleotide analog" means a nucleic acid capable of replacing nucleotides, but is structurally different from adenine ribonucleotides, guanine ribonucleotides, cytosine ribonucleotides, uracil ribonucleotides, or thymus. The group of pyrimidine deoxyribonucleotides. Such as isonucleotides, bridged nucleic acid (BNA), or acyclic nucleotides. The methoxy-modified nucleotide refers to a nucleotide in which the 2'-hydroxyl of the ribose group is replaced by a methoxy group. Isonucleotide refers to a compound formed by changing the position of the base in the nucleotide on the ribose ring. In some embodiments, the ribonucleotide may be a compound formed by moving a base from the 1'-position to the 2'-position or the 3'-position of the ribose ring. BNA refers to constrained or inaccessible nucleotides. BNAs may contain five-membered, six-membered, or seven-membered ring-bridged structures with "fixed" C3'-endosugar constrictions. Typically the bridge is incorporated at the 2'-,4'-position of the ribose to provide a 2',4'-BNA nucleotide. In some embodiments, the BNA can be LNA, ENA, cET BNA, etc. Acyclic nucleotides are a type of nucleotide formed by opening the sugar ring of the nucleotide. In some embodiments, the acyclic nucleotide may be an unlocked nucleic acid (UNA) or a glycerol nucleic acid (GNA).

As non-limiting examples, an RNA molecule can also include at least one modified ribonucleoside including but not limited to a 2'-O-methyl modified nucleoside, a nucleoside comprising a 5' phosphorothioate group, a terminal nucleoside linked to a cholesteryl derivative or dodecanoic acid bisdecylamide group, a locked nucleoside, an abasic nucleoside, a 2'-deoxy-2'-fluoro modified nucleoside, a 2'-amino-modified nucleoside, 2'-alkyl-modified nucleoside, morpholino nucleoside, a phosphoramidate or a non-natural base comprising nucleoside, or any combination thereof. In some embodiments of the invention, an RNA molecule comprises at least 1, 2, 3, 4, 5, 6, 7, 8, 9, 10, 11, 12, 13, 14, 15, 16, 17, 18, 19, 20, or 21 up to the full length of the target gene dsRNA agent molecule's ribonucleosides that are modified ribonucleosides. The modifications need not be the same for each of such a plurality of modified ribonucleosides in an RNA molecule.

In some embodiments of the invention dsRNA agents, antisense polynucleotides, and/or sense polynucleotides of the invention may, in some embodiments comprise one or more independently selected modified nucleotide and/or one or more independently selected non-phosphodiester linkage. As used herein the term "independently selected" used in reference to a selected element, such as a modified nucleotide, non-phosphodiester linkage, etc., means that two or more selected elements can but need not be the same as each other. As used herein, a "nucleotide base," "nucleotide," or "nucleobase" is a heterocyclic pyrimidine or purine compound, which is a standard constituent of all nucleic acids, and includes the bases that form the nucleotides adenine (a), guanine (g), cytosine (c), thymine (t), and uracil (u). A nucleobase may further be modified to include, though not intended to be limiting: universal bases, hydrophobic bases, promiscuous bases, size-expanded bases, and fluorinated bases. The term "ribonucleotide" or "nucleotide" may be used herein to refer to an unmodified nucleotide, a modified nucleotide, or a surrogate replacement moiety. Those in the art will recognize that guanine, cytosine, adenine, and uracil can be replaced by other moieties without substantially altering the base pairing properties of an oligonucleotide comprising a nucleotide bearing such replacement moiety.

In one embodiment, modified RNAs contemplated for use in methods and compositions described herein are peptide nucleic acids (PNAs) that have the ability to form the required duplex structure and that permit or mediate the specific degradation of a target RNA via a RISC pathway. In certain embodiments of the invention, a RNA interference agent includes a single stranded RNA that interacts with a target gene RNA sequence to direct the cleavage of the target RNA.

Modified RNA backbones can include, for example, phosphorothioates, chiral phosphorothioates, phosphorodithioates, phosphotriesters, aminoalkylphosphotriesters, methyl and other alkyl phosphonates including 3'-alkylene phosphonates and chiral phosphonates, phosphinates, phosphoramidates including 3'-amino phosphoramidate and aminoalkylphosphoramidates, thionophosphoramidates, thionoalkylphosphonates, thionoalkylphosphotriesters, and boranophosphates having normal 3'-5' linkages, 2'-5' linked analogs of these, and those) having inverted polarity wherein the adjacent pairs of nucleoside units are linked 3'-5' to 5'-3' or 2'-5' to 5'-2'. Various salts, mixed salts and free acid forms are also included. Means of preparing phosphorus-containing linkages are routinely practiced in the art and such methods can be used to prepare certain modified dsRNA agents, certain modified antisense polynucleotides, and/or certain modified sense polynucleotides of the invention.

Modified RNA backbones that do not include a phosphorus atom therein have backbones that are formed by short chain alkyl or cycloalkyl internucleoside linkages, mixed heteroatoms and alkyl or cycloalkyl internucleoside linkages, or one or more short chain heteroatomic or heterocyclic internucleoside linkages. These include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; alkene containing backbones; sulfamate backbones; methyleneimino and methylenehydrazino backbones; sulfonate and sulfonamide backbones; amide backbones; and others having mixed N, O, S and CH2 component parts. Means of preparing modified RNA backbones that do not include a phosphorus atom are routinely practiced in the art and such methods can be used to prepare certain modified dsRNA agents, certain modifiedantisense polynucleotides, and/or certain modified sense polynucleotides of the invention.

In certain embodiments of the invention, RNA mimetics are included in dsRNAs, antisense polynucleotides, and/orsense polynucleotides, such as, but not limited to: replacement of the sugar and the internucleoside linkage, i.e., the backbone, of the nucleotide units with novel groups. In such embodiments, base units are maintained for hybridization with an appropriate nucleic acid target compound. One such oligomeric compound, an RNA mimetic that has been shown to have excellent hybridization properties, is referred to as a peptide nucleic acid (PNA). In PNA compounds, the sugar backbone of an RNA is replaced with an amide containing backbone, in particular an aminoethylglycine backbone. The nucleobases are retained and are bound directly or indirectly to aza nitrogen atoms of the amide portion of the backbone. Means of preparing RNA mimetics are routinely practiced in the art and such methods can be used to make certain modified dsRNA agents of the invention.

Some embodiments of the invention include RNAs with phosphorothioate backbones and oligonucleosides with heteroatom backbones, and in particular -CH₂-NH-CH₂-, -CH₂-N(CH₃)-O-CH₂-[known as a methylene (methylimino) or MMI backbone], -CH₂-O-N(CH₃)-CH₂-, -CH₂-N(CH₃)-N(CH₃)-CH₂- and -N(CH₃)-CH₂- [wherein the native phosphodiester backbone is represented as -O-P-O-CH₂-]. Means of preparing RNAs with phosphorothioate backbones and oligonucleosides with heteroatom backbones are routinely practiced in the art and such methods can be used to prepare certain modified dsRNA agents, certain antisense polynucleotides, and/or certain sense polynucleotides of the invention.

Modified RNAs can also contain one or more substituted sugar moieties. dsRNAs, antisense polynucleotides, and/or sense polynucleotides of the invention may comprise one of the following at the 2' position: OH; F; O-, S-, or N-alkyl; O-, S-, or N-alkenyl; O-, S- or N-alkynyl; or O-alkyl-O-alkyl, wherein the alkyl, alkenyl and alkynyl may be substituted or unsubstituted C1 to C10 alkyl or C2 to C10 alkenyl and alkynyl. Exemplary suitable modifications include O[(CH₂)ₙO]ₘCH₃, O(CH₂)ₙOCH₃, O(CH₂)ₙNH₂, O(CH₂)ₙCH₃, O(CH₂)ₙONH₂, and O(CH₂)ₙON[(CH₂)ₙCH₃)]₂, where n and m are from 1 to about 10. In other embodiments, dsRNAs include one of the following at the 2' position: C1 to C10 lower alkyl, substituted lower alkyl, alkaryl, aralkyl, O-alkaryl or O-aralkyl, SH, SCH₃, OCN, Cl, Br, CN, CF₃, OCF₃, SOCH₃, SO₂CH₃, ONO₂, NO₂, N₃, NH₂, heterocycloalkyl, heterocycloalkaryl, aminoalkylamino, polyalkylamino, substituted silyl, an RNA cleaving group, a reporter group, an intercalator, a group for improving the pharmacokinetic properties of a dsRNA agent, or a group for improving the pharmacodynamic properties of a dsRNA agent, antisense polynucleotide, and/or sense polynucleotide, and other substituents having similar properties. In some embodiments, the modification includes a 2'-methoxyethoxy (2'-O-CH₂CH₂OCH₃, also known as 2'-O-(2-methoxyethyl) or 2'-MOE) (Martin et al., Helv. Chim. Acta, 1995, 78:486-504) i.e., an alkoxy-alkoxy group. Another exemplary modification is 2'-dimethyl aminooxyethoxy, i.e., a O(CH₂) ₂ON(CH₃)₂ group, also known as 2'-DMAOE, as described in examples herein below, and 2'-dimethylaminoethoxyethoxy (also known in the art as 2'-O-dimethylaminoethoxyethyl or 2'-DMAEOE), i.e., 2'-O-CH₂-O- CH₂-N(CH₂)₂. Means of preparing modified RNAs such as those described are routinely practiced in the art and such methods can be used to prepare certain modified dsRNA agents of the invention.

Similar modifications can also be made at other positions on the RNA of a dsRNA agent, antisense polynucleotide, and/or sense polynucleotide of the invention, particularly the 3' position of the sugar on the 3' terminal nucleotide or in 2'-5' linked dsRNAs, antisense polynucleotides, or sense polynucleotides, and the 5' position of 5' terminal nucleotide. dsRNA agents, antisense polynucleotides, and/or sense polynucleotides may also have sugar mimetics such as cyclobutyl moieties in place of the pentofuranosyl sugar. Means of preparing modified RNAs such as those described are routinely practiced in the art and such methods can be used to prepare certain modified dsRNA agents, antisense polynucleotides, and/or sense polynucleotides of the invention.

In certain embodiments of the invention, dsRNA agent, antisense polynucleotide, and/or sense polynucleotide may, in some embodiments, include nucleobase (often referred to in the art simply as "base") modifications or substitutions. As used herein, "unmodified" or "natural" nucleobases include the purine bases adenine (A) and guanine (G), and the pyrimidine bases thymine (T), cytosine (C) and uracil (U). Modified nucleobases include other synthetic and natural nucleobases such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl uracil and cytosine, 6-azo uracil, cytosine and thymine, 5-uracil (pseudouracil), 4-thiouracil, 8-halo, 8-amino, 8-thiol, 8-thioalkyl, 8-hydroxyl anal other 8-substituted adenines and guanines, 5-halo, particularly 5-bromo, 5-trifluoromethyl and other 5-substituted uracils and cytosines, 7-methylguanine and 7-methyladenine, 8-azaguanine and 8-azaadenine, 7-deazaguanine and 7-daazaadenine and 3-deazaguanine and 3-deazaadenine. Additional nucleobases that may be included in certain embodiments of dsRNA agents of the invention are known in the art, see for example: Modified Nucleosides in Biochemistry, Biotechnology and Medicine, Herdewijn, P. Ed. Wiley-VCH, 2008; The Concise Encyclopedia Of Polymer Science And Engineering, pages 858-859, Kroschwitz, J. L, Ed. John Wiley & Sons, 1990, English et al., Angewandte Chemie, International Edition, 1991, 30, 613, Sanghvi, Y S., Chapter 15, dsRNA Research and Applications, pages 289-302, Crooke, S. T. and Lebleu, B., Ed., CRC Press, 1993. Means of preparing dsRNAs, antisense strand polynucleotides and/or sense strand polynucleotides that comprise nucleobase modifications and/or substitutions such as those described herein are routinely practiced in the art and such methods can be used to prepare certain modified dsRNA agents, sense polynucleotides, and/or antisense polynucleotides of the invention.

Certain embodiments of dsRNA agents, antisense polynucleotides, and/or sense polynucleotides of the invention include RNA modified to include one or more locked nucleic acids (LNA). A locked nucleic acid is a nucleotide with a modified ribose moiety comprising an extra bridge connecting the 2' and 4' carbons. This structure effectively "locks" the ribose in the 3'-endo structural conformation. The addition of locked nucleic acids in a dsRNA agent, antisense polynucleotides, and/or sense polynucleotides of the invention may increase stability in serum, and to reduce off-target effects (Elmen, J. et al., (2005) Nucleic Acids Research 33(1):439-447; Mook, O R. et al., (2007)Mol Canc Ther 6(3):833-843; Grunweller, A. et al., (2003) Nucleic Acids Research 31(12):3185-3193). Means of preparing dsRNA agents, antisense polynucleotides, and/or sense polynucleotides that comprise locked nucleic acid(s) are routinely practiced in the art and such methods can be used to prepare certain modified dsRNA agents of the invention. Certain embodiments of dsRNA compounds, sense polynucleotides, and/or antisense polynucleotides of the invention, include at least one modified nucleotide, wherein the at least one modified nucleotide comprises: a 2'-O-methyl nucleotide,2'-Fluoro nucleotide, 2'-deoxy nucleotide, 2'3'-seco nucleotide mimic, locked nucleotide, 2'-F-Arabino nucleotide, 2'-methoyxyethyl nucleotide, 2'-amino-modified nucleotide, 2'-alkyl-modified nucleotide, mopholino nucleotide, and 3'-Ome nucleotide, a nucleotide comprising a 5'-phosphorothioate group, or a terminal nucleotide linked to a cholesteryl derivative or dodecanoic acid bisdecylamide group, a 2'-amino-modified nucleotide, a phosphoramidate, or a non-natural base comprising nucleotide. In some embodiments, a dsRNA compound includes an E-vinylphosphonate nucleotide at the 5' end of the antisense strand, also referred to herein as the guide strand.

Certain embodiments of dsRNA compounds, 3' and 5' end of sense polynucleotides, and/or 3' end of antisense polynucleotides of the invention, include at least one modified nucleotide, wherein the at least one modified nucleotide comprises: abasic nucleotide, ribitol, inverted nucleotide, inverted abasic nucleotide, inverted 2'-OMe nucleotide, inverted 2'-deoxy nucleotide. It is known to skilled in art, including an abasic or inverted abasic nucleotide at the end of oligonucleotide enhances stability (Czauderna et al. Structural variations and stabilizing modifications of synthetic siRNAs in mammalian cells. Nucleic Acids Res. 2003;31(11):2705-2716. doi:10.1093/nar/gkg393).

Certain embodiments of dsRNA compounds, antisense polynucleotides of the invention, include at least one modified nucleotide, wherein the at least one modified nucleotide comprises unlocked nucleic acid nucleotide (UNA) or/and glycol nucleic acid nucleotide (GNA). It is known to skilled in art, UNA and GNA are thermally destabilizing chemical modifications, can significantly improves the off-target profile of a siRNA compound (Janas, et al., Nat Commun. 2018;9(1):723. doi:10.1038/s41467-018-02989-4; Laursen et al., Mol BioSyst. 2010;6:862-70).

Certain embodiments of Unlocked nucleic acid (UNA) polynucleotides of the invention,. Unlocked nucleic acid (UNA) is an acyclic analog of RNA in which the bond between the C2' of the ribose ring and the target gene' atom has been severed. Incorporation of UNA has been shown to be well tolerated and in some cases even enhance the activity of siRNA gene silencing (Meghan A. et al. "Locked vs. unlocked nucleic acids (LNA vs. UNA): contrasting structures work towards common therapeutic goals". Chem. Soc. Rev., 2011, 40, 5680-5689).

UNA is a thermolabile modification, and replacing ribonucleotides with UNA reduces base-pairing strength and duplex stability. Strategically placing UNA at the seed region of the antisense strand of siRNA can reduce off-target activity in the mechanism of gene silencing mediated by microRNA (miRNA). miRNA mainly recognizes target genes through base pairing between the antisense seed region (2-8 from the 5' end) and target mRNA for gene suppression. Each miRNA potentially regulates a large number of genes. The siRNA antisense strand loaded by the RNA-induced silencing complex (RISC) can also potentially regulate a large number of unintended genes through miRNA-mediated mechanisms . Therefore, adding thermolabile nucleotides, such as UNA, to the seed region of siRNA can reduce off-target activity (Lam JK, Chow MY, Zhang Y, Leung SW. siRNA Versus miRNA as Therapeutics for Gene Silencing. Mol Ther Nucleic Acids. 2015 Sep 15;4(9):e252. doi: 10.1038/mtna.2015.23. PMID: 26372022; PMCID: PMC4877448.). In particular, such RNA oligonucleotides or complexes of RNA oligonucleotides contain at least one UNA nucleotide monomer in the seed region (Narendra Vaish et al. "Improved specificity of gene silencing by siRNAs containing unlocked nucleobase analog". Nucleic Acids Research, 2011, Vol. 39, No. 5 1823-1832).

According to the present technology, potential advantages of incorporating UNA in RNA oligonucleotides or complexes of RNA oligonucleotides include, but are not limited to:
1. Reduce off-target activity. The addition of UNA in the siRNA seed region will reduce the base-pairing strength of the seed region, thereby reducing the potential off-target activity caused by the micro-RNA mechanism.
2. UNA is well tolerated in terms of siRNA activity. In some cases, UNA can lead to enhanced activity.

Exemplary UNA monomers that can be used in this technical solution include, but are not limited to:

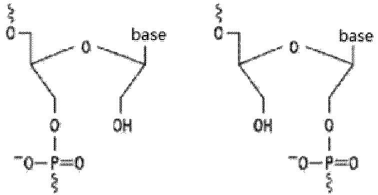

Invab is an inverted abasic (deoxyribose) residue. For example, in some embodiments, the sense or antisense strand can include an "end cap," which, as used herein, can be at one or more ends of a strand of an RNAi agent disclosed herein The incorporated non-nucleotide compounds or other moieties, and in some cases may confer certain advantageous properties on the RNAi agent, such as, for example, protection against degradation by exonucleases. In some embodiments, inverted abasic residues (Invab) are added as end caps (see, Czauderna et al. Structural variations and stabilizing modifications of synthetic siRNAs in mammalian cells. Nucleic Acids Res. 2003;31(11):2705-2716). In some embodiments, a dsRNA compound includes one or more inverted abasic residues (invab) at either 3'-end or 5'-end, or both 3'-end and 5'-end. Exemplified inverted abasic residues (invab) include, but are not limited to the following:
when at the 3'-end: 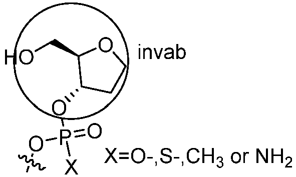 link to 3'-end of oligonucleotide

Another modification that may be included in the RNA of certain embodiments of dsRNA agents, antisense polynucleotides, and/or sense polynucleotides of the invention, comprises chemically linking to the RNA one or more ligands, moieties or conjugates that enhance one or more characteristics of the dsRNA agent, antisense polynucleotide, and/or sense polynucleotide, respectively. Non-limiting examples of characteristics that may be enhanced are: dsRNA agent, antisense polynucleotide, and/or sense polynucleotide activity, cellular distribution, delivery of a dsRNA agent, pharmacokinetic properties of a dsRNA agent, and cellular uptake of the dsRNA agent. In some embodiments of the invention, a dsRNA agent comprises one or more targeting groups or linking groups, which in certain embodiments of dsRNA agents of the invention are conjugated to the sense strand. A non-limiting example of a targeting group is a compound comprising N-acetyl-galactosamine (GalNAc). The terms "targeting group", "targeting agent", "linking agent", "targeting compound", and "targeting ligand" may be used interchangeably herein. In certain embodiments of the invention a dsRNA agent comprises a targeting compound that is conjugated to the 5'-terminal end of the sense strand. In certain embodiments of the invention a dsRNA agent comprises a targeting compound that is conjugated to the 3'-terminal end of the sense strand. In some embodiments of the invention, a dsRNA agent comprises a targeting group that comprises GalNAc. In certain embodiments of the invention a dsRNA agent does not include a targeting compound conjugated to one or both of the 3'-terminal end and the 5'-terminal end of the sense strand. In certain embodiments of the invention a dsRNA agent does not include a GalNAc containing targeting compound conjugated to one or both of the 5'-terminal end and the 3'-terminal end of the sense strand.

Additional targeting and linking agents are well known in the art, for example, targeting and linking agents that may be used in certain embodiments of the invention include but are not limited to lipid moieties such as a cholesterol moiety (Letsinger et al., Proc. Natl. Acid. Sci. USA, 1989, 86: 6553-6556), cholic acid (Manoharan et al., Biorg. Med. Chem. Let., 1994, 4:1053-1060), a thioether, e.g., beryl-S-tritylthiol (Manoharan et al., Ann. N.Y. Acad. Sci., 1992, 660:306-309; Manoharan et al., Biorg. Med. Chem. Let., 1993, 3:2765-2770), a thiocholesterol (Oberhauser et al., Nucl. Acids Res., 1992, 20:533-538), an aliphatic chain, e.g., dodecandiol or undecyl residues (Saison-Behmoaras et al., EMBO J, 1991, 10:1111-1118; Kabanov et al., FEBS Lett., 1990, 259:327-330; Svinarchuk et al., Biochimie, 1993, 75:49-54), a phospholipid, e.g., di-hexadecyl-rac-glycerol or triethyl-ammonium 1,2-di-O-hexadecyl-rac-glycero-3-phosphonate (Manoharan et al., Tetrahedron Lett., 1995, 36:3651-3654; Shea et al., Nucl. Acids Res., 1990, 18:3777-3783), a polyamine or a polyethylene glycol chain (Manoharan et al., Nucleosides & Nucleotides, 1995, 14:969-973), or adamantane acetic acid (Manoharan et al., Tetrahedron Lett., 1995, 36:3651-3654), a palmityl moiety (Mishra et al., Biochim. Biophys. Acta, 1995, 1264:229-237), or an octadecylamine or hexylamino-carbonyloxycholesterol moiety (Crooke et al., J. Pharmacol. Exp. Ther., 1996, 277:923-937).

Certain embodiments of a composition comprising a dsRNA agent, antisense polynucleotide, and/or sense polynucleotide may comprise a ligand that alters distribution, targeting. In some embodiments of a composition comprising a dsRNA agent of the invention, the ligand increases affinity for a selected target, e.g., molecule, cell or cell type, compartment, e.g., a cellular or organ compartment, tissue, organ or region of the body, as, e.g., compared to a species absent such a ligand. A ligand useful in a composition and/or method of the invention may be a naturally occurring substance, such as a protein (e.g., human serum albumin (HSA), low-density lipoprotein (LDL), or globulin); a carbohydrate (e.g., a dextran, pullulan, chitin, chitosan, inulin, cyclodextrin or hyaluronic acid); or a lipid. A ligand may also be a recombinant or synthetic molecule, such as a synthetic polymer, e.g., a synthetic polyamino acid or polyamine. Examples of polyamino acids are a polylysine (PLL), poly L-aspartic acid, poly L-glutamic acid, styrene-maleic acid anhydride copolymer, poly(L-lactide-co-glycolied) copolymer, divinyl ether-maleic anhydride copolymer, N-(2-hydroxypropyl)methacrylamide copolymer (HMPA), polyethylene glycol (PEG), polyvinyl alcohol (PVA), polyurethane, poly(2-ethylacryllic acid), N-isopropylacrylamide polymers, or polyphosphazine. Example of polyamines include: polyethylenimine, polylysine (PLL), spermine, spermidine, polyamine, pseudopeptide-polyamine, peptidomimetic polyamine, dendrimer polyamine, arginine, amidine, protamine, cationic lipid, cationic porphyrin, quaternary salt of a polyamine, or an alpha helical peptide.

A ligand included in a composition and/or method of the invention may comprise a targeting group, non-limiting examples of which are a cell or tissue targeting agent, e.g., a lectin, glycoprotein, lipid or protein, e.g., an antibody that binds to a specified cell type such as a kidney cell or a liver cell. A targeting group can be a thyrotropin, melanotropin, lectin, glycoprotein, surfactant protein A, Mucin carbohydrate, multivalent lactose, multivalent galactose, N-acetyl-galactosamine, N-acetyl-gulucosamine multivalent mannose, multivalent fucose, glycosylated polyaminoacids, multivalent galactose, transferrin, bisphosphonate, polyglutamate, polyaspartate, a lipid, cholesterol, a steroid, bile acid, folate, vitamin B12, vitamin A, biotin, or an RGD peptide or RGD peptide mimetic.

Other examples of ligands include dyes, intercalating agents (e.g. acridines), cross-linkers (e.g. psoralene, mitomycin C), porphyrins (TPPC4, texaphyrin, Sapphyrin), polycyclic aromatic hydrocarbons (e.g., phenazine, dihydrophenazine), artificial endonucleases (e.g. EDTA), lipophilic molecules, e.g., cholesterol, cholic acid, adamantane acetic acid, 1-pyrene butyric acid, dihydrotestosterone, 1,3-Bis-O(hexadecyl)glycerol, geranyloxyhexyl group, hexadecylglycerol, borneol, menthol, 1,3-propanediol, heptadecyl group, palmitic acid, myristic acid, O3-(oleoyl)lithocholic acid, O3-(oleoyl)cholenic acid, dimethoxytrityl, or phenoxazine) and peptide conjugates (e.g., antennapedia peptide, Tat peptide), alkylating agents, phosphate, amino, mercapto, PEG (e.g., PEG-40K), MPEG, [MPEG]2, polyamino, alkyl, substituted alkyl, radiolabeled markers, enzymes, haptens (e.g., biotin), transport/absorption facilitators (e.g., aspirin, vitamin E, folic acid), synthetic ribonucleases (e.g., imidazole, bisimidazole, histamine, imidazole clusters, acridine-imidazole conjugates, Eu3+ complexes of tetraazamacrocycles), dinitrophenyl, HRP, or AP.

A ligand included in a composition and/or method of the invention may be a protein, e.g., glycoprotein, or peptide, for example a molecule with a specific affinity for a co-ligand, or an antibody, for example an antibody, that binds to a specified cell type such as a cancer cell, endothelial cell, cardiac cell, or bone cell. A ligand useful in an embodiment of a composition and/or method of the invention can be a hormone or hormone receptor. A ligand useful in an embodiment of a composition and/or method of the invention can be a lipid, lectin, carbohydrates, vitamin, cofactos, multivalent lactose, multivalent galactose, N-acetyl-galactosamine, N-acetyl-gulucosamine multivalent mannose, or multivalent fucose. A ligand useful in an embodiment of a composition and/or method of the invention can be a substance that can increase uptake of the dsRNA agent into the cell, for example, by disrupting the cell's cytoskeleton, e.g., by disrupting the cell's microtubules, microfilaments, and/or intermediate filaments.

In some embodiments, a ligand attached to a dsRNA agent of the invention functions as a pharmacokinetic (PK) modulator. An example of a PK modulator that may be used in compositions and methods of the invention includes but is not limited to: a lipophiles, a bile acid, a steroid, a phospholipid analogue, a peptide, a protein binding agent, PEG, a vitamin, cholesterol, a fatty acid, cholic acid, lithocholic acid, dialkylglycerides, diacylglyceride, a phospholipid, a sphingolipid, naproxen, ibuprofen, vitamin E, biotin, an aptamer that binds a serum protein, etc. Oligonucleotides comprising a number of phosphorothioate linkages are also known to bind to serum protein, thus short oligonucleotides, e.g., oligonucleotides of about 5 bases, 10 bases, 15 bases or 20 bases, comprising multiple of phosphorothioate linkages in the backbone may also be used in compositions and/or methods of the invention as ligands.

### Delivery of dsRNA agents and ntisense polynucleotide agents

As used herein the term, "delivery" means facilitating or effecting uptake or absorption into the cell. Absorption or uptake of a dsRNA agent can occur through unaided diffusive or active cellular processes, or by use of delivery agents, targeting agents, etc. that may be associated with a dsRNA agent of the invention. Delivery means that are suitable for use in methods of the invention include, but are not limited to: *in vivo* delivery, in which a dsRNA agent is in injected into a tissue site or administered systemically.

Non-limiting examples of methods that can be used to deliver dsRNA agents to cells, tissues and/or subjects include: dsRNA-GalNAc conjugates, SAMiRNA technology, LNP-based delivery methods, and naked RNA delivery. These and other delivery methods have been used successfully in the art to deliver therapeutic RNAi agents for treatment of various diseases and conditions, such as but not limited to: liver diseases, acute intermittent porphyria (AIP), hemophilia, pulmonary fibrosis, etc. Details of various delivery means are found in publications such as: Nikam, R.R. & K. R. Gore (2018) Nucleic Acid Ther, 28 (4), 209-224 Aug 2018; Springer A.D. & S.F. Dowdy (2018) Nucleic Acid Ther. Jun 1; 28(3): 109-118; Lee, K. et al., (2018) Arch Pharm Res, 41(9), 867-874; and Nair, J.K. et al., (2014) J. Am. Chem. Soc. 136:16958-16961, the content each of which is incorporated by reference herein.

Some embodiments of the invention comprise use of lipid nanoparticles (LNPs) to deliver a dsRNA agent of the invention to a cell, tissue, and/or subject. LNPs are routinely used for *in vivo* delivery of dsRNA agents, including therapeutic dsRNA agents. One benefit of using an LNP or other delivery agent is an increased stability of the RNA agent when it is delivered to a subject using the LNP or other delivery agent. In some embodiments of the invention an LNP comprises a cationic LNP that is loaded with one or more RNAi molecules of the invention. The LNP comprising the RNAi molecule(s) is administered to a subject, the LNPs and their attached RNAi molecules are taken up by cells via endocytosis, their presence results in release of RNAi trigger molecules, which mediate RNAi.

Another non-limiting example of a delivery agent that may be used in embodiments of the invention to delivery a dsRNA agent of the invention to a cell, tissue and/or subject is an agent comprising GalNAc that is attached to a dsRNA agent of the invention and delivers the dsRNA agent to a cell, tissue, and/or subject. Examples of certain additional delivery agents comprising GalNAc that can be used in certain embodiments of methods and composition of the invention are disclosed in PCT Application: WO2020191183A1(incorporated herein in its entirety). A non-limiting example of a GalNAc targeting ligand that can be used in compositions and methods of the invention to deliver a dsRNA agent to a cell is a targeting ligand cluster. Examples of targeting ligand clusters that are presented herein are referred to as: GalNAc Ligand with phosphodiester link (GLO) and GalNAc Ligand with phosphorothioate link (GLS).

In addition to certain delivery means described herein, it will be understood that RNAi delivery means, such as but not limited to those described herein and those used in the art, can be used in conjunction with embodiments of RNAi agents and treatment methods described herein.

### Vector Encoded dsRNAs

In certain embodiments of the invention, a dsRNA agent can be delivered into a cell using a vector. dsRNA agent transcription units can be included in a DNA or RNA vector. Prepare and use of such vectors encoding transgenes for delivering sequences into a cell and or subject are well known in the art. Vectors can be used in methods of the invention that result in transient expression of dsRNA, for example for at least 1, 2, 3, 4, 5, 6, 7, 8, 9, 10 or more hours, 1, 2, 3, 4, 5, 6, 7, 8, 9, 10 or more weeks. The length of the transient expression can be determined using routine methods based on elements such as, but not limited to the specific vector construct selected and the target cell and/or tissue. Such transgenes can be introduced as a linear construct, a circular plasmid, or a viral vector, which can be an integrating or non-integrating vector. The transgene can also be constructed to permit it to be inherited as an extrachromosomal plasmid (Gassmann, et al., Proc. Natl. Acad. Sci. USA (1995) 92:1292).

An individual strand or strands of a dsRNA agent can be transcribed from a promoter on an expression vector. Where two separate strands are to be expressed to generate, for example, a dsRNA, two separate expression vectors can be co-introduced to a cell using means such as transfection or infection. In certain embodiments each individual strand of a dsRNA agent of the invention can be transcribed by promoters that are both included on the same expression vector. In certain embodiments of the invention a dsRNA agent is expressed as inverted repeat polynucleotides joined by a linker polynucleotide sequence such that the dsRNA agent has a stem and loop structure.

Non-limiting examples of RNA expression vectors are DNA plasmids or viral vectors. Expression vectors useful in embodiments of the invention can be compatible with eukaryotic cells. Eukaryotic cell expression vectors are routinely used in the art and are available from a number of commercial sources. Delivery of dsRNA expressing vectors can be systemic, such as by intravenous or intramuscular administration, by administration to target cells ex-planted from a subject followed by reintroduction into the subject, or by any other means that allows for introduction into a desired target cell.

Viral vector systems that may be included in an embodiment of a method of the include, but are not limited to, (a) adenovirus vectors; (b) retrovirus vectors, including but not limited to lentiviral vectors, moloney murine leukemia virus, etc.; (c) adeno-associated virus vectors; (d) herpes simplex virus vectors; (e) SV 40 vectors; (f) polyoma virus vectors; (g) papilloma virus vectors; (h) picornavirus vectors; (i) pox virus vectors such as an orthopox, e.g., vaccinia virus vectors or avipox, e.g. canary pox or fowl pox; and (j) a helper-dependent or gutless adenovirus. Constructs for the recombinant expression of a dsRNA agent may include regulatory elements, such as promoters, enhancers, etc., which may be selected to provide constitutive or regulated/inducible expression. Viral vector systems, and the use of promoters and enhancers, etc. are routine in the art and can be used in conjunction with methods and compositions described herein.

Certain embodiments of the invention include use of viral vectors for delivery of dsRNA agents into cells. Numerous adenovirus-based delivery systems are routinely used in the art for deliver to, for example, lung, liver, the central nervous system, endothelial cells, and muscle. Non-limiting examples of viral vectors that may be used in methods of the invention are: AAV vectors, a pox virus such as a vaccinia virus, a Modified Virus Ankara (MVA), NYVAC, an avipox such as fowl pox or canary pox.

Certain embodiments of the invention include methods of delivering dsRNA agents into cells using a vector and such vectors may be in a pharmaceutically acceptable carrier that may, but need not, include a slow release matrix in which the gene delivery vehicle is imbedded. In some embodiments, a vector for delivering a dsRNA can be produced from a recombinant cell, and a pharmaceutical composition of the invention may include one or more cells that produced the dsRNA delivery system.

### Treatment

As used herein, the term "preventing" or "preventing", when used to refer to a disease, disorder, or condition thereof that would benefit from reduced expression of a target gene, means that a subject develops a disease, disorder, or condition associated with such disease, disorder or condition. The likelihood of associated symptoms, such as symptoms associated with a disease or disorder caused by or associated with the activation of a target gene, is reduced. The likelihood of developing a disease is reduced, for example, when an individual has one or more risk factors for a disease that fails to develop A related disease, disorder or condition, or a reduction in the development of symptoms associated with such a disease, disorder or condition (eg, a reduction of at least about 10% on a scale of clinically having the disease or condition), or delayed symptoms Prophylaxis is considered effective if its manifestations are delayed by days, weeks, months, or years.

As used herein, the terms "treat", "treated", or "treating" when used with respect to a target gene- associated disease or condition may refer to a prophylactic treatment that decreases the likelihood of a subject developing the target gene-associated disease or condition, and also may refer to a treatment after the subject has developed a target gene-associated disease or condition in order to eliminate or reduce the level of the target gene-associated disease or condition, prevent the target gene-associated disease or condition from becoming more advanced (e.g., more severe), and/or slow the progression of the target gene-associated disease or condition in a subject compared to the subject in the absence of the therapy to reduce activity in the subject of target gene polypeptide.

Certain embodiments of agents, compositions, and methods of the invention can be used to inhibit target gene expression. As used herein in reference to expression of a target gene, the terms "inhibit," "silence," "reduce," "down-regulate," and "knockdown" mean the expression of the target gene, as measured by one or more of: a level of RNA transcribed from the gene, a level of activity of target expressed, and a level of target polypeptide, protein or protein subunit translated from the mRNA in a cell, group of cells, tissue, organ, or subject in which the target gene is transcribed, is reduced when the cell, group of cells, tissue, organ, or subject is contacted with (e.g., treated with) a target gene dsRNA agent or target gene antisense polynucleotide agent of the invention, compared to a control level of RNA transcribed from the target gene, a level of activity of expressed target gene, or a level of target gene translated from the MRNA, respectively. In some embodiments, a control level is a level in a cell, tissue, organ or subject that has not been contacted with (e.g. treated with) the dsRNA agent or antisense polynucleotide agent.

### Effective amount

As used herein terms such as: "pharmacologically effective amount," "therapeutically effective amount" and "effective amount" refers to that amount of a dsRNA agent or antisense polynucleotide agent of the invention to produce the intended pharmacological, therapeutic or preventive result. For example, if a given clinical treatment is considered effective when there is at least a 10% reduction in a measurable parameter associated with a disease or disorder, a therapeutically effective amount of a drug for the treatment of that disease or disorder is the amount necessary to effect at least a 10% reduction in that parameter. For example, a therapeutically effective amount of a dsRNA agent or antisense polynucleotide agent can reduce polypeptide levels by at least 10%.

The following examples are provided to illustrate specific instances of the practice of the present invention and are not intended to limit the scope of the invention. As will be apparent to one of ordinary skill in the art, the present invention will find application in a variety of compositions and methods.

### Examples

The following examples are provided to illustrate specific examples for practicing the invention and are not intended to limit the scope of the invention. As will be apparent to those of ordinary skill in the art, the present invention will find use in a variety of compositions and methods.

### Example 1. Synthesis of RNAi Agents

RNAi agent duplexes shown in Table1-2, above, were synthesized in accordance with the following general procedures:
Sense and antisense strand sequences of siRNA were synthesized on oligonucleotide synthesizers using a well-established solid phase synthesis method based on phosphoramidite chemistry. Oligonucleotide chain propagation is achieved through 4-step cycles: a deprotection, a condensation, a capping and an oxidation or a sulfurization step for addition of each nucleotide. Syntheses were performed on a solid support made of controlled pore glass (CPG, 500Å or1000 Å). Monomer phosphoramidites may be purchased from commercial sources or Phosphoramidites with GalNAc ligand cluster (GLPA1, GLPA2 and GLPA15 as non-limiting examples) were synthesized according to the procedures of Examples 2-3 herein.In the case where the GalNAc ligand is attached at 3'-end of sense strand, GalNAc ligand attached CPG solid support was used. In the case where the GalNAc ligand is attached at 5'-end of sense strand, a GalNAc phosphoramidite (GLPA1, GLPA2 or GLPA15 as a non-limiting example) was used for the last coupling reaction. Trichloroacetic acid (TCA) 3% in dichloromethane was used for deprotection of 4,4'-dimethoxytrityl protecting group (DMT). 5-Ethylthio-1H-tetrazole was used as an activator. I2 in THF/Py/H2O and phenylacetyl disulfide (PADS) in pyridine/MeCN was used for oxidation and sulfurization reactions, respectively. After the final solid phase synthesis step, solid support bound oligomer was cleaved and protecting groups were removed by treating with a solution of 20 wt. % methylamine in water and 28% ammonium hydroxide solution treat at 65°C for 2 hours.Removal of Nucleobase Protecting Groups and Cleavage of Solid Support-Bound Oligomers. Crude single strand product was further purified by ion pairing reversed phase HPLC (IP-RP-HPLC). Purified single strand oligonucleotide product from IP-RP-HPLC was converted to sodium salt by dissolving in 1.0 M NaOAc and precipitation by addition of ice cold EtOH. Annealing of equimolar complementary sense stand and antisense strand oligonucleotide in water was performed to form the double strand siRNA product, which was lyophilized to afford a fluffy white solid.

### Example 2. Preparation of Intermediate-A and Intermediate-B

As shown in Scheme 1 below, Intermediate-A was synthesized by treating commercially available galactosamine pentaacetate with trimethylsilyl trifluoromethanesulfonate (TMSOTf) in dichloromethane (DCM). This was followed by glycosylation with Cbz protected 2-(2-aminoethoxy)ethan-1-ol to give Compound II. The Cbz protecting group was removed by hydrogenation to afford Intermediate-A as a trifluoroacetate (TFA) salt. Intermediate B was synthesized based on the same scheme except Cbz protected 2-(2-(2-aminoethoxy)ethoxy)ethan-1-ol was used as the starting material.

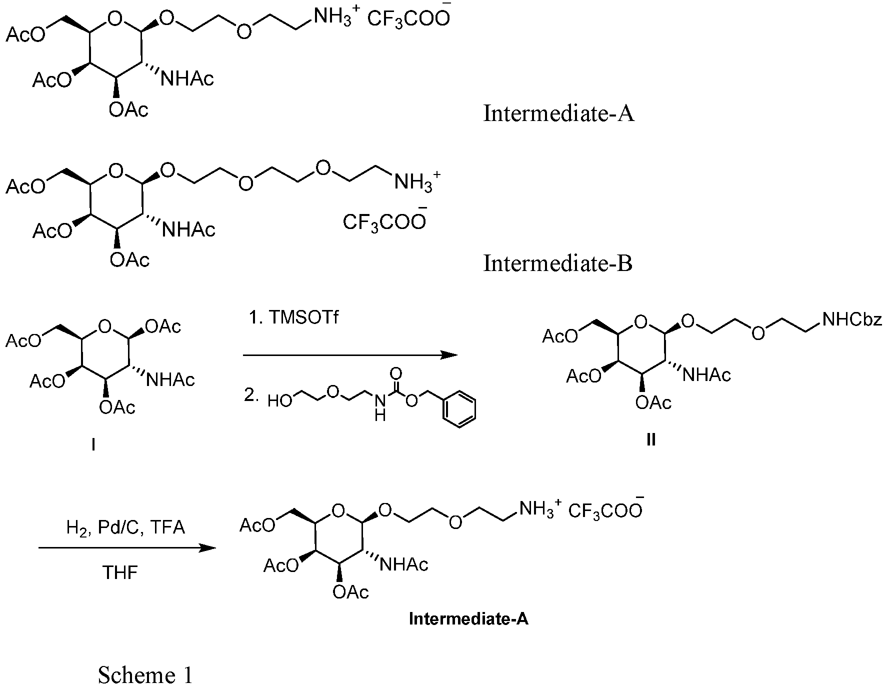

To a solution of Compound I (20.0 g, 51.4 mmol) in 100 mL 1,2-dichloroethane (DCE) was added TMSOTf (17.1 g, 77.2 mmol). The resulting reaction solution was stirred at 60 °C for 2 hrs, and then at 25 °C for 1 hr. Cbz protected 2-(2-aminoethoxy)ethan-1-ol (13.5 g, 56.5 mmol) in DCE (100 mL) dried over 4 Å powder molecular sieves (10 g) was added dropwise to the above mentioned reaction solution at 0 °C under N₂ atmosphere. The resulting reaction mixture was stirred at 25 °C for 16 hrs under N₂ atmosphere. The reaction mixture was filtered and washed with sat. NaHCO₃ (200 mL), water (200 mL) and sat. brine (200 mL). The organic layer was dried over anhydrous Na₂SO₄, filtered and concentrated under reduced pressure to give a crude product, which was triturated with 2-Methyltetrahydrofuran/heptane (5/3, v/v, 1.80 L) for 2 hrs. Resulting mixture was filtered and dried to give Compound II (15.0 g, 50.3% yield) as a white solid.

To a dried and argon purged hydrogenation bottle was carefully added 10% Pd/C (1.50 g), followed by 10 mL tetrahydrofuran (THF) and then a solution of Compound II (15.0 g, 26.4 mmol) in THF (300 mL) and TFA (trifluoroacetic acid, 3.00 g, 26.4 mmol). The resulting mixture was degassed and purged with H₂ three times and stirred at 25 °C for 3 hrs under H₂ (45 psi) atmosphere. Thin-layer chromatography (TLC, solvent: DCM:MeOH = 10:1) indicated Compound II was consumed completely. The reaction mixture was filtered and concentrated under reduced pressure. Residue was dissolved in anhydrous DCM (500 mL) and concentrated. This process was repeated 3 times to give Intermediate-A (14.0 g, 96.5% yield) as a foamy white solid. ¹H NMR (400 MHz DMSO-*d₆*): δ ppm 7.90 (d, *J =* 9.29 Hz, 1 H), 7.78 (br s, 3 H), 5.23 (d, *J =* 3.26 Hz, 1 H), 4.98 (dd, *J =* 11.29, 3.26 Hz, 1 H), 4.56 (d, *J =* 8.53 Hz, 1 H), 3.98 - 4.07 (m, 3 H), 3.79 - 3.93 (m, 2 H), 3.55 - 3.66 (m, 5 H), 2.98 (br d, *J =* 4.77 Hz, 2 H), 2.11 (s, 3 H), 2.00 (s, 3 H), 1.90 (s, 3 H), 1.76 (s, 3 H).

Intermediate-B was synthesized using similar procedures for synthesis of Intermediate-A. ¹H NMR (400 MHz DMSO-*d₆*): δ ppm 7.90 (br d, *J* = 9.03 Hz, 4 H), 5.21 (d, *J =* 3.51 Hz, 1 H), 4.97 (dd, *J =* 11.1 Hz, 1 H), 4.54 (d, *J =* 8.53 Hz, 1 H), 3.98 - 4.06 (m, 3 H), 3.88 (dt, *J =* 10.9 Hz, 1 H), 3.76 - 3.83 (m, 1 H), 3.49 - 3.61 (m, 9 H), 2.97 (br s, 2 H), 2.10 (s, 3 H), 1.99 (s, 3 H), 1.88 (s, 3 H), 1.78 (s, 3 H). Mass calc. for C₂₀H₃₄N₂O₁₁: 478.22; found: 479.3 (M+H⁺).

### Example 3. Synthesis of GalNAc ligand cluster phosphoramidite GLPA1, GLPA2 and GLPA15

Scheme 2 below was followed to prepare GLPA1 and GLPA2. Starting from benzyl protected propane-1,3-diamine, it was alkylated with *tert*-butyl 2-bromoacetate to afford triester Compound I. The benzyl protecting group was removed by hydrogenation to afford secondary amine Compound II. Amide coupling with 6-hydroxyhexanoic acid afforded Compound III. tert-Butyl protecting groups were then removed upon treatment of HCl in dioxane to generate triacid Compound IV. Amide coupling between triacid compound IV and Intermediate-A or Intermediate-B was performed to afford Compound Va or Vb. Phosphoramidite GLPA1 or GLPA2 was synthesized by phosphitylation of Compound Va or Vb with 2-Cyanoethyl N,N-diisopropylchlorophosphoramidite and a catalytic amount of 1H-tetrazole.

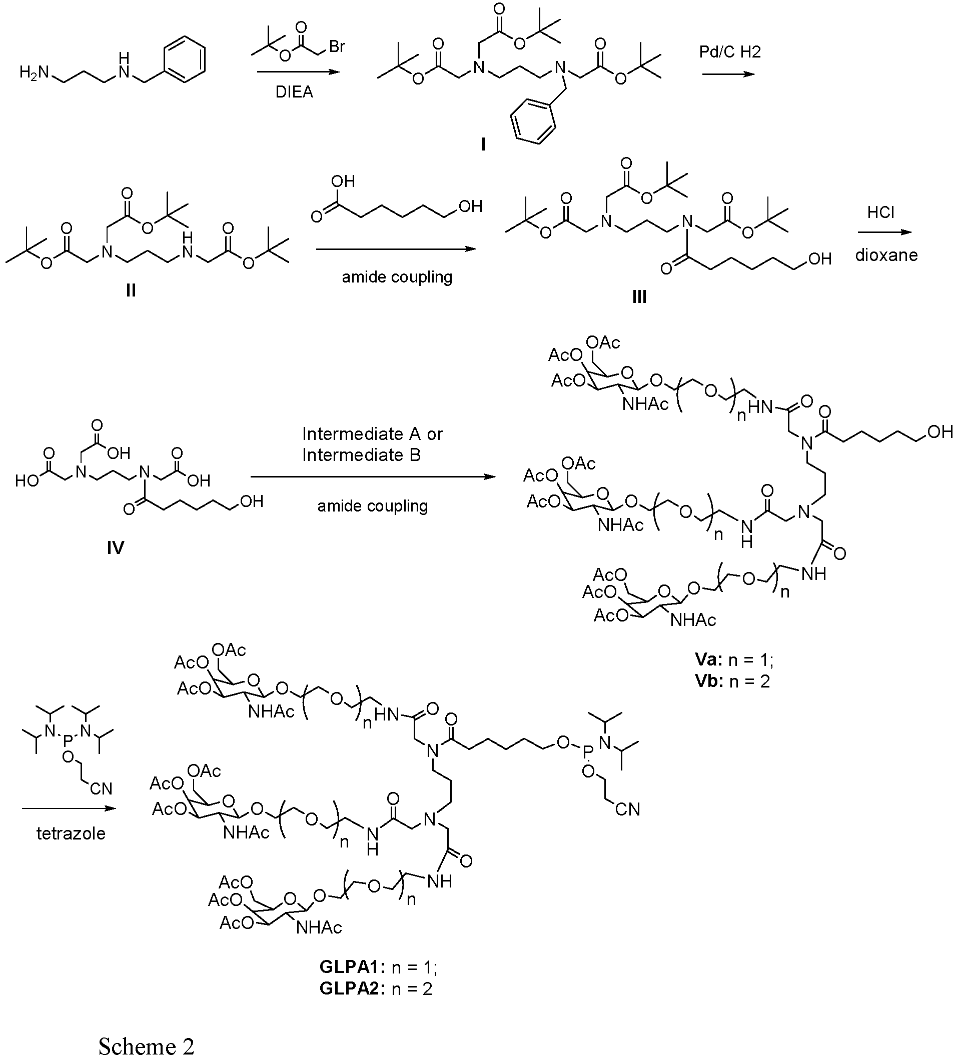

To a solution of N-Benzyl-1,3-propanediamine (5.00 g, 30.4 mmol) in dimethylformamide (DMF, 100 mL) was added *tert*-butyl 2-bromoacetate (23.7 g, 121 mmol), followed by addition of diisopropylethylamine (DIEA, 23.61 g, 182 mmol) dropwise. The resulting reaction mixture was stirred at 25-30 °C for 16 hrs. LCMS showed N-Benzyl-1,3-propanediamine was consumed completely. Reaction mixture was diluted with H₂O (500 mL) and extracted with EtOAc (500 mL x 2). The combined organics were washed with sat. brine (1 L), dried over anhydrous Na₂SO₄, filtered, and concentrated under reduced pressure to give crude product, which was purified by silica gel column chromatography (gradient: petroleum ether:ethyl acetate from 20:1 to 5:1). Compound I (12.1 g, 78.4% yield) was obtained as a colorless oil. ¹H NMR (400 MHz, CDCl₃): δ ppm 7.26 - 7.40 (m, 5 H), 3.79 (s, 2 H), 3.43 (s, 4 H), 3.21 (s, 2 H), 2.72 (dt, *J =* 16.9, 7.34 Hz, 4 H), 1.70 (quin, *J =* 7.2 Hz, 2 H), 1.44 - 1.50 (m, 27 H).

A dried hydrogenation bottle was purged with Argon three times. Pd/C (200 mg, 10%) was added, followed by MeOH (5 mL) and then a solution of Compound I (1.00 g, 1.97 mmol) in MeOH (5 mL). The reaction mixture was degassed under vacuum and refilled with H₂. This process was repeated three times. The mixture was stirred at 25°C for 12 hrs under H₂ (15 psi) atmosphere. LCMS showed Compound I was consumed completely. The reaction mixture was filtered under reduced pressure under N₂ atmosphere. Filtrate was concentrated under reduced pressure to give Compound II (655 mg, 79.7% yield) as yellow oil, which was used for the next step without further purification. ¹H NMR (400 MHz, CDCl₃): δ ppm 3.44 (s, 4 H), 3.31 (s, 2 H), 2.78 (t, *J =* 7.1 Hz, 2 H), 2.68 (t, *J =* 6.9 Hz, 2 H), 1.88 (br s, 1 H), 1.69 (quin, *J =* 7.03 Hz, 2 H), 1.44 - 1.50 (s, 27 H).

A mixture of Compound II (655 mg, 1.57 mmol) , 6-hydroxyhexanoic acid (249 mg, 1.89 mmol), DIEA (1.02 g, 7.86 mmol) , 1-ethyl-3-(3-dimethylaminopropyl) carbodiimide hydrochloride (EDCI, 904 mg, 4.72 mmol), and 1-Hydroxybenzotriazole (HOBt, 637 mg, 4.72 mmol) in DMF (6 mL) was degassed and purged with N₂ three times, and then was stirred at 25°C for 3 hrs under N₂ atmosphere. LCMS indicated desired product. The reaction mixture was diluted with H₂O (10 mL) and extracted with EtOAc 20 mL (10 mL x 2). Organics were combined and washed with sat. brine (20 mL), dried over anhydrous Na₂SO₄, filtered, and concentrated to give crude product, which was purified by silica gel column chromatography (gradient: petroleum ether:ethyl acetate from 5:1 to 1:1) to afford Compound III (650 mg, 77.8% yield) as a yellow oil. ¹H NMR (400 MHz, CDCl₃): δ ppm 3.90 - 3.95 (s, 2 H), 3.63 (t, *J =* 6.40 Hz, 2 H), 3.38 - 3.45 (m, 6 H), 2.72 (t, *J =* 6.65 Hz, 2 H), 2.40 (t, *J =* 7.28 Hz, 2 H), 1.55 - 1.75 (m, 8 H), 1.44 (s, 27 H). Mass calc. for C₂₇H₅₀N₂O₈: 530.36; found: 531.3 (M+H⁺).

A mixture of Compound III (5.5 g, 10.3 mmol) in HCl/dioxane (2M, 55 mL) was stirred at 25 °C for 3 hrs. LCMS showed complete consumption of Compound III. Reaction mixture was filtered, washed with EtOAc (50 mL), and dried under reduced pressure to give crude product. It was dissolved in CH₃CN (50 mL), volatiles were removed under vacuum. This process was repeated three times to give Compound IV (2.05 g, 54.5% yield) as a white solid. ¹H NMR (400 MHz, D₂O): δ ppm 4.21 (s, 1 H), 4.07 (d, *J =* 4.5 Hz, 4 H), 3.99 (s, 1 H), 3.45 - 3.52 (m, 3 H), 3.42 (t, *J =* 6.5 Hz, 1 H), 3.32 - 3.38 (m, 1 H), 3.24 - 3.31 (m, 1 H), 2.37 (t, *J =* 7.4 Hz, 1 H), 2.24 (t, *J =* 7.4 Hz, 1 H), 1.99 (dt, *J =* 15.5, 7.53 Hz, 1 H), 1.85 - 1.94 (m, 1 H), 1.85 - 1.94 (m, 1 H), 1.39 - 1.56 (m, 4 H), 1.19 - 1.31 (m, 2 H).

A mixture of Compound IV (500 mg, 1.05 mmol), Intermediate-A (2.02 g, 3.67 mmol), DIEA (813 mg, 6.30 mmol), EDCI (704 mg, 3.67 mmol) and HOBt (496 mg, 3.67 mmol) in DMF (10 mL) was degassed and purged with N₂ for 3 times, and then the mixture was stirred at 25 °C for 3 hrs under N₂ atmosphere. LCMS indicated desired product. The reaction mixture was quenched by addition of H₂O (10 mL), extracted with DCM (10 mL x 2). The combined organics were extracted with 10% citric acid (20 mL). The aqueous phase was neutralized with saturated NaHCO₃ solution and re-extracted with DCM (10 mL x 2). Organics were dried over sodium sulfate, filtered and concentrated under reduced pressure to give Compound Va (570 mg, 0.281 mmol, 26.8% yield) as a white solid. ¹H NMR: (400 MHz, CDCl₃) ppm δ 7.84 - 8.12 (m, 3 H), 6.85 - 7.15 (m, 2 H), 6.66 - 6.81 (m, 1 H), 5.36 (br d, *J* = 2.7 Hz, 3 H), 5.11 - 5.27 (m, 3 H), 4.63 - 4.85 (m, 3 H), 3.90 - 4.25 (m, 18 H), 3.37 - 3.75 (m, 28 H), 3.15 - 3.28 (m, 4 H), 2.64 (br d, *J= 6.53* Hz, 2 H), 2.30 - 2.46 (m, 2 H), 2.13 - 2.18 (m, 9 H), 2.05 (s, 9 H), 1.94 - 2.03 (m, 18 H), 1.68 (br s, 2 H), 1.45 (br s, 2 H), 1.12 (br t, *J =* 7.0 Hz, 2 H).

To a solution of Compound Va (260 mg, 0.161 mmol) in anhydrous DCM (5 mL) was added diisopropylammonium tetrazolide (30.3 mg, 0.177 mmol), followed by dropwise addition of 3-bis(diisopropylamino)phosphanyloxypropanenitrile (194 mg, 0.645 mmol) at ambient temperature under N₂. The reaction mixture was stirred at 20 ~ 25 °C for 2 hrs. LCMS indicated Compound Va was consumed completely. After cooling to -20 °C, the reaction mixture was added to a stirred solution of brine/saturated aq. NaHCO₃ (1:1, 5 mL) at 0 °C. After stirring for 1 min, DCM (5 mL) was added. Layers were separated. Organics were washed with brine/saturated aq. NaHCO₃ solution (1:1, 5 mL), dried over Na₂SO₄, filtered, and concentrated to ~ 1 mL of volume. The residue solution was added dropwise to 20 mL methyl tert-butyl ether (MTBE) with stirring. This resulted in precipitation of white solid. The mixture was centrifuged, and solid was collected. The solid was redissolved in 1 mL of DCM and precipitated by addition of MTBE (20 mL). Solid was again isolated by centrifuge. The solid collected was dissolved in anhydrous CH₃CN. Volatiles were removed. This process was repeated two more times to afford GalNAc ligand phosphoramidite compound GLPA1 (153 mg, 84.4 µmol) as a white solid. ¹H NMR (400 MHz, CDCl₃): ppm δ 7.71 - 8.06 (m, 2 H), 6.60 - 7.06 (m, 3 H), 5.37 (br d, J = 3.0 Hz, 3 H), 5.18 - 5.32 (m, 3 H), 4.70 - 4.86 (m, 3 H), 3.92 - 4.25 (m, 18 H), 3.42 - 3.85 (m, 30 H), 3.25 (m, 4 H), 2.59 - 2.75 (m, 4 H), 2.27 - 2.44 (m, 2 H), 2.15 - 2.20 (s, 9 H) 2.07 (s, 9 H), 1.96 - 2.03 (m, 18 H), 1.65 (br s, 4 H), 1.44 (br d, J = 7.28 Hz, 2 H), 1.14 - 1.24 (m, 12 H). ³¹P NMR (CDCl₃): ppm δ 147.15.

GalNAc ligand phosphoramidite compound GLPA2 was synthesized using the same procedure except Intermediate-B was used. ¹H NMR (400 MHz, CDCl₃): ppm δ 7.94 - 8.18 (m, 1 H), 7.69 (br s, 1 H), 6.66 - 7.10 (m, 3 H), 5.35 (d, *J =* 3.5 Hz, 3 H), 5.07 - 5.25 (m, 3 H), 4.76 - 4.86 (m, 3 H), 4.01 - 4.31 (m, 10 H), 3.91 - 4.01 (m, 8 H), 3.74 - 3.86 (m, 4 H), 3.52 - 3.71 (m, 30 H), 3.42 - 3.50 (m, 6 H), 3.15 - 3.25 (m, 4 H), 2.52 - 2.70 (m, 4 H), 2.22 - 2.45 (m, 2 H), 2.15 - 2.22 (s, 9 H), 2.06 (s, 9 H), 1.95 - 2.03 (m, 18 H), 1.77 (br s, 2 H), 1.58 - 1.66 (m, 4 H), 1.40 (m, 2 H), 1.08 - 1.24 (m, 12 H). ³¹P NMR (CDCl₃): ppm δ 147.12.

Scheme 3 below was followed to prepare GLPA15.

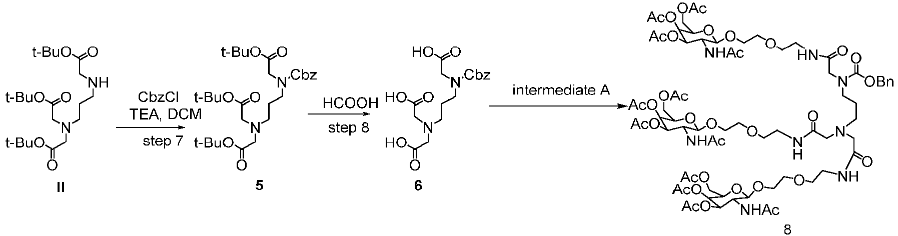

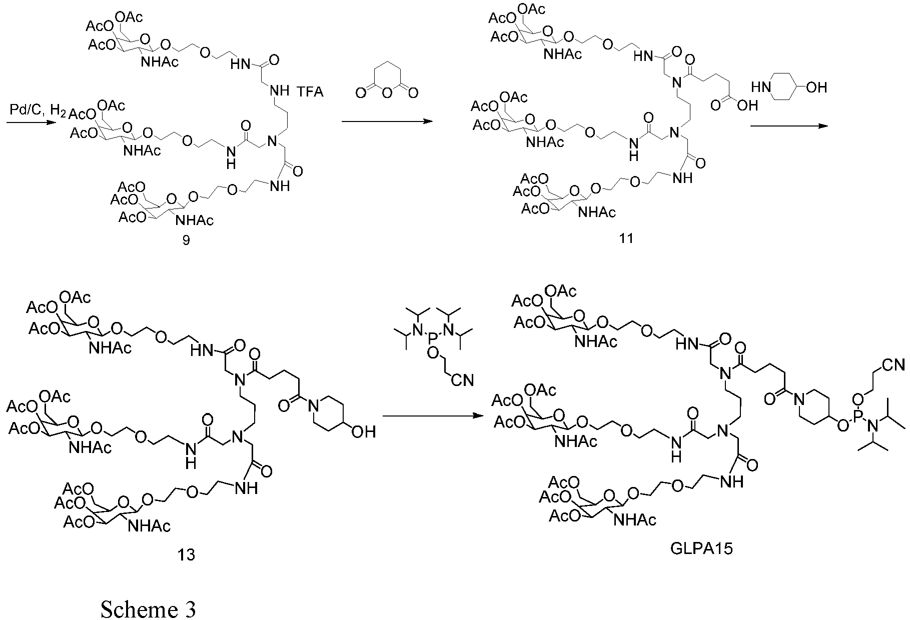

To a solution of intermediate compound II(275g, 660 mmol, 1.00 *eq.)* in dichloromethane(2.75L) was added triethylamine(133g, 1.32mol, 2.00 *eq.),* followed by dropwise addition of Cbz-Cl (169g, 990mmol, 1.50 *eq*.). The reaction solution was stirred at 25° C for 2 hours, and LCMS showed that compound II was completely converted. The reaction solution was washed successively with NaHCO₃₍800mL) saturated solution and saturated brine(500mL), and the organic phase was dried with anhydrous Na₂SO₄. After removing the desiccant by filtration, the filtrate was concentrated to dryness. The crude product was subjected to column chromatography(SiO₂, petroleum ether (PE)/ethyl acetate (EA) =100/1 to 5/1,v/v) to obtain a colorless oily compound 5(290g, 527mmol, yield 75.7 %). ¹H NMR (400 MHz in DMSO-d₆): δ ppm 7.23 - 7.40 (m, 5 H), 5.00 - 5.12 (m, 2 H), 3.86 - 3.95 (m, 2 H), 3.23 - 3.39 (m, 6 H), 2.55 - 2.67 (m, 2 H), 1.56 - 1.64 (m, 2 H), 1.31 - 1.46 (m, 27 H). MS (ESI) [M+H]⁺ m/z: 551.6.

HCOOH(2.9L) was added to compound 5(145g, 263mmol, 1.00 *eq),* and the solution was stirred at 60°C for 12 hours. LCMS showed that the conversion of compound 5 was complete. Add 1.5L toluene and 1.5L acetonitrile to the reaction solution, concentrate under reduced pressure to about 500mL, then add toluene/acetonitrile(1:1, v/v, about 750mL) and concentrate to about 500mL, then add acetonitrile(about 1000mL) and concentrated to dryness, the crude product was pulverized at 60°C for 2 hours with 700mL of acetonitrile, and filtered. The solid was collected and dried to obtain white solid compound 6(105 g, quantitative). ¹H NMR (400 MHz in DMSO-d₆): δ ppm 7.26 - 7.40 (m, 5 H), 5.02 - 5.10 (m, 2 H), 3.89 - 4.00 (m, 2 H), 3.36 - 3.45 (m, 4 H), 3.24 - 3.34 (m, 2 H), 2.59 - 2.72 (m, 2 H), 1.40 (s, 2 H). MS (ESI) [M+H]⁺m/z: 383.0.

To a DMF(1.0L) solution of compound 6(100g, 261mmol) and Intermediate-A (502g, 915.mmol, 3.50 *eq.*) was added O-benzotriazole-N,N,N',N'- Tetramethyluronium tetrafluoroboric acid (TBTU) (327g, 1.02mol, 3.90 *eq.*)*,* triethylamine(212g, 2.09mol, 8.00 *eq.),* the reaction was carried out at 25°C for 1 hour, LCMS showed that compound 6 was converted finish. The reaction solution was added to 4000mL of water, and extracted with methyl tert-butyl ether(2000mL twice) to remove impurities, and the remaining aqueous phase was extracted with dichloromethane(3000mL twice). The dichloromethane phase was washed successively with 10% citric acid aqueous solution(2000mL divided into two times), saturated NaHCO ₃ (2.0L divided into two times), saturated brine(2.0L), and dried over anhydrous Na₂SO₄. The filtrate was filtered and concentrated under reduced pressure to obtain white solid compound 8(260g, 159mmol, yield 60.9%). ¹H NMR (400 MHz in DMSO-d₆): δ ppm 7.99 - 8.08 (m, 2 H), 7.93 (br d, *J=5.50* Hz, 1 H), 7.79 - 7.86 (m, 3 H), 7.26 - 7.39 (m, 5 H), 5.22 (d, *J*=3.13 Hz, 3 H), 4.95 - 5.08 (m, 5 H), 4.54 (br d, *J*=8.38 Hz, 3 H), 4.03 (s, 9 H), 3.81 - 3.93 (m, 5 H), 3.76 (br d, *J*=4.88 Hz, 3 H), 3.44 - 3.62 (m, 10 H), 3.34 - 3.43 (m, 6 H), 3.24 (br d, *J*=6.13 Hz, 7 H), 3.02 - 3.09 (m, 4 H), 2.40 - 2.47 (m, 2 H), 2.10 (s, 9 H), 1.99 (s, 9 H), 1.89 (s, 9 H), 1.77 (s, 9 H), 1.57 - 1.68 (m, 2 H)_{∘} MS (ESI) [M+H]⁺ m/z: 816.4.

2L hydrogenation kettle with argon and carefully add dry Pd/C(9g), add MeOH(50mL) to wet Pd/C, then slowly add compound 8(90g, 55.1mmol, 1.00 *eq.)* under argon atmosphere and trifluoroacetic acid(6.29 g, 55.1 mmol, 1.00 *eq.)* in MeOH(850mL). The mixture was degassed/replaced by adding H₂ three times to a hydrogen atmosphere and stirred at 25° C for 10h. LCMS showed that the conversion of compound 8 was complete, Pd/C was removed by filtration, and the filtrate was concentrated under reduced pressure to obtain compound 9(80 g, yield 90.2%). ¹H NMR (400 MHz in DMSO-d₆): δ ppm 9.12 (br s, 2 H), 8.50 (br t, *J*=5.19 Hz, 1 H), 8.10 (br t, *J=5.50* Hz, 2 H), 7.85 - 7.91 (m, 3 H), 5.22 (d, *J*=3.25 Hz, 3 H), 4.95 - 5.01 (m, 3 H), 4.52 - 4.58 (m, 3 H), 4.03 (s, 9 H), 3.84 - 3.93 (m, 3 H), 3.75 - 3.83 (m, 3 H), 3.39 - 3.61 (m, 16 H), 3.23 - 3.32 (m, 6 H), 3.15 - 3.18 (m, 3 H), 2.97 - 3.05 (m, 2 H), 2.54 - 2.61 (m, 2 H), 2.10 (s, 9 H), 2.00 (s, 9 H), 1.89 (s, 9 H), 1.77 - 1.80 (m, 9 H), 1.70 - 1.76 (m, 2 H). MS (ESI) [M+H]⁺ m/z: 749.3.

To dichloromethane (2.7L) solution of compound 9(270 g, 168 mmol, 1.00 *eq.)* and glutaric anhydride(28.6 g, 252 mmol, 1.50 *eq)* was added triethylamine(67.8 g, 672 mmol, 4.00 *eq.),* the solution was stirred at 25°C for 1 hour, and LCMS showed that compound 9 was completely converted to compound 11. 4-Hydroxypiperidine(42.4g, 420mmol, 2.50 *eq.)* and TBTU(107g, 335mmol, 2.00 *eq.)* were added to the reaction solution, and stirring was continued at 25°C for 1 hour. LCMS showed complete conversion of compound 11. The reaction was quenched by slowly adding saturated NH₄Cl (3.0L), the layers were separated, the aqueous phase was extracted with dichloromethane (2×1000 mL) and combined with the previous organic phase. The combined organic phases were washed with a 1:1 (v/v) mixture(3.0L) of saturated NaHCO₃(aq) and saturated brine, dried over anhydrous Na₂SO₄, filtered and concentrated under reduced pressure. The crude product was dissolved in 1.5L of dichloromethane and added dropwise to methyl tert-butyl ether (7.5L), A translucent white precipitate gradually formed during the dropwise addition. The precipitate was filtered in vacuo, and the solid was collected and dried in vacuo to obtain compound 13(207g, yield 72.8%) as a white solid. ¹H NMR (400 MHz in DMSO-d₆): δ ppm 8.05 (br d, *J*=2.00 Hz, 2 H), 7.82 (br d, *J*=7.38 Hz, 3 H), 5.21 (br s, 3 H), 4.98 (br d, *J*=10.26 Hz, 3 H), 4.72 (br s, 1 H), 4.54 (br d, *J*=7.88 Hz, 3 H), 4.03 (br s, 9 H), 3.74 - 3.94 (m, 9 H), 3.45 - 3.71 (m, 12 H), 3.40 (br s, 6 H), 3.24 (br s, 7 H), 3.07 (br d, *J=14.13* Hz, 5 H), 2.91 - 3.01 (m, 1 H), 2.24 - 2.44 (m, 5 H), 2.20 (br s, 1 H), 2.10 (s, 9 H), 1.96 - 2.04 (m, 9 H), 1.89 (br s, 9 H), 1.74 - 1.81 (m, 9 H), 1.51 - 1.73 (m, 6 H), 1.07 - 1.36 (m, 3 H). MS (ESI) [M+H]⁺ m/z: 848.0.

To the dichloromethane (2.0L) solution of compound 13(200g,118mmol,1.00eq.) and tetrazole diisopropylammonium(8.08g,47.2mmol,0.40*eq.)* was added 3-double(diisopropyl amino) phosphonyl oxypropionitrile (53.3g,177mmol,1.50*eq*.) , the reaction solution was stirred at 40°C for 2 hours, and LCMS showed that the conversion of compound 13 was complete. The reaction solution was washed with a 1:1 mixture(2.0L) of saturated NaHCO₃ and saturated brine, dried over anhydrous Na₂SO₄, and the crude product obtained after the filtrate was concentrated was dissolved in dichloromethane (1.2L), added dropwise to the stirred Methyl tert-butyl ether(6.0L), filter the suspension, rinse the filter cake with tert-butyl ether, collect the solid and dry it in vacuum, dissolve the product in dichloromethane (1.0L) and concentrate to dryness, repeated the operation 4 times to remove residual tert-butyl ether to obtain GLPA15(164g, yield 73.3%). ¹H NMR (400 MHz in DMSO-d₆): δ ppm 8.05 (br d, *J=* 6.50 Hz, 2 H), 7.81 (br d, *J*=9.01 Hz, 3 H), 5.22 (d, *J*=3.25 Hz, 3 H), 4.98 (dd, *J*=11.26, 3.25 Hz, 3 H), 4.55 (br d, *J*=8.50 Hz, 3 H), 4.03 (s, 9 H), 3.64 - 3.97 (m, 12 H), 3.55 - 3.63 (m, 6 H), 3.50 (br s, 5 H), 3.40 (br d, *J*=6.13 Hz, 6 H), 3.17 - 3.30 (m, 9 H), 3.07 (br d, *J*=14.26 Hz, 4 H), 2.76 (t, *J*=5.82 Hz, 2 H), 2.18 - 2.47 (m, 6 H), 2.10 (s, 9 H), 1.99 (s, 9 H), 1.89 (s, 9 H), 1.78 (s, 9 H), 1.52 - 1.74 (m, 6 H), 1.12 - 1.19 (m, 12 H). ³¹P NMR (DMSO-d₆): ppm δ 145.25.

In certain studies, methods are provided for attaching a targeting group comprising GalNAc (also referred to herein as a GalNAc delivery compound) to the 5'- end of the sense strand, which involves GalNAc phosphoramidite (GLPA1) is used in the coupling step, using a synthetic process such as that used in oligonucleotide chain elongation (i.e. addition of nucleotides at the 5' end of the sense strand) to ligate them to the 5'- end of the sense strand.

In some studies, a method of attaching a targeting group comprising GalNAc to the 3'-end of a sense strand comprised the use of a solid support (CPG) that included a GLO-n. In some studies, the method of attaching a targeting group comprising GalNAc to the 3'-end of a sense strand included linking the GalNAc targeting group to a CPG solid support through an ester bond, and synthesizing the sense strand When using the resulting CPG with an attached GalNAc targeting group, this results in the GalNAc targeting group being attached at the 3'- end of the sense strand.

Other GalNAc phosphoramidite compounds(GLPAn) can also be obtained by using a reasonable corresponding intermediate, using a method similar to this article or well-known in the art, and connected to a suitable position of the siRNA duplex as a targeting group.

### Example 4. In vivo efficacy of different F-substituion modified RNAi agents

### In vivo test of FXII siRNA duplexes

To assess the *in vivo* activity of FXII siRNA, female C57BL/6 mice, 6 weeks old, specific pathogen-free, were used and purchased from Shanghai Slac Laborotory Animal Co., Ltd.

### a) Compounds

i. Solvent: PBS
ii. Test compound: AD00539, AD00540, AD00542, AD00549, AD00550, AD00552

### b) Experimental protocol

The animal experimental protocol is shown as Table 3.

**Table 3 Experimental protocol**

| Group | Number of animals (number/group) | Administration regimen | | | Time of blood collection | Detected indicators |
|---|---|---|---|---|---|---|
| | | Compound | Administration dosage (mg/kg) | Frequency of administration | | |
| 1 | 4, C57BL/6, female | PBS | NA | SC, administered once on day 0 | On days 7 and 14 | Plasma: FXII protein level |
| 2 | | AD00539 | 1 | | | |
| 3 | | AD00540 | 1 | | | |
| 5 | | AD00542 | 1 | | | |
| 12 | | AD00549 | 1 | | | |
| 13 | | AD00550 | 1 | | | |
| 15 | | AD00552 | 1 | | | |

c) Definition of experimental days: The day when mice were first administrated was defined as experimental day 0, one day forward as day -1, one day backward as day 1, and so on.

d) All compounds to be tested were prepared into 5 mg/mL stock solutions with PBS before administration. A small amount of 5 mg/mL stock solution was diluted 20 times, and the OD values were measured by Nanodrop. Afte converting the OD value to obtain the actual concentration of the stock solution, an appropriate amount of the stock solution was diluted to the working solution concentration (0.2 mg/mL) for administration.

e) After a 6-day adaptation period, all mice (C57BL/6, female, 6 weeks) were subjected to subcutaneous injection according to the above table on day 0.

f) On days 7 and 14, blood was collected from all mice through the submandibular vein to collect plasma for detection of FXII protein levels.

### Sample detection and analysis

a) The FXII protein levels in mouse plasma were detected using an ELISA kit (Molecular Innovations, IMSFXIIKTT). In short, the plasma samples collected by EDTA-K₂ were diluted 30,000 times and added to the detection plate coated with capture antibody for incubation, then the detection antibody and HRP-coupld secondary antibody were added in sequence, and finally TMB was used for color development. The absorbance value at 450 nm was read. The standard curve was fitted using the four-parameter method, and the OD value of the detected sample was substituted to calculate the FXII protein content of each sample, which was multiplied by the dilution factor to obtain the FXII protein concentration in the original plasma. The data results are shown as Table 4.

**Table 4. Relative expression levels of FXII proteins using compounds corresponding to the sequences, chemical modifications and delivery shown in Table 2.**

| AD# | Relative percentage levels of FXII protein expression remaining in mouse plasma on Day 7 | SD | Relative percentage levels of FXII protein expression remaining in mouse plasma on Day 14 | SD |
|---|---|---|---|---|
| AD00539 | 20% | 0.01 | 20% | 0.02 |
| AD00540 | 16% | 0.01 | 16% | 0.03 |
| AD00542 | 20% | 0.03 | 17% | 0.02 |
| AD00549 | 41% | 0.04 | 40% | 0.06 |
| AD00550 | 36% | 0.04 | 28% | 0.03 |
| AD00552 | 38% | 0.06 | 32% | 0.07 |

As shown in Table 4, the duplexes AD00540 and AD00542 have the same unmodified sense strand and antisense strand, and their antisense strands have the same 2'-F modified positions (2, 7, 12, 14, and 16). Compared with AD00539 (positive control), the 2'-F modification at positions 8, 11, 13 or 9, 11, 13 of the sense strand achieves better silencing effect at position 1938 of the FXII target gene, Similarly, the duplexes AD00550 and AD00552 have the same unmodified sense strand and antisense strand, and their antisense strands have the same 2'-F modified positions (2, 7, 12, 14 and 16). AD00550 and AD00552, with the 2'-F modification at position 8, 11, 13 or 9, 11, 13 of the sense strand, achieve better silencing effect at position 307 of the FXII gene as well.

### Example 5. In vivo efficacy of different F-substituion modified RNAi agents

### Experimental materials

### 1) Animals

Female C57BL/6 mice, 6 weeks old, specific pathogen-free, purchased from Shanghai Slac Laborotory Animal Co., Ltd.

### 2) Compounds

Solvent: PBS
Test compound: AD00716, AD00722, AD00723

### 3) Experimental protocol

The animal experimental protocol is shown as Table 5.

**Table 5 Experimental protocol**

| Group | Number of animals (number/group) | Administration regimen | | | Time of blood collection | Detected indicators |
|---|---|---|---|---|---|---|
| | | Compound | Administration dosage (mg/kg) | Frequency of administration | | |
| 1 | 4, C57BL/6, female | PBS | NA | SC, administered once on day 0 | On days 7 and 14 | Plasma: FXII protein level; Liver: 2-100 mg, FXII mRNA expression (optional) |
| 2 | | AD00716 | 1.5 | | | |
| 3 | | AD00722 | 1.5 | | | |
| 4 | | AD00723 | 1.5 | | | |

| | | | | | | |
|---|---|---|---|---|---|---|
| Definition of experimental days: The day when mice were first administrated was defined as experimental day 0, one day forward as day -1, one day backward as day 1, and so on. | | | | | | |

a) All compounds to be tested were prepared into 5 mg/mL stock solutions with PBS before administration. A small amount of 5 mg/mL stock solution was diluted 20 times, and the OD values were measured by Nanodrop. Afte converting the OD value to obtain the actual concentration of the stock solution, an appropriate amount of the stock solution was diluted to the working solution concentration (0.2 mg/mL or 0.3 mg/mL) for administration.
b) After a 6-day adaptation period, all mice (C57BL/6, female, 6 weeks) were subjected to subcutaneous injection according to the above table on day 0.
c) On days 7 and 14, blood was collected from all mice through the submandibular vein to collect plasma for detection of FXII protein levels.

### 4) Sample detection and analysis

The FXII protein levels in mouse plasma were detected using an ELISA kit.

The FXII protein levels in mouse plasma were detected using an ELISA kit (Molecular Innovations, IMSFXIIKTT). In short, the plasma samples collected by EDTA-K₂ were diluted 30,000 times and added to the detection plate coated with capture antibody for incubation, then the detection antibody and HRP-coupld secondary antibody were added in sequence, and finally TMB was used for color development. The absorbance value at 450 nm was read. The standard curve was fitted using the four-parameter method, and the OD value of the detected sample was substituted to calculate the FXII protein content of each sample, which was multiplied by the dilution factor to obtain the FXII protein concentration in the original plasma.

**Table 6. Relative expression levels of FXII proteins using compounds corresponding to the sequences, chemical modifications and delivery shown in Table 2.**

| AD | Relative percentage levels of FXII protein expression remaining in mouse plasma on Day 7 | SD | Relative percentage levels of FXII protein expression remaining in mouse plasma on Day 14 | SD |
|---|---|---|---|---|
| AD00716 | 34.3% | 0.090 | 24.2% | 0.023 |
| AD00722 | 30.2% | 0.032 | 21.1% | 0.043 |
| AD00723 | 29.6% | 0.025 | 22.9% | 0.025 |

As shown in table 6, the duplexes AD00722 and AD00723 with 2'-F modification at positions 8, 11, 13 of sense strands achieve superior FXII gene silencing effect compared to the positive control AD00716.

### Example 6. In vivo efficacy of different F-substituion modified RNAi agents

The *in vivo* efficacy of RNAi agents was performed by the experimental method as in Example 5.

**Table 7 Experimental protocol**

| Group | Number of animals (number/group) | Administration regimen | | | Time of blood collection | Detected Indicators |
|---|---|---|---|---|---|---|
| | | Compound | Administration dosage (mg/kg) | Frequency of administration | | |
| 1 | 4, C57BL/6, female | PBS | | SC, administered once on day 0 | On days 7 and 14 | FXII protein level; |
| 2 | | AD00549 | 1.5 mpk | | | |
| 3 | | AD00716 | 1.5 mpk | | | |
| 4 | | AD00550 | 1.5 mpk | | | Liver: 2-100 mg, FXII mRNA expression |
| 5 | | AD00552 | 1.5 mpk | | | |
| 6 | | AD00810 | 1.5 mpk | | | |
| 7 | | AD00813 | 1.5 mpk | | | |

**Table 8. Relative expression of FXII mRNA using compounds corresponding to the sequences, chemical modifications and delivery shown in Table 2.**

| AD | Relative percentage of FXII mRNA remaining in mouse liver on Day 7 | SD | Relative percentage of FXII mRNA remaining in mouse liver on Day 14 | SD |
|---|---|---|---|---|
| AD00549 | 43% | 0.03 | 46% | 0.09 |
| AD00716 | 35% | 0.03 | 40% | 0.09 |
| AD00550 | 33% | 0.03 | 29% | 0.03 |
| AD00552 | 35% | 0.06 | 34% | 0.07 |
| AD00810 | 25% | 0.05 | 17% | 0.02 |
| AD00813 | 31% | 0.03 | 23% | 0.04 |

Experiments show that duplexes AD00550 and AD00552 further verified that their antisense strands with the same 2'-F modified positions (2, 7, 12, 14 and 16) achieved better silencing effect at position 1938 of the FXII target gene. The duplexes AD00810, AD00813 and positive control AD00716 show that their antisense strands with the same 2'-F modified positions (2, 5, 12, 14, 18) achieved better silencing effect.

### Example 7. In vivo efficacy of different F-substituion modified RNAi agents

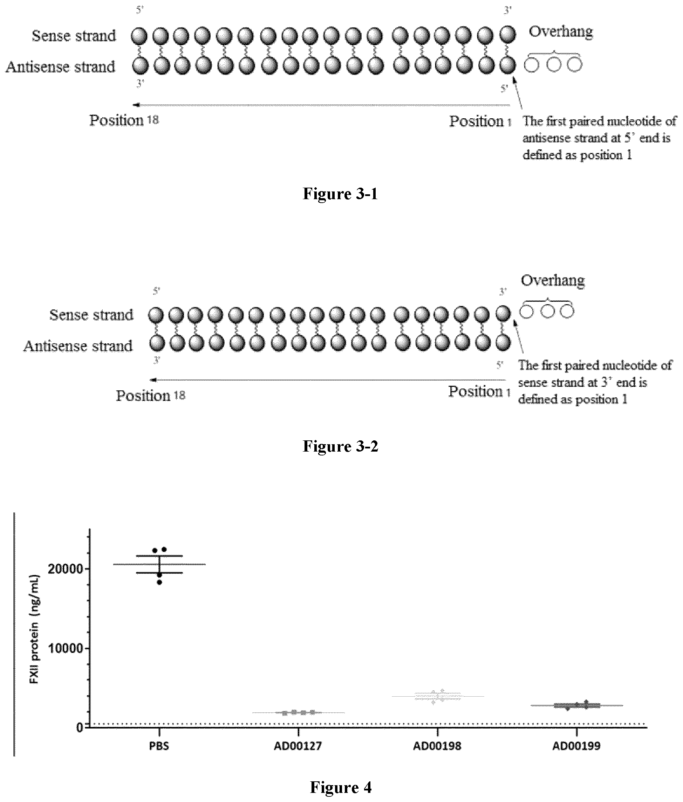

Mice were given a single subcutaneous injection of 1 mg/kg of PBS or siRNA compounds AD00127, AD00198, AD00199, with the GalNAc ligand clusters conjugated to the 3' end of the sense strand. In this study, AD00198 and AD00199 with the modifications disclosed in the prior art were used as positive controls. Plasma samples were collected before and on 14 days after administration. The concentration of mouse FXII protein was measured by ELISA assay according to literature procedures. Seen Liu et al. "An investigational RNAi therapeutic targeting Factor XII (ALN-FXII) for the treatment of hereditary angioedema". RNA. 2019 Feb;25(2):255-263. doi: 10.1261/rna.068916.118). The results are shown in figure 4, indicating that the RNAi agent with the modification characteristics of the present invention has better activity than that of the positive control.

### Example 8. In vivo efficacy of different F-substituion modified RNAi agents

The *in vivo* efficacy of 1 mpk or 1.5 mpk RNAi agents was performed by the experimental method as in Example 5, and the experimental results are shown as Table 9.

**Table 9. Relative expression of FXII mRNA using compounds corresponding to the sequences, chemical modifications and delivery shown in Table 2.**

| AD | Relative percentage ±SD of FXII mRNA remaining in mouse liver on Day 7 | Relative percentage ±SD of FXII mRNA remaining in mouse liver on Day 14 | Administration dosage (mg/kg) |
|---|---|---|---|
| AD00699 | 0.20±0.05 | 0.17±0.04 | 1.5mpk |
| AD00716 | 0.35±0.05 | 0.30±0.05 | |
| AD00864 | 0.28±0.04 | 0.23±0.05 | |
| AD00865 | 0.35±0.10 | 0.26±0.08 | |
| AD00866 | 0.31±0.02 | 0.28±0.04 | |
| AD00867 | 0.37±0.03 | 0.30±0.05 | |
| AD00868 | 0.42±0.07 | 0.37±0.08 | |
| AD00869 | 0.36±0.02 | 0.25±0.05 | |
| AD00870 | 0.33±0.03 | 0.29±0.02 | |
| AD00871 | 0.34±0.05 | 0.26±0.03 | |

Experiments of Table 9 show that the duplexes with the same 2'-F modified positions (9, 11, 13) of the sense strand achieved better silencing effect.

**Table 10. Relative expression of FXII mRNA using compounds corresponding to the sequences, chemical modifications, and delivery shown in Table 2.**

| AD | Relative percentage ±SD of FXII mRNA remaining in mouse liver on Day 7 | Relative percentage ±SD of FXII mRNA remaining in mouse liver on Day 14 | Administration dosage (mg/kg) |
|---|---|---|---|
| AD00699 | 0.17±0.05 | 0.14±0.03 | 1mpk |
| AD00700 | 0.20±0.01 | 0.14±0.01 | |
| AD00928 | 0.21±0.02 | 0.14±0.02 | |
| AD00929 | 0.23±0.01 | 0.17±0.02 | |
| AD00930 | 0.16±0.04 | 0.12±0.04 | |
| AD00933 | 0.08±0.01 | 0.08±0.01 | |
| AD00934 | 0.17±0.02 | 0.14±0.01 | |
| AD00935 | 0.22±0.02 | 0.17±0.02 | |
| AD00936 | 0.30±0.01 | 0.21±0.02 | |
| AD00937 | 0.30±0.03 | 0.24±0.03 | |
| AD00938 | 0.24±0.04 | 0.17±0.02 | |
| AD00939 | 0.20±0.03 | 0.17±0.01 | |
| AD00940 | 0.17±0.03 | 0.17±0.04 | |
| AD00941 | 0.27±0.03 | 0.17±0.02 | |
| AD00942 | 0.31±0.02 | 0.21±0.03 | |
| AD00943 | 0.29±0.02 | 0.25±0.01 | |

Experiments of Table 10 show that the duplexes with the same 2'-F modified positions (9, 11, 12) of the sense strand achieved better silencing effect.

**Table 11. Relative expression of FXII mRNA using compounds corresponding to the sequences, chemical modifications, and delivery shown in Table 2.**

| AD | Relative percentage ±SD of FXII mRNA remaining in mouse liver on Day 7 | Relative percentage ±SD of FXII mRNA remaining in mouse liver on Day 14 | Administration dosage (mg/kg) |
|---|---|---|---|
| AD00699 | 0.27±0.04 | 0.14±0.02 | 1mpk |
| AD00700 | 0.23±0.04 | 0.19±0.01 | |
| AD00933 | 0.21±0.06 | 0.16±0.05 | |
| AD00934 | 0.29±0.04 | 0.17±0.04 | |
| AD00935 | 0.27±0.04 | 0.20±0.04 | |
| AD00936 | 0.32±0.07 | 0.17±0.07 | |
| AD00545 | 0.19±0.07 | 0.11±0.04 | |
| AD00548 | 0.18±0.03 | 0.14±0.05 | |
| AD01029 | 0.17±0.06 | 0.13±0.02 | |
| AD01031 | 0.23±0.06 | 0.12±0.04 | |
| AD01032 | 0.26±0.05 | 0.11±0.03 | |
| AD01033 | 0.39±0.05 | 0.19±0.03 | |
| AD01034 | 0.27±0.04 | 0.14±0.04 | |
| AD01035 | 0.36±0.04 | 0.21±0.02 | |
| AD01036 | 0.33±0.08 | 0.17±0.02 | |
| AD01037 | 0.36±0.10 | 0.21±0.09 | |

**Table 12. Relative expression of FXII mRNA using compounds corresponding to the sequences, chemical modifications, and delivery shown in Table 2.**

| AD | Relative percentage ±SD of FXII mRNA remaining in mouse liver on Day 7 | Relative percentage ±SD of FXII mRNA remaining in mouse liver on Day 14 | Administration dosage (mg/kg) |
|---|---|---|---|
| AD00699 | 0.17±0.03 | 0.14±0.02 | 1mpk |
| AD01222 | 0.09±0.01 | 0.08±0.02 | |
| AD01223 | 0.12±0.02 | 0.09±0.01 | |
| AD01224 | 0.15±0.02 | 0.14±0.03 | |
| AD01225 | 0.16±0.02 | 0.14±0.03 | |
| AD01226 | 0.13±0.01 | 0.11±0.02 | |
| AD01227 | 0.08±0.01 | 0.08±0.01 | |
| AD01228 | 0.22±0.03 | 0.19±0.03 | |
| AD01229 | 0.16±0.02 | 0.12±0.02 | |
| AD01230 | 0.19±0.03 | 0.17±0.01 | |
| AD01232 | 0.14±0.02 | 0.13±0.01 | |
| AD01233 | 0.20±0.04 | 0.21±0.02 | |
| AD01234 | 0.22±0.05 | 0.20±0.03 | |
| AD01235 | 0.18±0.02 | 0.17±0.02 | |
| AD01236 | 0.23±0.02 | 0.23±0.01 | |
| AD01237 | 0.18±0.03 | 0.19±0.03 | |
| AD01238 | 0.17±0.02 | 0.16±0.03 | |

**Table 13. Relative expression of FXII mRNA using compounds corresponding to the sequences, chemical modifications, and delivery shown in Table 2.**

| AD | Relative percentage ±SD of FXII mRNA remaining in mouse liver on Day 7 | Relative percentage ±SD of FXII mRNA remaining in mouse liver on Day 14 | Administration dosage (mg/kg) |
|---|---|---|---|
| AD00699 | 0.21±0.02 | 0.14±0.01 | 1 mpk |
| AD01222 | 0.25±0.04 | 0.23±0.04 | 0.5 mpk |
| AD01222 | 0.11±0.02 | 0.09±0.01 | 1 mpk |
| AD01223 | 0.26±0.04 | 0.23±0.03 | 0.5 mpk |
| AD01224 | 0.32±0.04 | 0.33±0.05 | 0.5 mpk |
| AD01225 | 0.29±0.01 | 0.30±0.01 | 0.5 mpk |
| AD01227 | 0.22±0.02 | 0.21±0.04 | 0.5 mpk |
| AD01230 | 0.45±0.06 | 0.40±0.05 | 0.5 mpk |

### Example 9. In vivo efficacy of different F-substituion modified RNAi agents

The *in vivo* efficacy of 1 mpk or 0.5 mpk RNAi agents was performed by the experimental method as in Example 5. On days 7, 14, 21 and 28, blood was collected from all mice through the submandibular vein to collect plasma for detection of FXII protein levels. The experimental results are shown as Table 14.

**Table 14. Relative expression of FXII mRNA using compounds corresponding to the sequences, chemical modifications, and delivery shown in Table 2.**

| AD | Relative percentage ±SD of FXII mRNA remaining in mouse liver on Day 7 | Relative percentage ±SD of FXII mRNA remaining in mouse liver on Day 14 | Relative percentage ±SD of FXII mRNA remaining in mouse liver on Day 21 | Relative percentage ±SD of FXII mRNA remaining in mouse liver on Day 28 | Administ ration dosage (mg/kg) |
|---|---|---|---|---|---|
| AD00699 | 0.34±0.04 | 0.27±0.02 | 0.26±0.03 | 0.40±0.03 | 0.5 |
| AD01987 | 0.24±0.03 | 0.23±0.04 | 0.23±0.06 | 0.21±0.04 | 0.5 |
| AD01988 | 0.32±0.02 | 0.26±0.06 | 0.21±0.03 | 0.19±0.02 | 0.5 |
| AD00699 | 0.17±0.02 | 0.13±0.02 | 0.13±0.02 | 0.25±0.04 | 1 |
| AD01987 | 0.21±0.05 | 0.14±0.01 | 0.14±0.02 | 0.10±0.02 | 1 |
| AD01988 | 0.18±0.03 | 0.12±0.03 | 0.12±0.03 | 0.14±0.03 | 1 |

### Equivalents

Although several embodiments of the present invention have been described and illustrated herein, those of ordinary skill in the art will readily envision a variety of other means and/or structures for performing the functions and/or obtaining the results and/or one or more of the advantages described herein, and each of such variations and/or modifications is deemed to be within the scope of the present invention. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings of the present invention is/are used. Those skilled in the art will recognize, or be able to ascertain using no more than routine experimentation, many equivalents to the specific embodiments of the invention described herein. It is, therefore, to be understood that the foregoing embodiments are presented by way of example only and that, within the scope of the appended claims and equivalents thereto; the invention may be practiced otherwise than as specifically described and claimed. The present invention is directed to each individual feature, system, article, material, and/or method described herein. In addition, any combination of two or more such features, systems, articles, materials, and/or methods, if such features, systems, articles, materials, and/or methods are not mutually inconsistent, is included within the scope of the present invention.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Other elements may optionally be present other than the elements specifically identified by the "and/or" clause, whether related or unrelated to those elements specifically identified, unless clearly indicated to the contrary.

All references, patents and patent applications and publications that are cited or referred to in this application are incorporated herein in their entirety herein by reference.

## Claims

1. An RNAi agent for inhibiting expression of a target gene, wherein the RNAi agent is a double-stranded ribonucleic acid (dsRNA) agent comprising a sense strand and an antisense strand complementary to at least a portion of an mRNA corresponding to the target gene, and the antisense strand has 18-30 nucleotides and comprises a sequence represented by formula (X) in the direction of 3'-5':
3'-(N_{X})ₙ N_{X} N_{X} N_{X} N_{X} N_{F} N_{X} N_{X} N_{X} N_{X} N_{X} N_{X} N_{X} N_{X} N_{X}N_{X} N_{X} N_{F} N_{X}-5' formula (X)
wherein, each N_{F} represents a 2'-fluoro modified nucleotide;
each N_{X} independently represents a modified or an unmodified nucleotide, wherein no more than three N_{X} are 2'-fluoro modified nucleotides, or only one N_{X} is a 2'-fluoro modified nucleotide; and
n is an integer from 0 to 7.

2. The dsRNA agent according to claim 1, wherein the antisense strand comprises a sequence represented by formula (I) in the direction of 3'-5' :
3'-(N_{X})ₙ N_{X} N_{X} N_{X} N_{X} N_{F} N_{X} N_{X} N_{X} N_{X} N_{X} N_{X} N_{X} N_{X} N_{X}N_{X} N_{X} N_{F} N_{X}-5' formula (I)
wherein, each N_{F} represents a 2'-fluoro modified nucleotide;
each N_{X} independently represents a modified or an unmodified nucleotide, wherein no more than three N_{X} are 2'-fluoro modified nucleotides, or only one N_{X} is a 2'-fluoro modified nucleotide; and
n is an integer from 0 to 7.

3. The dsRNA agent according to claim 1 or 2, wherein the antisense strand comprises a sequence represented by formula (I-1) in the direction of 3' to 5':
3'-(N_{L})ₙ N_{M1} N_{L} N_{M2} N_{L}N_{F} N_{L} N_{M3} N_{L} N_{M4} N_{L} N_{M5} N_{M6} N_{L} N_{M7} N_{M8} N_{L} N_{F} N_{L}-5' formula (I-1)
wherein, each N_{F} represents a 2'-fluoro modified nucleotide;
N_{M1}, N_{M2}, N_{M3}, N_{M4}, N_{M5}, N_{M6}, N_{M7}, and N_{M8} independently represent a modified or an unmodified nucleotide, wherein only three of N_{M1}, N_{M2}, N_{M3}, N_{M4}, N_{M5}, N_{M6}, N_{M7}, and N_{M8} are 2'-fluoro modified nucleotides, or only one of N_{M1}, N_{M2}, N_{M3}, N_{M4}, N_{M5}, N_{M6}, N_{M7}, and N_{M8} is a 2'-fluoro modified nucleotide;
each N_{L} independently represents a modified or an unmodified nucleotide, wherein the modified nucleotide is not a 2'-fluoro modified nucleotide; and
n is an integer from 0 to 7.

4. The dsRNA agent according to claim 3, wherein in the antisense strand represented by formula (I-1), only three of N_{M1}, N_{M2}, N_{M3}, N_{M4}, N_{M6}, N_{M7}, and N_{M8} are 2'-fluoro modified nucleotides, and N_{M5} is not a 2'-F modified nucleotide.

5. The dsRNA agent according to any one of claims 1-3, wherein the antisense strand comprises a sequence represented by formula (II) in the direction of 3'-5':
3'-(N_{L})ₙ N_{M1} N_{L} N_{M2} N_{L} N_{F} N_{L} N_{M3} N_{L} N_{M4} N_{L} N_{M5} N_{F} N_{L} N_{M7} N_{M8} N_{L} N_{F} N_{L}-5' formula (II)
wherein, each N_{F} represents a 2'-fluoro modified nucleotide;
N_{M1}, N_{M2}, N_{M3}, N_{M4}, N_{M5}, N_{M7}, and N_{M8} independently represent a modified or an unmodified nucleotide, wherein only two of N_{M1}, N_{M2}, N_{M3}, and N_{M4} are 2'-fluoro modified nucleotides, and N_{M5}, N_{M7} and N_{M8} are not 2'-fluoro modified nucleotides;
each N_{L} independently represents a modified or an unmodified nucleotide, wherein the modified nucleotide is not a 2'-fluoro modified nucleotide; and
n is an integer from 0 to 7.

6. The dsRNA agent according to any one of claims 1-3, wherein the antisense strand comprises a sequence represented by formula (III) in the direction of 3'-5':
3'-(N_{L})ₙ N_{M1} N_{L} N_{M2} N_{L}N_{F} N_{L}N_{F} N_{L} N_{M4} N_{L} N_{M5} N_{M6} N_{L} N_{M7} N_{M8} N_{L}N_{F} N_{L}-5' formula (III)
wherein, each N_{F} represents a 2'-fluoro modified nucleotide;
N_{M1}, N_{M2}, N_{M4}, N_{M5}, N_{M6}, N_{M7}, and N_{M8} independently represent a modified or an unmodified nucleotide, wherein only two of N_{M1}, N_{M2}, N_{M6}, and N_{M7} are 2'-fluoro modified nucleotides, and N_{M4}, N_{M5} and N_{M8} are not 2'-fluoro modified nucleotides;
each N_{L} independently represents a modified or an unmodified nucleotide, wherein the modified nucleotide is not a 2'-fluoro modified nucleotide; and
n is an integer from 0 to 7.

7. The dsRNA agent according to any one of claims 3-6, wherein the dsRNA agent has 2'-fluoro modified nucleotides at positions 2, 7, 12, 14, and 16 of the antisense strand represented by formula (I-1) counting from the first paired nucleotide at 5' end.

8. The dsRNA agent according to any one of claims 3-6, wherein the dsRNA agent has 2'-fluoro modified nucleotides at positions 2, 7, 12, 14, and 18 of the antisense strand represented by formula (I-1) counting from the first paired nucleotide at 5' end.

9. The dsRNA agent according to any one of claims 3-6, wherein the dsRNA agent has 2'-fluoro modified nucleotides at positions 2, 5, 12, 14, and 18 of the antisense strand represented by formula (I-1) counting from the first paired nucleotide at 5' end.

10. The dsRNA agent according to any one of claims 3-6, wherein the dsRNA agent has 2'-fluoro modified nucleotides at positions 2, 4, 10, 14, and 16 of the antisense strand represented by formula (I-1) counting from the first paired nucleotide at 5' end.

11. The dsRNA agent according to any one of claims 3-6, wherein the dsRNA agent has 2'-fluoro modified nucleotides at positions 2, 7, 10, 14, and 16 of the antisense strand represented by formula (I-1) counting from the first paired nucleotide at 5' end.

12. The dsRNA agent according to any one of claims 3-6, wherein the dsRNA agent has 2'-fluoro modified nucleotides at positions 2, 5, 12, 14, and 16 of the antisense strand represented by formula (I-1) counting from the first paired nucleotide at 5' end.

13. The dsRNA agent according to any one of claims 3-6, wherein the dsRNA agent has 2'-fluoro modified nucleotides at positions 2, 5, 10, 14, and 16 of the antisense strand represented by formula (I-1) counting from the first paired nucleotide at 5' end.

14. The dsRNA agent according to claim 1 or 2, wherein in the antisense strand represented by formula (I), only one of N_{M1}, N_{M2}, N_{M3}, N_{M5}, N_{M6}, and N_{M8} is a 2'-fluoro modified nucleotide, and N_{M4} and N_{M7} are not 2'-F modified nucleotides.

15. The dsRNA agent according to claim 14, wherein the antisense strand comprises a sequence represented by formula (IV) in the direction of 3'-5' :
3'-(N_{L})ₙ N_{M1} N_{L} N_{M2} N_{L} N_{F} N_{L} N_{M3} N_{L} N_{M4} N_{L} N_{M5} N_{M6} N_{L} N_{M7} N_{M8} N_{L} N_{F} N_{L}-5' formula (IV)
wherein, each N_{F} represents a 2'-fluoro modified nucleotide;
N_{M1}, N_{M2}, N_{M3}, N_{M4}, N_{M5}, N_{M6}, N_{M7}, and N_{M8} independently represent a modified or an unmodified nucleotide, wherein only one of N_{M1}, N_{M2}, N_{M3}, N_{M5}, N_{M6}, and N_{M8} is a 2'-fluoro modified nucleotide, and N_{M4} and N_{M7} are not 2'-fluoro modified nucleotides;
each N_{L} independently represents a modified or an unmodified nucleotide, wherein the modified nucleotide is not a 2'-fluoro modified nucleotide; and
n is an integer from 0 to 7.

16. The dsRNA agent according to claim 15, wherein the dsRNA agent has 2'-fluoro modified nucleotides at positions 2, 4, and 14 of the antisense strand represented by formula (IV) counting from the first paired nucleotide at 5' end.

17. The dsRNA agent according to claim 15, wherein the dsRNA agent has 2'-fluoro modified nucleotides at positions 2, 8, and 14 of the antisense strand represented by formula (IV) counting from the first paired nucleotide at 5' end.

18. The dsRNA agent according to claim 15, wherein the dsRNA agent has 2'-fluoro modified nucleotides at positions 2, 12 and 14 of the antisense strand represented by formula (IV) counting from the first paired nucleotide at 5' end.

19. The dsRNA agent according to claim 15, wherein the dsRNA agent has 2'-fluoro modified nucleotides at positions 2, 14, and 16 of the antisense strand represented by formula (IV) counting from the first paired nucleotide at 5' end.

20. The dsRNA agent according to claim 15, wherein the dsRNA agent has 2'-fluoro modified nucleotides at positions 2, 14, and 18 of the antisense strand represented by formula (IV) counting from the first paired nucleotide at 5' end.

21. The dsRNA agent according to claim 15, wherein the dsRNA agent has 2'-fluoro modified nucleotides at positions 2, 6, and 14 of the antisense strand represented by formula (IV) counting from the first paired nucleotide at 5' end.

22. The dsRNA agent according to claim 1 or 2, wherein the antisense strand comprises a sequence represented by the formula (I-2) in the direction of 3' to 5':
3'-(N_{L})ₙ N_{M1} N_{L} N_{M2} N_{L} N_{F} N_{L} N_{M3} N_{M9} N_{M4} N_{M10} N_{M5} N_{M6} N_{L} N_{M7} N_{M8} N_{L} N_{F} N_{L} -5' formula (I-2)
wherein, each N_{F} represents a 2'-fluoro modified nucleotide;
N_{M1}, N_{M2}, N_{M3}, N_{M4}, N_{M5}, N_{M6}, N_{M7}, N_{M8}, N_{M9} and N_{M10} independently represent a modified or an unmodified nucleotide, wherein only three of N_{M1}, N_{M2}, N_{M3}, N_{M4}, N_{M5}, N_{M6}, N_{M7}, N_{M8}, N_{M9} and N_{M10} are 2'-fluoro modified nucleotides;
each N_{L} independently represents a modified or an unmodified nucleotide, wherein the modified nucleotide is not a 2'-fluoro modified nucleotide; and
n is an integer from 0 to 7.

23. The dsRNA agent according to claim 22, wherein the dsRNA agent has 2'-fluoro modified nucleotides at positions 2, 7, 11, 14, and 16 of the antisense strand represented by formula (I-2) counting from the first paired nucleotide at 5' end.

24. The dsRNA agent according to claim 22, wherein the dsRNA agent has 2'-fluoro modified nucleotides at positions 2, 7, 11, 14, and 18 of the antisense strand represented by formula (I-2) counting from the first paired nucleotide at 5' end.

25. The dsRNA agent according to claim 22, wherein the dsRNA agent has 2'-fluoro modified nucleotides at positions 2, 8, 12, 14, and 16 of the antisense strand represented by formula (I-2) counting from the first paired nucleotide at 5' end.

26. The dsRNA agent according to claim 22, wherein the dsRNA agent has 2'-fluoro modified nucleotides at positions 2, 9, 12, 14, and 16 of the antisense strand represented by formula (I-2) counting from the first paired nucleotide at 5' end.

27. The dsRNA agent according to any one of claims 3-26, wherein in the antisense strand represented by formula (I-1), formula (I-2), formula (II), formula (III) and/or formula (IV), one or more of N_{L} are modified nucleotides independently selected from the group consisting of 2'-O-methyl modified nucleotide, 2'-deoxy nucleotide, 2',3'-seco nucleotide mimic, locked nucleotide (LNA), unlocked nucleic acid nucleotide (UNA), glycol nucleic acid nucleotide (GNA), bicyclo nucleic acid (BNA), 2'-F-arabino nucleotide, 2'-methoyxyethyl nucleotide, abasic nucleotide, ribitol, inverted nucleotide, inverted abasic nucleotide (Invab), inverted 2'-OMe nucleotide, inverted 2'-deoxy nucleotide, 2'-amino modified nucleotide, 2'-alkyl modified nucleotide, morpholino nucleotide, 3'-OMe nucleotide, phosphorothioate modified nucleotide, terminal nucleotide linked to a cholesteryl derivative or dodecanoic acid bisdecylamide group, 2'-amino modified nucleotide, phosphoramidate, and non-natural base comprising nucleotide; preferably, N_{L} in the dsRNA agent is a modified nucleotide selected from the group consisting of 2'-O-methyl modified nucleotide, UNA, and Invab.

28. The dsRNA agent according to claim 1 or 2, wherein in the antisense strand represented by formula (I) and/or formula (X), one or more of N_{X} are modified nucleotides independently selected from the group consisting of 2'-fluoro modified nucleotide, 2'-O-methyl modified nucleotide, 2'-deoxy nucleotide, 2',3'-seco nucleotide mimic, locked nucleotide (LNA), unlocked nucleic acid nucleotide (UNA), glycol nucleic acid nucleotide (GNA), bicyclo nucleic acid (BNA), 2'-F-arabino nucleotide, 2'-methoyxyethyl nucleotide, abasic nucleotide, ribitol, inverted nucleotide, inverted abasic nucleotide (Invab), inverted 2'-OMe nucleotide, inverted 2'-deoxy nucleotide, 2'-amino modified nucleotide, 2'-alkyl modified nucleotide, morpholino nucleotide, 3'-OMe nucleotide, phosphorothioate modified nucleotide, terminal nucleotide linked to a cholesteryl derivative or dodecanoic acid bisdecylamide group, 2'-amino modified nucleotide, phosphoramidate, and non-natural base comprising nucleotide; preferably, N_{L} in the dsRNA agent is a modified nucleotide selected from the group consisting of 2'-O-methyl modified nucleotide, UNA, and Invab.

29. The dsRNA agent according to any one of claims 1-28, wherein the antisense strand has an E-vinylphosphonate nucleotide at 5' end of the antisense strand.

30. The dsRNA agent according to any one of claims 3-28, wherein in the antisense strand represented by formula (I-1), formula (I-2), formula (II), formula (III) and/or formula (IV), N_{M1}, N_{M2}, N_{M3}, N_{M4}, N_{M5}, N_{M6}, N_{M7}, N_{M8}, N_{M9} and/or N_{M10} are modified nucleotides independently selected from the group consisting of 2'-fluoro modified nucleotide, 2'-O-methyl modified nucleotide, 2'-deoxy nucleotide, 2',3'-seco nucleotide mimic, locked nucleotide (LNA), unlocked nucleic acid nucleotide (UNA), glycol nucleic acid nucleotide (GNA), bicyclo nucleic acid (BNA), 2'-F-arabino nucleotide, 2'-methoyxyethyl nucleotide, abasic nucleotide, ribitol, inverted nucleotide, inverted abasic nucleotide (Invab), inverted 2'-OMe nucleotide, inverted 2'-deoxy nucleotide, 2'-amino modified nucleotide, 2'-alkyl modified nucleotide, morpholino nucleotide, 3'-OMe nucleotide, phosphorothioate modified nucleotide, terminal nucleotide linked to a cholesteryl derivative or dodecanoic acid bisdecylamide group, 2'-amino modified nucleotide, phosphoramidate, and non-natural base comprising nucleotide.

31. The dsRNA agent according to claim 30, wherein in the antisense strand represented by formula (I-1), N_{M1}, N_{M2}, N_{M3}, N_{M4}, N_{M5}, N_{M6}, N_{M7}, and N_{M8} are independently 2'-O methyl modified nucleotides in case of not being 2'-fluoro modified nucleotides.

32. The dsRNA agent according to any one of claims 1-31, wherein the dsRNA agent has a sense strand complementary or substantially complementary to the antisense strand, and the complementary region is 18-25 nucleotides in length, preferably 18-23 nucleotides in length, preferably 19-21 nucleotides in length, preferably 18, 19, 20, 21, 22, 23, 24, or 25 nucleotides in length.

33. The dsRNA agent according to any one of claims 1-32, wherein the dsRNA agent has a sense strand fully complementary to the antisense strand.

34. The dsRNA agent according to any one of claims 1-33, wherein n is 0, 1, 2, 3, 4, 5, 6, or 7.

35. The dsRNA agent according to any one of claims 1-34, wherein the sense strand is no more than 40 nucleotides in length; preferably, each strand of the dsRNA agent is no more than 30 nucleotides in length, preferably no more than 25 nucleotides in length, preferably no more than 23 nucleotides in length, preferably 19, 20, or 21 nucleotides in length.

36. The dsRNA agent according to any one of claims 1-35, wherein either strand of the dsRNA agent comprises one or more of an overhang at the 3' or 5' end or both ends, and the overhang is 1-5 nucleotides in length.

37. The dsRNA agent according to any one of claims 1-36, wherein the antisense strand of the dsRNA agent has a blunt end at 3' end.

38. The dsRNA agent according to any one of claims 1-37, wherein the antisense strand of the dsRNA agent has an overhang of 1, 2, 3, 4, or 5 unpaired nucleotides at 3' end, and/or the sense strand of the dsRNA agent has an overhang of 1, 2, 3, 4, or 5 unpaired nucleotides at 5' end.

39. The dsRNA agent according to any one of claims 1-38, wherein the sense strand of the dsRNA agent has a blunt end at 3' end, and/or the antisense strand of the dsRNA agent has a blunt end at 5' end.

40. The dsRNA agent according to any one of claims 1-39, wherein the dsRNA agent comprises at least one phosphorothioate internucleoside linkage; preferably, the sense strand of the dsRNA agent comprises at least one phosphorothioate internucleoside linkage; preferably, the antisense strand of the dsRNA agent comprises at least one phosphorothioate internucleoside linkage; preferably, the sense strand and/or antisense strand of the dsRNA agent comprises 1, 2, 3, 4, 5, or 6 phosphorothioate internucleoside linkages; preferably, the dsRNA agent comprises 1 or 2 phosphorothioate internucleoside linkages at 3' end and/or 5' end of the sense strand and/or the antisense strand.

41. The dsRNA agent according to any one of claims 1-40, wherein the dsRNA agent further comprises one or more targeting groups or linking groups.

42. The dsRNA agent according to claim 41, wherein the one or more targeting groups or linking groups are conjugated to the sense strand.

43. The dsRNA agent according to claim 41, wherein the targeting group or linking group is selected from the group consisting of N-acetyl-galactosamine (GalNAc) and a lipophilic molecule.

44. The dsRNA agent according to claim 41, wherein the targeting group or linking group is conjugated to 5' end of the sense strand, and the targeting group has a structure selected from the group consisting of:

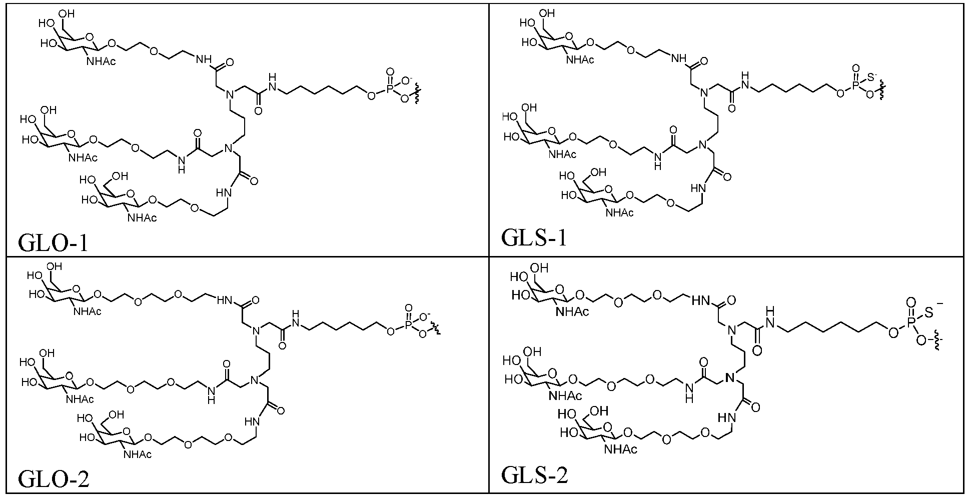

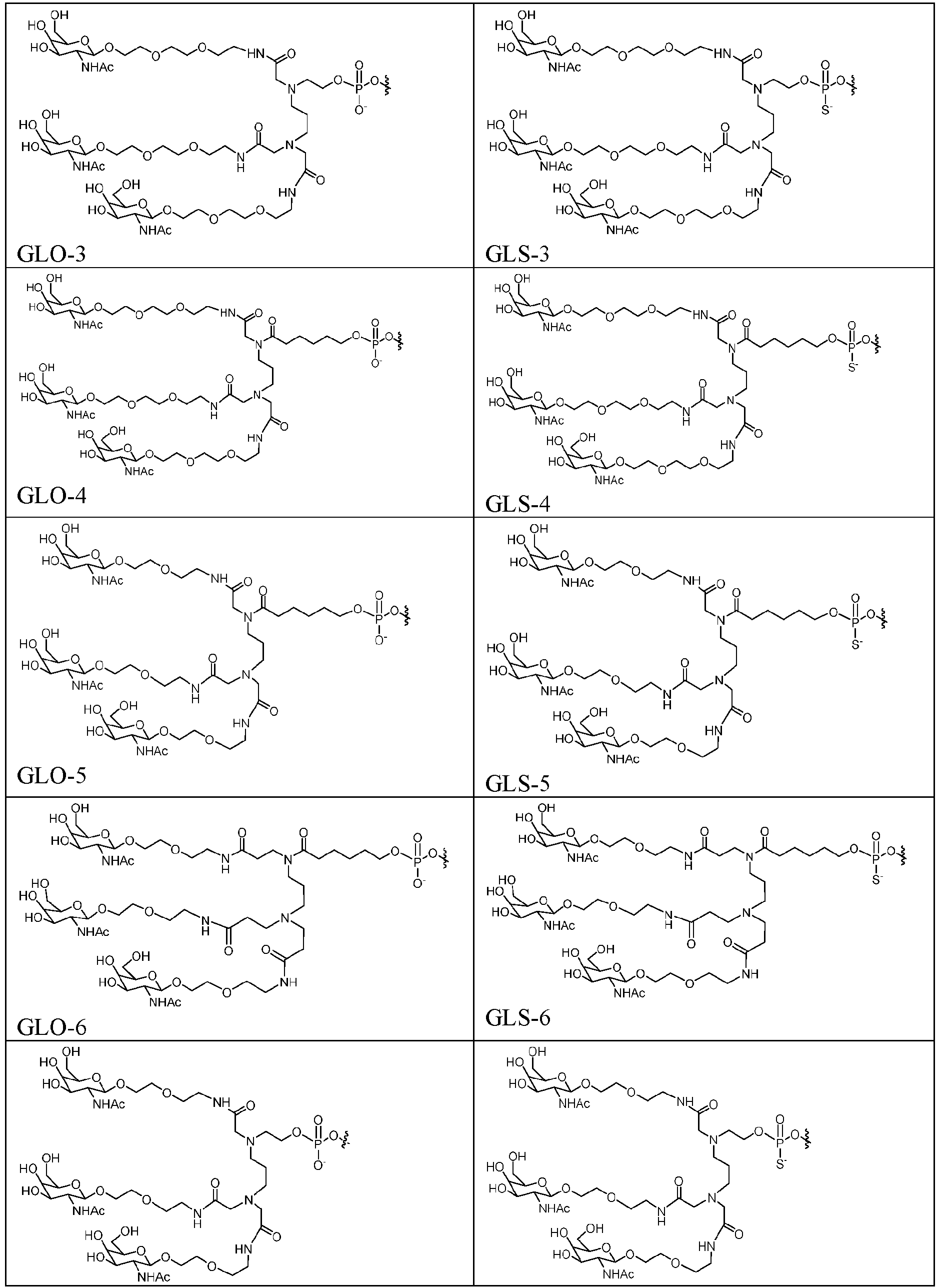

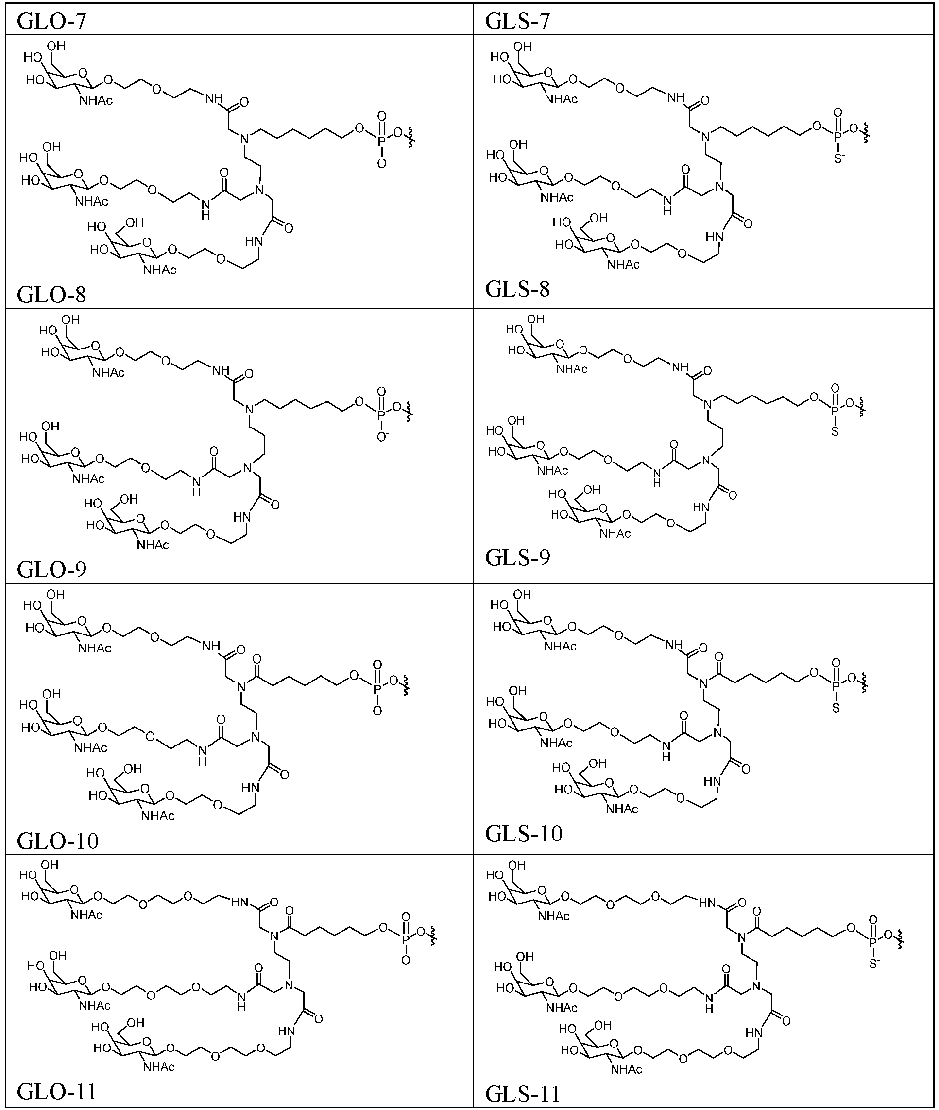

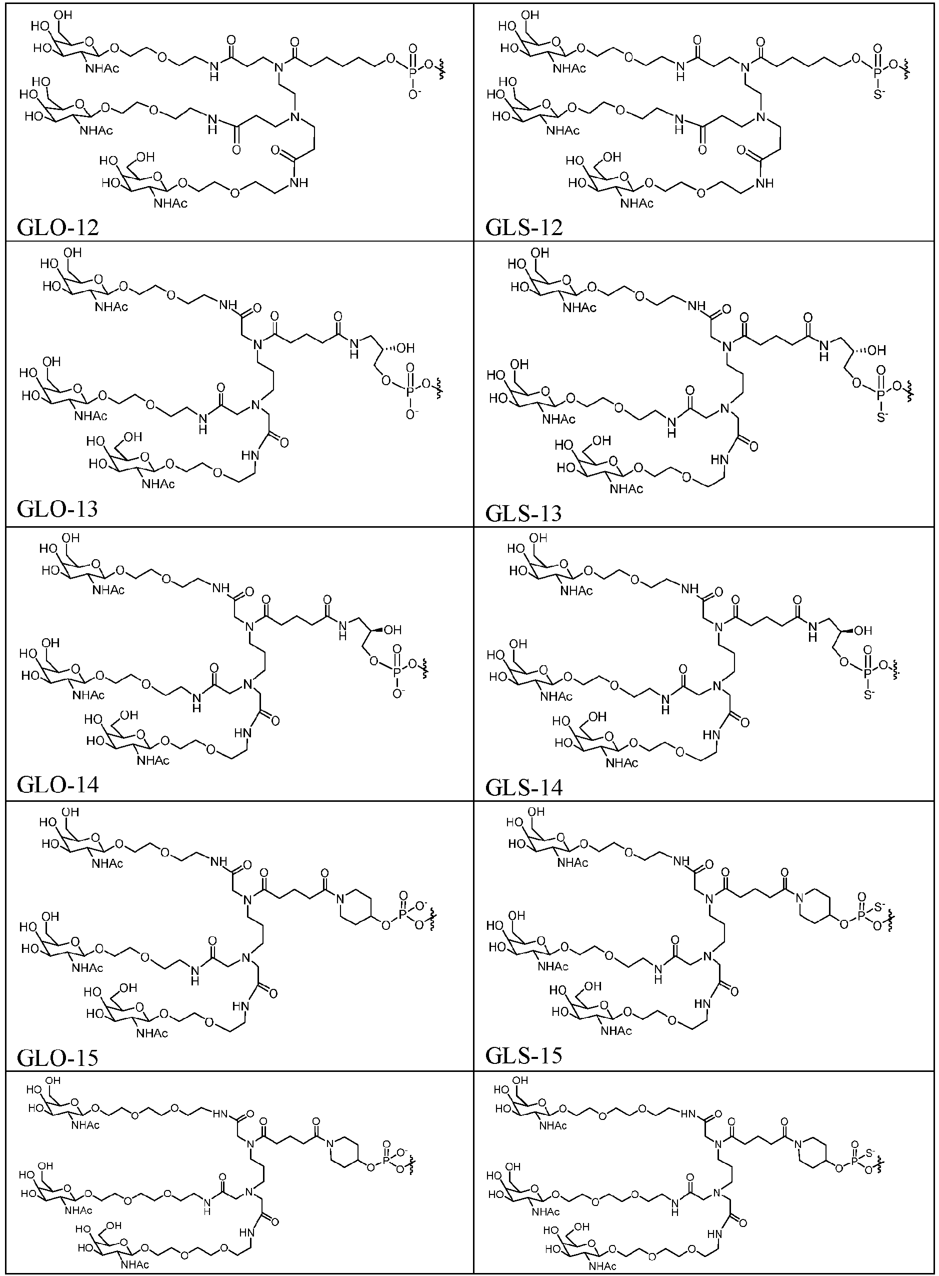

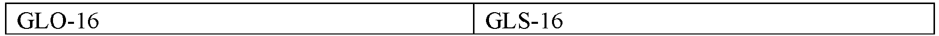

45. The dsRNA agent according to any one of claims 1-44, wherein the antisense strand of the dsRNA agent comprises an inverted abasic residue at 3' end; preferably, the sense strand of the dsRNA agent comprises one or two inverted abasic residues at 3' end or/and 5' end.

46. An RNAi agent for inhibiting expression of a target gene, wherein the RNAi agent is a double-stranded ribonucleic acid (dsRNA) agent comprising a sense strand and an antisense strand complementary to at least a portion of an mRNA corresponding to the target gene, and the sense strand has 18-40 nucleotides and comprises a sequence represented by formula (V) in the direction of 5'-3':
5'-(N'_{L})_{n'} N'_{L} N'_{L} N'_{L} N'_{N1} N'_{N2} N'_{N3} N'_{N4} N'_{F} N'_{L} N'_{N5}N'_{N6} N'_{L} N'_{L} N'_{L} N'_{L} N'_{L} N'_{L} N'_{L}-3' formula (V)
wherein, each N'_{F} represents a 2'-fluoro modified nucleotide;
N'_{N1}, N'_{N2}, N'_{N3}, N'_{N4}, N'_{N5}, and N'_{N6} independently represent a modified or an unmodified nucleotide, wherein at least two of N'_{N1}, N'_{N2}, N'_{N3}, N'_{N4}, N'_{N5}, and N'_{N6} are 2'-fluoro modified nucleotides;
formula (V) has no motif of three or more contiguous 2'-fluoro modified nucleotides and has no more than six 2'-fluoro modified nucleotides;
each N'_{L} independently represents a modified or an unmodified nucleotide; and
n' is an integer from 0 to 7.

47. The dsRNA agent according to claim 46, wherein the sense strand comprises a sequense represented by formula (V') in the direction of 5'-3':
5'-(N'_{L})_{n'} N'_{L} N'_{L} N'_{L} N'_{N1} N'_{N2} N'_{N3} N'_{N4} N'_{F} N'_{L} N'_{N5}N'_{N6} N'_{L} N'_{L} N'_{L} N'_{L} N'_{L} N'_{L} N'_{L} -3' formula (V')
wherein, each N'_{F} represents a 2'-fluoro modified nucleotide;
N'_{N1}, N'_{N2}, N'_{N3}, N'_{N4}, N'_{N5}, and N'_{N6} independently represent a modified or an unmodified nucleotide, wherein only two of N'_{N1}, N'_{N2}, N'_{N3}, N'_{N4}, N'_{N5}, and N'_{N6} are 2'-fluoro modified nucleotides.
each N'_{L} independently represents a modified or an unmodified nucleotide, wherein the modified nucleotide is not a 2'-fluoro modified nucleotide; and
n' is an integer from 0 to 7.

48. The dsRNA agent according to claim 47, wherein the dsRNA agent has 2'-fluoro modified nucleotides at positions 9, 11, and 13 of the sense strand represented by formula (V') counting from the first paired nucleotide at 3' end of the sense strand.

49. The dsRNA agent according to claim 47, wherein the dsRNA agent has 2'-fluoro modified nucleotides at positions 9, 11 and 12 of the sense strand represented by formula (V') counting from the first paired nucleotide at 3' end of the sense strand.

50. The dsRNA agent according to claim 47, wherein the dsRNA agent has 2'-fluoro modified nucleotides at positions 8, 11 and 13 of the sense strand represented by formula (V') counting from the first paired nucleotide at 3' end of the sense strand.

51. The dsRNA agent according to claim 47, wherein the dsRNA agent has 2'-fluoro modified nucleotides at positions 11, 12 and 14 of the sense strand represented by formula (V') counting from the first paired nucleotide at 3' end of the sense strand.

52. The dsRNA agent according to claim 47, wherein the dsRNA agent has 2'-fluoro modified nucleotides at positions 11, 12 and 15 of the sense strand represented by formula (V') counting from the first paired nucleotide at 3' end of the sense strand.

53. An RNAi agent for inhibiting expression of a target gene, wherein the RNAi agent is a double-stranded ribonucleic acid (dsRNA) agent comprising a sense strand and an antisense strand complementary to at least a portion of an mRNA corresponding to the target gene, and the sense strand has 18-40 nucleotides and comprises a sequence represented by formula (IX) in the direction of 5'-3':
5'-(N'_{L})_{n'} N'_{L} N'_{L} N'_{L} N'_{N1} N'_{N2} N'_{N3} N'_{N4} N'_{F} N'_{L} N'_{N5}N'_{N6} N'_{N7} N'_{L} N'_{L} N'_{L} N'_{L} N'_{L} N'_{L}-3' formula (IX)
wherein, each N'_{F} represents a 2'-fluoro modified nucleotide;
N'_{N1}, N'_{N2}, N'_{N3}, N'_{N4}, N'_{N5}, N'_{N6}, and N'_{N7} independently represent a modified or an unmodified nucleotide, wherein at least two of N'_{N1}, N' _{N2}, N'_{N3}, N'_{N4}, N'_{N5}, N'_{N6}, and N'_{N7} are 2'-fluoro modified nucleotides;
formula (IX) has no motif of three or more contiguous 2'-fluoro modified nucleotides and has no more than six 2'-fluoro modified nucleotides;
each N'_{L} independently represents a modified or an unmodified nucleotide; and
n' is an integer from 0 to 7.

54. The dsRNA agent according to claim 53, wherein the dsRNA agent has 2'-fluoro modified nucleotides at positions 7, 9, 11 and 13 of the sense strand represented by formula (IX) counting from the first paired nucleotide at 3' end of the sense strand.

55. The dsRNA agent according to any one of claims 46-54, wherein in the sense strand represented by formula (V) or formula (IX), one or more of N'_{L} are modified nucleotides independently selected from the group consisting of 2'-fluoro modified nucleotide, 2'-O-methyl modified nucleotide, 2'-deoxy nucleotide, 2',3'-seco nucleotide mimic, locked nucleotide (LNA), unlocked nucleic acid nucleotide (UNA), glycol nucleic acid nucleotide (GNA), bicyclo nucleic acid (BNA), 2'-F-arabino nucleotide, 2'-methoyxyethyl nucleotide, abasic nucleotide, ribitol, inverted nucleotide, inverted abasic nucleotide (Invab), inverted 2'-OMe nucleotide, inverted 2'-deoxy nucleotide, 2'-amino modified nucleotide, 2'-alkyl modified nucleotide, morpholino nucleotide, 3'-OMe nucleotide, phosphorothioate modified nucleotide, terminal nucleotide linked to a cholesteryl derivative or dodecanoic acid bisdecylamide group, 2'-amino modified nucleotide, phosphoramidate, and non-natural base comprising nucleotide; preferably, N'_{L} in formula (V) is a modified nucleotide selected from the group consisting of 2'-O-methyl modified nucleotide, UNA, and Invab.

56. The dsRNA agent according to any one of claims 46-55, the dsRNA agent has an E-vinylphosphonate nucleotide at 5' end of the antisense strand.

57. The dsRNA agent according to any one of claims 46-56, wherein in the sense strand represented by formula (V) or formula (IX), N'_{N1}, N'_{N2}, N'_{N3}, N'_{N4}, N'_{N5}, N'_{N6}, and N'_{N7} are modified nucleotides independently selected from the group consisting of 2'-fluoro modified nucleotide, 2'-O-methyl modified nucleotide, 2'-deoxy nucleotide, 2',3'-seco nucleotide mimic, locked nucleotide (LNA), unlocked nucleic acid nucleotide (UNA), glycol nucleic acid nucleotide (GNA), bicyclo nucleic acid (BNA), 2'-F-arabino nucleotide, 2'-methoyxyethyl nucleotide, abasic nucleotide, ribitol, inverted nucleotide, inverted abasic nucleotide (Invab), inverted 2'-OMe nucleotide, inverted 2'-deoxy nucleotide, 2'-amino modified nucleotide, 2'-alkyl modified nucleotide, morpholino nucleotide, 3'-OMe nucleotide, phosphorothioate modified nucleotide, terminal nucleotide linked to a cholesteryl derivative or dodecanoic acid bisdecylamide group, 2'-amino modified nucleotide, phosphoramidate, and non-natural base comprising nucleotide.

58. The dsRNA agent according to claim 57, N'_{N1}, N'_{N2}, N'_{N3}, N'_{N4}, N'_{N5}, N'_{N6}, and N'_{N7} are independently 2'-O methyl modified nucleotides in case of not being 2'-fluoro modified nucleotides.

59. The dsRNA agent according to any one of claims 46-58, wherein the dsRNA agent has an antisense strand complementary or substantially complementary to the sense strand, and the complementary region is 18-25 nucleotides in length, preferably 18-23 nucleotides in length, preferably 19-21 nucleotides in length, preferably 18, 19, 20, 21, 22, 23, 24, or 25 nucleotides in length.

60. The dsRNA agent according to any one of claims 46-59, wherein the dsRNA agent has an antisense strand fully complementary to the sense strand.

61. The dsRNA agent according to any one of claims 46-60, wherein n' is 0, 1, 2, 3, 4, 5, 6, or 7.

62. The dsRNA agent according to any one of claims 46-61, wherein the antisense strand is no more than 30 nucleotides in length; preferably, each strand of the dsRNA agent is no more than 30 nucleotides in length, preferably no more than 25 nucleotides in length, preferably no more than 23 nucleotides in length, preferably 19, 20, or 21 nucleotides in length.

63. The dsRNA agent according to any one of claims 46-62, wherein the sense strand represented by formula (V) or formula (IX) has an overhang with unpaired nucleotide at one or two positions at 5' end.

64. The dsRNA agent according to any one of claims 46-63, wherein the sense strand represented by formula (V) or formula (IX) has a blunt end at 5' end.

65. The dsRNA agent according to any one of claims 46-64, wherein the antisense strand of the dsRNA agent has an overhang of 1, 2, 3, 4, 5, 6, 7, 8, 9, or 10 unpaired nucleotides at 3' end.

66. The dsRNA agent according to any one of claims 46-65, wherein the antisense strand of the dsRNA has a blunt end at 5' end, and/or the sense strand of the dsRNA has a blunt end at 3' end.

67. The dsRNA agent according to any one of claims 46-66, wherein the dsRNA agent comprises at least one phosphorothioate internucleoside linkage; preferably, the sense strand of the dsRNA agent comprises at least one phosphorothioate internucleoside linkage; preferably, the antisense strand of the dsRNA agent comprises at least one phosphorothioate internucleoside linkage; preferably, the sense strand and/or antisense strand of the dsRNA agent comprises 1, 2, 3, 4, 5, or 6 phosphorothioate internucleoside linkages; preferably, the dsRNA agent comprises 1 or 2 phosphorothioate internucleoside linkages at 3' end and/or 5' end of the sense strand and/or the antisense strand.

68. The dsRNA agent according to any one of claims 46-67, wherein the dsRNA agent further comprises one or more targeting groups or linking groups.

69. The dsRNA agent according to claim 68, wherein the one or more targeting groups or linking groups are conjugated to the sense strand.

70. The dsRNA agent according to claim 68, wherein the targeting group or linking group is selected from the group consisting of N-acetyl-galactosamine (GalNAc) and a lipophilic molecule, and the targeting group or linking group is conjugated to 5' end of the sense strand;
preferably, the targeting group has a structure selected from the group consisting of:

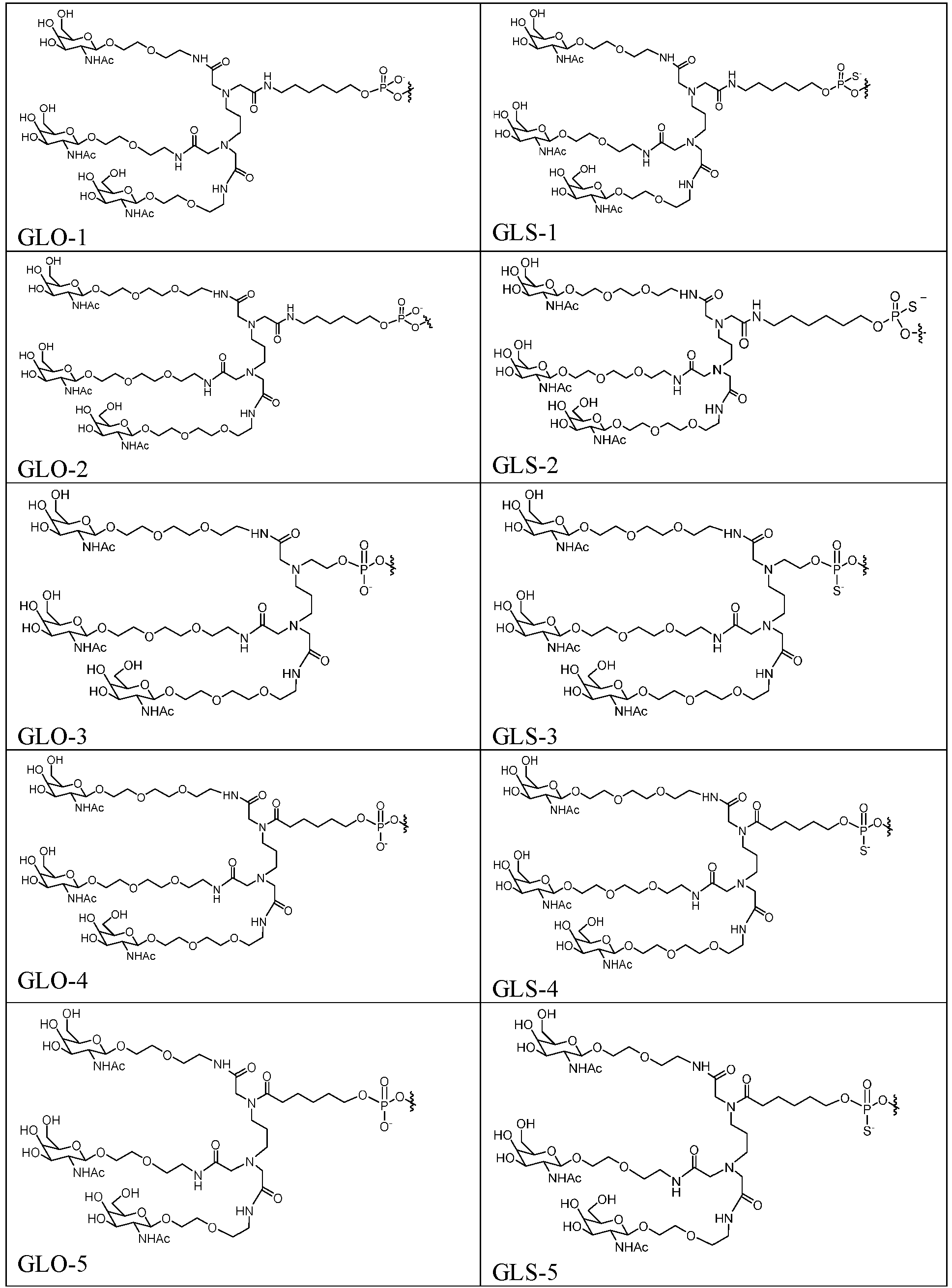

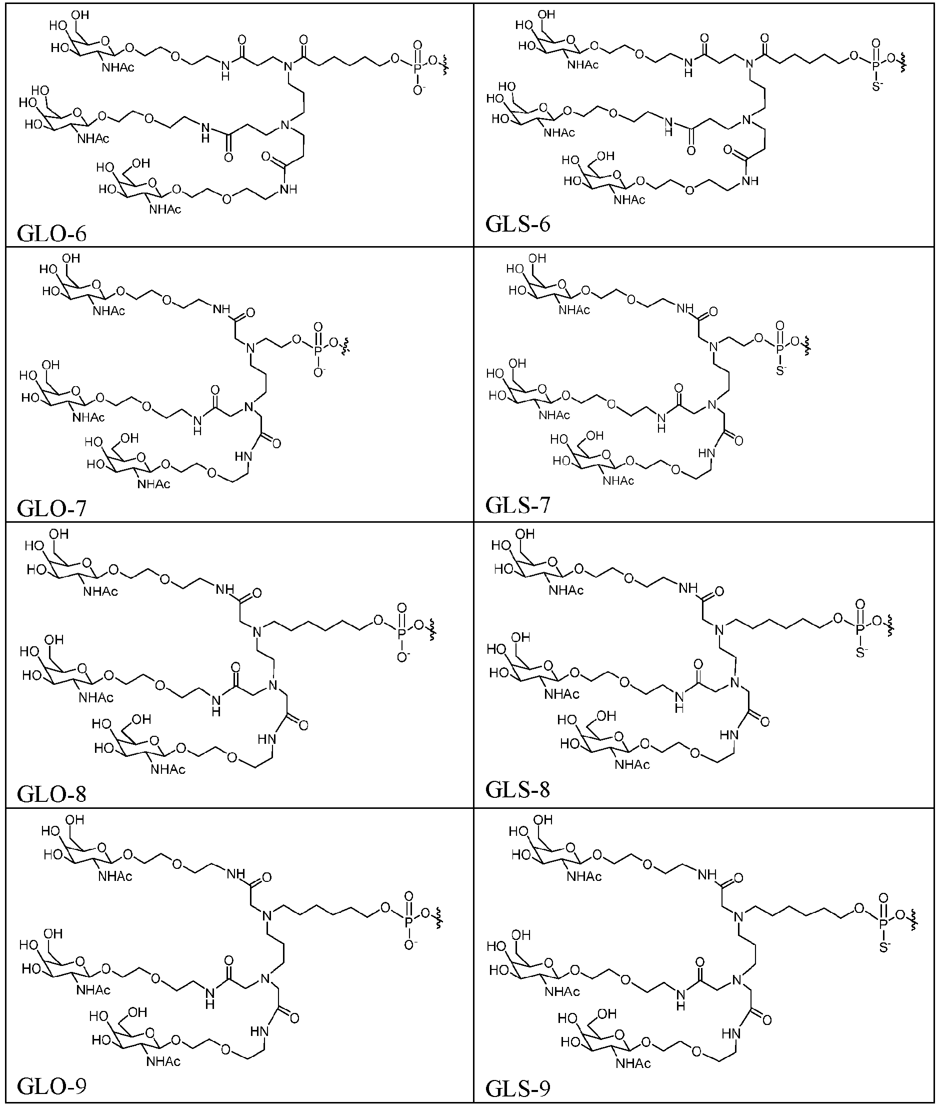

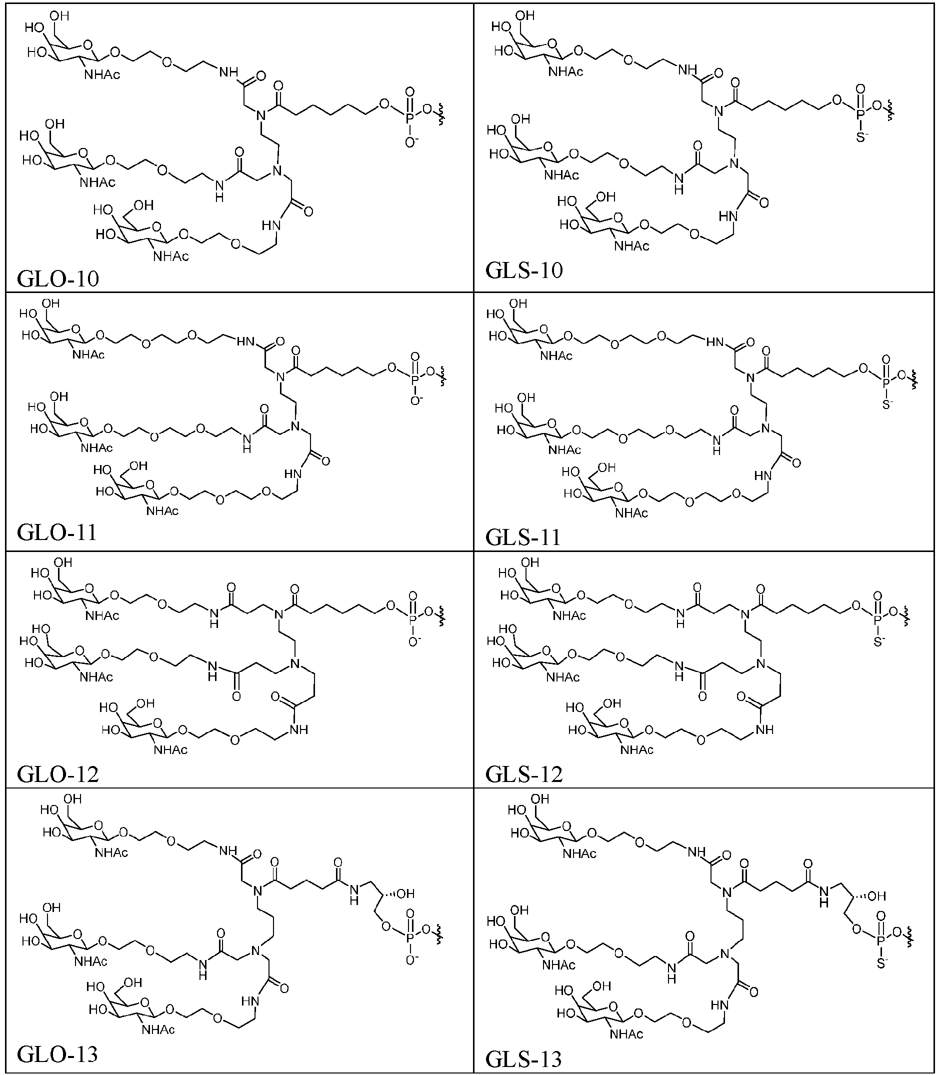

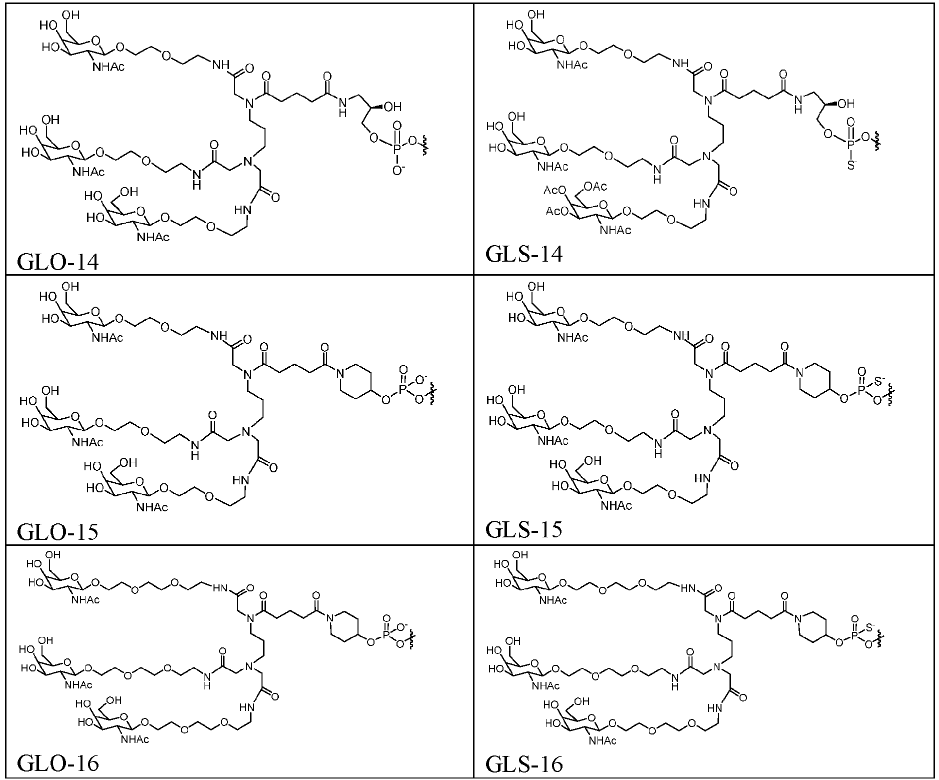

71. The dsRNA agent according to any one of claims 46-70, wherein the sense strand of the dsRNA agent comprises an inverted abasic residue at 3' end.

72. The dsRNA agent according to any one of claims 46-70, wherein the sense strand of the dsRNA agent comprises one or two inverted abasic residues 3' end or/and 5' end.

73. The dsRNA agent according to any one of claims 1-72, wherein the dsRNA agent is siRNA.

74. An RNAi agent for inhibiting expression of a target gene, wherein the RNAi agent is a double-stranded ribonucleic acid (dsRNA) agent, comprising an antisense strand of the dsRNA agent according to any one of claims 1-45 and a sense strand of the dsRNA agent according to any one of claims 46-72.

75. A composition comprising the dsRNA agent according to any one of claims 1-74.

76. The composition according to claim 75, further comprising a pharmaceutically acceptable carrier.

77. The composition according to claim 76, further comprising one or more additional therapeutic agents.

78. The composition according to claim 76, wherein the composition is packaged in a kit, container, pack, dispenser, pre-filled syringe, or vial.

79. The composition according to claim 76, wherein the composition is formulated for ophthalmic, vaginal, rectal, intranasal, transdermal, subcutaneous, intravenous, intraarterial, intralymphatic, intrabronchial, intrapleural, intraperitoneal, intracerobrospinal, intramuscular, pulmonary, intrathecal, or intraventricular administration.

80. A cell comprising the dsRNA agent according to any one of claims 1-74.

81. A method for inhibiting expression of a target gene in a cell, comprising
delivering the dsRNA agent according to any one of claims 1-74 into a subject, such that the dsRNA agent is delivered to a specific target in the subject.

82. The method according to claim 81, wherein the dsRNA agent is administered to the subject by ophthalmic, vaginal, rectal, intranasal, transdermal, subcutaneous, intravenous, intraarterial, intralymphatic, intrabronchial, intrapleural, intraperitoneal, intracerobrospinal, intramuscular, pulmonary, intrathecal, or intraventricular administration.

83. The method according to claim 81, wherein the target gene is selected from the group consisting of LPA, PNPLA3, ASGR1, F7, F12, FXI, APOCIII, APOB, APOL1, TTR, PCSK9, SCAP, KRAS, CD274, PDCD1, C5, ALAS1, HAO1, LDHA, ANGPTL3, SERPINA1, AGT, HAMP, LECT2, EGFR, VEGF, KIF11, AT3, CTNNB1, HMGB1, HIF1A, APP, ATXN2, C9orf72, TARDBP, MAPT (Tau), HTT, SNCA, FUS, ATXN3, SCN9A, SCN10A, CACNA1B, ATXN1, SCAl, SCA7, SCA8, MeCP2, PRNP, SODI, DMPK and STAT3.

84. Use of the dsRNA agent according to any one of claims 1-73 in the manufacture of a medicament for treating a viral disease, neuromuscular disease, bacterial infection, inflammation, immune disease, metabolic disease, liver disease, kidney disease, cardiovascular disease, ophthalmic disease, lung disease, or a rare disease.
